(19) Europäisches Patentamt
European Patent Office
Office européen des brevets

(11) EP 3 237 387 B1

(12) **EUROPÄISCHE PATENTSCHRIFT**

(45) Veröffentlichungstag und Bekanntmachung des Hinweises auf die Patenterteilung:
**06.09.2023 Patentblatt 2023/36**

(21) Anmeldenummer: **15801677.4**

(22) Anmeldetag: **24.11.2015**

(51) Internationale Patentklassifikation (IPC):
***C07D 487/06*** *(2006.01)* ***H10K 85/60*** *(2023.01)*
***C07D 487/16*** *(2006.01)* ***C07D 513/06*** *(2006.01)*
***C07D 487/22*** *(2006.01)* ***C07D 498/06*** *(2006.01)*
*H10K 50/11 (2023.01)* *H10K 50/16 (2023.01)*
*H10K 50/18 (2023.01)*

(52) Gemeinsame Patentklassifikation (CPC):
**C07D 487/06; C07D 487/16; C07D 487/22; C07D 498/06; C07D 513/06; H10K 85/6572;**
Y02E 10/549

(86) Internationale Anmeldenummer:
**PCT/EP2015/002359**

(87) Internationale Veröffentlichungsnummer:
**WO 2016/102039 (30.06.2016 Gazette 2016/26)**

(54) **HETEROCYCLISCHE VERBINDUNGEN MIT DIBENZAZAPIN-STRUKTUREN**

HETEROCYCLIC COMPOUNDS WITH DIBENZAZAPINE STRCTURES

COMPOSÉS HÉTÉROCYCLIQUES À STRUCTURES DE DIBENZAZÉPINE

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **23.12.2014 EP 14004390**

(43) Veröffentlichungstag der Anmeldung:
**01.11.2017 Patentblatt 2017/44**

(73) Patentinhaber: **Merck Patent GmbH**
**64293 Darmstadt (DE)**

(72) Erfinder:
- **PARHAM, Amir Hossain**
  **60486 Frankfurt am Main (DE)**
- **EBERLE, Thomas**
  **76829 Landau (DE)**
- **JATSCH, Anja**
  **60489 Frannkfurt am Main (DE)**
- **GROSSMANN, Tobias**
  **64297 Darmstadt (DE)**
- **KROEBER, Jonas Valentin**
  **60311 Frankfurt am Main (DE)**

(56) Entgegenhaltungen:
**EP-A1- 2 175 005**
**WO-A1-00/33617**
**WO-A1-2016/052962**
**WO-A1-2016/080791**
**JP-A- 2014 160 813**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

EP 3 237 387 B1

## Beschreibung

Die vorliegende Erfindung betrifft heterocyclischeVerbindungen mit Dibenzazepin-Strukturen, welche sich für den Einsatz in elektronischen Vorrichtungen eignen. Weiterhin betrifft die vorliegende Erfindung Verfahren zu deren Herstellung und elektronische Vorrichtungen.

Elektronische Vorrichtungen, welche organische, metallorganische und/oder polymere Halbleiter enthalten, gewinnen zunehmend an Bedeutung, wobei diese aus Kostengründen und aufgrund ihrer Leistungsfähigkeit in vielen kommerziellen Produkten eingesetzt werden. Als Beispiele seien hier Ladungstransportmaterialien auf organischer Basis (z.B. Lochtransporter auf Triarylamin-Basis) in Kopiergeräten, organischen oder polymeren Leuchtdioden (OLEDs oder PLEDs) und in Anzeige- und Displayvorrichtungen oder organische Photorezeptoren in Kopierern genannt. Organische Solarzellen (O-SC), organische FeldeffektTransistoren (O-FET), organische Dünnfilm-Transistoren (O-TFT), organische Schaltelemente (O-IC), organische optische Verstärker und organische Laserdioden (O-Laser) sind in einem fortgeschrittenen Entwicklungsstand und können in der Zukunft große Bedeutung erlangen.

Viele dieser elektronischen Vorrichtungen weisen unabhängig von dem jeweiligen Verwendungszweck folgenden allgemeinen Schichtaufbau auf, der für die jeweilige Anwendung angepasst werden kann:

(1) Substrat,
(2) Elektrode, häufig metallisch oder anorganisch, aber auch aus organischen bzw. polymeren, leitfähigen Materialien,
(3) Ladungsinjektionsschicht/en bzw. Zwischenschicht/en, beispielsweise zum Ausgleich von Unebenheiten der Elektrode ("planarisation layer"), häufig aus einem leitfähigen, dotierten Polymer,
(4) Organische Halbleiter,
(5) evtl. weitere Ladungstransport-, Ladungsinjektions- bzw. Ladungsblockierschichten,
(6) Gegenelektrode, Materialien wie unter (2) genannt,
(7) Verkapselung.

Die obige Anordnung stellt den allgemeinen Aufbau einer organischen, elektronischen Vorrichtung dar, wobei verschiedene Schichten zusammengefasst werden können, so dass im einfachsten Fall eine Anordnung aus zwei Elektroden resultiert, zwischen denen sich eine organische Schicht befindet. Die organische Schicht erfüllt in diesem Fall alle Funktionen, einschließlich der Emission von Licht im Fall von OLEDs. Ein derartiges System ist beispielsweise in der WO 90/13148 A1 auf der Basis von Poly-(p-phenylenen) beschrieben.

Elektronische Vorrichtungen, die Verbindungen mit Dibenzazepin-Strukturen enthalten, sind unter anderem aus der Veröffentlichung JP 2014-160813 A bekannt. In WO00/33617 werden Verbindungen mit symmetrischer molekularer Struktur beschrieben, deren Endgruppen lochtransportierende Diaryleinheiten tragen. In EP2175005 spezielle Triphenylenverbindungen beschrieben, die unter anderem mit Dibenzazepinstruktureinheiten substituiert sein können.

Bekannte elektronische Vorrichtungen weisen ein brauchbares Eigenschaftsprofil auf. Allerdings besteht die dauerhafte Notwendigkeit, die Eigenschaften dieser Vorrichtungen zu verbessern.

Zu diesen Eigenschaften gehört insbesondere die Energieeffizienz, mit der eine elektronische Vorrichtung die vorgegebene Aufgabe löst. Bei organischen Leuchtdioden, die sowohl auf niedermolekularen Verbindungen als auch auf polymeren Materialien basieren können, sollte insbesondere die Lichtausbeute hoch sein, so dass zum Erreichen eines bestimmten Lichtflusses möglichst wenig elektrische Leistung aufgebracht werden muss. Weiterhin sollte auch zum Erzielen einer vorgegebenen Leuchtdichte eine möglichst geringe Spannung notwendig sein. Ein weiteres Problem stellt insbesondere die Lebensdauer der elektronischen Vorrichtungen dar.

Aufgabe der vorliegenden Erfindung ist daher die Bereitstellung neuer Verbindungen, welche zu Elektronischen Vorrichtungen mit verbesserten Eigenschaften führen. Insbesondere ist die Aufgabe Lochtransportmaterialien, Lochinjektionsmaterialien, Lochblockiermaterialien, Elektroneninjektionsmaterialien, Elektronenblockiermaterialien und/oder Elektronentransportmaterialien bereitzustellen, welche verbesserte Eigenschaften in Bezug auf Effizienz, Betriebsspannung und/oder Lebensdauer zeigen. Weiterhin sollten sich die Verbindungen möglichst einfach verarbeiten lassen, insbesondere eine gute Löslichkeit und Filmbildung zeigen. Beispielsweise sollten die Verbindungen eine erhöhte Oxidationsstabilität und eine verbesserte Glasübergangstemperatur zeigen.

Eine weitere Aufgabe kann darin gesehen werden, elektronische Vorrichtungen mit einer ausgezeichneten Leistungsfähigkeit möglichst kostengünstig und in konstanter Qualität bereitzustellen Weiterhin sollten die elektronischen Vorrichtungen für viele Zwecke eingesetzt oder angepasst werden können. Insbesondere sollte die Leistungsfähigkeit der elektronischen Vorrichtungen über einen breiten Temperaturbereich erhalten bleiben.

Überraschenderweise wurde gefunden, dass diese sowie weitere nicht explizit genannte Aufgaben, die jedoch aus den hierin einleitend diskutierten Zusammenhängen ohne Weiteres ableitbar oder erschließbar sind, durch Verbindungen mit allen Merkmalen des Patentanspruchs 1 gelöst werden. Zweckmäßige Abwandlungen der erfindungsgemäßen Verbindungen werden in den auf Anspruch 1 rückbezogenen abhängigen Ansprüchen unter Schutz gestellt.

Gegenstand der Erfindung ist somit eine Verbindung, umfassend Strukturen der Formel (II) gemäß Anspruch 1 wobei für die verwendeten Symbole gilt:

X ist bei jedem Auftreten gleich oder verschieden $CR^1$ oder C für die Anbindungsstelle des Restes $R^a$;

W ist eine Bindung, $NR^1$, $C(R^1)_2$, O oder S;

$R^a$ ist D, F, Cl, Br, I, $B(OR^1)_2$, CHO, $C(=O)R^1$, $CR^1=C(R^1)_2$, CN, $C(=O)OR^1$, $C(=O)N(R^1)_2$, $Si(R^1)_3$, $N(R^1)_2$, $NO_2$, $P(=O)(R^1)_2$, $OSO_2R^1$, $OR^1$, $S(=O)R^1$, $S(=O)_2R^1$, eine geradkettige Alkyl-, Alkoxy- oder Thioalkoxygruppe mit 1 bis 40 C-Atomen oder eine verzweigte oder cyclische Alkyl-, Alkoxy- oder Thioalkoxygruppe mit 3 bis 40 C-Atomen, die jeweils mit einem oder mehreren Resten $R^1$ substituiert sein kann, wobei eine oder mehrere nicht benachbarte $CH_2$-Gruppen durch $-R^1C=CR^1-$, -C≡C-, $Si(R^1)_2$, C=O, C=S, $C=NR^1$, -C(=O)O-, $-C(=O)NR^1-$, $NR^1$, $P(=O)(R^1)$, -O-, -S-, SO oder $SO_2$ ersetzt sein können und wobei ein oder mehrere H-Atome durch D, F, Cl, Br, I, CN oder $NO_2$ ersetzt sein können, oder ein aromatisches oder heteroaromatisches Ringsystem mit 5 bis 40 aromatischen Ringatomen, das jeweils durch einen oder mehrere Reste $R^1$ substituiert sein kann, oder eine Aryloxy- oder Heteroaryloxygruppe mit 5 bis 40 aromatischen Ringatomen, die durch einen oder mehrere Reste $R^1$ substituiert sein kann, oder eine Kombination dieser Systeme;

$R^b$ ist definiert gemäß Anspruch 1;

$R^1$ ist bei jedem Auftreten gleich oder verschieden H, D, F, Cl, Br, I, $B(OR^2)_2$, CHO, $C(=O)R^2$, $CR^2=C(R^2)_2$, CN, $C(=O)OR^2$, $C(=O)N(R^2)_2$, $Si(R^2)_3$, $N(R^2)_2$, $NO_2$, $P(=O)(R^2)_2$, $OSO_2R^2$, $OR^2$, $S(=O)R^2$, $S(=O)_2R^2$, eine geradkettige Alkyl-, Alkoxy- oder Thioalkoxygruppe mit 1 bis 40 C-Atomen oder eine verzweigte oder cyclische Alkyl-, Alkoxy- oder Thioalkoxygruppe mit 3 bis 40 C-Atomen, die jeweils mit einem oder mehreren Resten $R^2$ substituiert sein kann, wobei eine oder mehrere nicht benachbarte $CH_2$-Gruppen durch $-R^2C=CR^2-$, -C=C-, $Si(R^2)_2$, $Ge(R^2)_2$, $Sn(R^2)_2$, C=O, C=S, C=Se, $C=NR^2$, -C(=O)O-, $-C(=O)NR^2-$, $NR^2$, $P(=O)(R^2)$, -O-, -S-, SO oder $SO_2$ ersetzt sein können und wobei ein oder mehrere H-Atome durch D, F, Cl, Br, I, CN oder $NO_2$ ersetzt sein können, oder ein aromatisches oder heteroaromatisches Ringsystem mit 5 bis 40 aromatischen Ringatomen, das jeweils durch einen oder mehrere Reste $R^2$ substituiert sein kann, oder eine Aryloxy- oder Heteroaryloxygruppe mit 5 bis 40 aromatischen Ringatomen, die durch einen oder mehrere Reste $R^2$ substituiert sein kann, oder eine Kombination dieser Systeme;

$R^2$ ist bei jedem Auftreten gleich oder verschieden H, D, F, Cl, Br, I, $B(OR^3)_2$, CHO, $C(=O)R^3$, $CR^3=C(R^3)_2$, CN, $C(=O)OR^3$, $C(=O)N(R^3)_2$, $Si(R^3)_3$, $N(R^3)_2$, $NO_2$, $P(=O)(R^3)_2$, $OSO_2R^3$, $OR^3$, $S(=O)R^3$, $S(=O)_2R^3$, eine geradkettige Alkyl-, Alkoxy- oder Thioalkoxygruppe mit 1 bis 40 C-Atomen oder eine verzweigte oder cyclische Alkyl-, Alkoxy- oder Thioalkoxygruppe mit 3 bis 40 C-Atomen, die jeweils mit einem oder mehreren Resten $R^3$ substituiert sein kann, wobei eine oder mehrere nicht benachbarte $CH_2$-Gruppen durch $-R^3C=CR^3-$, -C=C-, $Si(R^3)_2$, $Si(R^2)_2$, $Ge(R^3)_2$, $Sn(R^3)_2$, C=O, C=S, C=Se, $C=NR^3$, -C(=O)O-, $-C(=O)NR^3-$, $NR^3$, $P(=O)(R^3)$, -O-, -S-, SO oder $SO_2$ ersetzt sein können und wobei ein oder mehrere H-Atome durch D, F, Cl, Br, I, CN oder $NO_2$ ersetzt sein können, oder ein aromatisches oder heteroaromatisches Ringsystem mit 5 bis 40 aromatischen Ringatomen, das jeweils durch einen oder mehrere Reste $R^3$ substituiert sein kann, oder eine Aryloxy- oder Heteroaryloxygruppe mit 5 bis 40 aromatischen Ringatomen, die durch einen oder mehrere Reste $R^3$ substituiert sein kann, oder eine Kombination dieser Systeme; dabei können zwei oder mehrere benachbarte Substituenten $R^2$ auch miteinander ein mono- oder poly-cyclisches, aliphatisches oder aromatisches Ringsystem bilden;

$R^3$ ist bei jedem Auftreten gleich oder verschieden H, D, F oder ein aliphatischer, aromatischer und/oder heteroaromatischer Kohlenwasserstoffrest mit 1 bis 20 C-Atomen, in dem auch H-Atome durch F ersetzt sein können; dabei können zwei oder mehrere benachbarte Substituenten $R^3$ auch miteinander ein mono- oder polycyclisches, aliphatisches oder aromatisches Ringsystem bilden.

Dabei bedeutet "benachbarte Kohlenstoffatome", dass die Kohlenstoffatome direkt aneinander gebunden sind. Weiterhin bedeutet "benachbarte Reste" in der Definition der Reste, dass diese Reste an dasselbe Kohlenstoffatom oder an benachbarte Kohlenstoffatome gebunden sind. Diese Definitionen gelten entsprechend unter anderem für die Begriffe "benachbarte Gruppen" und "benachbarte Substituenten".

Insbesondere weist $R^b$ eine aromatische und/oder heteroaromatische Gruppe auf, die mindestens zwei benachbarte aromatische und/oder heteroaromatische Ringe umfasst. Demgemäß können die Ringe über eine Bindung miteinander verbunden sind, so dass die Reste $R^a$ und/oder $R^b$ beispielsweise eine Biphenylgruppe umfassen können. Weiterhin können die Ringe kondensiert sein, so dass beispielsweise zwei C-Atome zu den mindenstens zwei aromatischen oder heteroaromatischen Ringen zugehören, wie dies beispielsweise in einer Naphthylgruppe gegeben ist. Ferner können die Gruppen in $R^b$ über ein Atom miteinander benachbart sein. So kann $R^b$ beispielsweise eine Diarylaminverbindung umfassen, wobei mindestens zwei Arylgruppen über ein Stickstoffatom benachbart sind. Vorzugsweise umfassen aromatische und/oder heteroaromatische Gruppen, die mindestens zwei benachbarte aromatische und/oder heteroaromatische Ringe aufweisen, zwei Arylgruppen, die über eine Bindung miteinander verbunden oder kondensiert sind. Besonders bevorzugt umfassen aromatische und/oder heteroaromatische Gruppen, die mindestens zwei benachbarte aromatische und/oder heteroaromatische Ringe aufweisen, zwei Arylgruppen, die über eine Bindung miteinander verbunden sind.

Eine Arylgruppe im Sinne dieser Erfindung enthält 6 bis 40 C-Atome; eine Heteroarylgruppe im Sinne dieser Erfindung enthält 2 bis 40 C-Atome und mindestens ein Heteroatom, mit der Maßgabe, dass die Summe aus C-Atomen und Heteroatomen mindestens 5 ergibt. Die Heteroatome sind bevorzugt ausgewählt aus N, O und/oder S. Dabei wird unter einer Arylgruppe bzw. Heteroarylgruppe entweder ein einfacher aromatischer Cyclus, also Benzol, bzw. ein einfacher heteroaromatischer Cyclus, beispielsweise Pyridin, Pyrimidin, Thiophen, etc., oder eine kondensierte Aryl- oder Heteroarylgruppe, beispielsweise Naphthalin, Anthracen, Phenanthren, Chinolin, Isochinolin, etc., verstanden.

Ein aromatisches Ringsystem im Sinne dieser Erfindung enthält 6 bis 60 C-Atome im Ringsystem. Ein heteroaromatisches Ringsystem im Sinne dieser Erfindung enthält 1 bis 60 C-Atome und mindestens ein Heteroatom im Ringsystem, mit der Maßgabe, dass die Summe aus C-Atomen und Heteroatomen mindestens 5 ergibt. Die Heteroatome sind bevorzugt ausgewählt aus N, O und/oder S. Unter einem aromatischen oder heteroaromatischen Ringsystem im Sinne dieser Erfindung soll ein System verstanden werden, das nicht notwendigerweise nur Aryl- oder Heteroarylgruppen enthält, sondern in dem auch mehrere Aryl- oder Heteroarylgruppen durch eine nicht-aromatische Einheit (bevorzugt weniger als 10 % der von H verschiedenen Atome), wie z. B. ein C-, N- oder O-Atom oder eine Carbonylgruppe, unterbrochen sein können. So sollen beispielsweise auch Systeme wie 9,9'-Spirobifluoren, 9,9-Diarylfluoren, Triarylamin, Diarylether, Stilben, etc. als aromatische Ringsysteme im Sinne dieser Erfindung verstanden werden, und ebenso Systeme, in denen zwei oder mehrere Arylgruppen beispielsweise durch eine lineare oder cyclische Alkylgruppe oder durch eine Silylgruppe unterbrochen sind. Weiterhin sollen Systeme, in denen zwei oder mehrere Aryl- oder Heteroarylgruppen direkt aneinander gebunden sind, wie z. B. Biphenyl oder Terphenyl, ebenfalls als aromatisches bzw. heteroaromatisches Ringsystem verstanden werden.

Unter einer cyclischen Alkyl-, Alkoxy- oder Thioalkoxygruppe im Sinne dieser Erfindung wird eine monocyclische, eine bicyclische oder eine polycyclische Gruppe verstanden.

Im Rahmen der vorliegenden Erfindung werden unter einer $C_1$- bis $C_{40}$-Alkylgruppe, in der auch einzelne H-Atome oder $CH_2$-Gruppen durch die oben genannten Gruppen substituiert sein können, beispielsweise die Reste Methyl, Ethyl, n-Propyl, i-Propyl, Cyclopropyl, n-Butyl, i-Butyl, s-Butyl, t-Butyl, Cyclobutyl, 2-Methylbutyl, n-Pentyl, s-Pentyl, t-Pentyl, 2-Pentyl, neo-Pentyl, Cyclopentyl, n-Hexyl, s-Hexyl, t-Hexyl, 2-Hexyl, 3-Hexyl, neo-Hexyl, Cyclohexyl, 1-Methylcyclopentyl, 2-Methylpentyl, n-Heptyl, 2-Heptyl, 3-Heptyl, 4-Heptyl, Cycloheptyl, 1-Methylcyclohexyl, n-Octyl, 2-Ethylhexyl, Cyclooctyl, 1-Bicyclo[2,2,2]octyl, 2-Bicyclo[2,2,2]-octyl, 2-(2,6-Dimethyl)octyl, 3-(3,7-Dimethyl)octyl, Adamantyl, Trifluormethyl, Pentafluorethyl, 2,2,2-Trifluorethyl, 1,1-Dimethyl-n-hex-1-yl-, 1,1-Dimethyl-n-hept-1-yl-, 1,1-Dimethyl-n-oct-1-yl-, 1,1-Dimethyl-n-dec-1-yl-, 1,1-Dimethyl-n-dodec-1-yl-, 1,1-Dimethyl-n-tetradec-1-yl-, 1,1-Dimethyl-n-hexadec-1-yl-, 1,1-Dimethyl-n-octadec-1-yl-, 1,1-Diethyl-n-hex-1-yl-, 1,1-Diethyl-n-hept-1-yl-, 1,1-Diethyl-n-oct-1-yl-, 1,1-Diethyl-n-dec-1-yl-, 1,1-Diethyl-n-dodec-1-yl-, 1,1-Diethyl-n-tetradec-1-yl-, 1,1-Diethyln-n-hexadec-1-yl-, 1,1-Diethyl-n-octadec-1-yl-, 1-(n-Propyl)-cyclohex-1-yl-, 1-(n-Butyl)-cyclohex-1-yl-, 1-(n-Hexyl)-cyclohex-1-yl-, 1-(n-Octyl)-cyclohex-1-yl- und 1-(n-Decyl)-cyclohex-1-yl- verstanden. Unter einer Alkenylgruppe werden beispielsweise Ethenyl, Propenyl, Butenyl, Pentenyl, Cyclopentenyl, Hexenyl, Cyclohexenyl, Heptenyl, Cycloheptenyl, Octenyl, Cyclooctenyl oder Cyclooctadienyl verstanden. Unter einer Alkinylgruppe werden beispielsweise Ethinyl, Propinyl, Butinyl, Pentinyl, Hexinyl, Heptinyl oder Octinyl verstanden. Unter einer $C_1$- bis $C_{40}$-Alkoxygruppe werden beispielsweise Methoxy, Trifluormethoxy, Ethoxy, n-Propoxy, i-Propoxy, n-Butoxy, i-Butoxy, s-Butoxy, t-Butoxy oder 2-Methylbutoxy verstanden.

Unter einem aromatischen oder heteroaromatischen Ringsystem mit 5 - 60 aromatischen Ringatomen, welches noch jeweils mit den oben genannten Resten substituiert sein kann und welches über beliebige Positionen am Aromaten bzw. Heteroaromaten verknüpft sein kann, werden beispielsweise Gruppen verstanden, die abgeleitet sind von Benzol, Naphthalin, Anthracen, Benzanthracen, Phenanthren, Benzophenanthren, Pyren, Chrysen, Perylen, Fluoranthen, Benzfluoranthen, Naphthacen, Pentacen, Benzpyren, Biphenyl, Biphenylen, Terphenyl, Terphenylen, Fluoren, Spirobifluoren, Dihydrophenanthren, Dihydropyren, Tetrahydropyren, cis- oder trans-Indenofluoren, cis- oder trans-Monobenzoindenofluoren, cis- oder trans-Dibenzoindenofluoren, Truxen, Isotruxen, Spirotruxen, Spiroisotruxen, Furan, Benzofuran, Isobenzofuran, Dibenzofuran, Thiophen, Benzothiophen, Isobenzothiophen, Dibenzothiophen, Pyrrol, Indol, Isoindol, Carbazol, Indolocarbazol, Indenocarbazol, Pyridin, Chinolin, Isochinolin, Acridin, Phenanthridin, Benzo-5,6-chinolin, Benzo-6,7-chinolin, Benzo-7,8-chinolin, Phenothiazin, Phenoxazin, Pyrazol, Indazol, Imidazol, Benzimidazol, Naphthimidazol, Phenanthrimidazol, Pyridimidazol, Pyrazinimidazol, Chinoxalinimidazol, Oxazol, Benzoxazol, Naphthoxazol, Anthroxazol, Phenanthroxazol, Isoxazol, 1,2-Thiazol, 1,3-Thiazol, Benzothiazol, Pyridazin, Benzopyridazin, Pyrimidin, Benzpyrimidin, Chinoxalin, 1,5-Diazaanthracen, 2,7-Diazapyren, 2,3-Diazapyren, 1,6-Diazapyren, 1,8-Diazapyren, 4,5-Diazapyren, 4,5,9,10-Tetraazaperylen, Pyrazin, Phenazin, Phenoxazin, Phenothiazin, Fluorubin, Naphthyridin, Azacarbazol, Benzocarbolin, Phenanthrolin, 1,2,3-Triazol, 1,2,4-Triazol, Benzotriazol, 1,2,3-Oxadiazol, 1,2,4-Oxadiazol, 1,2,5-Oxadiazol, 1,3,4-Oxadiazol, 1,2,3-Thiadiazol, 1,2,4-Thiadiazol, 1,2,5-Thiadiazol, 1,3,4-Thiadiazol, 1,3,5-Triazin, 1,2,4-Triazin, 1,2,3-Triazin, Tetrazol, 1,2,4,5-Tetrazin, 1,2,3,4-Tetrazin, 1,2,3,5-Tetrazin, Purin, Pteridin, Indolizin und Benzothiadiazol.

Die erfindungsgemäßen Verbindungen umfassen Strukturen der Formel (II)

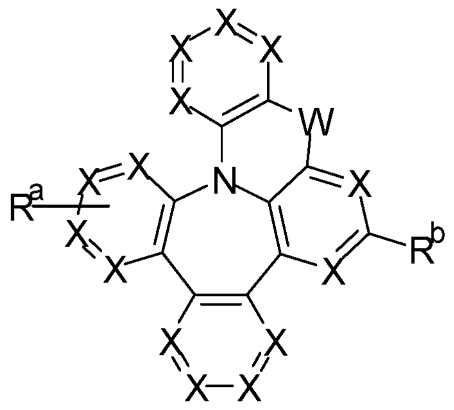

Formel (II),

wobei die verwendeten Symbole die zuvor genannte Bedeutung aufweisen und der Rest $R^b$ eine aromatische Gruppe mit 10 bis 40 C-Atomen oder eine heteroaromatische Gruppe mit 6 bis 40 C-Atomen ist, wobei die aromatische und/oder heteroaromatische Gruppe mindestens zwei benachbarte aromatische und/oder heteroaromatische Ringe umfasst, die jeweils kondensiert oder nicht-kondensiert und/oder durch einen oder mehrere Reste $R^1$ substituiert sein können.

Vorzugsweise können die erfindungsgemäßen Verbindungen Strukturen gemäß Formel (IV)

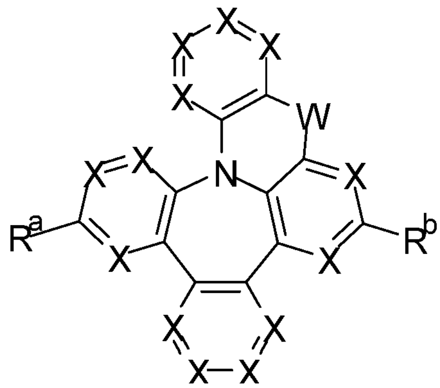

Formel (IV)

umfassen, wobei die verwendeten Symbole die zuvor genannte Bedeutung aufweisen und mindestens der Rest $R^b$, vorzugsweise beide Reste Reste $R^a$ und $R^b$ eine aromatische Gruppe mit 10 bis 40 C-Atomen oder eine heteroaromatische Gruppe mit 6 bis 40 C-Atomen ist/sind, wobei die aromatische und/oder heteroaromatische Gruppe mindestens zwei benachbarte aromatische und/oder heteroaromatische Ringe umfasst, die jeweils kondensiert oder nicht-kondensiert und/oder durch einen oder mehrere Reste $R^1$ substituiert sein können.

Darüber hinaus sind Verbindungen bevorzugt, die dadurch gekennzeichnet sind, dass mindestens einer der Reste $R^a$ und/oder $R^b$ in den Formeln (II) und/oder (IV) eine Lochtransportgruppe oder eine Elektronentransportgruppe darstellt.

Abgesehen von den Anbindungsstellen der Gruppen Reste $R^a$ und/oder $R^b$ in der Formel (II), an denen X für C steht, stellen alle X eine Gruppe $CR^1$ dar wobei vorzugsweise höchstens 4, besonders bevorzugt höchstens 3 und speziell bevorzugt höchstens 2 der Gruppen $CR^1$ für die X steht, ungleich der Gruppe CH ist.

Besonders bevorzugt sind Verbindungen, umfassend Strukturen der Formel (V)

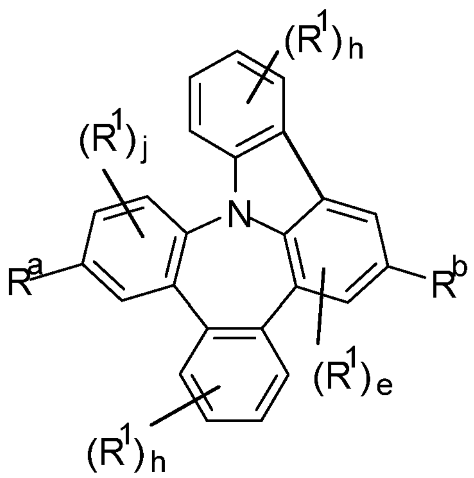

Formel (V)

worin $R^a$, $R^b$ und $R^1$ die zuvor, insbesondere in Bezug auf Formel (II) dargelegten Bedeutungen haben,
e 0, 1 oder 2, vorzugsweise 0 oder 1, besonders bevorzugt 0 ist,
j 0, 1, 2 oder 3, vorzugsweise 0, 1 oder 2, besonders bevorzugt 0 oder 1 ist,
h 0, 1, 2, 3 oder 4, vorzugsweise 0, 1 oder 2, besonders bevorzugt 0 oder 1 ist,
wobei mindestens einer der Reste $R^a$, $R^b$ eine Lochtransportgruppe und/oder eine Elektronentransportgruppe darstellt, wobei vorzugsweise beide Reste $R^a$ und Rest $R^b$ jeweils eine Lochtransportgruppe und/oder eine Elektronentransportgruppe darstellen.

Ferner sind Verbindungen ganz besonders bevorzugt, umfassend Strukturen der Formel (VI)

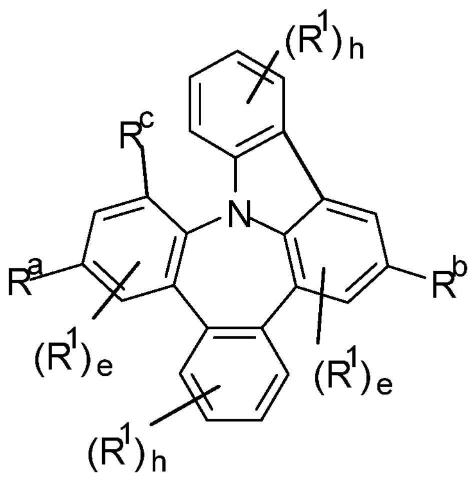

Formel (VI)

worin $R^a$, $R^b$ und $R^1$ die zuvor, insbesondere in Bezug auf Formel (II) dargelegten Bedeutungen haben,
e 0, 1 oder 2, vorzugsweise 0 oder 1, besonders bevorzugt 0 ist,
h 0, 1, 2, 3 oder 4, vorzugsweise 0, 1 oder 2, besonders bevorzugt 0 oder 1 ist,
wobei der Rest $R^b$ eine Lochtransportgruppe oder eine Elektronentransportgruppe darstellt und der Rest $R^c$ eine aromatische Gruppe mit 10 bis 40 C-Atomen, oder eine heteroaromatische Gruppe mit 6 bis 40 C-Atomen ist, wobei die aromatische und/oder heteroaromatische Gruppe mindestens zwei benachbarte aromatische und/oder heteroaromatische Ringe umfasst, die jeweils kondensiert oder nicht-kondensiert und/oder durch einen oder mehrere Reste $R^1$ substituiert sein können. Besonders bevorzugt kann der Rest $R^c$ in Formel (VI) eine Lochtransportgruppe und/oder eine Elektronentransportgruppe darstellen.

Besonders bevorzugt sind Verbindungen, umfassend Strukturen der Formel (VII)

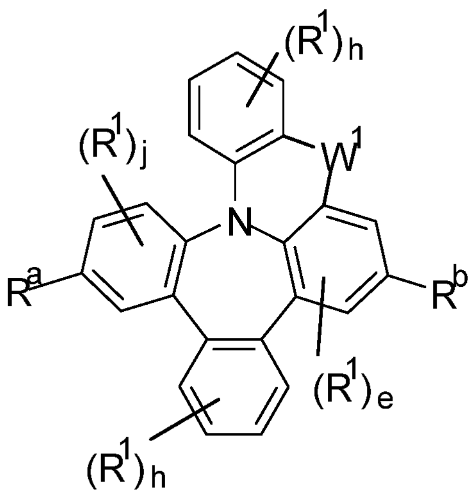

Formel (VII)

worin $R^a$, $R^b$ und $R^1$ die zuvor, insbesondere in Bezug auf Formel (II) dargelegten Bedeutungen haben,
e 0, 1 oder 2, vorzugsweise 0 oder 1, besonders bevorzugt 0 ist,
j 0, 1, 2 oder 3, vorzugsweise 0, 1 oder 2, besonders bevorzugt 0 oder 1 ist,
h 0, 1, 2, 3 oder 4, vorzugsweise 0, 1 oder 2, besonders bevorzugt 0 oder 1 ist,
wobei mindestens einer der Reste $R^a$, $R^b$ eine Lochtransportgruppe und/oder eine Elektronentransportgruppe darstellt und $W^1$ $NR^1$, $C(R^1)_2$, O oder S ist.

Weiterhin sind Verbindungen ganz besonders bevorzugt, umfassend Strukturen der Formel (VIII)

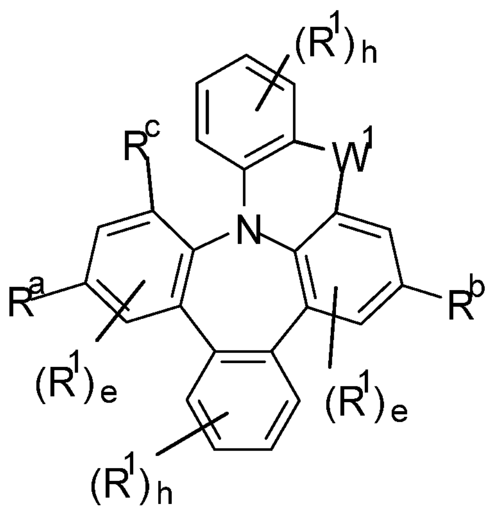

Formel (VIII)

worin $R^a$, $R^b$ und $R^1$ die zuvor, insbesondere in Bezug auf Formel (II) dargelegten Bedeutungen haben,
e 0, 1 oder 2, vorzugsweise 0 oder 1, besonders bevorzugt 0 ist,
h 0, 1, 2, 3 oder 4, vorzugsweise 0, 1 oder 2, besonders bevorzugt 0 oder 1 ist,
wobei der Rest $R^b$ eine Lochtransportgruppe oder eine Elektronentransportgruppe darstellt und der Rest $R^c$ eine Arylgruppe mit 10 bis 40 C-Atomen, welche mindestens zwei Ringe umfasst, oder eine Heteroarylgruppe mit 6 bis 40 C-Atomen darstellt, welche mindestens zwei Ringe umfasst, wobei die jeweilige Gruppe jeweils durch einen oder mehrere Reste $R^1$ substituiert sein kann und $Y^1$ $NR^1$, $C(R^1)_2$, O oder S ist. Besonders bevorzugt kann der Rest $R^c$ in Formel (VIII) eine Lochtransportgruppe und/oder eine Elektronentransportgruppe darstellen.

Darüber hinaus kann vorgesehen sein, dass der Rest $R^b$ in einer Verbindung umfassend Strukturen der Formeln (II), (IV), (V), (VI), (VII) und/oder (VIII) eine Lochtransportgruppe und der Rest $R^a$ in dieser Struktur der identischen Formel, ausgewählt aus einer der Formeln (II), (IV), (V), (VI), (VII) und/oder (VIII) eine Lochtransportgruppe ist.

Ferner kann vorgesehen sein, dass der Rest $R^b$ in einer Verbindung umfassend Strukturen der Formeln (II), (IV), (V), (VI), (VII) und/oder (VIII) eine Elektronentransportgruppe und der Rest $R^a$ in dieser Struktur der identischen Formel, ausgewählt aus einer der Formeln (II), (IV), (V), (VI), (VII) und/oder (VIII) eine Lochtransportgruppe ist.

Weiterhin kann vorgesehen sein, dass der Rest $R^b$ in einer Verbindung umfassend Strukturen der Formeln (II), (IV), (V), (VI), (VII) und/oder (VIII) eine Lochtransportgruppe und der Rest $R^a$ in dieser Struktur der identischen Formel, ausgewählt aus einer der Formeln (II), (IV), (V), (VI), (VII) und/oder (VIII) eine Elektronentransportgruppe ist.

Desweiteren kann vorgesehen sein, dass der Rest $R^b$ in einer Verbindung umfassend Strukturen der Formeln (II), (IV), (V), (VI), (VII) und/oder (VIII) eine Elektronentransportgruppe und der Rest $R^a$ in dieser Struktur der identischen Formel, ausgewählt aus einer der Formeln (II), (IV), (V), (VI), (VII) und/oder (VIII) eine Elektronentransportgruppe ist.

Gemäß einer besonderen Ausführungsform einer erfindungsgemäßen Verbindung kann vorgesehen sein, dass die Summe der Indices e, h und j in den Strukturen der Formeln (V), (VI), (VII) und/oder (VIII) bevorzugt jeweils höchstens 3, vorzugsweise höchstens 2 und besonders bevorzugt höchstens 1 beträgt.

Bevorzugt sind Verbindungen umfassend Strukturen der Formel (II), in denen mindestens ein Rest $R^1$, $R^a$, $R^b$ und/oder $R^c$ in den Strukturen gemäß Formeln (II), (IV), (V), (VI), (VII) und/oder (VIII) für eine Gruppe steht, die ausgewählt ist aus den Formeln ($R^1$-1) bis ($R^1$- 72)

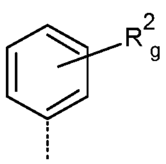

Formel ($R^1$-1)

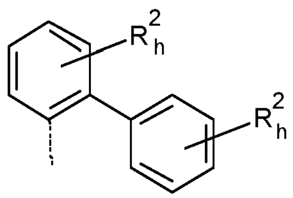

Formel ($R^1$-2)

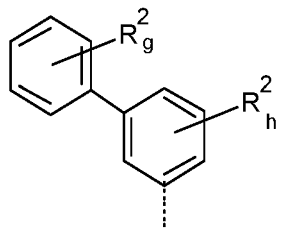

Formel ($R^1$-3)

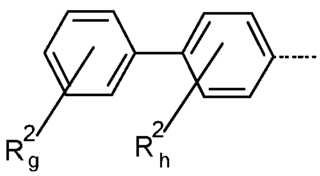

Formel ($R^1$-4)

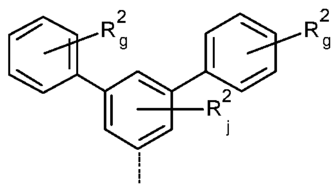

Formel ($R^1$-5)

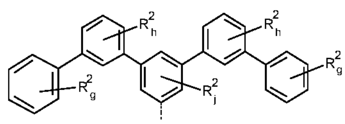

Formel ($R^1$-6)

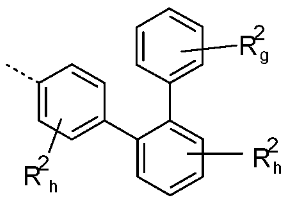

Formel ($R^1$-7)

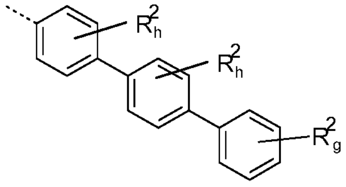

Formel ($R^1$-8)

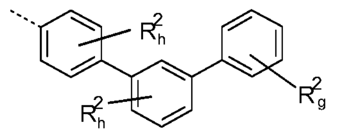

Formel ($R^1$-9)

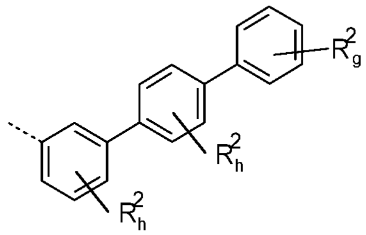

Formel ($R^1$-10)

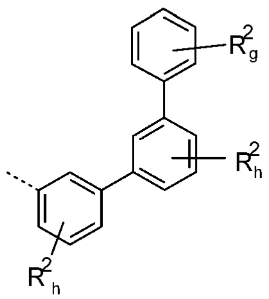

Formel ($R^1$-11)

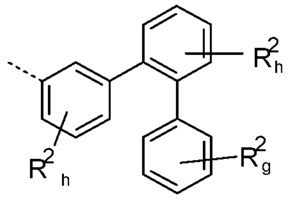

Formel ($R^1$-12)

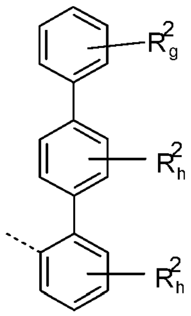

Formel ($R^1$-13)

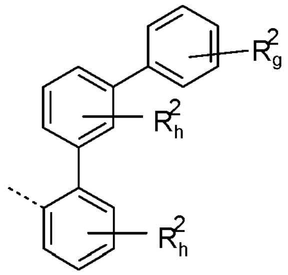

Formel ($R^1$-14)

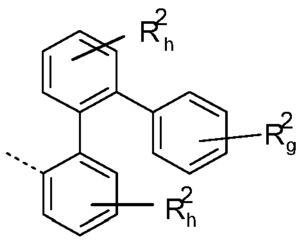

Formel ($R^1$-15)

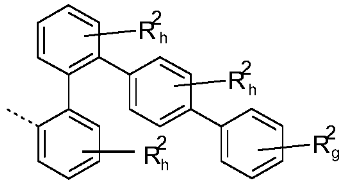

Formel ($R^1$-16)

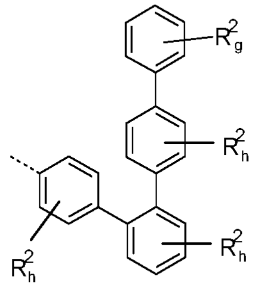

Formel ($R^1$-17)

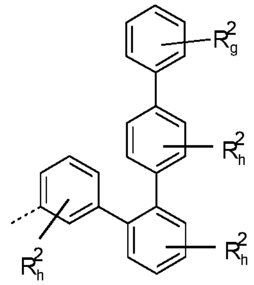

Formel ($R^1$-18)

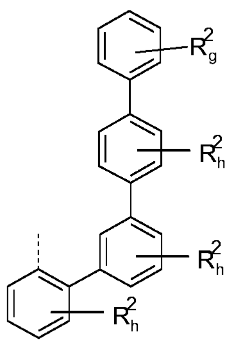

Formel ($R^1$-19)

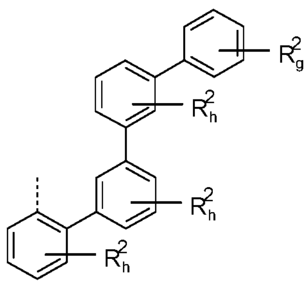

Formel ($R^1$-20)

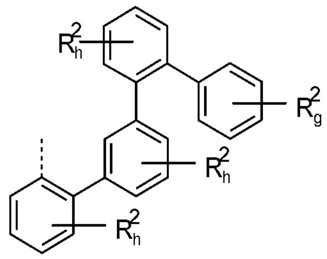

Formel ($R^1$-21)

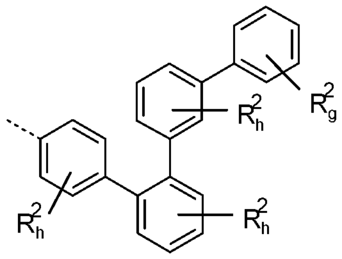

Formel ($R^1$-22)

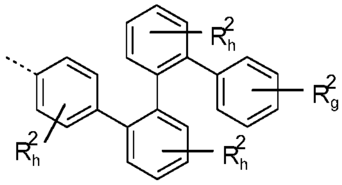

Formel ($R^1$-23)

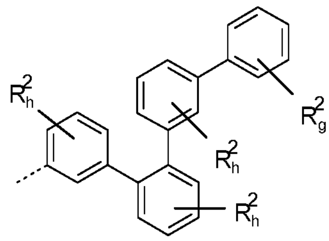

Formel ($R^1$-24)

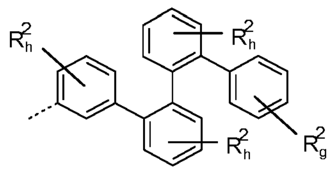

Formel ($R^1$-25)

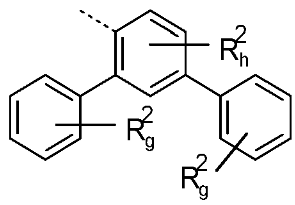

Formel ($R^1$-26)

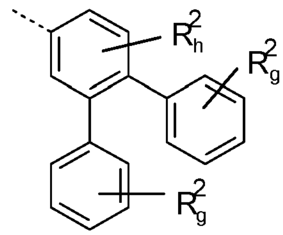

Formel ($R^1$-27)

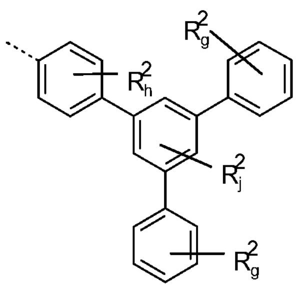

Formel ($R^1$-28)

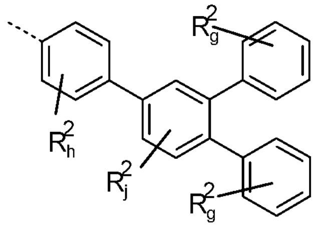

Formel ($R^1$-29)

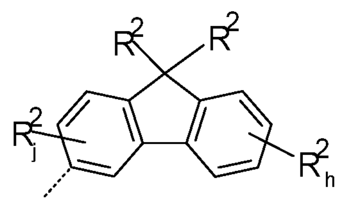

Formel ($R^1$-30)

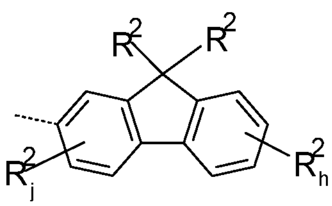

Formel ($R^1$-31)

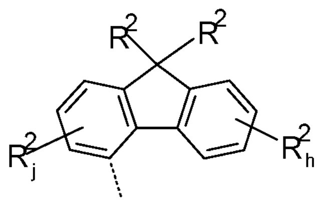

Formel ($R^1$-32)

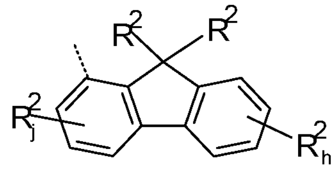

Formel ($R^1$-33)

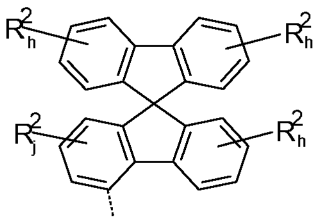

Formel ($R^1$-34)

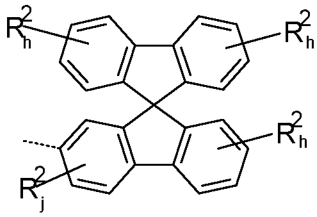

Formel ($R^1$-35)

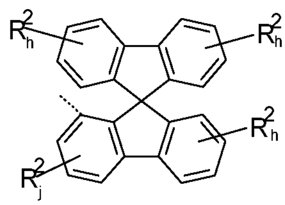

Formel ($R^1$-36)

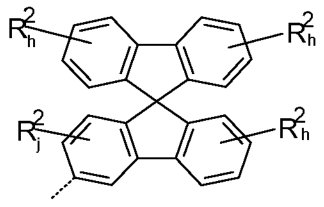

Formel ($R^1$-37)

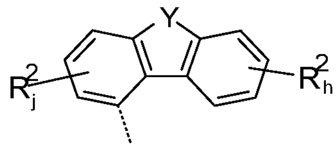

Formel ($R^1$-38)

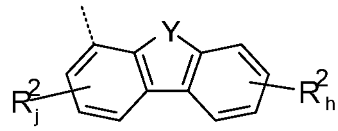

Formel ($R^1$-39)

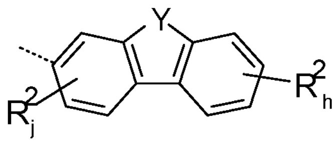

Formel ($R^1$-40)

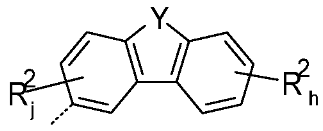

Formel ($R^1$-41)

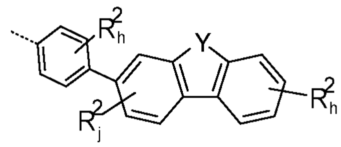

Formel ($R^1$-42)

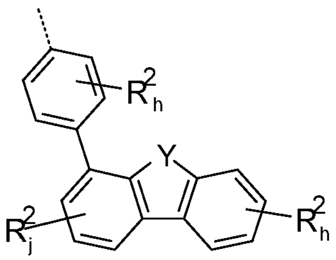

Formel ($R^1$-43)

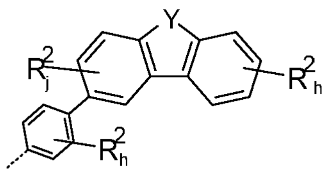

Formel ($R^1$-44)

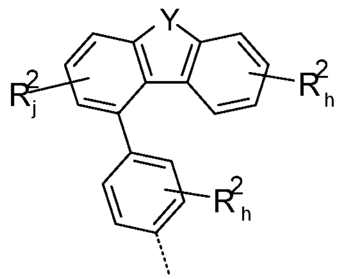

Formel ($R^1$-45)

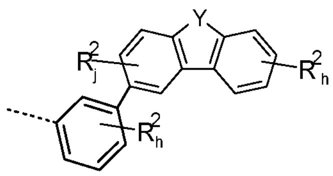

Formel ($R^1$-46)

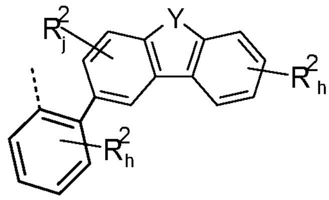

Formel ($R^1$-47)

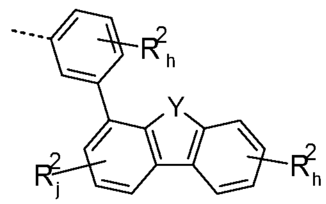

Formel ($R^1$-48)

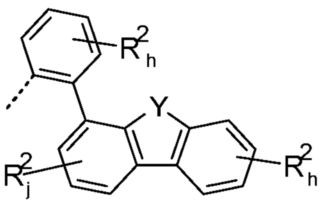

Formel ($R^1$-49)

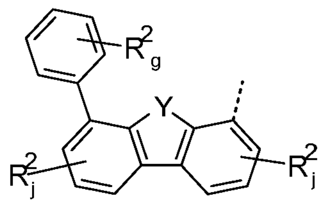

Formel ($R^1$-50)

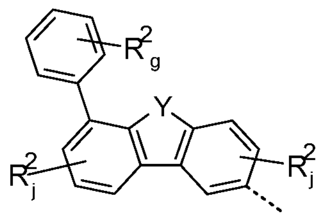

Formel ($R^1$-51)

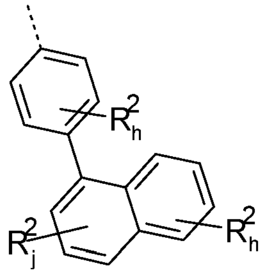

Formel ($R^1$-52)

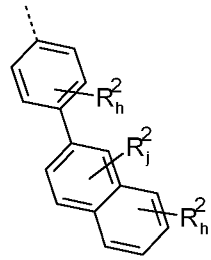

Formel ($R^1$-53)

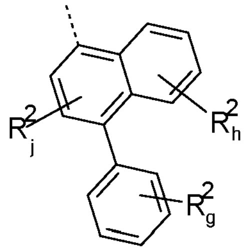

Formel ($R^1$-54)

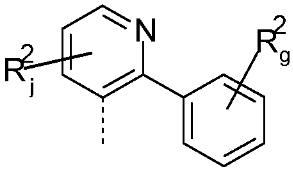

Formel ($R^1$-55)

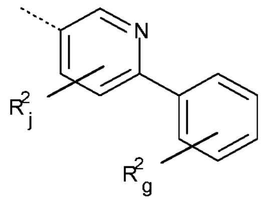

Formel ($R^1$-56)

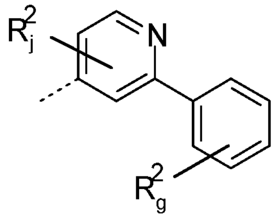

Formel ($R^1$-57)

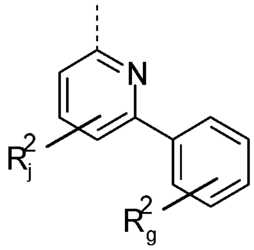

Formel ($R^1$-58)

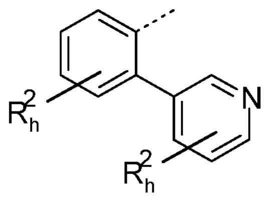

Formel ($R^1$-59)

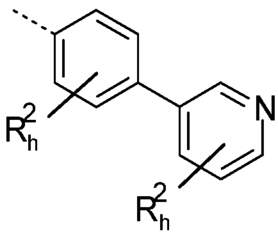

Formel ($R^1$-60)

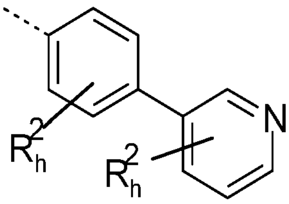

Formel ($R^1$-61)

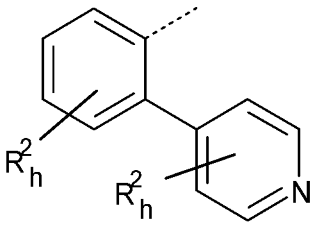

Formel ($R^1$-62)

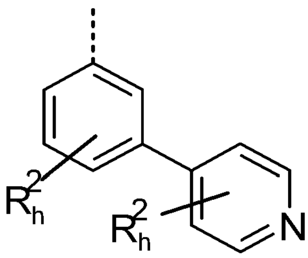

Formel ($R^1$-63)

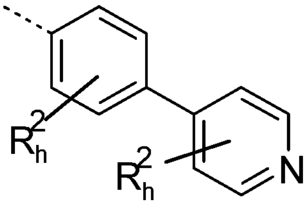

Formel ($R^1$-64)

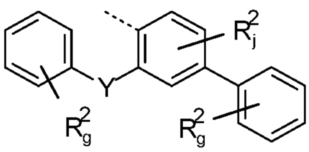

Formel ($R^1$-65)

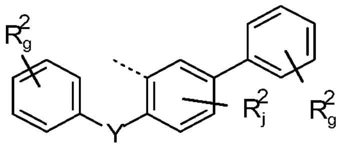

Formel ($R^1$-66)

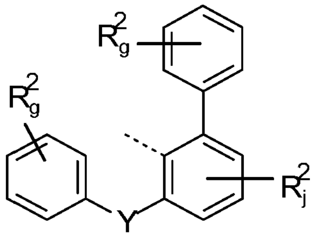

Formel ($R^1$-67)

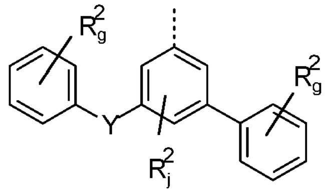

Formel ($R^1$-68)

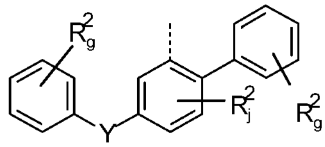

Formel ($R^1$-69)

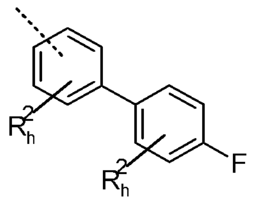

Formel ($R^1$-70)

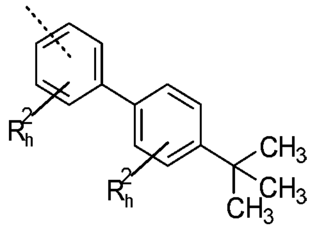

Formel ($R^1$-71)

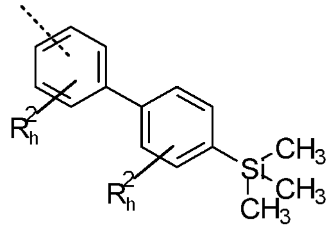

Formel ($R^1$-72),

wobei für die verwendeten Symbole gilt:

Y ist O, S oder $NR^2$, vorzugsweise O oder S;
j ist bei jedem Auftreten unabhängig 0, 1, 2 oder 3;
h ist bei jedem Auftreten unabhängig 0, 1, 2, 3 oder 4;
g ist bei jedem Auftreten unabhängig 0, 1, 2, 3, 4 oder 5;

die gestrichelte Bindung markiert die Anbindungsposition; und

$R^2$ hat die zuvor genannte Bedeutung.

Vorzugsweise kann vorgesehen sein, dass die Summe der Indices g, h und j in den Strukturen der Formel ($R^1$-1) bis ($R^1$-72) jeweils höchstens 3, vorzugsweise höchstens 2 und besonders bevorzugt höchstens 1 beträgt.

Besonders bevorzugt kann die Gruppe $R^1$, $R^a$ und/oder $R^c$ gemäß Formel (II), (IV), (V), (VI), (VII) und/oder (VIII) einen aromatischen Rest mit 6 bis 18, vorzugsweise 6 bis 12 Kohlenstoffatomen darstellen.

Die Struktur gemäß Formel (II) oder einer der bevorzugten Ausführungsformen dieser Struktur umfasst mindestens eine Gruppe $R^a$ und/oder $R^b$, wie diese zuvor näher dargelegt wurde. Die Art der funktionellen Gruppe $R^a$ und/oder $R^b$ beieinflusst die Eigenschaften der Verbindung, wobei diese Eigenschaften über einen weiten Bereich eingestellt werden können.

In diesem Zusammenhang ist festzuhalten, dass die erhaltenen Verbindungen im Allgemeinen ein wesentlich besseres Eigenschaftsprofil durch die Gegenwart des Dibenzazepin-Strukturelement aufweisen als vergleichbare Verbindungen gemäß dem Stand der Technik. Besonders bevorzugt sind insbesondere Verbindung, umfassend Strukturen der Formel (II), bzw. die zuvor oder nachfolgend aufgeführten bevorzugten Ausführungsformen, die als Matrixmaterial, als Lochtransportmaterial oder als Elektronentransportmaterial eingesetzt werden können. Hierzu können erfindungsgemäße Verbindungen, insbesondere die Reste $R^a$, $R^b$ in Strukturen gemäß den Formeln (II), (IV), (V), (VI), (VII) und/oder (VIII) eine Lochtransportgruppe und/oder eine Elektronentransportgruppe darstellen.

Ferner kann der Rest $R^a$ in Strukturen gemäß den Formeln (II), (IV), (V), (VI), (VII) und/oder (VIII) bevorzugt eine Pyridin-, Pyrimidin-, Pyrazin-, Pyridazin-, Triazin-, Dibenzofuran-, Dibenzothiophen-, Fluoren-, Spirobifluoren-, Anthracen- oder Benzimidazolgruppe darstellen. Verbindungen umfassend Strukturen gemäß den Formeln (II), (IV), (V), (VI), (VII) und/oder (VIII) mit mindestens einer Pyridin-, Pyrimidin-, Pyrazin- , Pyridazin-, Triazin-, Dibenzofuran-, Dibenzothiophen-, Fluoren-, Spirobifluoren-, Anthracen- oder Benzimidazolgruppe können mit Vorteil als Elektronentransportmaterial (ETM) verwendet werden.

Vorzugsweise kann vorgesehen sein, dass eine Elektronentransportgruppe mindestens eine Struktur umfasst, die aus der Gruppe Triazine, Pyrimidine, Pyrazine, Imidazole, Benzimidazole und Pyridine ausgewählt ist, wobei Triazinstrukturen besonders bevorzugt sind.

Ferner kann vorgesehen sein, dass dass die Elektronentransportgruppe mindestens eine Struktur gemäß den

Formeln (E-1), (E-5) bis (E-10)

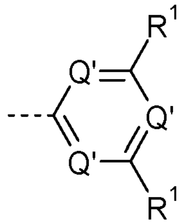

Formel (E-1)

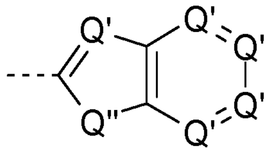

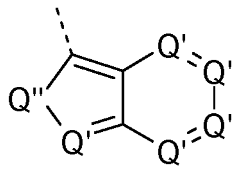

Formel (E-5) Formel (E-6)

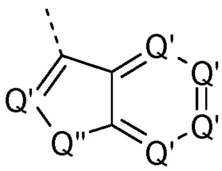

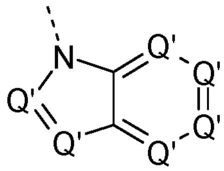

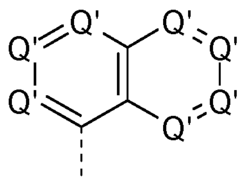

Formel (E-7) Formel (E-8) Formel (E-9)

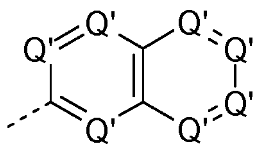

Formel (E-10),

aufweist, wobei die gestrichelte Bindung die Anbindungsposition markiert,

Q‘ bei jedem Auftreten gleich oder verschieden $CR^1$ oder N darstellt, und

Q" $NR^1$, O oder S darstellt;

wobei wenigstens ein Q’ gleich N und/oder wenigstens ein Q" gleich $NR^1$ ist und

$R^1$ wie zuvor definiert ist.

Besonders bevorzugt kann vorgesehen sein, dass die Elektronentransportgruppe mindestens eine Struktur gemäß den Formeln (E-11) bis (E-23)

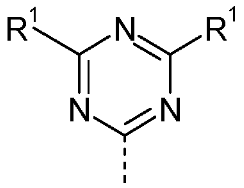

Formel (E-11)

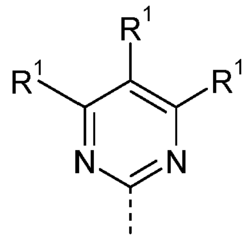

Formel (E-12)

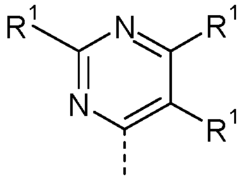

Formel (E-13)

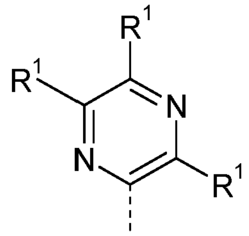

Formel (E-14)

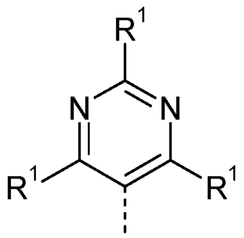

Formel (E-15)

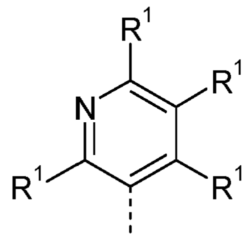

Formel (E-16)

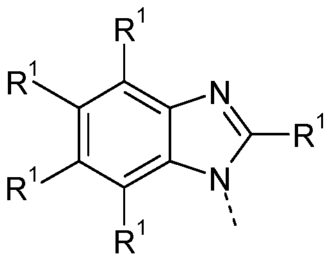

Formel (E-17)

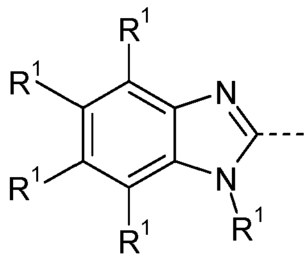

Formel (E-18)

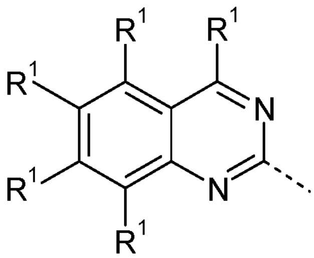

Formel (E-19)

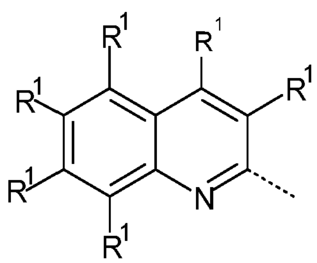

Formel (E-20)

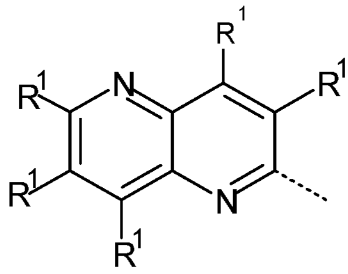

Formel (E-21)

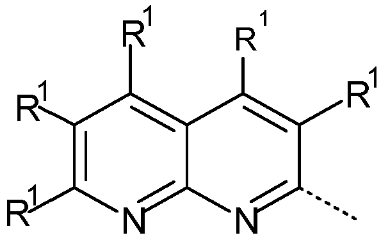

Formel (E-22)

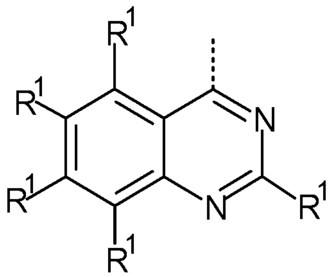

Formel (E-23)

aufweist, wobei die gestrichelte Bindung die Anbindungsposition markiert und $R^1$ die zuvor genannte Bedeutung aufweist.

Vorzugsweise kann für Verbindungen mit Strukturen gemäß Formel (E-1), Formel (E-5) bis Formel (E-23) mindestens einer vorzugsweise mindestens zwei der Reste $R^1$ für $Ar^a$ stehen, wobei $Ar^a$ bei jedem Auftreten gleich oder verschieden eine Arylgruppe mit 6 bis 40 C-Atomen oder eine Heteroarylgruppe mit 3 bis 40 C-Atomen ist, welche jeweils durch einen oder mehrere Reste $R^2$ substituiert sein kann.

Die Substituenten $R^1$ in der elektronentransportierenden Gruppe E sind vorzugsweise ausgewählt aus der Gruppe bestehend aus H oder ein aromatisches oder heteroaromatisches Ringsystem mit 5 bis 60 aromatischen Ringatomen, das jeweils durch einen oder mehrere Reste $R^2$ substituiert sein kann, wobei die Gruppen der Formel (E-11), (E-17) und (E-18) noch bevorzugter sind und die Gruppe der Formel (E-11) am meisten bevorzugt ist.

Beispiele ganz besonders bevorzugter elektronentransportierender Gruppen E sind die folgenden Gruppen, die mit einem oder mehreren voneinander unabhängigen Resten $R^2$ substituiert sein können, wobei die gestrichelten Bindungen die Anbindungsposition kennzeichnen.

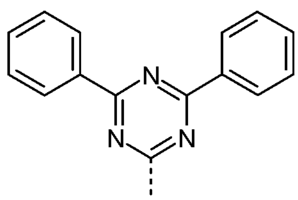

Formel (E-24)

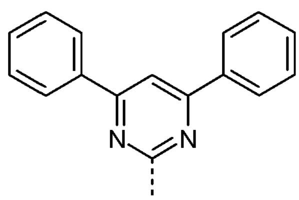

Formel (E-25)

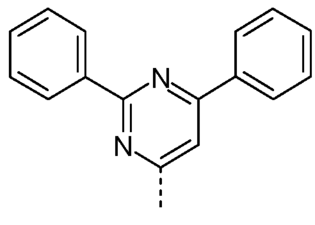

Formel (E-26)

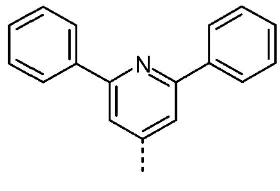

Formel (E-27)

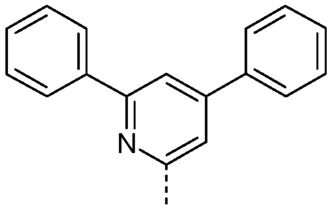

Formel (E-28)

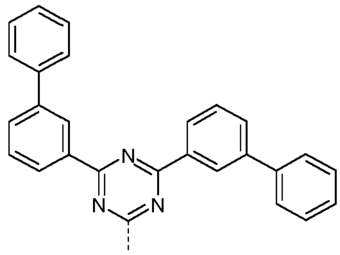

Formel (E-29)

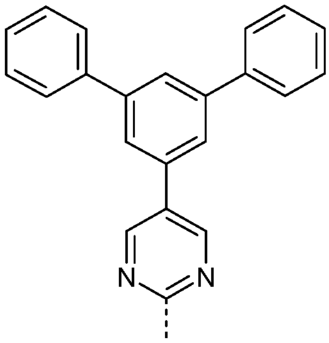

Formel (E-30)

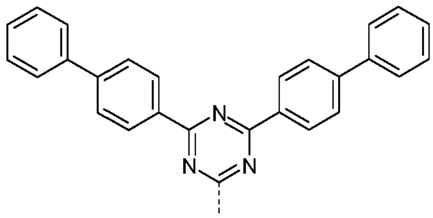

Formel (E-31)

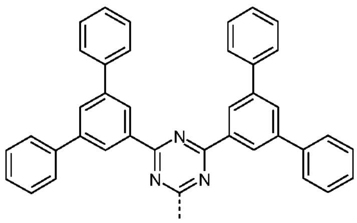

Formel (E-32)

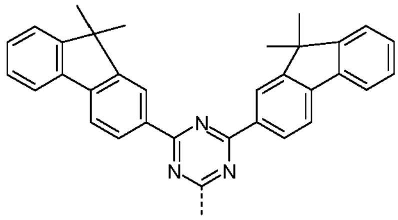

Formel (E-33)

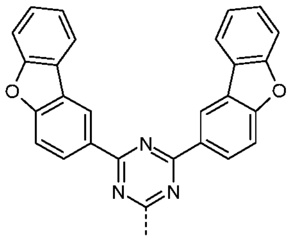

Formel (E-34)

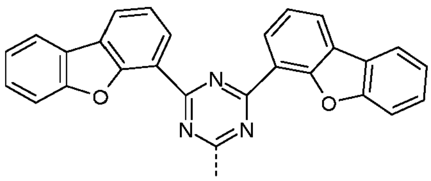

Formel (E-35)

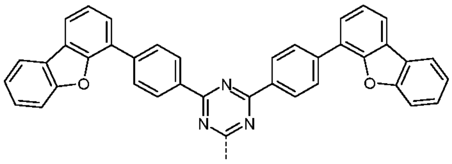

Formel (E-36)

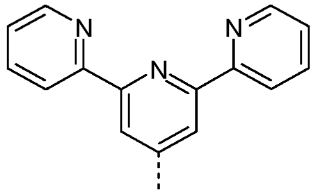

Formel (E-37)

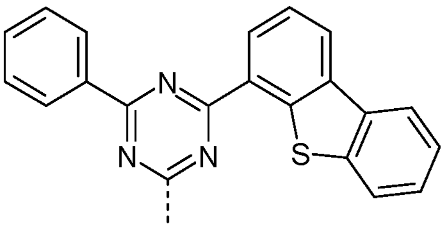

Formel (E-38)

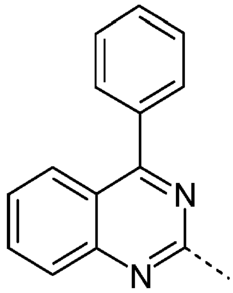

Formel (E-39)

Gemäß einer besonderen Ausführungsform der vorliegenden Erfindung kann mindestens einer der Reste $R^a$, $R^b$ in Strukturen gemäß den Formeln (II), (IV), (V), (VI), (VII) und/oder (VIII) bevorzugt eine Carbazol-, Indenocarbazol-, Indolocarbazol-, Arylamin- oder eine Diarylamingruppe darstellen. Verbindungen der Formel (II) mit mindestens einer Carbazol-, Indenocarbazol-, Indolocarbazol-, Arylamin- oder eine Diarylamingruppe können bevorzugt als Matrixmaterial eingesetzt werden.

Vorzugsweise kann vorgesehen sein, dass eine Lochtransportgruppe mindestens eine Struktur umfasst, die aus der Gruppe Triarylamine, Carbazole, Indenocarbazole und Indolocarbazole ausgewählt ist.

Ferner kann vorgesehen sein, dass dass die Lochtransportgruppe mindestens eine Struktur gemäß den Formeln (L-2) bis (L-9)

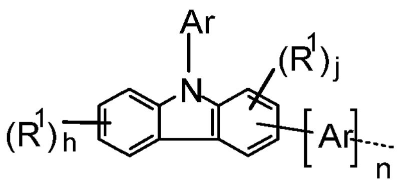

Formel (L-2)

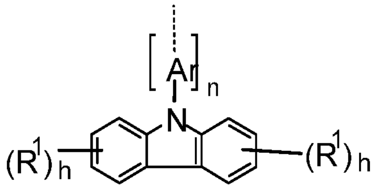

Formel (L-3)

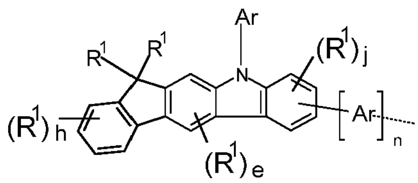

Formel (L-4)

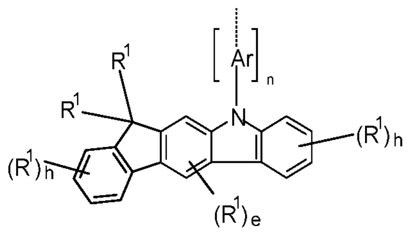

Formel (L-5)

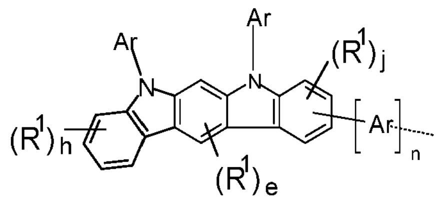

Formel (L-6)

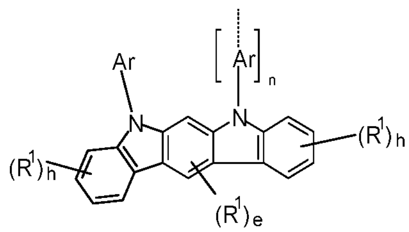

Formel (L-7)

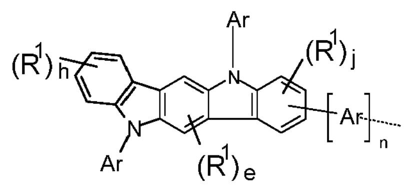

Formel (L-8)

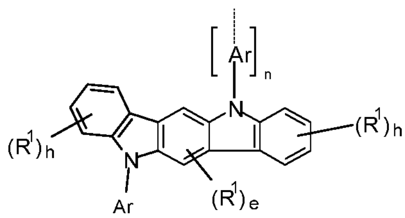

Formel (L-9)

aufweist, wobei die gestrichelte Bindung die Anbindungsposition markiert, e 0, 1 oder 2 ist, j 0, 1, 2 oder 3 ist, h 0, 1, 2, 3 oder 4 ist, n 0 oder 1 ist, Ar eine Arylgruppe mit 6 bis 40 C-Atomen oder eine Heteroarylgruppe mit 3 bis 40 C-Atomen darstellt, die durch einen oder mehrere Reste $R^1$ substituiert sein kann, und $R^1$ die zuvor genannte Bedeutung hat.

Falls eine erfindungsgemäße Verbindung mit einer oder mehreren Strukturen gemäß Formeln (II), (IV), (V), (VI), (VII) und/oder (VIII) sowohl eine Elektronentransportgruppe als auch eine Lochtransportgruppe aufweist, wobei besonders bevorzugt mindestens einer der Reste $R^a$ und/oder $R^b$ eine Elektronentransportgruppe und/oder eine Lochtransportgruppe darstellt, so können diese Verbindungen vorzugsweise als Matrixmaterial eingesetzt werden, das sowohl lochleitende als auch elektronenleitende Eigenschaften aufweist.

Vorzugsweise kann die Verbindung mit Strukturen gemäß Formel (II) oder die zuvor und nachfolgend dargestellten Ausführungsformen Reste $R^1$ umfassen, bei denen dieser Rest $R^1$ bei jedem Auftreten gleich oder verschieden bevorzugt ausgewählt ist aus der Gruppe bestehend aus H, D, F, Br, I, CN, $Si(R^2)_3$, $B(OR^2)_2$, $C(=O)Ar^1$, einer geradkettigen Alkylgruppe mit 1 bis 10 C-Atomen oder einer geradkettigen Alkoxygruppe mit 1 bis 10 C-Atomen oder einer Alkenylgruppe mit 2 bis 10 C-Atomen oder einer verzweigten oder cyclischen Alkoxygruppe mit 3 bis 10 C-Atomen oder einer verzweigten oder cyclischen Alkylgruppe mit 3 bis 10 C-Atomen, die jeweils mit einem oder mehreren Resten $R^2$ substituiert sein kann, wobei ein oder mehrere H-Atome durch D oder F ersetzt sein können, oder einem aromatischen oder heteroaromatischen Ringsystem mit 5 bis 30 aromatischen Ringatomen, das jeweils durch einen oder mehrere Reste $R^2$ substituiert sein kann.

Vorzugsweise kann die Verbindung mit Strukturen gemäß Formel (II) oder die zuvor und nachfolgend dargestellten Ausführungsformen Reste $R^2$ umfassen, bei denen diese Reste $R^2$ bei jedem Auftreten gleich oder verschieden bevorzugt ausgewählt sind aus der Gruppe bestehend aus H, D, F, Cl, Br, I, CHO, $C(=O)Ar^1$, $P(=O)(Ar^1)_2$, $S(=O)Ar^1$, $S(=O)_2Ar^1$, CN, $NO_2$, $Si(R^3)_3$, $B(OR^3)_2$, $OSO_2R^3$, eine geradkettige Alkyl-, Alkoxy- oder Thioalkoxygruppe mit 1 bis 10 C-Atomen oder eine verzweigte oder cyclische Alkyl-, Alkoxy- oder Thioalkoxygruppe mit 3 bis 10 C-Atomen, die jeweils mit einem oder mehreren Resten $R^3$ substituiert sein kann, wobei eine oder mehrere nicht benachbarte $CH_2$-Gruppen durch C≡C , $Si(R^3)_2$, $Ge(R^3)_2$, $Sn(R^3)_2$, C=O, C=S, C=Se, $P(=O)(R^3)$, SO, $SO_2$, O, S oder $CONR^3$ ersetzt sein können und wobei ein oder mehrere H-Atome durch D, F, Cl, Br, I, CN oder $NO_2$ ersetzt sein können,

oder ein aromatisches oder heteroaromatisches Ringsystem mit 5 bis 24 aromatischen Ringatomen, das jeweils durch einen oder mehrere Reste $R^3$ substituiert sein kann, oder eine Aryloxy- oder Heteroaryloxygruppe mit 5 bis 24 aromatischen Ringatomen, die durch einen oder mehrere Reste $R^3$ substituiert sein kann, oder eine Kombination dieser Systeme; dabei können zwei oder mehrere benachbarte Substituenten $R^2$ auch miteinander ein mono- oder polycyclisches, aliphatisches oder aromatisches Ringsystem bilden. Besonders bevorzugt kann mindestens einer der Reste $R^2$ in Formel (II) eine Arylgruppe oder eine Heteroarylgruppe mit 6 bis 18 Kohlenstoffatomen darstellen, die mit bis zu drei Resten $R^3$ substituiert sein kann.

Besonders bevorzugte Verbindungen umfassen Strukuren gemäß den folgenden Formeln:

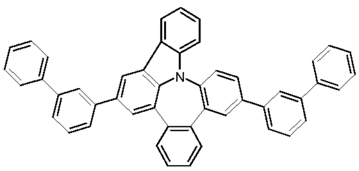

Formel 1

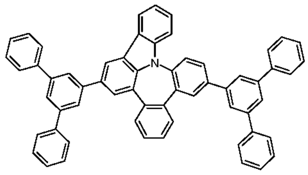

Formel 2

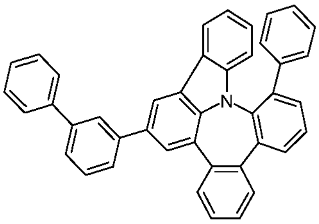

Formel 4

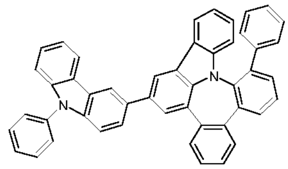

Formel 5

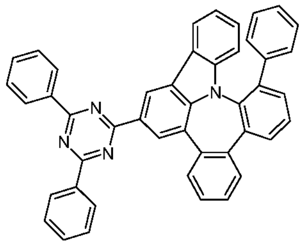

Formel 6

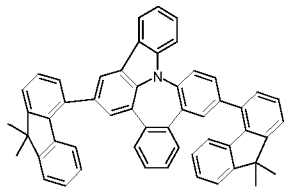

Formel 7

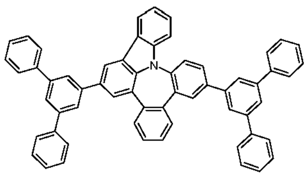

Formel 8

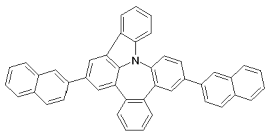

Formel 10

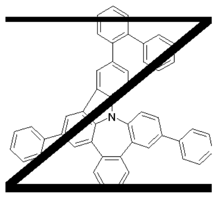

~~Formel 11~~

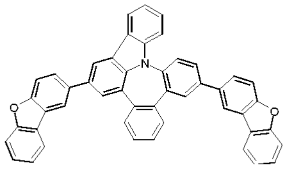
Formel 14

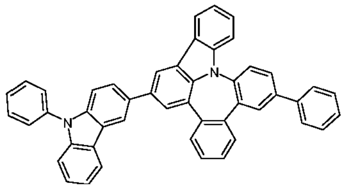
Formel 15

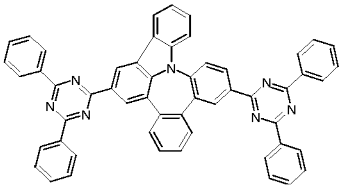
Formel 16

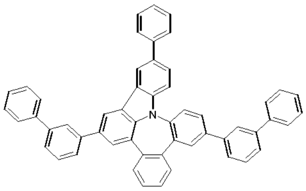
Formel 17

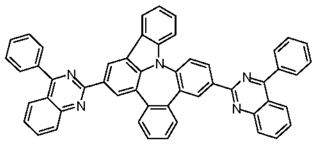
Formel 19

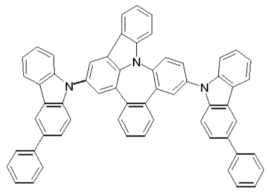
Formel 20

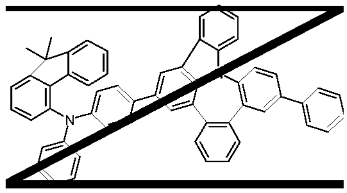
Formel 21

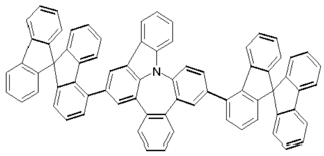
Formel 22

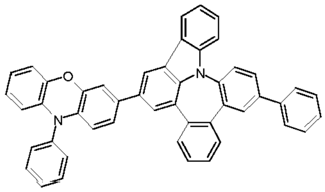
Formel 24

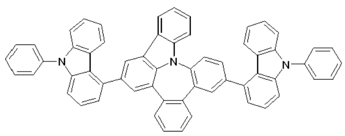
Formel 27

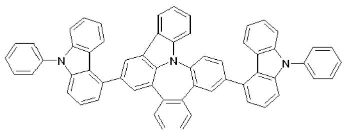
Formel 28

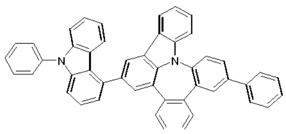
Formel 29

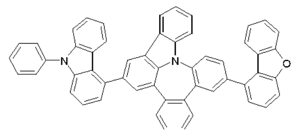
Formel 30

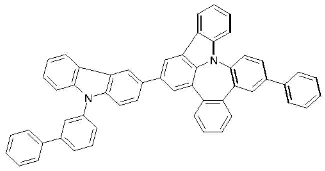
Formel 34

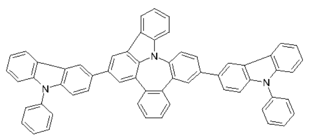
Formel 36

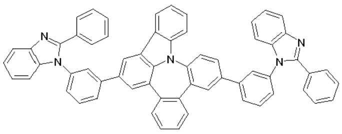
Formel 43

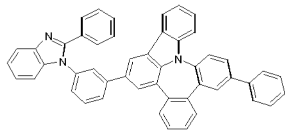
Formel 44

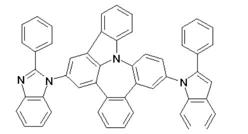
Formel 45

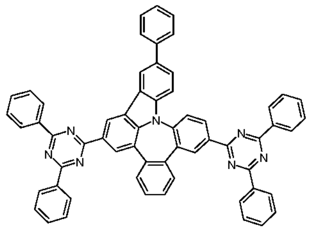
Formel 46

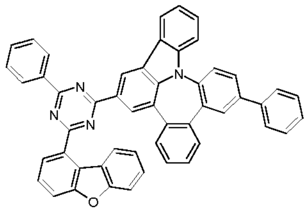
Formel 49

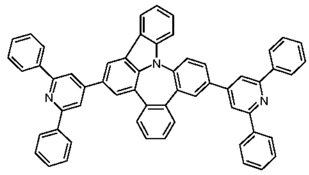
Formel 50

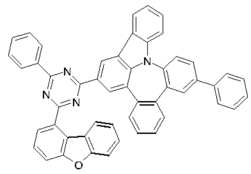
Formel 51

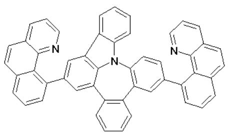
Formel 52

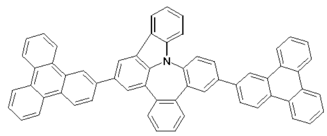
Formel 53

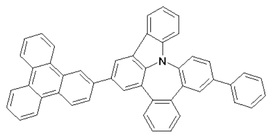
Formel 54

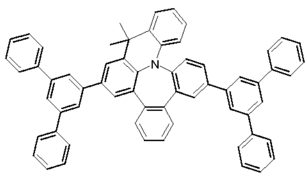
Formel 55

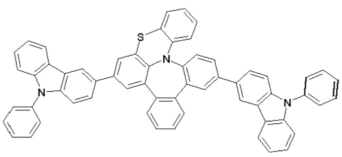
Formel 57

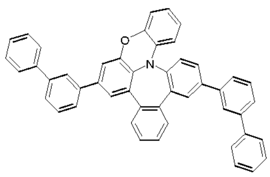
Formel 58

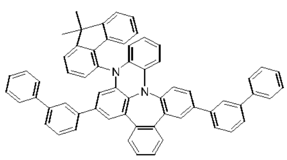
Formel 62

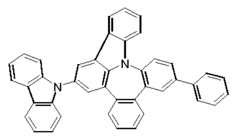
Formel 72

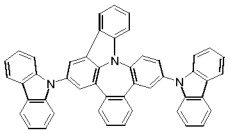
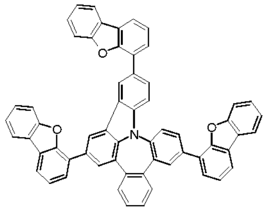
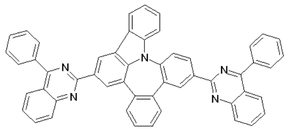

Formel 73 Formel 75 Formel 76

Bevorzugte Ausführungsformen von erfindungsgemäßen Verbindungen werden in den Beispielen näher ausgeführt, wobei diese Verbindungen allein oder in Kombination mit weiteren für alle erfindungsgemäßen Verwendungszwecke eingesetzt werden können.

Unter der Voraussetzung, dass die in Anspruch 1 genannten Bedingungen eingehalten werden, sind die oben genannten bevorzugten Ausführungsformen beliebig miteinander kombinierbar. In einer besonders bevorzugten Ausführungsform der Erfindung gelten die oben genannten bevorzugten Ausführungsformen gleichzeitig.

Die erfindungsgemäßen Verbindungen sind prinzipiell durch verschiedene Verfahren darstellbar. Es haben sich jedoch die im Folgenden beschriebenen Verfahren als besonders geeignet herausgestellt.

Daher ist ein weiterer Gegenstand der vorliegenden Erfindung ein Verfahren zur Herstellung der Verbindungen, umfassend Strukturen gemäß Formel (II), bei dem an einer Verbindung mit einem Azepinstrukturelement eine Ringschlussreaktion durchgeführt wird.

Verbindungen mit einem Azepinstrukturelement, beispielsweise 9H-Tribenz[b,d,f]azepin können vielfach kommerziell erhalten werden, wobei die in den Beispielen dargelegten Ausgangsverbindungen durch bekannte Verfahren erhältlich sind, so dass hierauf verwiesen wird.

Diese Verbindungen können durch bekannte Kupplungsreaktionen mit weiteren Arylverbindungen umgesetzt werden, wobei die erhaltenen Zwischenverbindungen nachfolgend einer Ringschlussreaktion unterzogen werden, um die erfindungsgemäßen Verbindungen, die Strukturen gemäß den Formeln (II), (IV), (V), (VI), (VII) und/oder (VIII) zu erhalten. Ferner können die nach einer Ringschlussreaktion erhaltenen Verbindungen in weiteren Reaktionen umgesetzt werden, um erfindungsgemäße Produkte zu erhalten.

Die notwendigen Bedingungen hierfür sind dem Fachmann bekannt, wobei die ausführlichen Angaben in den Beispielen den Fachmann zur Durchführung dieser Umsetzungen unterstützen.

Besonders geeignete und bevorzugte Kupplungsreaktionen, die alle zu C-C-Verknüpfungen und/oder C-N-Verknüpfungen führen, sind solche gemäß BUCHWALD, SUZUKI, YAMAMOTO, STILLE, HECK, NEGISHI, SONOGASHIRA und HIYAMA. Diese Reaktionen sind weithin bekannt, wobei die Beispiele dem Fachmann weitere Hinweise bereitstellen.

In allen folgenden Syntheseschemata sind die Verbindungen zur Vereinfachung der Strukturen mit einer geringen Anzahl an Substituenten gezeigt gezeigt. Dies schließt das Vorhandensein von beliebigen weiteren Substituenten in den Verfahren nicht aus.

Eine Umsetzung ergibt sich beispielhaft gemäß folgenden Schemata, ohne dass hierdurch eine Beschränkung erfolgen soll. Die Teilschritte der einzelnen Schemata können hierbei beliebig kombiniert werden.

Beispielsweise kann gemäß Schema 1, ausgehend von einer 9H-Tribenz[b,d,f]azepin-Verbindung ein reaktives Zwischenprodukt durch eine Buchwald-Kupplung hergestellt werden. Das erhaltene Zwischenprodukt kann mittels eines Katalysators, beispielsweise $Pd(OAc)_2$ in einer Ringschlussreaktion unterzogen werden, um eine erfindungsgemäße Verbindung zu erhalten.

## Schema 1

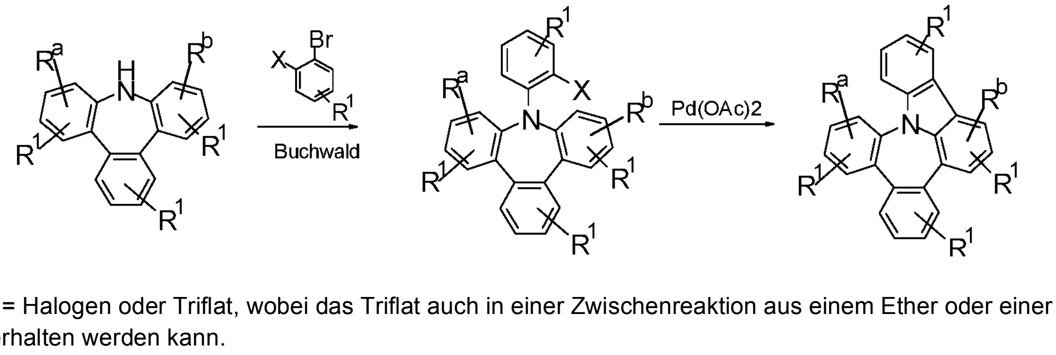


X = Halogen oder Triflat, wobei das Triflat auch in einer Zwischenreaktion aus einem Ether oder einer Hydroxygruppe erhalten werden kann.

In Umsetzungen nach Schema 2 wird, ausgehen von einem Edukt mit einem Azepinstrukturelement eine Reaktion mit einer Fluorarylverbindung, die eine Nitrogruppe umfasst, durchgeführt, beispielsweise unter Verwendung von $Cs_2CO_3$. Die erhaltene Zwischenverbindung wird zunächst reduziert, um die Nitrogruppe der Zwischenverbindung in eine Aminogruppe umzusetzen, wobei beispielsweise $SnCl_2$ als Reduktionsmittel eingesetzt werden kann. Das hierdurch erhältliche Zwischenprodukt mit einer Aminogruppe kann nachfolgend beispielsweise mittel $NaNO_2$ über eine Ringschlussreaktion in eine erfindungsgemäße Verbindung überführt werden.

## Schema 2

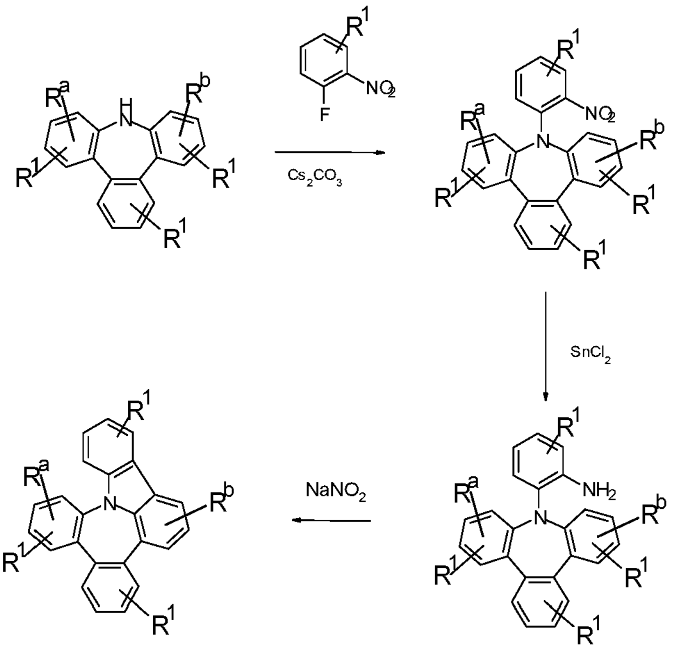


Gemäß Schema 3 kann eine Azepin-Verbindung mit einem Arylhalogenid umgesetzt werden, welches eine Estergruppe aufweist. Die Estergruppe der erhaltenen Zwischenstufe kann anschließend zu einem Alkohol reduziert werden, wobei beispielsweise eine metallorganische Verbindung, unter anderem Methyllithium eingesetzt werden kann. Das hergestellte Zwischenprodukt kann anschließend einer Ringschlussreaktion unterzogen werden, wobei unter anderem eine Säure Verwendung finden kann.

## Schema 3

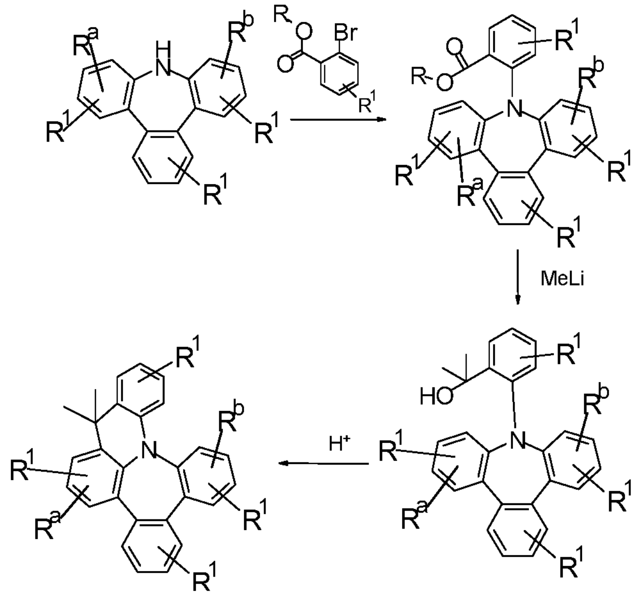


Gemäß Schema 4 kann eine Azepin-Verbindung mit einer Abgangsgruppe X, die in Nachbarschaft zum Stickstoffatom der Azepin-Verbindung steht, in einer Buchwald-Kupplung umgesetzt werden, wobei hierfür eine Arylverbindung mit einer Nitrogruppe eingesetzt wird, die in ortho-Stellung zur Nitrogruppe eine Abgangsgruppe X aufweist. Die Nitrogruppe der erhaltenen Zwischenstufe kann anschließend zu einer Aminogruppe reduziert werden, wobei beispielsweise $SnCl_2$ eingesetzt werden kann. Das hergestellte Zwischenprodukt kann anschließend einer Ringschlussreaktion unterzogen werden, wobei der Ringschluss durch eine Buchwald-Kupplung bewirkt werden kann. Hierdurch wird eine Diarylaminostruktur erhalten, die in einer weiteren Buchwald-Kupplung unter Verwendung einer Arylverbindung mit einer Abgangsgruppe X umgesetzt werden kann.

## Schema 4

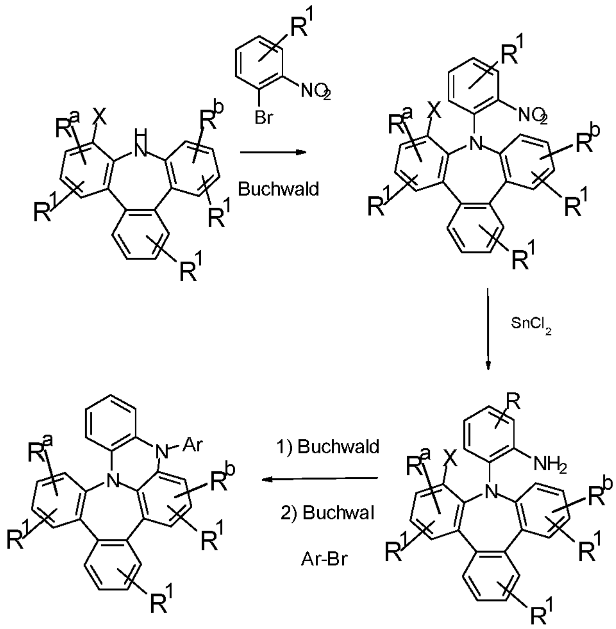


Ar = Arylverbindung
X = Halogen oder Triflat, wobei das Triflat auch in einer Zwischenreaktion aus einem Ether oder einer Hydroxygruppe erhalten werden kann.

Die gezeigten Verfahren zur Synthese der erfindungsgemäßen Verbindungen sind exemplarisch zu verstehen.

Der Fachmann kann alternative Synthesewege im Rahmen seines allgemeinen Fachwissens entwickeln.

Die Grundlagen der zuvor dargelegten Herstellungsverfahren sind im Prinzip aus der Literatur für ähnliche Verbindungen bekannt und können vom Fachmann leicht zur Herstellung der erfindungsgemäßen Verbindungen angepasst werden. Weitere Informationen können den Beispielen entnommen werden.

Durch diese Verfahren, gegebenenfalls gefolgt von Aufreinigung, wie z. B. Umkristallisation oder Sublimation, lassen sich die erfindungsgemäßen Verbindungen, umfassend Strukturen gemäß Formel (II) in hoher Reinheit, bevorzugt mehr als 99 % (bestimmt mittels $^1$H-NMR und/oder HPLC) erhalten.

Die erfindungsgemäßen Verbindungen können auch geeignete Substituenten aufweisen, beispielsweise durch längere Alkylgruppen (ca. 4 bis 20 C-Atome), insbesondere verzweigte Alkylgruppen, oder gegebenenfalls substituierte Arylgruppen, beispielsweise Xylyl-, Mesityl- oder verzweigte Terphenyl- oder Quaterphenylgruppen, die eine Löslichkeit in gängigen organischen Lösemitteln bewirken, wie beispielsweise Toluol oder Xylol bei Raumtemperatur in ausreichender Konzentration löslich, um die Verbindungen aus Lösung verarbeiten zu können. Diese löslichen Verbindungen eignen sich besonders gut für die Verarbeitung aus Lösung, beispielsweise durch Druckverfahren. Weiterhin ist festzuhalten, dass die erfindungsgemäßen Verbindungen, umfassend mindestens eine Struktur der Formel (II) bereits eine gesteigerte Löslichkeit in diesen Lösungsmitteln besitzen.

Die erfindungsgemäßen Verbindungen können auch mit einem Polymer gemischt werden. Ebenso ist es möglich, diese Verbindungen kovalent in ein Polymer einzubauen. Dies ist insbesondere möglich mit Verbindungen, welche mit reaktiven Abgangsgruppen, wie Brom, Iod, Chlor, Boronsäure oder Boronsäureester, oder mit reaktiven, polymerisierbaren Gruppen, wie Olefinen oder Oxetanen, substituiert sind. Diese können als Monomere zur Erzeugung entsprechender Oligomere, Dendrimere oder Polymere Verwendung finden. Die Oligomerisation bzw. Polymerisation erfolgt dabei bevorzugt über die Halogenfunktionalität bzw. die Boronsäurefunktionalität bzw. über die polymerisierbare Gruppe. Es ist weiterhin möglich, die Polymere über derartige Gruppen zu vernetzen. Die erfindungsgemäßen Verbindungen und Polymere können als vernetzte oder unvernetzte Schicht eingesetzt werden.

Weiterer Gegenstand der Erfindung sind daher Oligomere, Polymere oder Dendrimere enthaltend eine oder mehrere der oben aufgeführten Strukturen der Formel (II) oder erfindungsgemäße Verbindungen, wobei ein oder mehrere Bindungen der erfindungsgemäßen Verbindungen oder der Strukturen der Formel (II) zum Polymer, Oligomer oder Dendrimer vorhanden sind. Je nach Verknüpfung der Strukturen der Formel (II) bzw. der Verbindungen bilden diese daher eine Seitenkette des Oligomers oder Polymers oder sind in der Hauptkette verknüpft. Die Polymere, Oligomere oder Dendrimere können konjugiert, teilkonjugiert oder nicht-konjugiert sein. Die Oligomere oder Polymere können linear, verzweigt oder dendritisch sein. Für die Wiederholeinheiten der erfindungsgemäßen Verbindungen in Oligomeren, Dendrimeren und Polymeren gelten dieselben Bevorzugungen, wie oben beschrieben.

Zur Herstellung der Oligomere oder Polymere werden die erfindungsgemäßen Monomere homopolymerisiert oder mit weiteren Monomeren copolymerisiert. Bevorzugt sind Copolymere, wobei die Einheiten gemäß Formel (II) bzw. die zuvor und nachfolgend ausgeführten bevor¬zugten Ausführungsformen zu 0.01 bis 99.9 mol%, bevorzugt 5 bis 90 mol%, besonders bevorzugt 20 bis 80 mol% vorhanden sind. Geeignete und bevorzugte Comonomere, welche das Polymergrundgerüst bilden, sind gewählt aus Fluorenen (z. B. gemäß EP 842208 oder WO 2000/022026), Spirobifluorenen (z. B. gemäß EP 707020, EP 894107 oder WO 2006/061181), Para-phenylenen (z. B. gemäß WO 92/18552), Carbazolen (z. B. gemäß WO 2004/070772 oder WO 2004/113468), Thiophenen (z. B. gemäß EP 1028136), Dihydrophenanthrenen (z. B. gemäß WO 2005/014689), cis- und trans-Indenofluorenen (z. B. gemäß WO 2004/041901 oder WO 2004/113412), Ketonen (z. B. gemäß WO 2005/040302), Phenanthrenen (z. B. gemäß WO 2005/104264 oder WO 2007/017066) oder auch mehreren dieser Einheiten. Die Polymere, Oligomere und Dendrimere können noch weitere Einheiten enthalten, beispielsweise Lochtransporteinheiten, insbesondere solche basierend auf Triarylaminen, und/oder Elektronentransporteinheiten.

Ferner können die vorliegenden Verbindungen ein relativ geringes Molekulargewicht aufweisen, beispielsweise ein Molekulargewicht von vorzugsweise kleiner oder gleich 10000 g/mol, bevorzugt kleiner oder gleich 5000 g/mol, besonders bevorzugt kleiner oder gleich 4000 g/mol, insbesondere bevorzugt kleiner oder gleich 3000 g/mol, speziell bevorzugt kleiner oder gleich 2000 g/mol und ganz besonders bevorzugt kleiner oder gleich 1000 g/mol aufweist.

Weiterhin zeichnen sich bevorzugte erfindungsgemäße Verbindungen dadurch aus, dass diese sublimierbar sind. Diese Verbindungen weisen im Allgemeinen eine Molmasse von weniger als ca. 1200 g/mol auf.

Von besonderem Interesse sind des Weiteren erfindungsgemäße Verbindungen, die sich durch eine hohe Glasübergangstemperatur auszeichnen. In diesem Zusammenhang sind insbesondere erfindungsgemäße Verbindungen umfassend Strukturen der allgemeinen Formel (II) bzw. die zuvor und nachfolgend ausgeführten bevor¬zugten Ausführungsformen bevorzugt, die eine Glasübergangstemperatur von mindestens 70°C, besonders bevorzugt von mindestens 110°C, ganz besonders bevorzugt von mindestens 125°C und insbesondere bevorzugt von mindestens 150°C aufweisen, bestimmt nach DIN 51005.

Nochmals ein weiterer Gegenstand der vorliegenden Erfindung ist eine Formulierung, enthaltend eine erfindungsgemäße Verbindung bzw. ein erfindungsgemäßes Oligomer, Polymer oder Dendrimer und mindestens ein Lösemittel. Eine weitere Verbindung in der Formulierung kann aber auch eine weitere organische oder anorganische Verbindung sein, die ebenfalls in der elektronischen Vorrichtung eingesetzt wird, beispielsweise ein Matrixmaterial. Diese weitere Verbindung kann auch polymer sein.

Geeignete und bevorzugte Lösungsmittel sind beispielsweise Toluol, Anisol, o-, m- oder p-Xylol, Methylbenzoat, Mesitylen, Tetralin, Veratrol, THF, Methyl-THF, THP, Chlorbenzol, Dioxan, Phenoxytoluol, insbesondere 3-Phenoxytoluol, (-)-Fenchon, 1,2,3,5-Tetramethylbenzol, 1,2,4,5-tetramethylbenzol, 1-Methylnaphtalin, 2-Methylbenzothiazol, 2-Phenoxyethanol, 2-Pyrrolidinon, 3-Methylanisol, 4-Methylanisol, 3,4-Dimethylanisol, 3,5-Dimethylanisol, Acetophenon, α-Terpineol, Benzothiazol, Butylbenzoat, Cumol, Cyclohexanol, Cyclohexanon, Cyclohexylbenzol, Decalin, Dodecylbenzol, Ethylbenzoat, Indan, Methylbenzoat, NMP, p-Cymol, Phenetol, 1,4-Diisopropylbenzol, Dibenzyl ether, Diethylenglycolbutylmethylether, Triethylenglycolbutylmethylether, Diethylenglycoldibutylether, Triethylenglycoldimethylether, Diethylen-glycolmonobutylether, Tripropylenglycoldimethylether, Tetraethylenglycol-dimethylether, 2-Isopropylnaphthalin, Pentylbenzol, Hexylbenzol, Heptylbenzol, Octylbenzol, 1,1-Bis(3,4-Dimethylphenyl)ethan oder Mischungen dieser Lösungsmittel.

Nochmals ein weiterer Gegenstand der vorliegenden Erfindung ist eine Zusammensetzung enthaltend eine erfindungsgemäße Verbindung und wenigstens ein weiteres organisch funktionelles Material nach Anspruch 23. Funktionelle Materialen sind generell die organischen oder anorganischen Materialien, welche zwischen Anode und Kathode eingebracht sind. Das organisch funktionelle Material kann ausgewählt aus der Gruppe bestehend aus fluoreszierenden Emittern, phosphoreszierenden Emittern, Host Materialien, Matrix-Materialien, Elektronentransportmaterialien, Elektroneninjektionsmaterialien, Lochleitermaterialien, Lochinjektionsmaterialien, n-Dotanden, Wide-Band-Gap-Materialien, Elektronenblockiermaterialien und Lochblockiermaterialien werden.

Die vorliegenden Erfindung betrifft daher auch eine Zusammensetzung enthaltend wenigstens eine Verbindung umfassend Strukuren gemäß Formel (II) bzw. die zuvor ausgeführten bevor¬zugten Ausführungsformen sowie wenigstens ein weiteres Matrixmaterial. Gemäß einem besonderen Aspekt der vorliegenden Erfindung weist das weitere Matrixmaterial lochtransportierende Eigenschaften auf.

Ein alternative Zusammensetzung enthält wenigstens eine Verbindung, umfassend mindestens eine Struktur gemäß Formel (II) bzw. die zuvor und nachfolgend ausgeführten bevor¬zugten Ausführungsformen sowie wenigstens ein Wide-Band-Gap-Material, wobei unter Wide-Band-Gap-Material ein Material im Sinne der Offenbarung von US 7,294,849 verstanden wird. Diese Systeme zeigen besondere vorteilhafte Leistungsdaten in elektrolumineszierenden Vorrichtungen.

Vorzugsweise kann die zusätzliche Verbindung eine Bandlücke (band gap) von 2,5 eV oder mehr, bevorzugt 3,0 eV oder mehr, ganz bevorzugt von 3,5 eV oder mehr aufweisen. Die Bandlücke kann unter anderem durch die Energieniveaus des highest occupied molecular orbital (HOMO) und des lowest unoccupied molecular orbital (LUMO) berechnet werden.

Molekülorbitale, insbesondere auch das highest occupied molecular orbital (HOMO) und das lowest unoccupied molecular orbital (LUMO), deren Energieniveaus sowie die Energie des niedrigsten Triplettzustands $T_1$ bzw. des niedrigsten angeregten Singulettzustands $S_1$ der Materialien werden über quantenchemische Rechnungen bestimmt. Zur Berechnung organischer Substanzen ohne Metalle wird zuerst eine Geometrieoptimierung mit der Methode "Ground State/Semi-empirical/Default Spin/AM1/Charge 0/Spin Singlet" durchgeführt. Im Anschluss erfolgt auf Grundlage der optimierten Geometrie eine Energierechnung. Hierbei wird die Methode "TD-SCF/DFT/Default Spin/B3PW91" mit dem Basissatz "6-31 G(d)" verwendet (Charge 0, Spin Singlet). Für metallhaltige Verbindungen wird die Geometrie über die Methode "Ground State/ Hartree-Fock/Default Spin/LanL2MB/Charge 0/Spin Singlet" optimiert. Die Energierechnung erfolgt analog zu der oben beschriebenen Methode für die organischen Substanzen mit dem Unterschied, dass für das Metallatom der Basissatz "LanL2DZ" und für die Liganden der Basissatz "6-31 G(d)" verwendet wird. Aus der Energierechnung erhält man das HOMO-Energieniveau HEh bzw. LUMO-Energieniveau LEh in Hartree-Einheiten. Daraus werden die anhand von Cyclovoltammetriemessungen kalibrierten HOMO- und LUMO-Energieniveaus in Elektronenvolt wie folgt bestimmt:

$$HOMO(eV) = ((HEh*27.212)-0.9899)/1.1206$$

$$LUMO(eV) = ((LEh*27.212)-2.0041)/1.385$$

Diese Werte sind im Sinne dieser Anmeldung als HOMO- bzw. LUMO-Energieniveaus der Materialien anzusehen.

Der niedrigste Triplettzustand $T_1$ ist definiert als die Energie des Triplettzustands mit der niedrigsten Energie, der sich aus der beschriebenen quantenchemischen Rechnung ergibt.

Der niedrigste angeregte Singulettzustand $S_1$ ist definiert als die Energie des angeregten Singulettzustands mit der niedrigsten Energie, der sich aus der beschriebenen quantenchemischen Rechnung ergibt.

Die hierin beschriebene Methode ist unabhängig von dem verwendeten Softwarepaket und liefert immer dieselben Ergebnisse. Beispiele oft benutzter Programme für diesen Zweck sind "Gaussian09W" (Gaussian Inc.) und Q-Chem 4.1 (Q-Chem, Inc.).

Die vorliegende Erfindung betrifft auch eine Zusammensetzung umfassend wenigstens eine Verbindung umfassend Strukuren gemäß Formel (II) bzw. die zuvor ausgeführten bevor¬zugten Ausführungsformen sowie wenigstens einen phosphoreszierende Emitter, wobei unter dem Begriff phosphoreszierende Emitter auch phosphoreszierende Dotanden verstanden werden.

Vom Begriff phosphoreszierende Dotanden sind typischerweise Verbindungen umfasst, bei denen die Lichtemission durch einen spinverbotenen Übergang erfolgt, beispielsweise einen Übergang aus einem angeregten Triplettzustand oder einem Zustand mit einer höheren Spinquantenzahl, beispielsweise einem Quintett-Zustand.

Als phosphoreszierende Dotanden eignen sich insbesondere Verbindungen, die bei geeigneter Anregung Licht, vorzugsweise im sichtbaren Bereich, emittieren und außerdem mindestens ein Atom der Ordnungszahl größer 20, bevorzugt größer 38 und kleiner 84, besonders bevorzugt größer 56 und kleiner 80 enthalten. Bevorzugt werden als phosphoreszierende Dotanden Verbindungen, die Kupfer, Molybdän, Wolfram, Rhenium, Ruthenium, Osmium, Rhodium, Iridium, Palladium, Platin, Silber, Gold oder Europium enthalten, verwendet, insbesondere Verbindungen, die Iridium, Platin oder Kupfer enthalten.

Dabei werden im Sinne der vorliegenden Anmeldung alle lumineszierenden Iridium-, Platin- oder Kupferkomplexe als phosphoreszierende Verbindungen angesehen. Beispiele für phosphoreszierende Dotanden sind in einem folgenden Abschnitt aufgeführt.

Unter einem Dotanden wird in einem System enthaltend ein Matrixmaterial und einen Dotanden diejenige Komponente verstanden, deren Anteil in der Mischung der kleinere ist. Entsprechend wird unter einem Matrixmaterial in einem System enthaltend ein Matrixmaterial und einen Dotanden diejenige Komponente verstanden, deren Anteil in der Mischung der größere ist.

Bevorzugte phosphoreszierende Dotanden zur Verwendung in Mixed-Matrix-Systemen sind die im Folgenden angebenen bevorzugten phosphoreszierenden Dotanden.

Beispiele für phosphoreszierende Dotanden können den Anmeldungen WO 2000/70655, WO 2001/41512, WO 2002/02714, WO 2002/15645, EP 1191613, EP 1191612, EP 1191614, WO 2005/033244, WO 2005/019373 und US 2005/0258742 entnommen werden. Generell eignen sich alle phosphoreszierenden Komplexe, wie sie gemäß dem Stand der Technik für phosphoreszierende OLEDs verwendet werden und wie sie dem Fachmann auf dem Gebiet der organischen Elektrolumineszenzvorrichtungen bekannt sind, zur Verwendung in den erfindungsgemäßen Vorrichtungen.

Explizite Beispiele für phosphoreszierende Dotanden sind in der folgenden Tabelle aufgeführt.

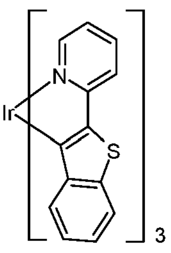
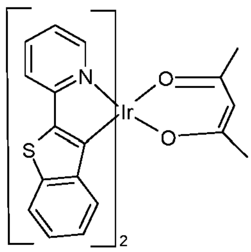
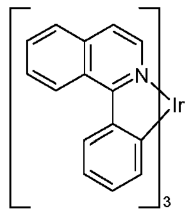

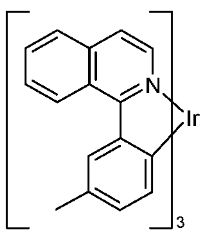
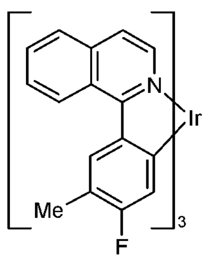
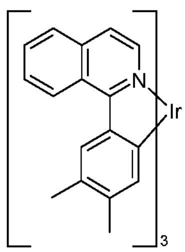

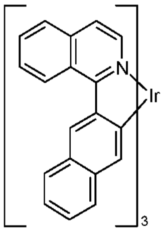

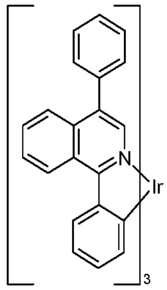

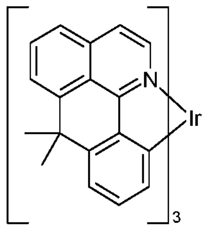

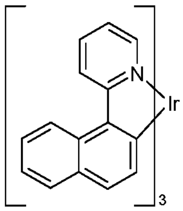

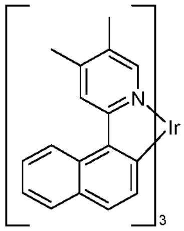

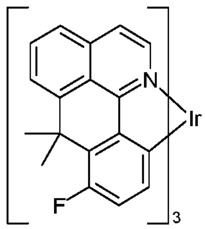

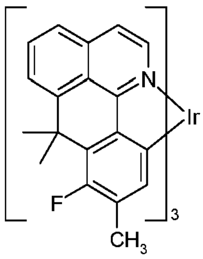

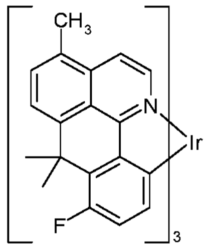

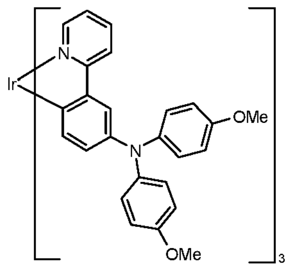

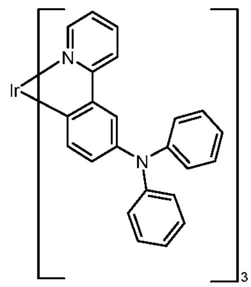

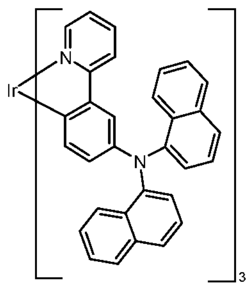

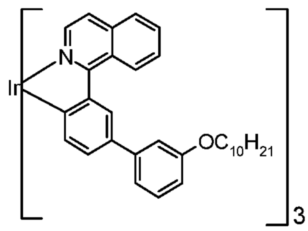

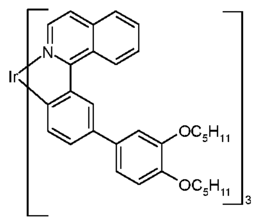

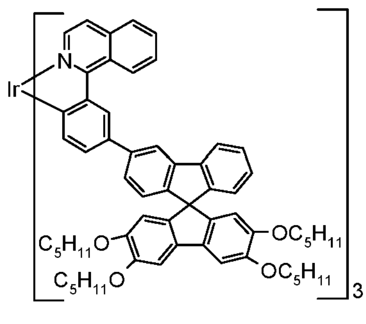

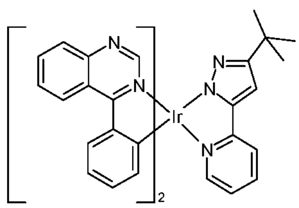

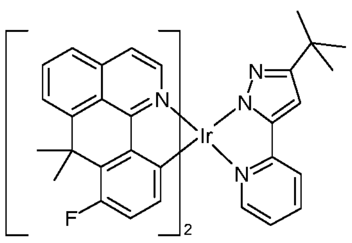
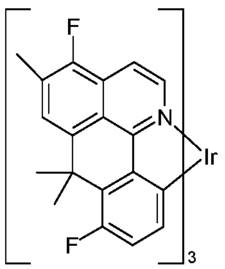
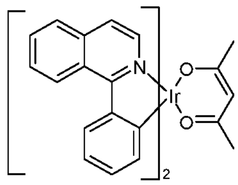

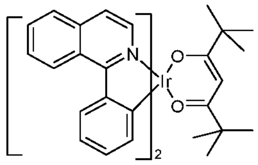
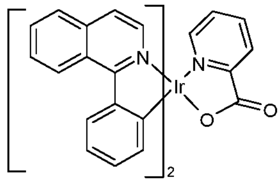
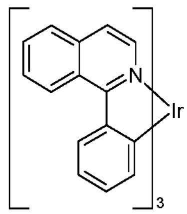

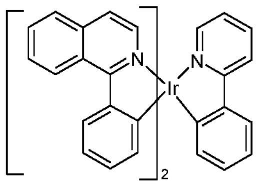
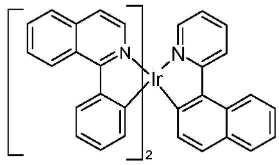
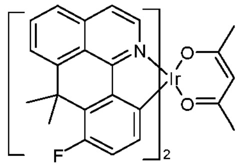

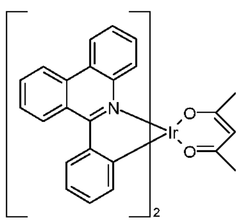
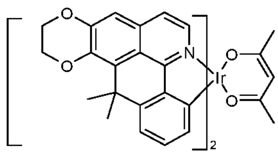
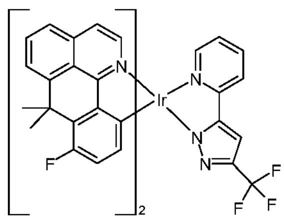

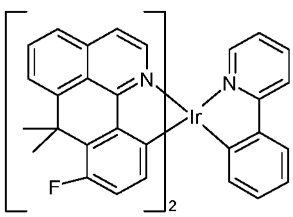
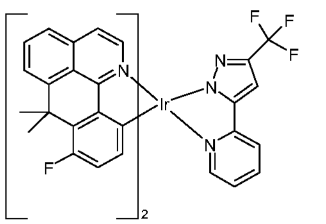
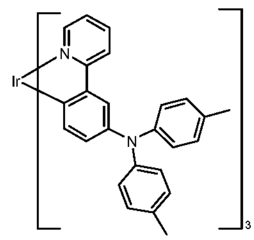

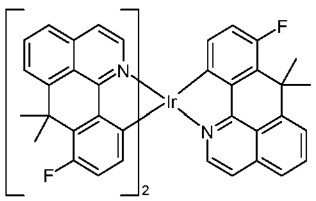
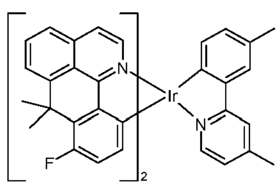
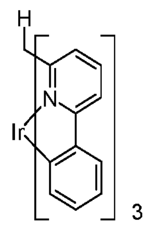

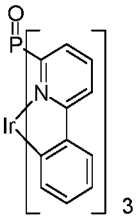
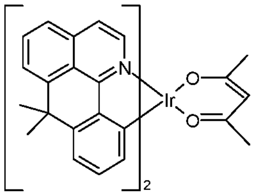
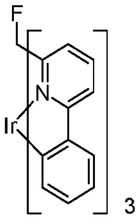

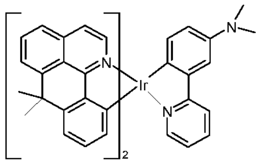
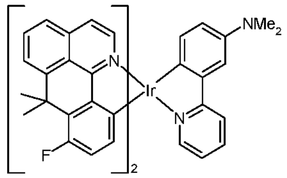
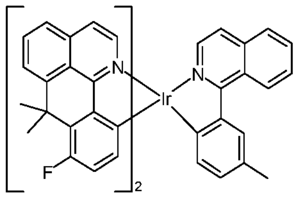

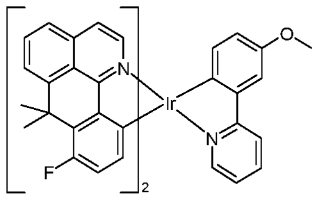
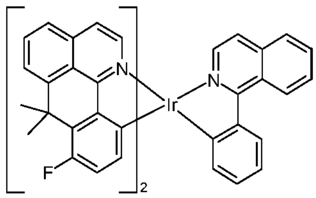
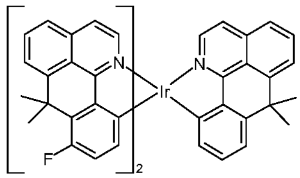

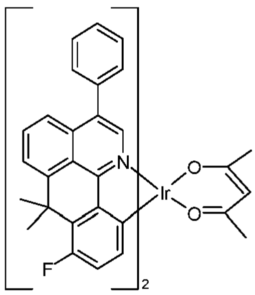
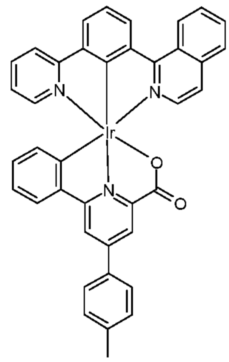
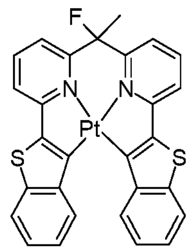

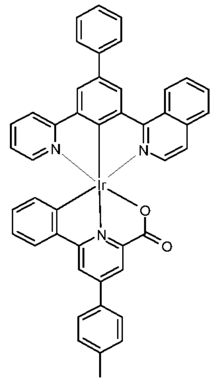
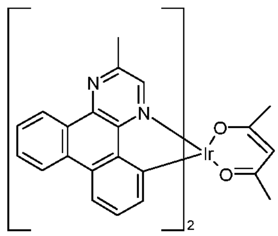
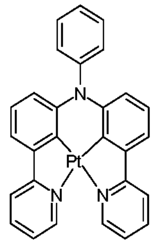

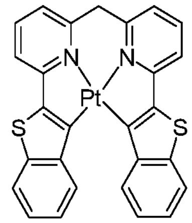
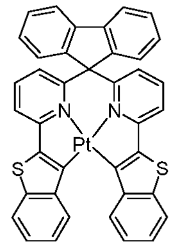
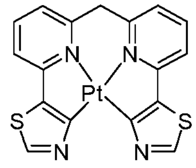

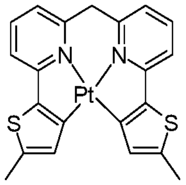
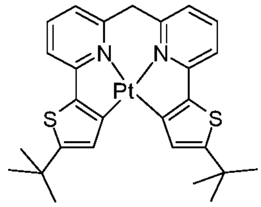
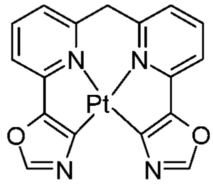

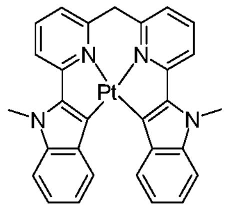
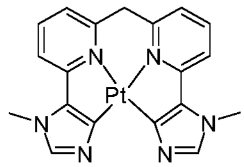
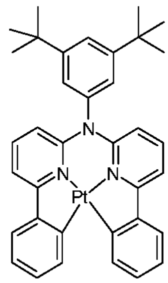

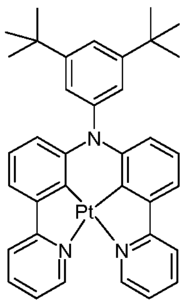
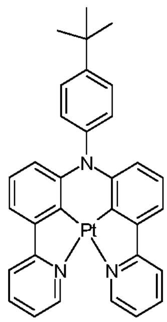
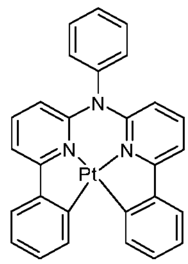

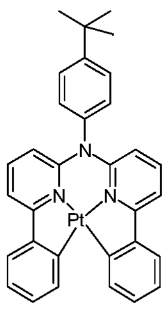
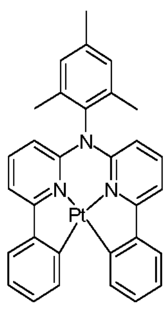
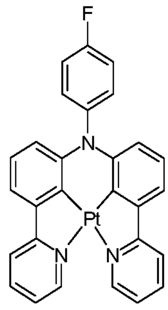

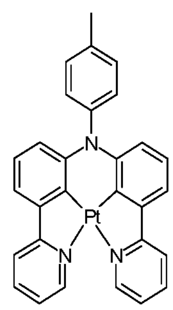
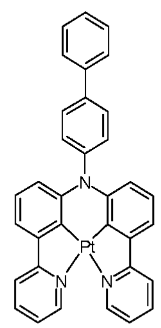
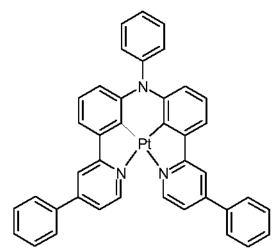

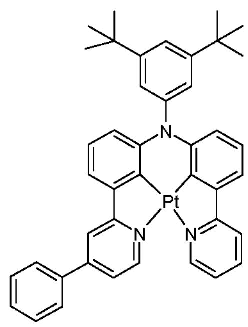
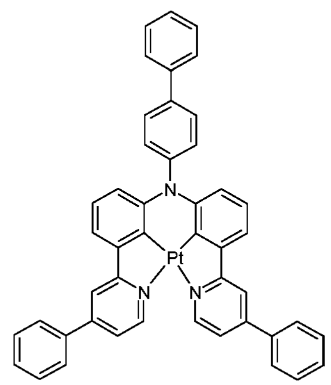
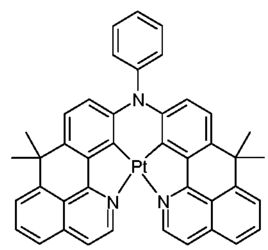

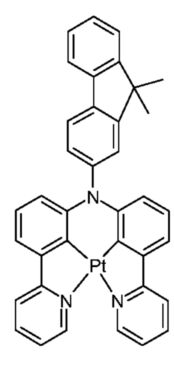
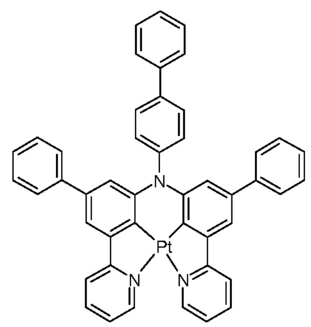
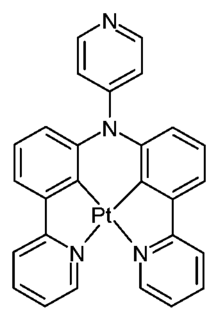

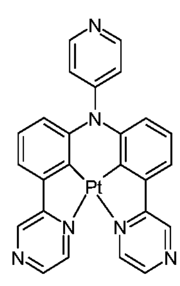
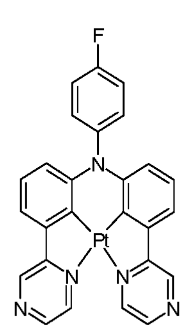
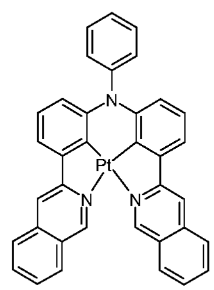

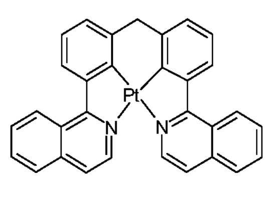
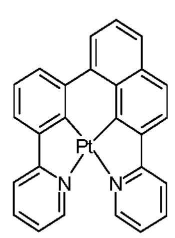
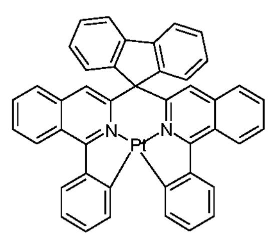

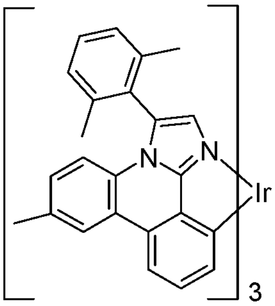
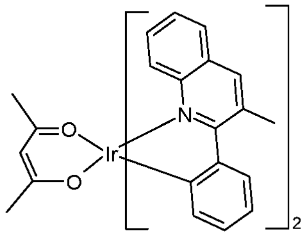
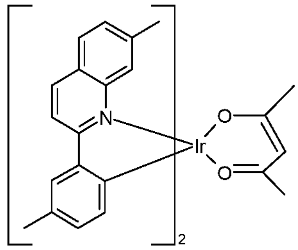

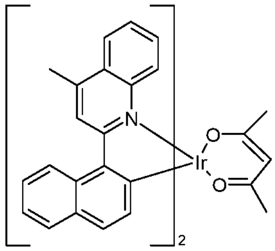
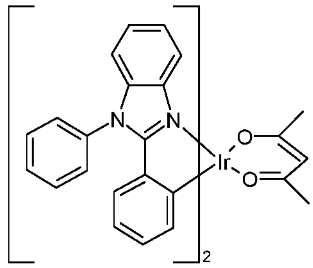
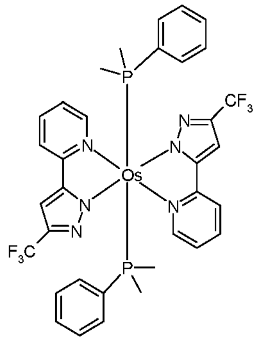

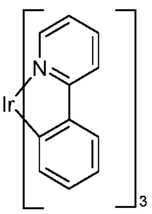
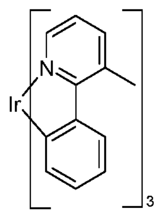
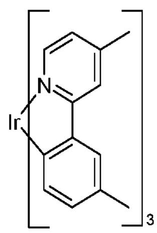

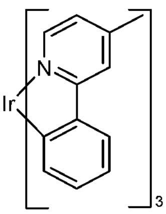
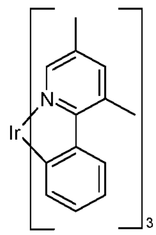
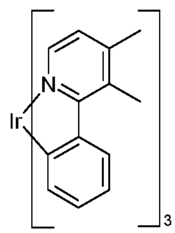

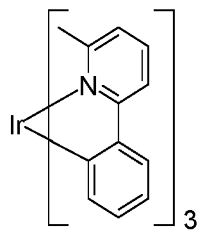
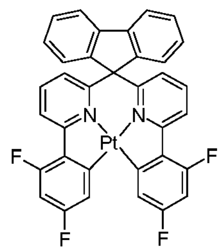
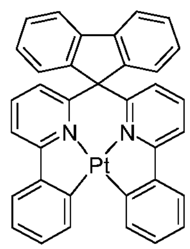

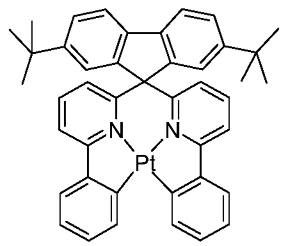
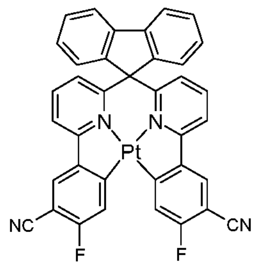
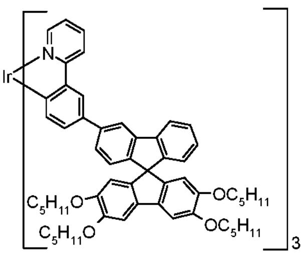

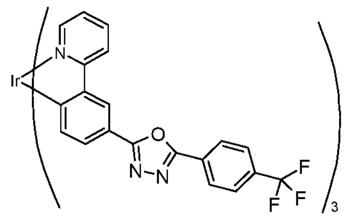
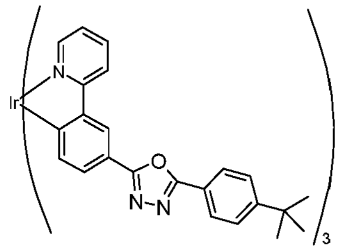
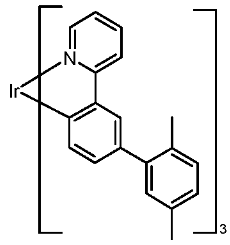

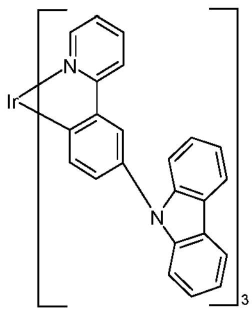
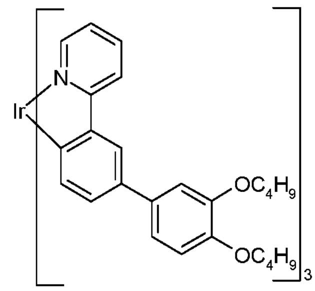
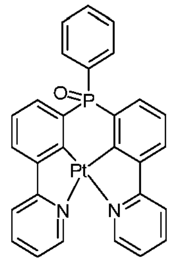

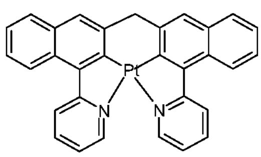
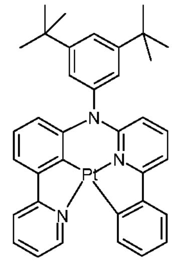
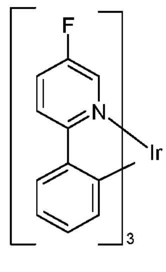

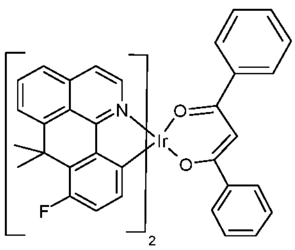
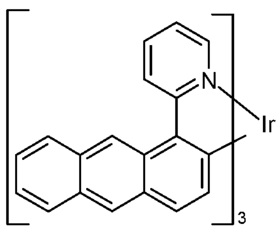
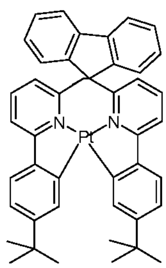

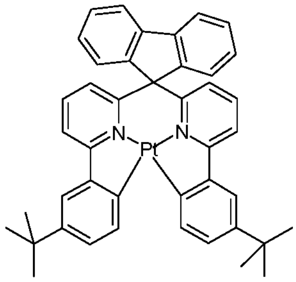
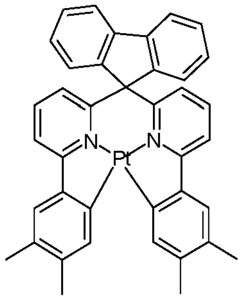
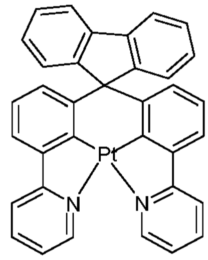

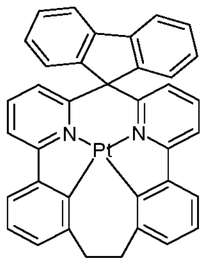
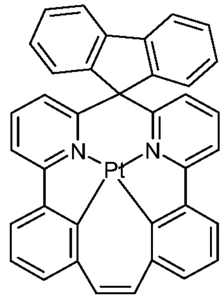
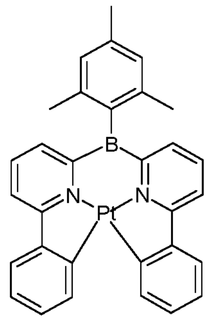

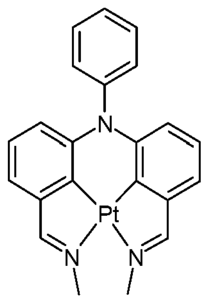
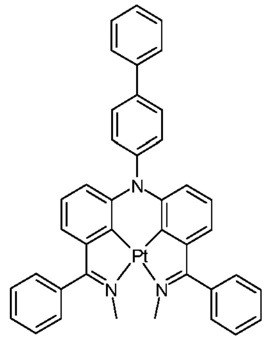
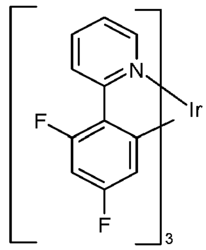

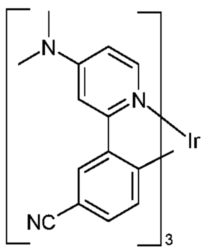
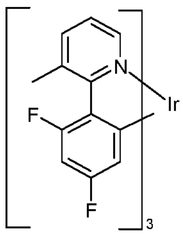
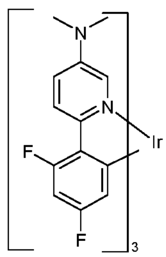

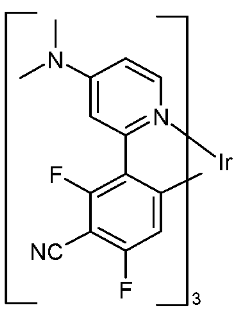
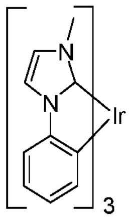
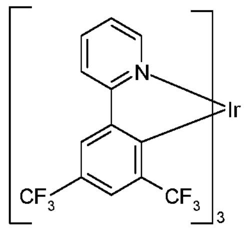

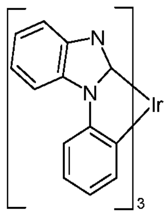
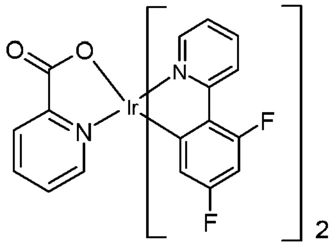
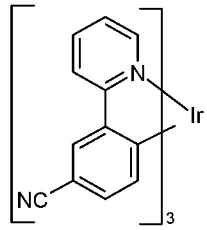

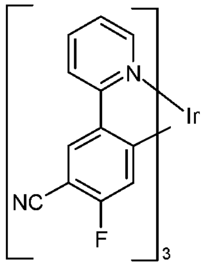
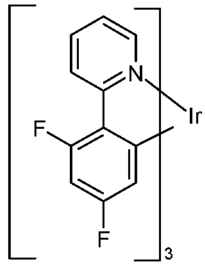
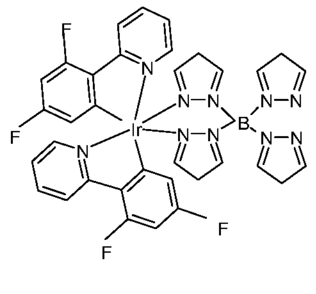

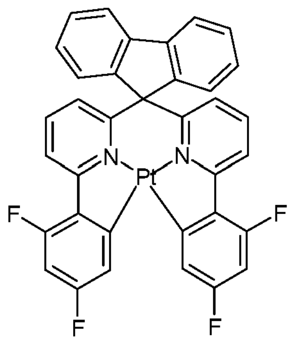
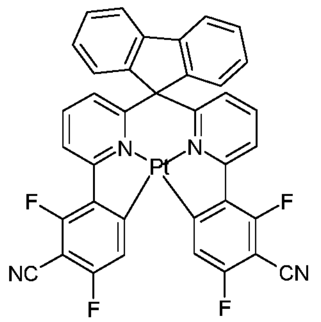
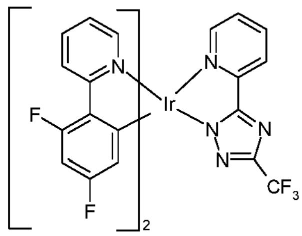

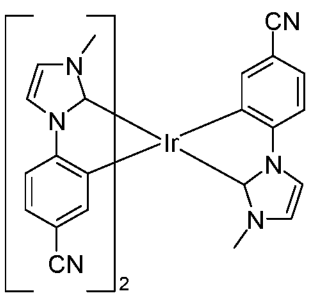
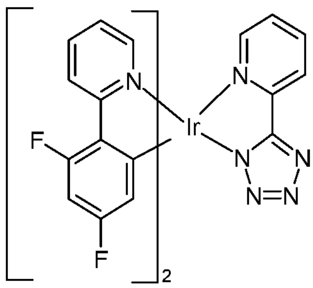
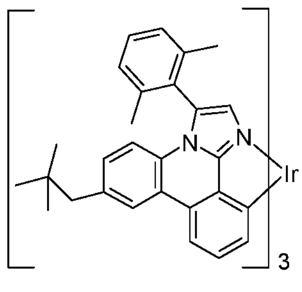

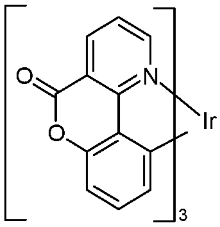
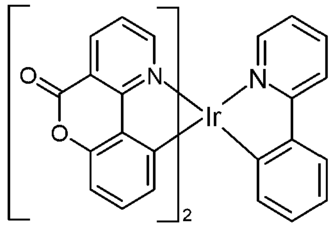
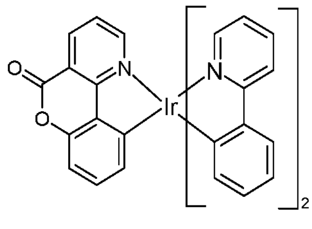

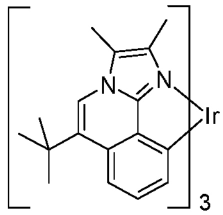
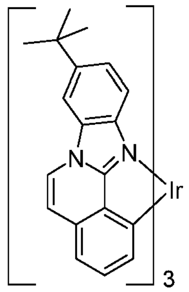
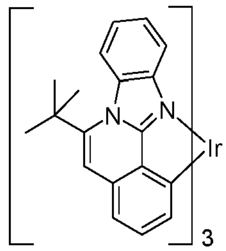

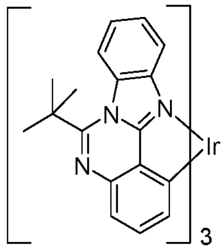
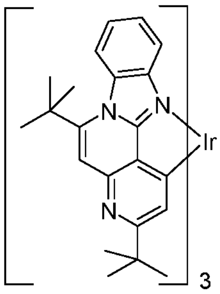
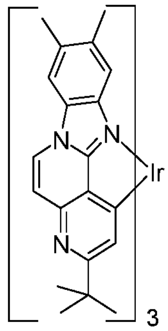

Die oben beschriebenen Verbindung, umfassend Strukturen der Formel (II), bzw. die oben aufgeführten bevorzugten Ausführungsformen können in einer elektronischen Vorrichtung bevorzugt als aktive Komponente verwendet werden. Unter einer elektronischen Vorrichtung wird eine Vorrichtung verstanden, welche Anode, Kathode und mindestens eine Schicht enthält, wobei diese Schicht mindestens eine organische bzw. metallorganische Verbindung enthält. Die erfindungsgemäße elektronische Vorrichtung enthält also Anode, Kathode und mindestens eine Schicht, welche mindestens eine Verbindung, umfassend Strukturen der Formel (II), enthält. Dabei sind bevorzugte elektronische Vorrichtungen ausgewählt aus der Gruppe bestehend aus organischen Elektrolumineszenzvorrichtungen (OLEDs, PLEDs), organischen integrierten Schaltungen (O-ICs), organischen Feld-Effekt-Transistoren (O-FETs), organischen Dünnfilmtransistoren (O-TFTs), organischen lichtemittierenden Transistoren (O-LETs), organischen Solarzellen (O-SCs), organischen optischen Detektoren, organischen Photorezeptoren, organischen Feld-Quench-Devices (O-FQDs), organischen elektrischen Sensoren, lichtemittierenden elektrochemischen Zellen (LECs) oder organischen Laserdioden (O-Laser), enthaltend in mindestens einer Schicht mindestens eine Verbindung, umfassend Strukturen der Formel (II). Besonders bevorzugt sind organische Elektrolumineszenzvorrichtungen. Aktive Komponenten sind generell die organischen oder anorganischen Materialien, welche zwischen Anode und Kathode eingebracht sind, beispielsweise Ladungsinjektions-, Ladungstransport- oder Ladungsblockiermaterialien, insbesondere aber Emissionsmaterialien und Matrixmaterialien.

Eine bevorzugte Ausführungsform der Erfindung sind organische Elektrolumineszenzvorrichtungen. Die organische Elektrolumineszenzvorrichtung enthält Kathode, Anode und mindestens eine emittierende Schicht. Außer diesen Schichten kann sie noch weitere Schichten enthalten, beispielsweise jeweils eine oder mehrere Lochinjektionsschichten, Lochtransportschichten, Lochblockierschichten, Elektronentransportschichten, Elektroneninjektionsschichten, Exzitonenblockierschichten, Elektronenblockierschichten, Ladungserzeugungsschichten und/oder organische oder anorganische p/n-Übergänge. Dabei ist es möglich, dass eine oder mehrere Lochtransportschichten p-dotiert sind, beispielsweise mit Metalloxiden, wie MoOs oder $WO_3$ oder mit (per)fluorierten elektronenarmen Aromaten, und/oder dass eine oder mehrere Elektronentransportschichten n-dotiert sind. Ebenso können zwischen zwei emittierende Schichten Interlayers eingebracht sein, welche beispielsweise eine Exzitonen-blockierende Funktion aufweisen und/oder die Ladungsbalance in der Elektrolumineszenzvorrichtung steuern. Es sei aber darauf hingewiesen, dass nicht notwendigerweise jede dieser Schichten vorhanden sein muss.

Dabei kann die organische Elektrolumineszenzvorrichtung eine emittierende Schicht enthalten, oder sie kann mehrere emittierende Schichten enthalten. Wenn mehrere Emissionsschichten vorhanden sind, weisen diese bevorzugt insgesamt mehrere Emissionsmaxima zwischen 380 nm und 750 nm auf, so dass insgesamt weiße Emission resultiert, d. h. in den emittierenden Schichten werden verschiedene emittierende Verbindungen verwendet, die fluoreszieren oder phosphoreszieren können. Insbesondere bevorzugt sind Dreischichtsysteme, wobei die drei Schichten blaue, grüne und orange oder rote Emission zeigen (für den prinzipiellen Aufbau siehe z. B. WO 2005/011013) bzw. Systeme, welche mehr als drei emittierende Schichten aufweisen. Es kann sich auch um ein Hybrid-System handeln, wobei eine oder mehrere Schichten fluoreszieren und eine oder mehrere andere Schichten phosphoreszieren.

In einer bevorzugten Ausführungsform der Erfindung enthält die organische Elektrolumineszenzvorrichtung die efindungsgemäße Verbindung umfassend Strukturen gemäß Formel (II) bzw. die oben aufgeführten bevorzugten Ausführungsformen als Matrixmaterial, vorzugsweise als elektronenleitendes Matrixmaterial in einer oder mehreren emittierenden Schichten, bevorzugt in Kombination mit einem weiteren Matrixmaterial, vorzugsweise einem lochleitenden Matrixmaterial. Eine emittierende Schicht umfasst mindestens eine emittierende Verbindung.

Als Matrixmaterial können generell alle Materialien eingesetzt werden, die gemäß dem Stand der Technik hierfür bekannt sind. Bevorzugt ist das Triplett-Niveau des Matrixmaterials höher als das Triplett-Niveau des Emitters.

Geeignete Matrixmaterialien für die erfindungsgemäßen Verbindungen sind Ketone, Phosphinoxide, Sulfoxide und Sulfone, z. B. gemäß WO 2004/013080, WO 2004/093207, WO 2006/005627 oder WO 2010/006680, Triarylamine, Carbazolderivate, z. B. CBP (N,N-Biscarbazolylbiphenyl), m-CBP oder die in WO 2005/039246, US 2005/0069729, JP 2004/288381, EP 1205527, WO 2008/086851 oder US 2009/0134784 offenbarten Carbazolderivate, Indolocarbazolderivate, z. B. gemäß WO 2007/063754 oder WO 2008/056746, Indenocarbazolderivate, z. B. gemäß WO 2010/136109 oder WO 2011/000455, Azacarbazole, z. B. gemäß EP 1617710, EP 1617711, EP 1731584, JP 2005/347160, bipolare Matrix-materialien, z. B. gemäß WO 2007/137725, Silane, z. B. gemäß WO 2005/111172, Azaborole oder Boronester, z. B. gemäß WO 2006/117052, Diazasilolderivate, z. B. gemäß WO 2010/054729, Diazaphospholderivate, z. B. gemäß WO 2010/054730, Triazinderivate, z. B. gemäß WO 2010/015306, WO 2007/063754 oder WO 2008/056746, Zinkkomplexe, z. B. gemäß EP 652273 oder WO 2009/062578, Dibenzofuranderivate, z. B. gemäß WO 2009/148015, oder verbrückte Carbazolderivate, z. B. gemäß US 2009/0136779, WO 2010/050778, WO 2011/042107 oder WO 2011/088877.

Es kann auch bevorzugt sein, mehrere verschiedene Matrixmaterialien als Mischung einzusetzen, insbesondere mindestens ein elektronenleitendes Matrixmaterial und mindestens ein lochleitendes Matrixmaterial. Ebenso bevorzugt ist die Verwendung einer Mischung aus einem ladungstransportierenden Matrixmaterial und einem elektrisch inerten Matrixmaterial, welches nicht bzw. nicht in wesentlichem Maße am Ladungstransport beteiligt ist, wie z. B. in WO 2010/108579 beschrieben.

Weiterhin bevorzugt ist es, eine Mischung aus zwei oder mehr Triplett-Emittern zusammen mit einer Matrix einzusetzen. Dabei dient der Triplett-Emitter mit dem kürzerwelligen Emissionsspektrum als Co-Matrix für den Triplett-Emitter mit dem längerwelligen Emissionsspektrum.

Besonders bevorzugt kann eine erfindungsgemäße Verbindung umfassend Strukuren gemäß Formel (II) in einer bevorzugten Ausführungsform als Matrixmaterial in einer Emissionsschicht einer organischen elektronischen Vorrichtung, insbesondere in einer organischen elektrolumineszierenden Vorrichtung, beispielsweise in einer OLED oder OLEC, eingesetzt werden. Dabei ist das Matrixmaterial enthaltend Verbindung umfassend Strukuren gemäß Formel (II) bzw. die zuvor ausgeführten bevor¬zugten Ausführungsformen in der elektronischen Vorrichtung in Kombination mit einem oder mehreren Dotanden, vorzugsweise phosphoreszierenden Dotanden, vorhanden.

Der Anteil des Matrixmaterials in der emittierenden Schicht beträgt in diesem Fall zwischen 50.0 und 99.9 Vol.-%, bevorzugt zwischen 80.0 und 99.5 Vol.-% und besonders bevorzugt für fluoreszierende emittierende Schichten zwischen 92.0 und 99.5 Vol.-% sowie für phosphoreszierende emittierende Schichten zwischen 85.0 und 97.0 Vol.-%.

Entsprechend beträgt der Anteil des Dotanden zwischen 0.1 und 50.0 Vol.-%, bevorzugt zwischen 0.5 und 20.0 Vol.-% und besonders bevorzugt für fluoreszierende emittierende Schichten zwischen 0.5 und 8.0 Vol.-% sowie für phosphoreszierende emittierende Schichten zwischen 3.0 und 15.0 Vol.-%.

Eine emittierende Schicht einer organischen Elektrolumineszenzvorrichtung kann auch Systeme umfassend mehrere Matrixmaterialien (Mixed-Matrix-Systeme) und/oder mehrere Dotanden enthalten. Auch in diesem Fall sind die Dotanden im Allgemeinen diejenigen Materialien, deren Anteil im System der kleinere ist und die Matrixmaterialien sind diejenigen Materialien, deren Anteil im System der größere ist. In Einzelfällen kann jedoch der Anteil eines einzelnen Matrixmaterials im System kleiner sein als der Anteil eines einzelnen Dotanden.

In einer weiteren bevorzugten Ausführungsform der Erfindung werden die Verbindungen umfassend Strukuren gemäß Formel (II) bzw. die zuvorausgeführten bevor¬zugten Ausführungsformen als eine Komponente von Mixed-Matrix-Systemen verwendet. Die Mixed-Matrix-Systeme umfassen bevorzugt zwei oder drei verschiedene Matrixmaterialien, besonders bevorzugt zwei verschiedene Matrixmaterialien. Bevorzugt stellt dabei eines der beiden Materialien ein Material mit lochtransportierenden Eigenschaften und das andere Material ein Material mit elektronentransportierenden Eigenschaften dar. Die gewünschten elektronentransportierenden und lochtransportierenden Eigenschaften der Mixed-Matrix-Komponenten können jedoch auch hauptsächlich oder vollständig in einer einzigen Mixed-Matrix-Komponente vereinigt sein, wobei die weitere bzw. die weiteren Mixed-Matrix-Komponenten andere Funktionen erfüllen. Die beiden unterschiedlichen Matrixmaterialien können dabei in einem Verhältnis von 1:50 bis 1:1, bevorzugt 1:20 bis 1:1, besonders bevorzugt 1:10 bis 1:1 und ganz besonders bevorzugt 1:4 bis 1:1 vorliegen. Bevorzugt werden Mixed-Matrix-Systeme in phosphoreszierenden organischen Elektrolumineszenzvorrichtungen eingesetzt. Genauere Angaben zu Mixed-Matrix-Systemen sind unter anderem in der Anmeldung WO 2010/108579 enthalten.

Ferner ist eine elektronische Vorrichtung, vorzugsweise eine organische Elektrolumineszenzvorrichtung Gegenstand der vorliegenden Erfindung, die eine oder mehrere erfindungsgemäße Verbindungen und/oder mindestens

ein erfindungsgemäßes Oligomer, Polymer oder Dendrimer in einer oder mehreren elektronenleitenden Schichten umfasst, als elektronenleitende Verbindung.

Als Kathode sind Metalle mit geringer Austrittsarbeit, Metalllegierungen oder mehrlagige Strukturen aus verschiedenen Metallen bevorzugt, wie beispielsweise Erdalkalimetalle, Alkalimetalle, Hauptgruppenmetalle oder Lanthanoide (z. B. Ca, Ba, Mg, Al, In, Mg, Yb, Sm, etc.). Weiterhin eignen sich Legierungen aus einem Alkali- oder Erdalkalimetall und Silber, beispielsweise eine Legierung aus Magnesium und Silber. Bei mehrlagigen Strukturen können auch zusätzlich zu den genannten Metallen weitere Metalle verwendet werden, die eine relativ hohe Austrittsarbeit aufweisen, wie z. B. Ag, wobei dann in der Regel Kombinationen der Metalle, wie beispielsweise Mg/Ag, Ca/Ag oder Ba/Ag verwendet werden. Es kann auch bevorzugt sein, zwischen einer metallischen Kathode und dem organischen Halbleiter eine dünne Zwischenschicht eines Materials mit einer hohen Dielektrizitätskonstante einzubringen. Hierfür kommen beispielsweise Alkalimetall- oder Erdalkalimetallfluoride, aber auch die entsprechenden Oxide oder Carbonate in Frage (z. B. LiF, $Li_2O$, $BaF_2$, MgO, NaF, CsF, $Cs_2CO_3$, etc.). Ebenso kommen hierfür organische Alkalimetallkomplexe in Frage, z. B. Liq (Lithiumchinolinat). Die Schichtdicke dieser Schicht beträgt bevorzugt zwischen 0.5 und 5 nm.

Als Anode sind Materialien mit hoher Austrittsarbeit bevorzugt. Bevorzugt weist die Anode eine Austrittsarbeit größer 4.5 eV vs. Vakuum auf. Hierfür sind einerseits Metalle mit hohem Redoxpotential geeignet, wie beispielsweise Ag, Pt oder Au. Es können andererseits auch Metall/Metalloxid-Elektroden (z. B. $Al/Ni/NiO_x$, $Al/PtO_x$) bevorzugt sein. Für einige Anwendungen muss mindestens eine der Elektroden transparent oder teiltransparent sein, um entweder die Bestrahlung des organischen Materials (O-SC) oder die Auskopplung von Licht (OLED/PLED, O-LASER) zu ermöglichen. Bevorzugte Anodenmaterialien sind hier leitfähige gemischte Metalloxide. Besonders bevorzugt sind Indium-Zinn-Oxid (ITO) oder Indium-Zink-Oxid (IZO). Bevorzugt sind weiterhin leitfähige, dotierte organische Materialien, insbesondere leitfähige dotierte Polymere, z. B. PEDOT, PANI oder Derivate dieser Polymere. Bevorzugt ist weiterhin, wenn auf die Anode ein p-dotiertes Lochtransportmaterial als Lochinjektionsschicht aufgebracht wird, wobei sich als p-Dotanden Metalloxide, beispielsweise $MoO_3$ oder $WO_3$, oder (per)fluorierte elektronenarme Aromaten eignen. Weitere geeignete p-Dotanden sind HAT-CN (Hexacyano-hexaazatriphenylen) oder die Verbindung NPD9 von Novaled. Eine solche Schicht vereinfacht die Lochinjektion in Materialien mit einem tiefen HOMO, also einem betragsmäßig großen HOMO.

In den weiteren Schichten können generell alle Materialien verwendet werden, wie sie gemäß dem Stand der Technik für die Schichten verwendet werden, und der Fachmann kann ohne erfinderisches Zutun jedes dieser Materialien in einer elektronischen Vorrichtung mit den erfindungsgemäßen Materialien kombinieren.

Die Vorrichtung wird entsprechend (je nach Anwendung) strukturiert, kontaktiert und schließlich hermetisch versiegelt, da sich die Lebensdauer derartiger Vorrichtungen bei Anwesenheit von Wasser und/oder Luft drastisch verkürzt.

Weiterhin bevorzugt ist eine elektronischen Vorrichtung, insbesondere eine organische Elektrolumineszenzvorrichtung, welche dadurch gekennzeichnet ist, dass eine oder mehrere Schichten mit einem Sublimationsverfahren beschichtet werden. Dabei werden die Materialien in Vakuum-Sublimationsanlagen bei einem Anfangsdruck von üblicherweise kleiner $10^{-5}$ mbar, bevorzugt kleiner $10^{-6}$ mbar aufgedampft. Es ist auch möglich, dass der Anfangsdruck noch geringer oder noch höher ist, beispielsweise kleiner $10^{-7}$ mbar.

Bevorzugt ist ebenfalls eine elektronischen Vorrichtung, insbesondere eine organische Elektrolumineszenzvorrichtung, welche dadurch gekennzeichnet ist, dass eine oder mehrere Schichten mit dem OVPD (Organic Vapour Phase Deposition) Verfahren oder mit Hilfe einer Trägergassublimation beschichtet werden. Dabei werden die Materialien bei einem Druck zwischen $10^{-5}$ mbar und 1 bar aufgebracht. Ein Spezialfall dieses Verfahrens ist das OVJP (Organic Vapour Jet Printing) Verfahren, bei dem die Materialien direkt durch eine Düse aufgebracht und so strukturiert werden (z. B. M. S. Arnold et al., Appl. Phys. Lett. 2008, 92, 053301).

Weiterhin bevorzugt ist eine elektronischen Vorrichtung, insbesondere eine organische Elektrolumineszenzvorrichtung, welche dadurch gekennzeichnet ist, dass eine oder mehrere Schichten aus Lösung, wie z. B. durch Spincoating, oder mit einem beliebigen Druckverfahren, wie z. B. Siebdruck, Flexodruck, Offsetdruck oder Nozzle-Printing, besonders bevorzugt aber LITI (Light Induced Thermal Imaging, Thermotransferdruck) oder Ink-Jet Druck (Tintenstrahldruck), hergestellt werden. Hierfür sind lösliche Verbindungen nötig, welche beispielsweise durch geeignete Substitution erhalten werden.

Die elektronischen Vorrichtung, insbesondere die organische Elektrolumineszenzvorrichtung kann auch als Hybridsystem hergestellt werden, indem eine oder mehrere Schichten aus Lösung aufgebracht werden und eine oder mehrere andere Schichten aufgedampft werden. So ist es beispielsweise möglich, eine emittierende Schicht enthaltend eine efindungsgemäße Verbindung umfassend Strukturen gemäß Formel (II) und ein Matrixmaterial aus Lösung aufzubringen und darauf eine Lochblockierschicht und/oder eine Elektronentransportschicht im Vakuum aufzudampfen.

Diese Verfahren sind dem Fachmann generell bekannt und können von ihm ohne Probleme auf elektronischen Vorrichtungen, insbesondere organische Elektrolumineszenzvorrichtungen enthaltend efindungsgemäße Verbindungen umfassend Strukturen gemäß Formel (II) bzw. die oben aufgeführten bevorzugten Ausführungsformen angewandt werden.

Die erfindungsgemäßen elektronischen Vorrichtungen, insbesondere organische Elektrolumineszenzvorrichtungen, zeichnen sich durch einen oder mehrere der folgenden überraschenden Vorteile gegenüber dem Stand der Technik aus:

1. Elektronischen Vorrichtungen, insbesondere organische Elektrolumineszenzvorrichtungen enthaltend Verbindungen, Oligomere, Polymere oder Dendrimere mit Strukturen gemäß Formel (II) bzw. die zuvor ausgeführten bevor¬zugten Ausführungsformen, insbesondere als elektronenleitende und/oder als lochleitende Materialien, weisen eine sehr gute Lebensdauer auf.

2. Elektronischen Vorrichtungen, insbesondere organische Elektrolumineszenzvorrichtungen enthaltend Verbindungen, Oligomere, Polymere oder Dendrimere mit Strukturen gemäß Formel (II) bzw. die zuvor ausgeführten bevor¬zugten Ausführungsformen als elektronenleitende und/oder als lochleitende Materialien weisen eine hervorragende Effizienz auf. Insbesondere ist die Effizienz deutlich höher gegenüber analogen Verbindungen, die keine Struktureinheit gemäß Formel (II) enthalten.

3. Die erfindungsgemäßen Verbindungen, Oligomere, Polymere oder Dendrimere mit Strukturen gemäß Formel (II) bzw. die zuvor ausgeführten bevor¬zugten Ausführungsformen zeigen eine sehr hohe Stabilität und führen zu Verbindungen mit einer sehr hohen Lebensdauer.

4. Mit Verbindungen, Oligomeren, Polymeren oder Dendrimeren mit Strukturen gemäß Formel (II) bzw. die zuvor ausgeführten bevor¬zugten Ausführungsformen kann in elektronischen Vorrichtungen, insbesondere organische Elektrolumineszenzvorrichtungen die Bildung von optischen Verlustkanäle vermieden werden. Hierdurch zeichnen sich diese Vorrichtungen durch eine hohe PL- und damit hohe EL-Effizienz von Emittern bzw. eine ausgezeichnete Energieübertragung der Matrices auf Dotanden aus.

5. Die Verwendung von Verbindungen, Oligomeren, Polymeren oder Dendrimeren mit Strukturen gemäß Formel (II) bzw. die zuvor ausgeführten bevor¬zugten Ausführungsformen in Schichten elektronischer Vorrichtungen, insbesondere organischer Elektrolumineszenzvorrichtungen, führt zu einer hohen Mobilität der Elektron-Leiterstrukturen und/oder der Loch-Leiterstrukturen.

6. Verbindungen, Oligomere, Polymere oder Dendrimere mit Strukturen gemäß Formel (II) bzw. die zuvor ausgeführten bevor¬zugten Ausführungsformen zeichnen sich durch eine ausgezeichnete thermische Stabilität aus, wobei Verbindungen mit einer Molmasse von weniger als ca. 1200 g/mol gut sublimierbar sind

7. Verbindungen, Oligomere, Polymere oder Dendrimere mit Strukturen gemäß Formel (II) bzw. die zuvor ausgeführten bevor¬zugten Ausführungsformen weisen eine ausgezeichnete Glasfilmbildung auf.

8. Verbindungen, Oligomere, Polymere oder Dendrimere mit Strukturen gemäß Formel (II) bzw. die zuvor ausgeführten bevor¬zugten Ausführungsformen bilden aus Lösungen sehr gute Filme.

9. Die Verbindungen, Oligomere, Polymere oder Dendrimere umfassend Strukturen gemäß Formel (II) bzw. die zuvor ausgeführten bevor¬zugten Ausführungsformen weisen ein überraschend hohes Triplett-Niveau $T_1$ auf, wobei dies insbesondere für Verbindungen gilt, die als elektronenleitende Materialien eingesetzt werden.

Diese oben genannten Vorteile gehen nicht mit einer Verschlechterung der weiteren elektronischen Eigenschaften einher.

Ein weiterer Gegenstand der vorliegenden Erfindung ist die Verwendung einer erfindungsgemäßen Verbindung und/oder eines erfindungsgemäßen Oligomers, Polymers oder Dendrimers in einer elektronischen Vorrichtung als als Lochtransportmaterial, Lochinjektionsmaterial, Lochblockiermaterial, Elektroneninjektionsmaterial, Elektronenblockiermaterial und/oder Elektronentransportmaterial.

Es sei darauf hingewiesen, dass Variationen der in der vorliegenden Erfindung beschriebenen Ausführungsformen unter den Umfang dieser Erfindung fallen. Jedes in der vorliegenden Erfindung offenbarte Merkmal kann, sofern dies nicht explizit ausgeschlossen wird, durch alternative Merkmale, die demselben, einem äquivalenten oder einem ähnlichen Zweck dienen, ausgetauscht werden. Somit ist jedes in der vorliegenden Erfindung offenbartes Merkmal, sofern nichts anderes gesagt wurde, als Beispiel einer generischen Reihe oder als äquivalentes oder ähnliches Merkmal zu betrachten.

Alle Merkmale der vorliegenden Erfindung können in jeder Art miteinander kombiniert werden, es sei denn dass sich bestimmte Merkmale und/oder Schritte sich gegenseitig ausschließen. Dies gilt insbesondere für bevorzugte Merkmale der vorliegenden Erfindung. Gleichermaßen können Merkmale nicht wesentlicher Kombinationen separat

verwendet werden (und nicht in Kombination).

Die mit der vorliegenden Erfindung offengelegte Lehre zum technischen Handeln kann abstrahiert und mit anderen Beispielen kombiniert werden.

Die Erfindung wird durch die nachfolgenden Beispiele näher erläutert, ohne sie dadurch einschränken zu wollen.

Der Fachmann kann aus den Schilderungen ohne erfinderisches Zutun weitere erfindungsgemäße elektronische Vorrichtungen herstellen und somit die Erfindung im gesamten beanspruchten Bereich ausführen.

## Beispiele

Die nachfolgenden Synthesen werden, sofern nicht anders angegeben, unter einer Schutzgasatmosphäre in getrockneten Lösungsmitteln durchgeführt. Die Metallkomplexe werden zusätzlich unter Ausschluss von Licht bzw. unter Gelblicht gehandhabt. Die Lösungsmittel und Reagenzien können z. B. von Sigma-ALDRICH bzw. ABCR bezogen werden. Die jeweiligen Angaben in eckigen Klammern bzw. die zu einzelnen Verbindungen angegebenen Nummern beziehen sich auf die CAS-Nummern der literaturbekannten Verbindungen.

## Hestellungsbeispiele

### a) 6,12-Dibromo-9H-9-aza-tribenz [b,d,f]azepin

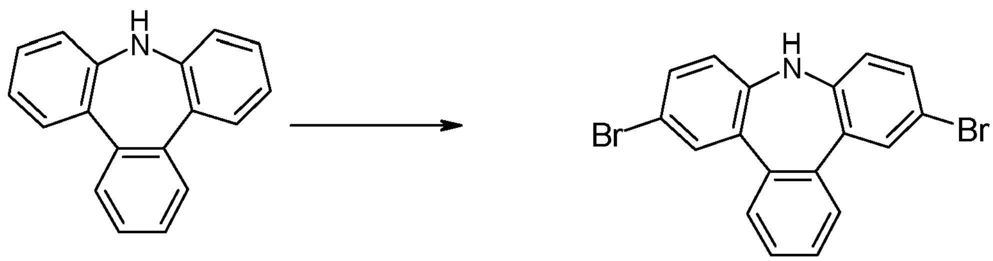

100 g (373,2 mmol) 9H-Tribenz[b,d,f]azepin werden in 800 mL DMF vorgelegt. Anschließend werden 132,8 g (746,4 mmol) NBS in portionsweise zugegeben und rührt 4 h weiter bei dieser Temperatur. Anschließend wird die Mischung mit 150 mL Wasser versetzt und mit $CH_2Cl_2$ extrahiert. Die organische Phase wird über $MgSO_4$ getrocknet und die Lösungsmittel im Vakuum entfernt. Das Produkt wird mit Hexan heiß ausgerührt und abgesaugt. Ausbeute: 122 g (295 mmol), 79 % der Theorie, Reinheit nach $^1$H-NMR ca. 97 %.

Analog dazu werden die folgenden Verbindungen hergestellt:

| | Edukt 1 | Produkt | Ausbeute |
|---|---|---|---|
| a1 | 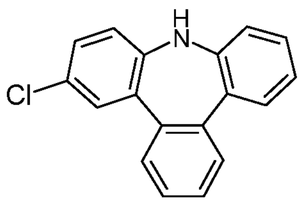 [163811-01-6] | 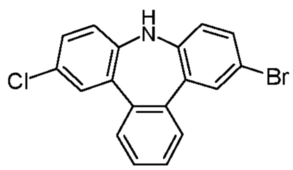 | 78% |
| a2 | 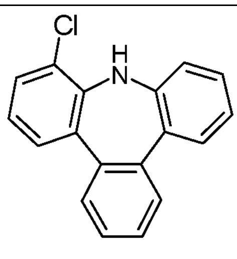 | 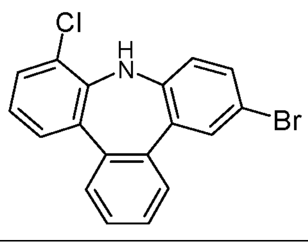 | 77% |

(fortgesetzt)

| | Edukt 1 | Produkt | Ausbeute |
|---|---|---|---|
| | [163811-00-5] | | |
| a3 | 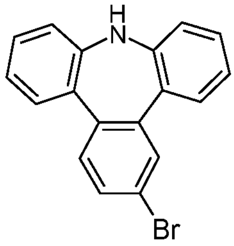 [1262523-61-4 ] | 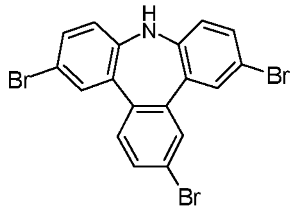 | 84% |
| a4 | 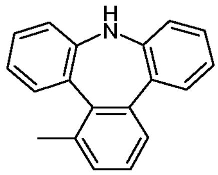 [332154-32-2 ] | 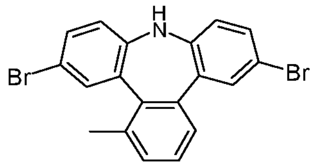 | 83% |
| a5 | 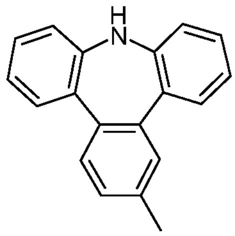 [204200-14-6 ] | 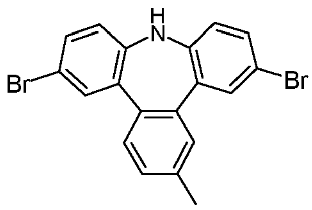 | 80% |

**b) 6,12-Bis-(9,9-dimethyl-9H-fluoren-4-yl)-9H-9-azatribenzo[a,c,e]cyclohepten**

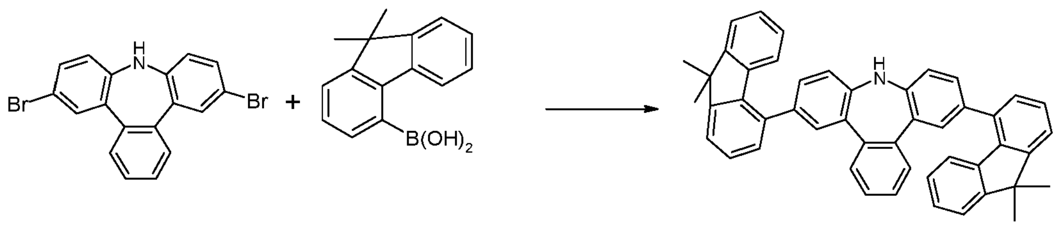

70,0 g (168 mmol) 6,12-Dibromo-9H-9-aza-tribenz [b,d,f]azepin, 71 g (343 mmol) 9,9'-Dimethylfluoren-4-boronsäure und 6,8 g (71 mmol) $K_2CO_3$ werden in 200 mL Toluol, 250 mL 1,4-Dioxam und 150 mL Wasser suspendiert. Zu dieser Suspension werden 9,6 g (8,3 mmol) Tetrakis(triphenylphosphin)palladium(0) zugegeben. Die Reaktionsmischung wird 16 h unter Rückfluss erhitzt. Nach Erkalten wird die organische Phase abgetrennt, dreimal mit 100 mL Wasser gewaschen und anschließend zur Trockene eingeengt. Der Rückstand wird mit Toluol heiß extrahiert, aus Toluol umkristallisiert und abschließend im Hochvakuum sublimiert. Die Ausbeute beträgt 61 g (98 mmol), 57 % der Theorie, Reinheit nach $^1$H-NMR ca. 98 %.

Analog dazu werden die folgenden Verbindungen hergestellt:

| | Edukt 1 | Edukt 2 | Produkt | Ausbeute |
|---|---|---|---|---|
| b1 | 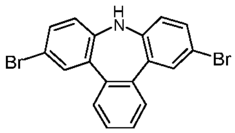 | 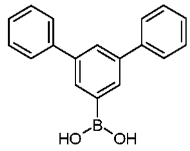 [1449431-83-7 ] | 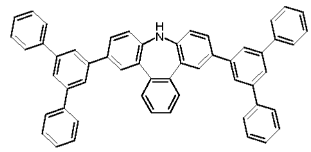 | 64% |
| b2 | 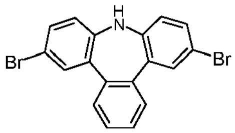 | 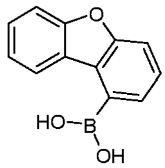 [162607-19-4] | 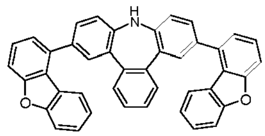 | 78% |
| b3 | 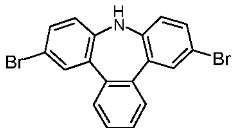 | 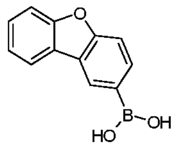 [402936-15-6 ] | 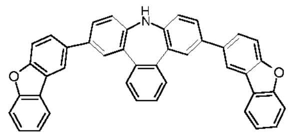 | 63% |
| b4 | 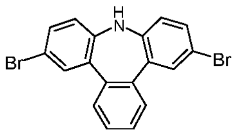 | 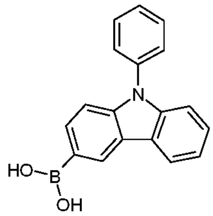 [854952-58-2] | 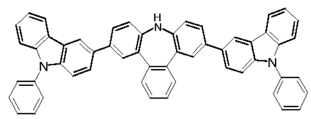 | 78% |
| b5 | 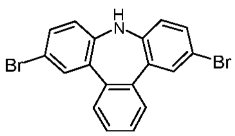 | 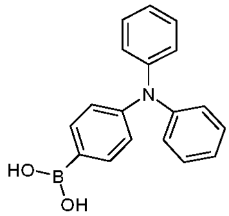 [201802-67-7] | 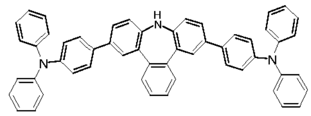 | 69% |
| b6 | 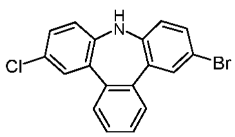 | 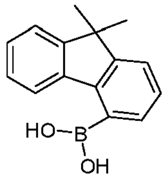 [1246022-50-3] | 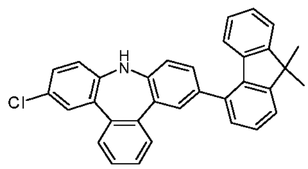 | 61% |
| b7 | 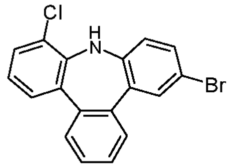 | 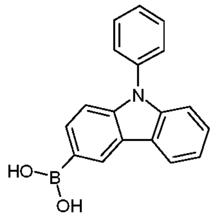 [854952-58-2] | 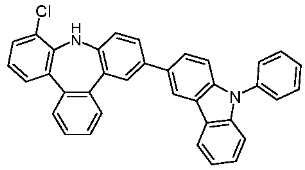 | 62% |

(fortgesetzt)

| | Edukt 1 | Edukt 2 | Produkt | Ausbeute |
|---|---|---|---|---|
| b8 | 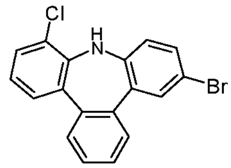 | 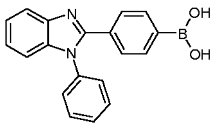 [952514-79-3] | 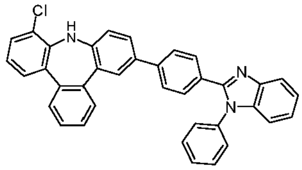 | 58% |
| b9 | 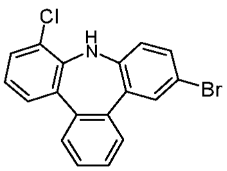 | 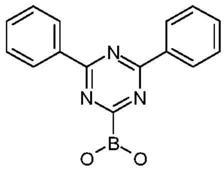 [1251825-65-6] | 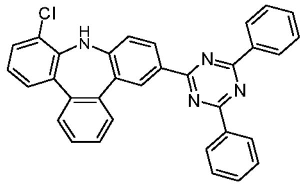 | 54% |
| b10 | 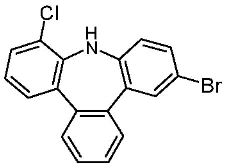 | 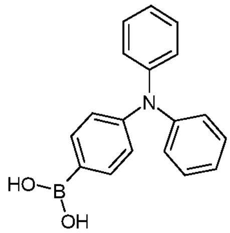 [201802-67-7] | 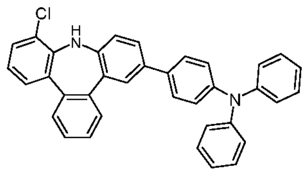 | 59% |
| b11 | 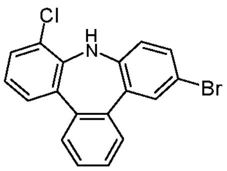 | 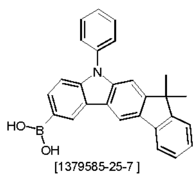 [1379585-25-7 ] | 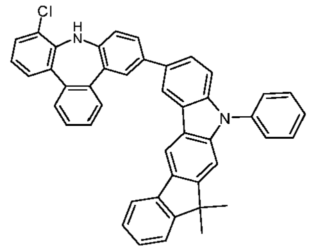 | 62% |
| b12 | 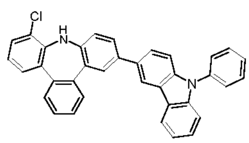 | 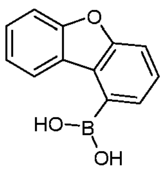 [162607-19-4] | 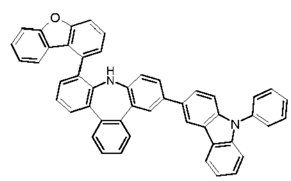 | 60% |
| b13 | 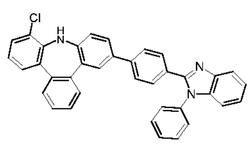 | 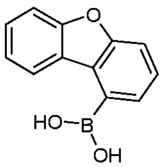 [162607-19-4] | 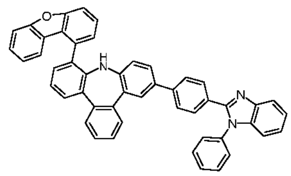 | 81% |
| b14 | 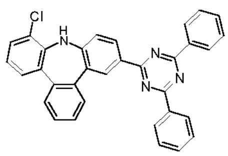 | 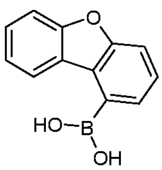 [162607-19-4] | 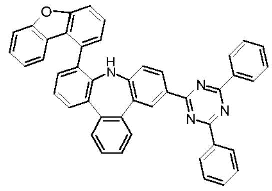 | 89% |

(fortgesetzt)

| | Edukt 1 | Edukt 2 | Produkt | Ausbeute |
|---|---|---|---|---|
| b15 | 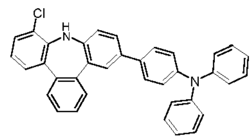 | 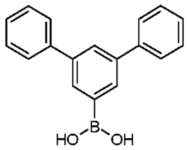 [1449431-83-7 ] | 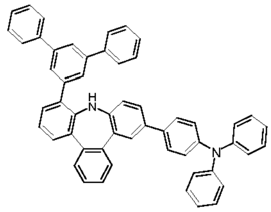 | 87% |
| b16 | 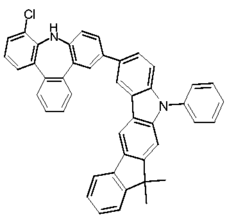 | 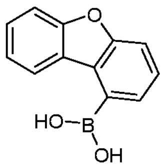 [162607-19-4] | 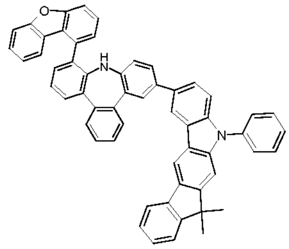 | 88% |
| b18 | 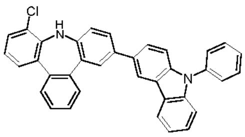 | 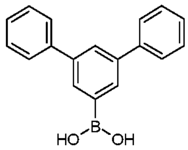 [1449431-83-7 ] | 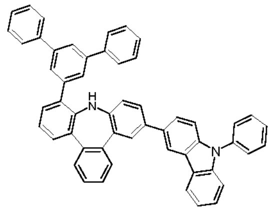 | 86% |
| b19 | 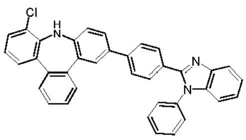 | 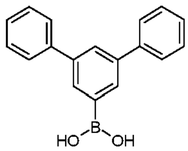 [1449431-83-7 ] | 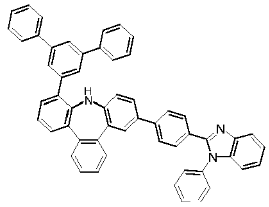 | 78% |
| b20 | 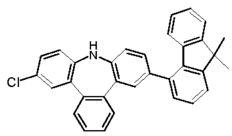 | 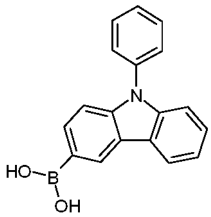 [854952-58-2] | 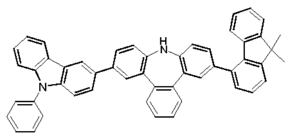 | 89% |
| b21 | 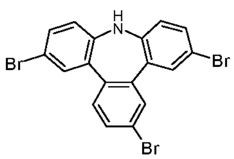 | 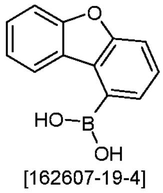 [162607-19-4] | 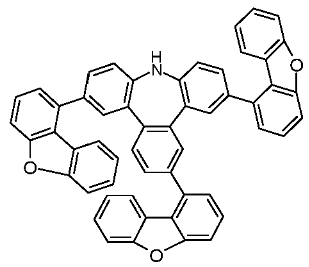 | 78% |

(fortgesetzt)

| | Edukt 1 | Edukt 2 | Produkt | Ausbeute |
|---|---|---|---|---|
| b22 | 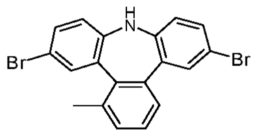 | 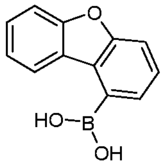 [162607-19-4] | 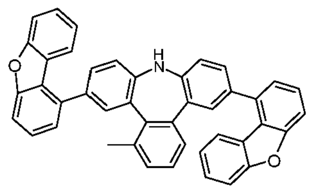 | 74% |
| b23 | 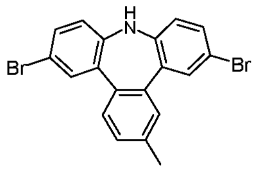 | 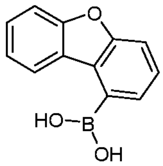 [162607-19-4] | 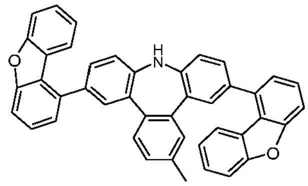 | 73% |
| b24 | 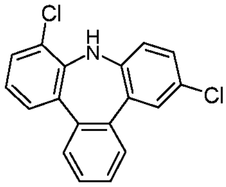 [163811-03-8] | 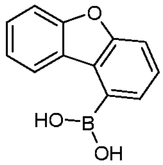 [162607-19-4] | 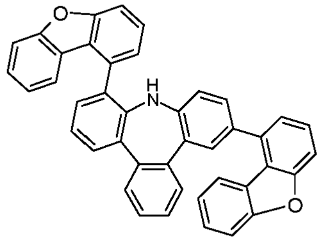 | 78% |
| b25 | 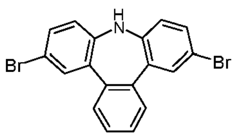 | 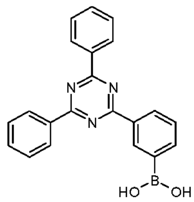 [1269508-31-7 ] | 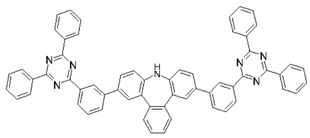 | 67% |
| b26 | 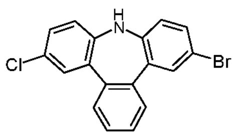 | 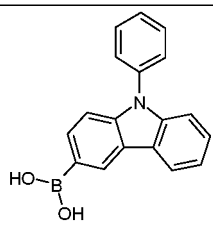 [854952-58-2] | 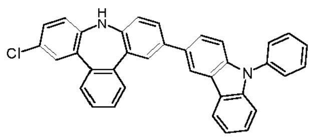 | 60% |
| b27 | 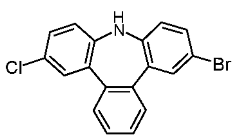 | 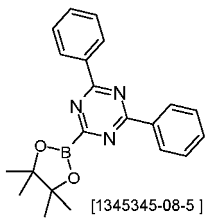 [1345345-08-5 ] | 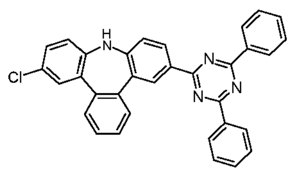 | 58% |

(fortgesetzt)

| | Edukt 1 | Edukt 2 | Produkt | Ausbeute |
|---|---|---|---|---|
| b28 | 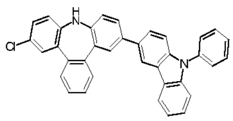 | 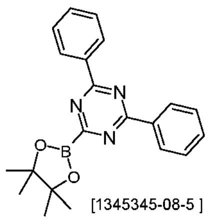 [1345345-08-5 ] | 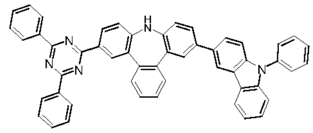 | 69% |

**Synthese von 6-Carbazol-9-yl-12-(4,6-diphenyl-[1,3,5]triazin-2-yl)-9H-9-aza-tribenzo[a,c,e]cyclohepten**

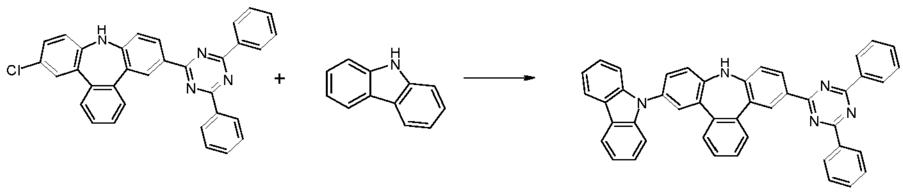

80 g (159,5 mmol) 6-Chloro-12-(4,6-diphenyl-[1,3,5]triazin-2-yl)-9H-9-aza-tribenzo[a,c,e]cycloheptenewerden in 150 mL Di-n-Butylether mit 67,7 g (145 mmol) Carbazol versetzt und die Lösung entgast. Anschließend wird die Mischung mit 10 g (0,158 mmol) Kupferpulver 1,38 g (0,007 mmol) Kupferiodid und 22 g (159,6 mmol) $K_2CO_3$ versetzt und 4 Tage unter Schutzgas bei 144°C gerührt. Die organische Phase wird über $MgSO_4$ getrocknet und das Lösungsmittel im Vakuum entfernt. Ausbeute: 32 g (50 mmol), 40% d. Th..

**c) 9-(2-Chloro-phenyl)-6,12-bis-(9,9-dimethyl-9H-fluoren-4-yl)-9H-9-aza-tribenzo[a,c,e]cycloheptene**

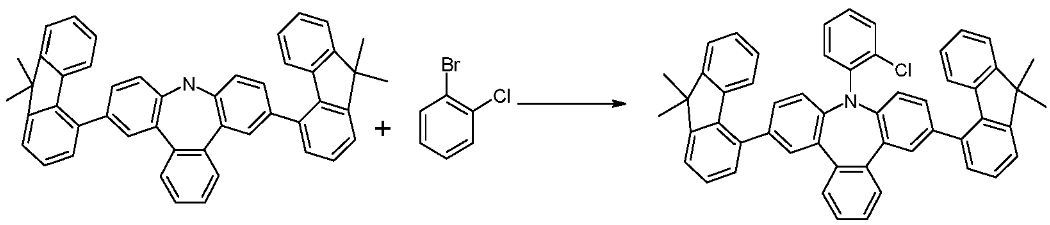

Unter Schutzgas werden 43,9 g (70 mmol) 6,12-Bis-(9,9-dimethyl-9H-fluoren-4-yl)-9H-9-aza-tribenzo[a,c,e] cyclohepten und 14 g (73 mmol)1-Brom-2-chlor-benzen, 8g (84 mmol) Natrium-tert-Butylat, 3,5 ml Tris-tert-butyl-phosphin (1M in Toluol) , 0,393 mg (1,7 mmol) Palladiumacetat in 300 ml p-Xylol suspendiert. Die Reaktionsmischung wird 12 h unter Rückfluss bei 110 °C erhitzt. Nach Erkalten wird die organische Phase abgetrennt, dreimal mit 200 mL Wasser gewaschen und anschließend zur Trockene eingeengt. Das Produkt wird via Säulenchromatographie an Kieselgel mit Toluol/Heptan (1:2) gereinigt. Die Ausbeute beträgt 45 g (61,6mmol), 88 % der Theorie, Reinheit nach $^1$H-NMR ca. 94 %.

Analog können folgende Verbindungen erhalten werden:

| Beispiel | Edukt 1 | Edukt 2 | Produkt | Ausbeute |
|---|---|---|---|---|
| c1 | 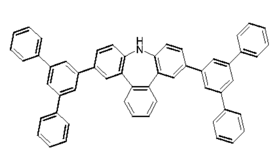 | 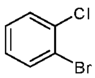 | 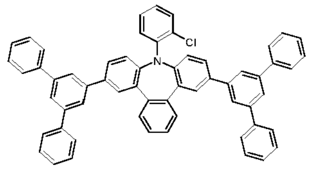 | 64% |
| c2 | 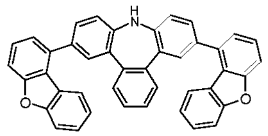 | 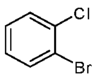 | 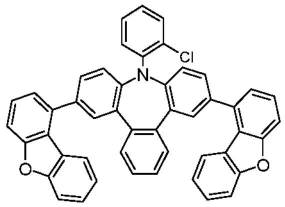 | 78% |
| c3 | 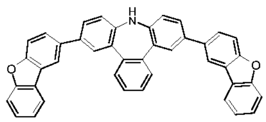 | 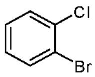 | 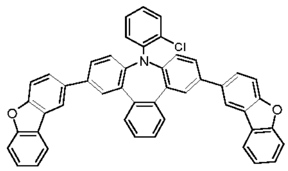 | 63% |
| c4 | 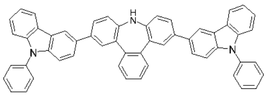. | 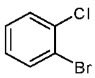 | 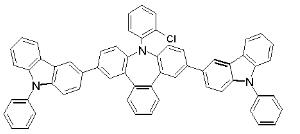. | 78% |
| c5 | 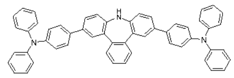 | 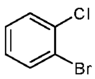 | 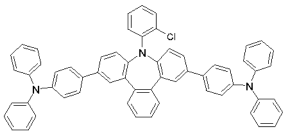. | 69% |
| c6 | 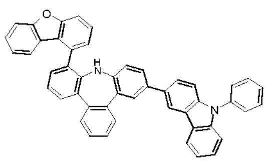 | 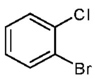 | 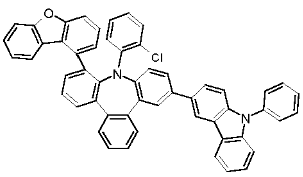 | 61% |
| c7 | 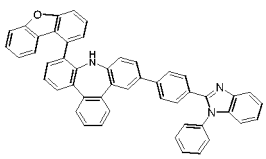 | 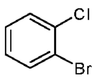 | 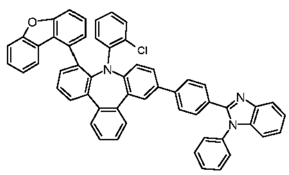 | 62% |
| c8 | 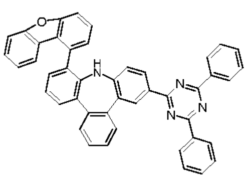 | 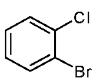 | 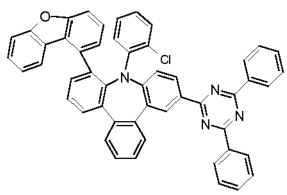 | 58% |

(fortgesetzt)

| Beispiel | Edukt 1 | Edukt 2 | Produkt | Ausbeute |
|---|---|---|---|---|
| c9 | 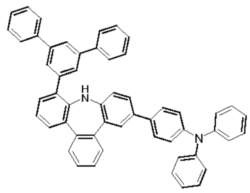 | 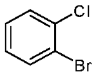 | 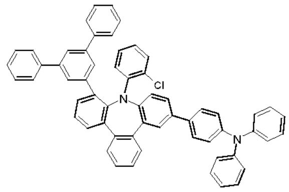 | 54% |
| c10 | 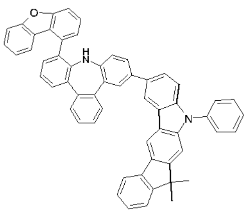 | 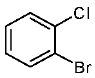 | 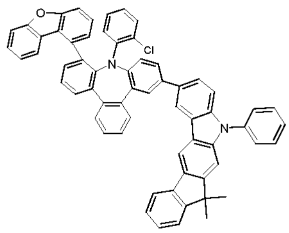 | 59% |
| c11 | 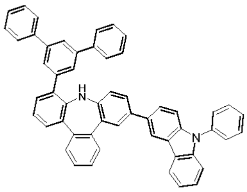 | 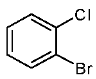 | 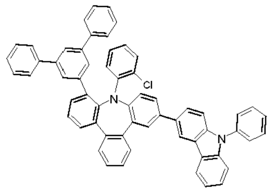 | 62% |
| c12 | 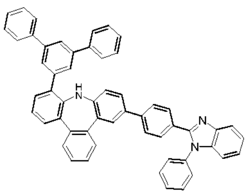 | 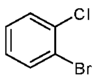 | 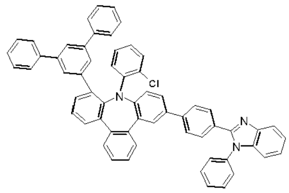 | 60% |
| c13 | 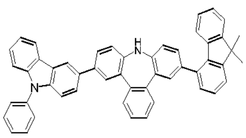 | 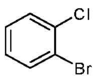 | 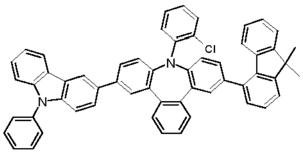 | 81% |
| c14 | 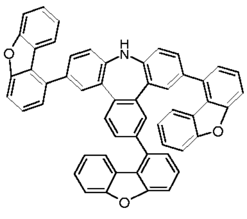 | 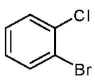 | 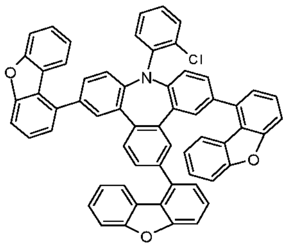 | 89% |
| c15 | 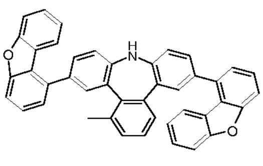 | 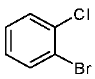 | 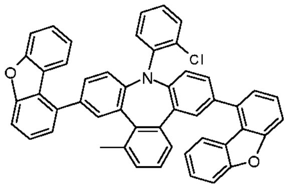 | 87% |

(fortgesetzt)

| Beispiel | Edukt 1 | Edukt 2 | Produkt | Ausbeute |
|---|---|---|---|---|
| c16 | 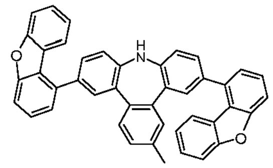 | 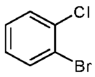 | 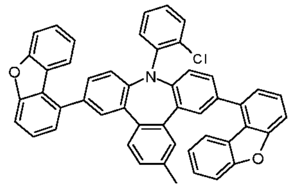 | 88% |
| C17 | 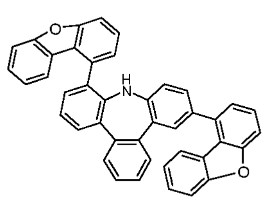 | 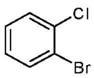 | 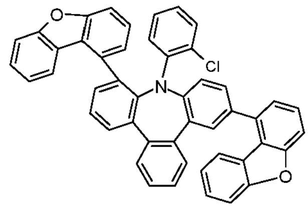 | 86% |
| c18 | 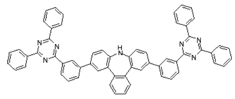 | 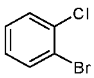 | 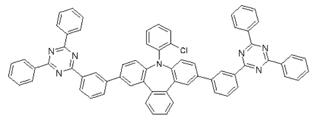 | 76% |
| c19 | 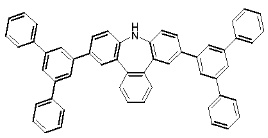 | 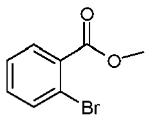 **[610-94-6]** | 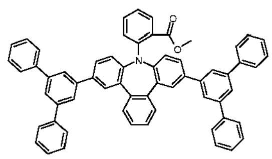 | 74% |
| c20 | 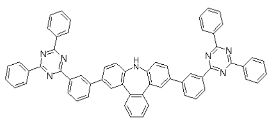 | 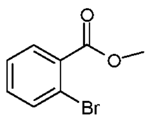 | 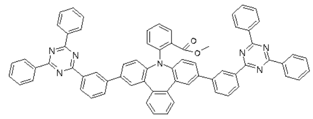 | 72% |
| c21 | 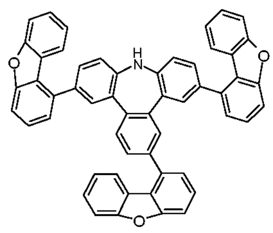 | 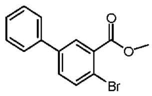 **[727408-92-6]** | 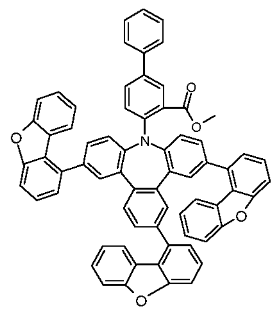 | 73% |
| c22 | 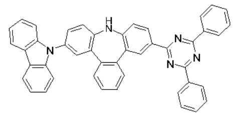 | 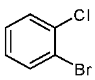 | 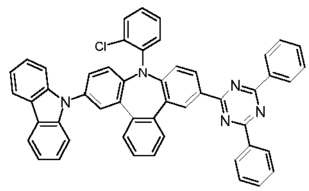 | 74% |
| c23 | 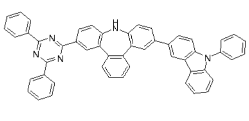 | 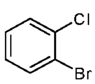 | 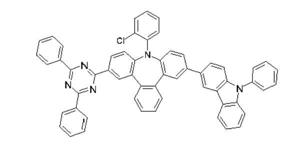 | 73% |

**d) Cyclisierung**

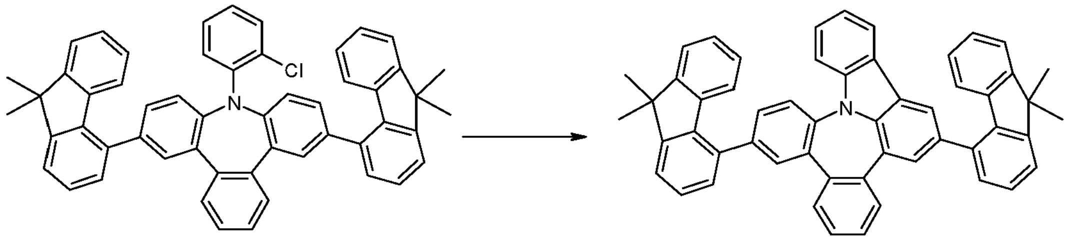

Unter Schutzgas werden 45 g (61 mmol) 9-(2-Chloro-phenyl)-6,12-bis-(9,9-dimethyl-9H-fluoren-4-yl)-9H-9-aza-tribenzo[a,c,e]cycloheptene in 250 mL Dimethylacetamid gelöst. Zur diese Lösung werden 21g (154 mmol)$K_2CO_3$, 10 ml Tri-tert-butylphosphin (1mol/L) und 2,7 g (12,5 mmol) Pd(OAC)2 und 1,8 g (18,5 mmol) Pivalinsäure zugegeben. Anschließend wird das Gemisch bei 130 °C für 80 h gerührt. Nach dieser Zeit wird das Reaktionsgemisch auf Raumtemperatur gekühlt mit Dichlormethan extrahiert. Die vereinigten organischen Phasen werden über $Na_2SO_4$ getrocknet und eingeengt. Der Rückstand wird mit Toluol heiß extrahiert, aus Toluol umkristallisiert und abschließend im Hochvakuum sublimiert. Die Ausbeute beträgt 37 g (52 mmol), 87 % der Theorie, Reinheit nach HPLC ca. 99,9 %.

Analog können folgende Verbindungen erhalten werden: (Verbindung d9 ist nicht erfindungsgemäß)

| | Edukt 1 | Produkt | Ausbeute |
|---|---|---|---|
| d1 | 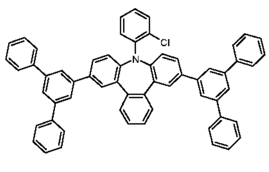 | 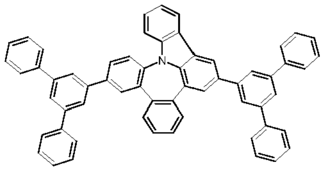 | 68% |
| d2 | 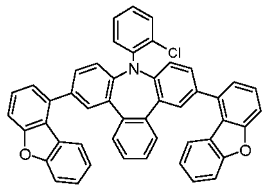 | 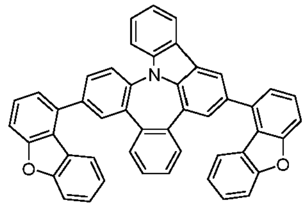 | 78% |
| d3 | 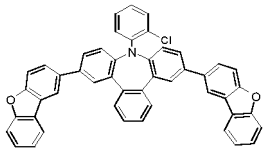 | 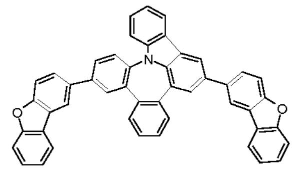 | 65% |
| d4 | 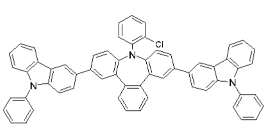 | 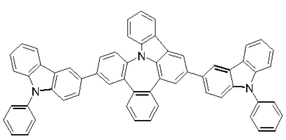 | 69% |
| d5 | 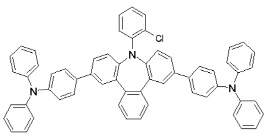 | 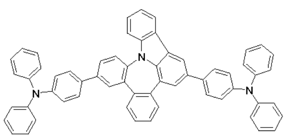 | 65% |

(fortgesetzt)

| | Edukt 1 | Produkt | Ausbeute |
|---|---|---|---|
| d6 | 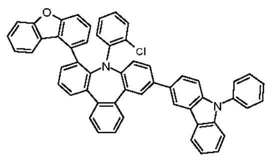 | 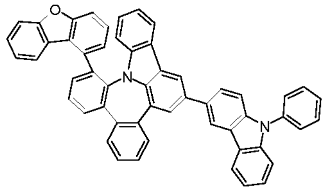 | 61% |
| d7 | 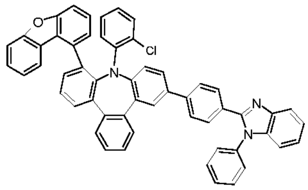 | 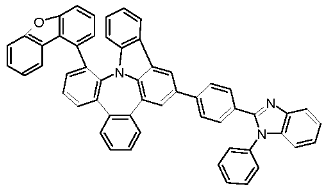 | 60% |
| d8 | 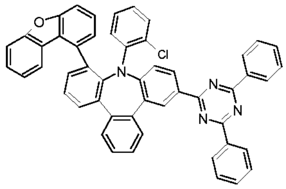 | 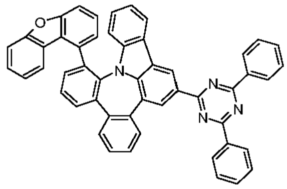 | 58% |
| d9 | 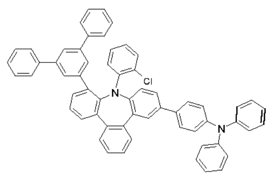 | 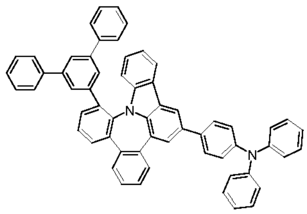 | 63% |
| d10 | 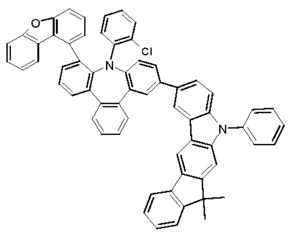 | 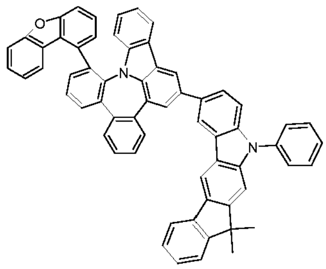 . . | 60% |
| d11 | 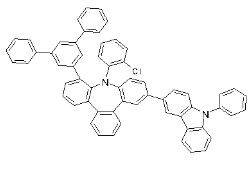 | 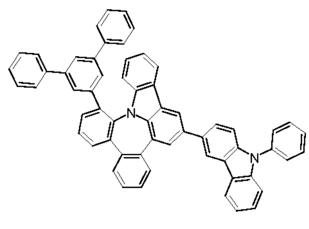 . . | 62% |
| d12 | 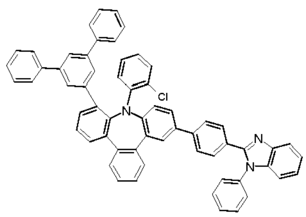 | 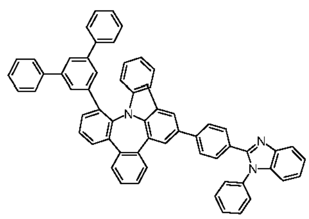 | 64% |

(fortgesetzt)

| | Edukt 1 | Produkt | Ausbeute |
|---|---|---|---|
| d13 | 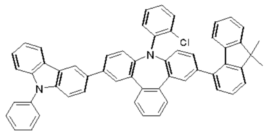 | 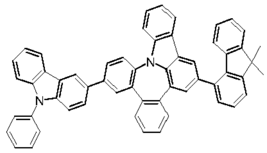 | 61% |
| d14 | 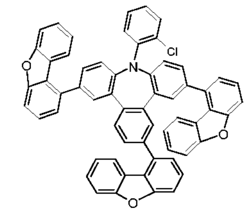 | 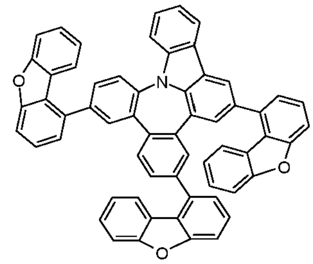 | 69% |
| d15 | 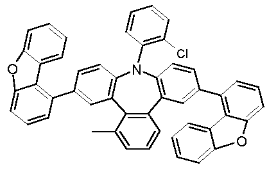 | 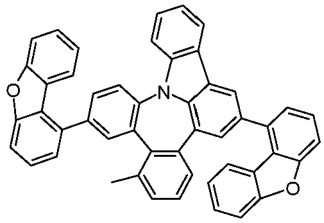 | 66% |
| d16 | 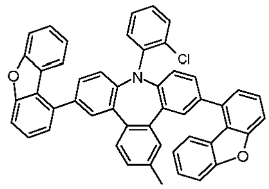 | 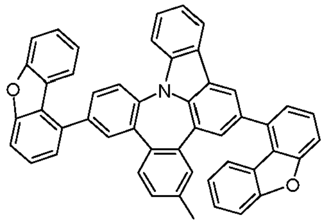 | 68% |
| d17 | 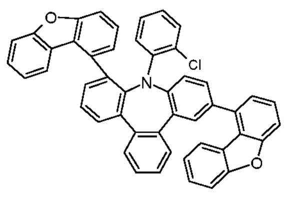 . | 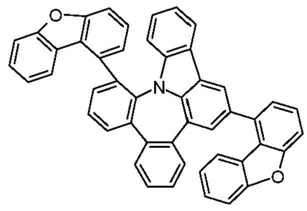 . | 72% |
| d18 | 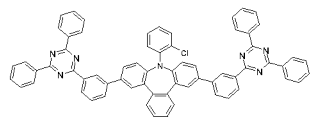 . | 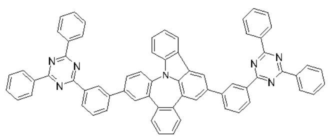 . | 83% |
| d19-a | 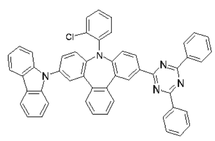 | 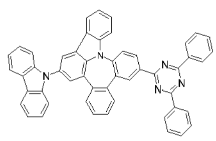 | 35% |
| d19-b | 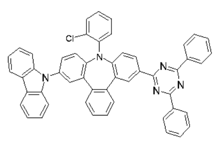 | 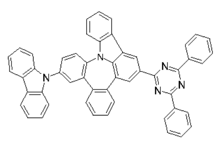 | 23% |

(fortgesetzt)

| | Edukt 1 | Produkt | Ausbeute |
|---|---|---|---|
| d20-a | 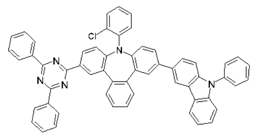 | 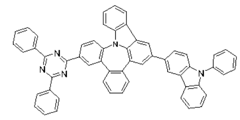 | 37% |
| d20-b | 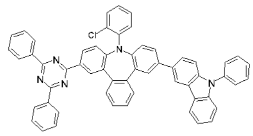 | 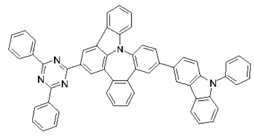 | 29% |

**e) 2-[2-(6,12-Bis-[1,1';3',1"]terphenyl-5'-yl-9-aza-tribenzo[a,c,e]cyclohepten-9-yl)-phenyl]-propan-2-ol**

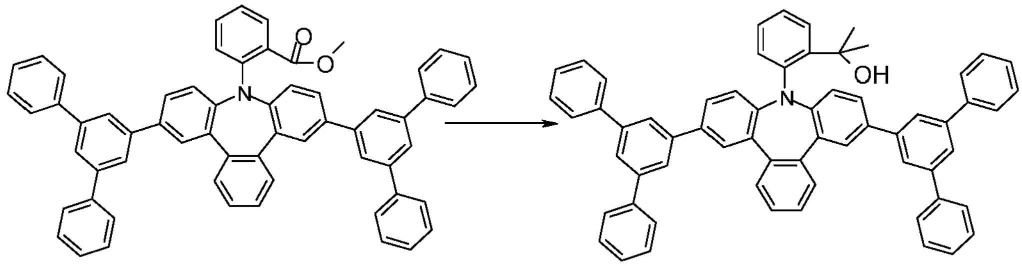

177 g (213 mmol) 2-(6,12-Bis-[1,1';3',1"]terphenyl-5'-yl-9-aza-tribenzo[a,c,e]-cyclohepten-9-yl)-benzoesäuremethylester werden in 1500 mL getrocknetem THF gelöst und entgast. Es wird auf -78 °C gekühlt und innerhalb von 40 min mit 569 mL (854 mmol) Methyllithium versetzt. Man lässt innerhalb 1 h bis auf -40 °C erwärmen und kontrolliert die Umsetzung via DC. Nach vollständiger Umsetzung wird bei -30 °C vorsichtig mit MeOH gequencht. Die Reaktionslösung wird auf 1/3 eingeengt und mit 1 L $CH_2Cl_2$ versetzt, gewaschen und die organische Phase über $MgSO_4$ getrocknet und eingeengt. Die Ausbeute beträgt 158 g (189 mmol), 89% der Theorie.

Analog kann die folgende Verbindung hergestellt werden.

| Beispiel | Edukt 1 | Produkt | Ausbeute |
|---|---|---|---|
| e1 | 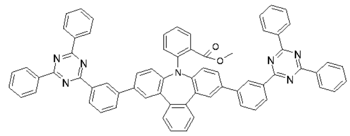 | 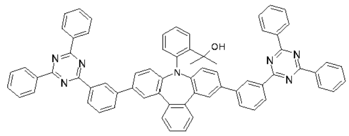 | 72% |
| e2 | 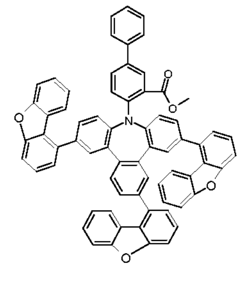 | 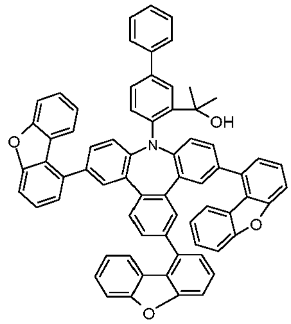 | 74% |

**f) Cyclisierung**

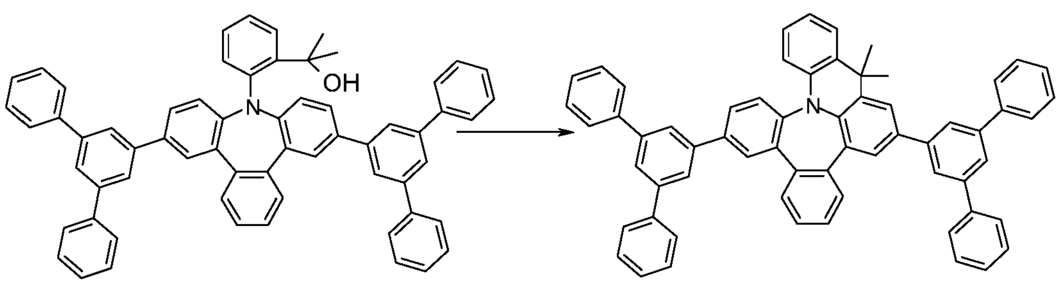

Molekulargewicht =834,08    Molekulargewicht =816,07

36 g (43.6 mmol) 2-[2-(6,12-Bis-[1,1';3',1"]terphenyl-5'-yl-9-azatribenzo[a,c,e] cyclohepten-9-yl)-phenyl]-propan-2-ol werden in 1200 mL entgastem Toluol gelöst und mit einer Suspension aus 40 g Polyphosphorsäure und 28 mL Methansulfonsäure versetzt und für 1 h auf 60 °C erhitzt. Der Ansatz wird abgekühlt und mit Wasser versetzt. Es fällt ein Feststoff aus, der in $CH_2Cl_2$/THF (1:1) gelöst wird. Die Lösung wird mit 20%iger NaOH vorsichtig alkalisiert, die Phasen werden getrennt und über $MgSO_4$ getrocknet. Der Rückstand wird mit Toluol heiß extrahiert, aus Toluol/Heptan (1:2) umkristallisiert und abschließend im Hochvakuum sublimiert. Die Ausbeute beträgt 28 g (35 mmol) 81% der Theorie.

Analog können folgende Verbindungen erhalten werden:

| Beispiel | Edukt 1 | Produkt | Ausbeute |
|---|---|---|---|
| f1 | 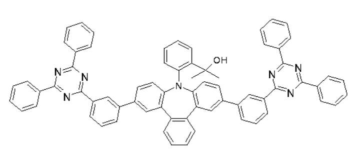 | 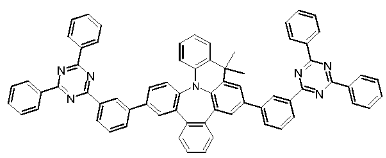 | 73% |
| f2 | 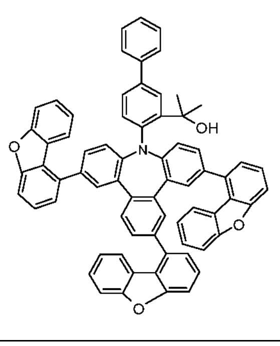 | 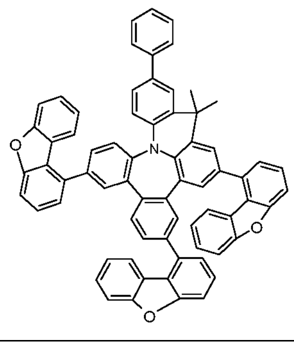 | 72% |

**g) 8-Chloro-9-(2-Nitro-phenyl)-9H-9-aza-tribenz [b,d,f]azepin**

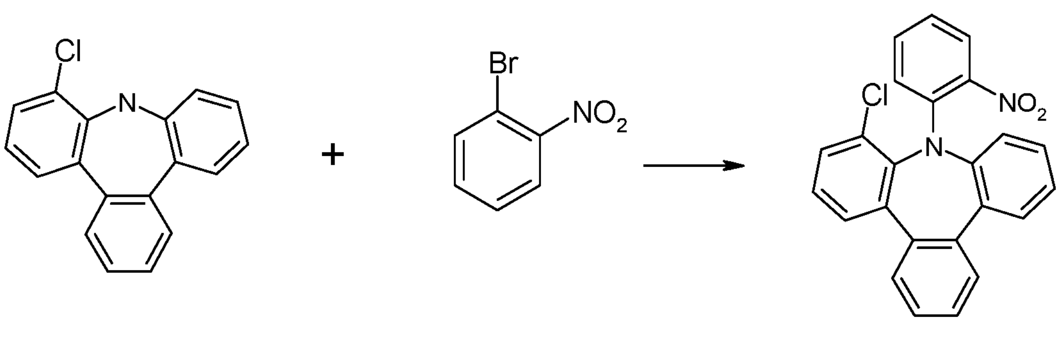

**[0148]** [163811-00-5] Unter Schutzgas werden 24,6g (89 mmol) 8-Chloro-9H-9-aza-tribenz [b,d,f]azepin, 17,9 g (89 mmol) 1,2 Bromnitrobenzol und 0,8 g (0,88 mmol) Tris(dibenzylideneaceton)-dipalladium, 1.79 g (7.9 mmol) Palladiumacetat in 500 ml Toluol suspendiert. Die Reaktionsmischung wird 8 h unter Rückfluss erhitzt. Nach Erkalten wird die organische Phase abgetrennt, dreimal mit 200 mL Wasser gewaschen und anschließend zur Trockene eingeengt. Die Reinheit beträgt 87 %. Ausbeute: 25,5 g (63 mmol) 72 % der Theorie.

Analog dazu werden die folgenden Verbindungen hergestellt.

| | Edukt 1 | Edukt 2 | Produkt | Ausbeute |
|---|---|---|---|---|
| g1 | 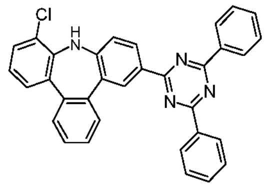 | 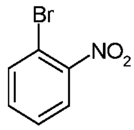 | 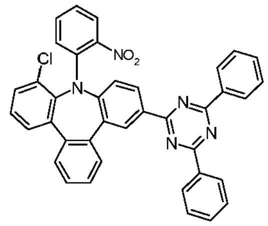 | 64% |
| g2 | 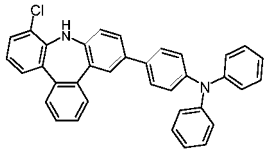 | 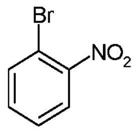 | 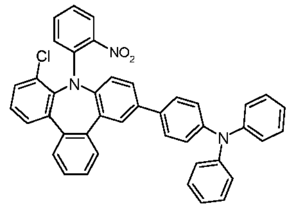 | 78% |
| g3 | 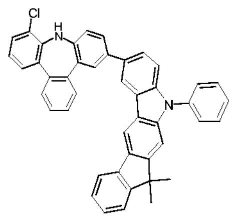 | 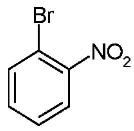 | 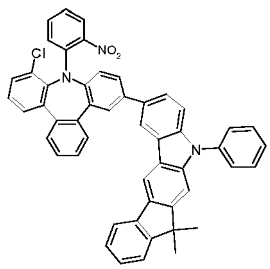 | |
| g4 | 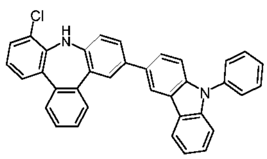 | 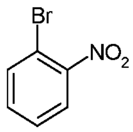 | 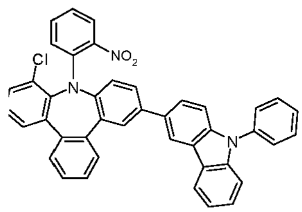 | |
| g5 | 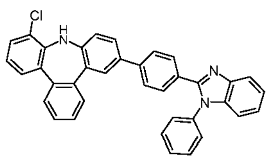 | 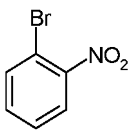 | 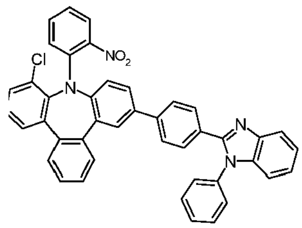 | |
| g6 | 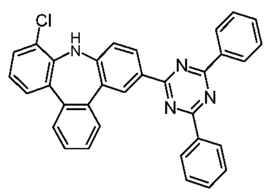 | 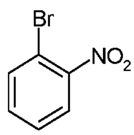 | 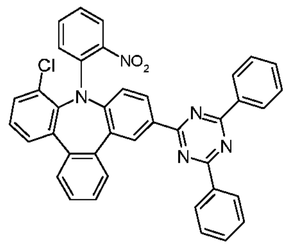 | |

**h) 2-(8-Chloro-9H-9-aza-tribenz [b,d,f]azepin-yl)-phenylamin**

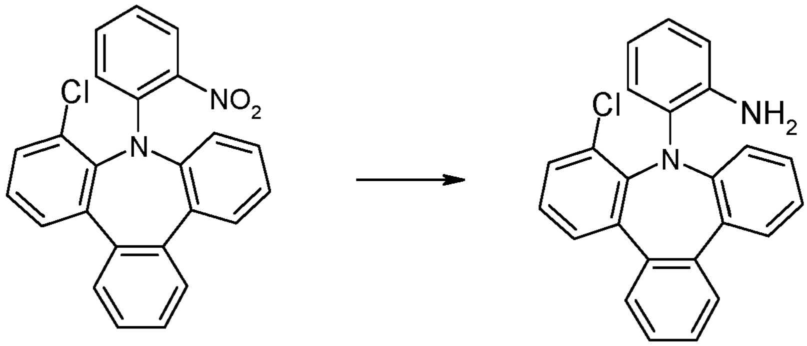

16,7 g (42 mmol) 8-Chloro-9-(2-Nitro-phenyl)-9H-9-aza-tribenz [b,d,f]azepin wird in 200 mL Ethanol suspendiert. Unter Rühren bei 60 °C werden 26 g (140 mmol) SnCl2 gelöst in 25 ml konzentriert HCl portionsweise hinzugefügt und 8 h unter Rückfluss gekocht. Danach wird der Niederschlag abfiltriert und im Vakuum getrocknet. Die Reinheit beträgt 90 %. Ausbeute: 14,2 g (38 mmol) 92 % der Theorie.

Analog können folgende Verbindungen erhalten werden:

| | Edukt 1 | Produkt | Ausbeute |
|---|---|---|---|
| h1 | 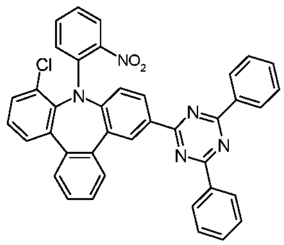 | 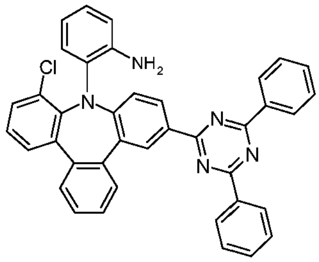 | 71% |
| h2 | 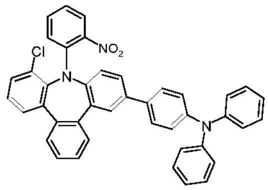 | 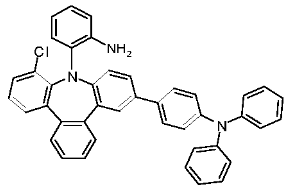 | 73% |
| h3 | 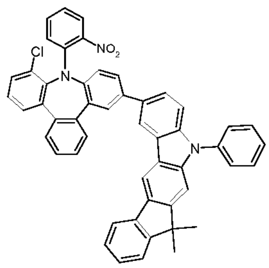 | 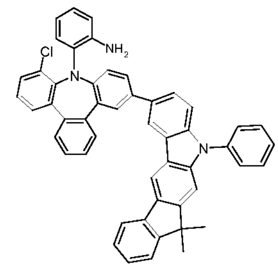 | 70% |
| h4 | 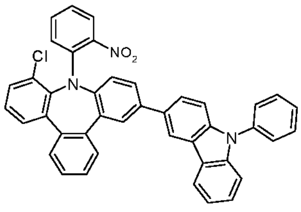 | 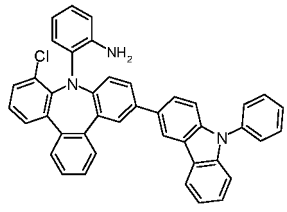 | 74% |

(fortgesetzt)

| | Edukt 1 | Produkt | Ausbeute |
|---|---|---|---|
| h5 | 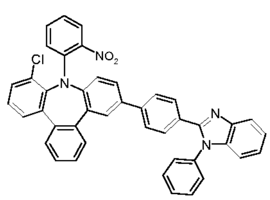 | 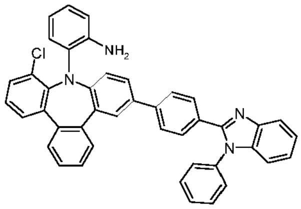 | 78% |
| h6 | 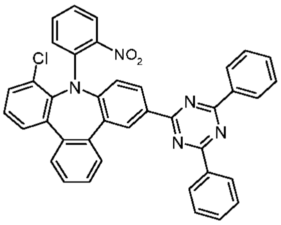 | 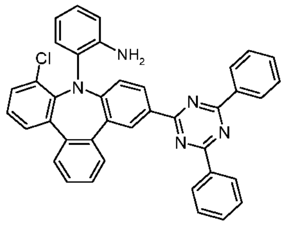 | 72% |

**i) Cyclisierung nicht erfindungsgemäß**

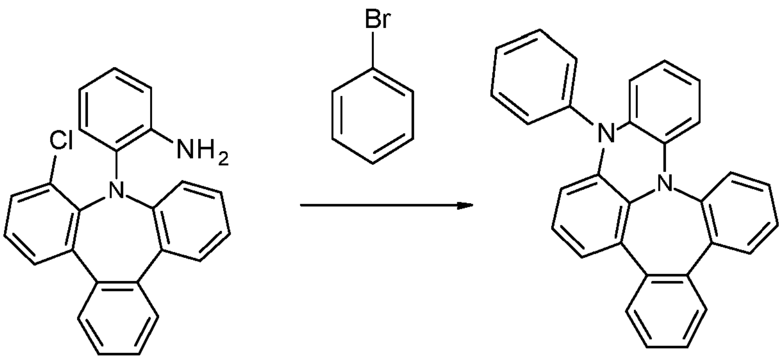

Unter Schutzgas werden 9,9 g (27 mmol) 2-(8-Chloro-9H-9-aza-tribenz [b,d,f]azepin-yl)-phenylamin, 0,24g (0,26 mmol) Tris(dibenzylideneaceton)-dipalladium, 0.53 g (2,37 mmol) Palladiumacetat in 150 ml Toluol suspendiert. Die Reaktionsmischung wird 8 h unter Rückfluss erhitzt. Anschließend wird 4 g (26 mmol) 4-Brombenzol und zu gegeben und weiter 8 h unter Rückfluß gekocht. Nach Erkalten wird die organische Phase abgetrennt, dreimal mit 80 mL Wasser gewaschen und anschließend zur Trockene eingeengt. Der Rückstand wird mit Toluol heiß extrahiert, aus Toluol/Heptan (1:2) umkristallisiert und abschließend im Hochvakuum sublimiert. Die Reinheit beträgt nach HPLC 99,9 %. Ausbeute: 7,9 g (19,4 mmol) 72 % der Theorie.

Analog dazu werden die folgenden Verbindungen nicht erfindungsgemäß hergestellt

| | Edukt 1 | Edukt 2 | Produkt | Aus-beute |
|---|---|---|---|---|
| i1 | 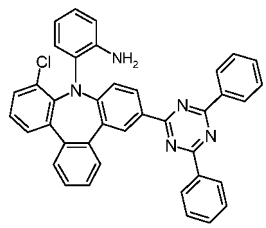 | 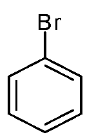 | 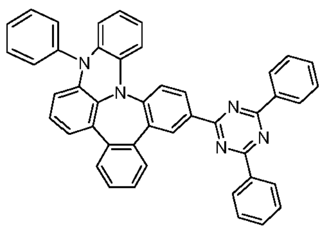 | 60% |

(fortgesetzt)

| | Edukt 1 | Edukt 2 | Produkt | Aus-beute |
|---|---|---|---|---|
| i2 | 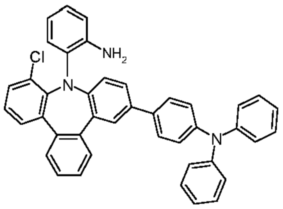 | 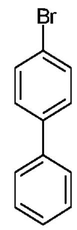 | 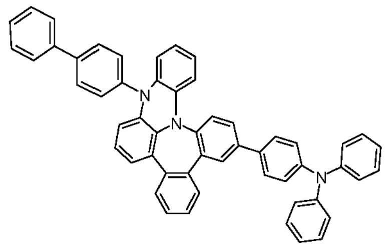 | 67% |
| i3 | 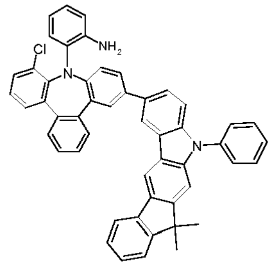 | 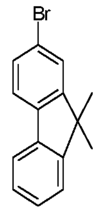 | 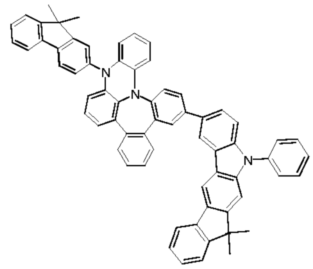 | 69% |
| i4 | 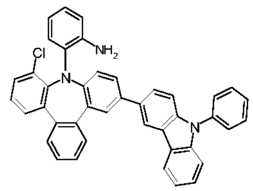 | 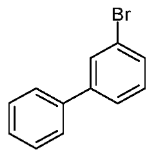 | 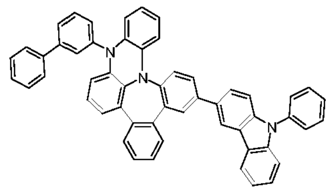 | 68% |
| i5 | 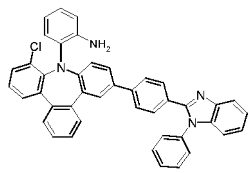 | 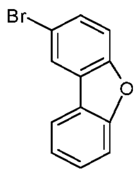 | 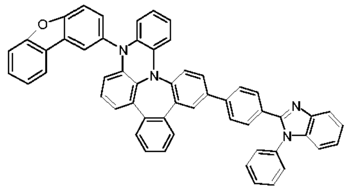 | 65% |
| i6 | 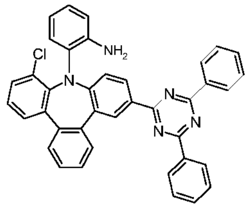 | 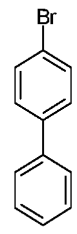 | 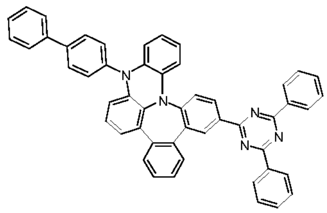 | 73% |

## Vorrichtungsbeispiele

### Herstellung der OLEDs

Die Herstellung von erfindungsgemäßen OLEDs sowie OLEDs nach dem Stand der Technik erfolgt nach einem allgemeinen Verfahren gemäß WO 2004/058911, das auf die hier beschriebenen Gegebenheiten (Schichtdickenvariation, Materialien) angepasst wird.

In den folgenden Beispielen V1-E6-4 (siehe Tabellen 1 und 2) werden die Daten verschiedener OLEDs vorgestellt. Gereinigte Glasplättchen (Reinigung in Laborspülmaschine, Reiniger Merck Extran), die mit strukturiertem ITO (Indium Zinn Oxid) der Dicke 50 nm beschichtet sind werden zur verbesserten Prozessierung mit 20 nm PEDOT:PSS beschichtet (Poly(3,4-ethylenedioxythiophene) poly(styrenesulfonate), bezogen als CLEVIOS™ P VP AI 4083 von Heraeus Precious Metals GmbH Deutschland, aus wässriger Lösung aufgeschleudert). Diese beschichteten Glasplättchen bilden die Substrate, auf welche die OLEDs aufgebracht werden.

Die OLEDs haben prinzipiell folgenden Schichtaufbau: Substrat / Optionale Lochinjektionsschicht (HIL) / Lochtransportschicht (HTL) / Optionale Zwischenschicht (IL) / Elektronenblockierschicht (EBL) / Emissionsschicht (EML) / Optionale Lochblockierschicht (HBL) / Elektronentransportschicht (ETL) / Optionale Elektroneninjektionsschicht (EIL) und abschließend eine Kathode. Die Kathode wird durch eine 100 nm dicke Aluminiumschicht gebildet. Der genaue Aufbau der OLEDs ist Tabelle 1 zu entnehmen. Die zur Herstellung der OLEDs benötigten Materialien sind in Tabelle 3 gezeigt.

Alle Materialien werden in einer Vakuumkammer thermisch aufgedampft. Dabei besteht die Emissionsschicht immer aus mindestens einem Matrixmaterial (Hostmaterial, Wirtsmaterial) und einem emittierenden Dotierstoff (Dotand, Emitter), der dem Matrixmaterial bzw. den Matrixmaterialien durch Coverdampfung in einem bestimmten Volumenanteil beigemischt wird. Eine Angabe wie ST1:CBP:TER1 (55%:35%:10%) bedeutet hierbei, dass das Material ST1 in einem Volumenanteil von 55%, CBP in einem Anteil von 35% und TER1 in einem Anteil von 10% in der Schicht vorliegt. Analog kann auch die Elektronentransportschicht aus einer Mischung von zwei Materialien bestehen.

Die OLEDs werden standardmäßig charakterisiert. Hierfür werden die Elektrolumineszenzspektren, die Stromeffizienz (gemessen in cd/A), in Abhängigkeit der Leuchtdichte, berechnet aus Strom-Spannungs-Leuchtdichte-Kennlinien (IUL-Kennlinien) unter Annahme einer lambertschen Abstrahlcharakteristik sowie die Lebensdauer bestimmt. Die Elektrolumineszenzspektren werden bei einer Leuchtdichte von 1000 cd/$m^2$ bestimmt und daraus die CIE 1931 x und y Farbkoordinaten berechnet. Die Angabe U1000 in Tabelle 2 bezeichnet die Spannung, die für eine Leuchtdichte von 1000 cd/$m^2$ benötigt wird. SE1000 bezeichnet die Stromeffizienz, die bei 1000 cd/$m^2$ erreicht werden.

Als Lebensdauer LD wird die Zeit definiert, nach der die Leuchtdichte bei Betrieb mit konstantem Strom von der Startleuchtdichte auf einen gewissen Anteil L1 absinkt. Eine Angabe von $L_0;j_0$ = 4000 cd/$m^2$ und L1 = 80% in Tabelle 2 bedeutet, dass die in Spalte LD angegebene Lebensdauer der Zeit entspricht, nach der die Anfangsleuchtdichte von 4000 cd/$m^2$ auf 3200 cd/$m^2$ absinkt. Analog bedeutet $L_0;j_0$ = 20mA/$cm^2$, L1 = 80%, dass die anfängliche Leuchtdichte bei Betrieb mit 20mA/$cm^2$ nach der Zeit LD auf 80% ihres Anfangswertes absinkt.

Die Werte für die Lebensdauer können mit Hilfe dem Fachmann bekannten Umrechnungsformeln auf eine Angabe für andere Startleuchtdichten umgerechnet werden. Hierbei ist die Lebensdauer für eine Startleuchtdichte von 1000 cd/$m^2$ eine übliche Angabe.

Die Daten der verschiedenen OLEDs sind in Tabelle 2 zusammengefasst. Die Beispiel V1-V5 sind Vergleichsbeispiele gemäß dem Stand der Technik, die Beispiele E1-E6-4 zeigen Daten von OLEDs mit erfindungsgemäßen Materialien.

Im folgenden werden einige der Beispiele näher erläutert, um die Vorteile der erfindungsgemäßen Verbindungen zu verdeutlichen. Es sei jedoch darauf hingewiesen, dass dies nur eine Auswahl der in Tabelle 2 gezeigten Daten darstellt. Wie sich der Tabelle entnehmen lässt, werden auch bei Verwendung der nicht näher ausgeführten erfindungsgemäßen Verbindungen deutliche Verbesserungen gegenüber dem Stand der Technik erzielt, teilweise in allen Parametern, in manchen Fällen ist aber nur eine Verbesserung von Effizienz oder Spannung oder Lebensdauer zu beobachten. Allerdings stellt bereits die Verbesserung einer der genannten Paramter einen signifikanten Fortschritt dar, weil verschiedene Anwendungen die Optimierung hinsichtlich unterschiedlicher Parameter erfordern.

Die OLEDs V1-V5 sind Vergleichsbeispiele gemäß dem Stand der Technik.

**Verwendung von erfindungsgemäßen Verbindungen als Elektronentransportmaterialien**

Durch den Einsatz von erfindungsgemäßen Verbindungen in der Elektronentransportschicht von OLEDs lassen sich deutliche Steigerungen hinsichtlich Betriebsspannung, externer Quanteneffizienz und damit vor allem auch der Leistungseffizienz erzielen. Weiterhin erhält man verbesserte Lebensdauern im Falle phosphoreszenter Dotanden.

**Verwendung von erfindungsgemäßen Verbindungen als Lochblockiermaterialien**

Durch den Einsatz von erfindungsgemäßen Verbindungen auf der Lochblockierseite von OLEDs erhält man also signifikante Verbesserungen bezüglich Betriebsspannung, Leistungseffizienz, Lebensdauer und Prozessierungsaufwand.

**Verwendung von erfindungsgemäßen Verbindungen als Matrixmaterialien in phosphoreszierenden OLEDs**

Die erfindungsgemäßen Materialien ergeben bei Einsatz als Matrixmaterialien in phosphoreszierenden OLEDs somit wesentliche Verbesserungen gegenüber dem Stand der Technik in allen Parametern, vor allem bezüglich Lebensdauer und teilweise auch in der Stromeffizienz.

Tabelle 1: Aufbau der OLEDs (Beispiele E4, E7 und E8 umfassend Verbindungen EG4, EG7 und EG8, sind nicht erfindungsgemäß)

| Bsp | HTL Dicke | IL Dicke | EBL Dicke | EML Dicke | HBL Dicke | ETL Dicke | EIL Dicke |
|---|---|---|---|---|---|---|---|
| V1 | SpA1 70nm | HATCN 5nm | SpMA1 90nm | SdT1:TEG1 (90%: 10%) 30nm | ST2 10nm | ST2:LiQ (50%: 50%) 30nm | --- |
| V2 | SpA1 70nm | HATCN 5nm | SpMA1 90nm | SdT2:TEG1 (90%: 10%) 30nm | ST2 10nm | ST2:LiQ (50%: 50%) 30nm | --- |
| V3 | SpA1 70nm | HATCN 5nm | SpMA1 90nm | SdT3:TEG1 (90%: 10%) 30nm | ST2 10nm | ST2:LiQ (50%: 50%) 30nm | --- |
| V4 | SpA1 70nm | HATCN 5nm | SpMA1 90nm | SdT4:IC1:TEG1 (45%:45%:10%) 30nm | ST2 10nm | ST2:LiQ (50%: 50%) 30nm | --- |
| V5 | SpA1 70nm | HATCN 5nm | SpMA1 90nm | SdT5:IC1:TEG1 (45%:45%:10%) 30nm | ST2 10nm | ST2:LiQ (50%: 50%) 30nm | --- |
| E1 | SpA1 70nm | HATCN 5nm | SpMA1 90nm | EG1:TEG1 (90%: 10%) 30nm | ST2 10nm | ST2:LiQ (50%: 50%) 30nm | --- |
| E2 | SpA1 70nm | HATCN 5nm | SpMA1 90nm | EG2:TEG1 (90%: 10%) 30nm | ST2 10nm | ST2:LiQ (50%: 50%) 30nm | --- |
| E3 | SpA1 70nm | HATCN 5nm | SpMA1 90nm | EG3:IC1:TEG1 (45%:45%:10%) 30nm | ST2 10nm | ST2:LiQ (50%: 50%) 30nm | --- |
| E4 | SpA1 70nm | HATCN 5nm | SpMA1 90nm | EG4:IC1:TEG1 (45%:45%:10%) 30nm | ST2 10nm | ST2:LiQ (50%: 50%) 30nm | --- |
| E1-1 | SpA1 90nm | HATCN 5nm | SpMA1 130nm | EG1:TER1(92%:8%) 30nm | --- | ST2:LiQ (50%: 50%) 40nm | --- |
| E2-1 | SpA1 90nm | HATCN 5nm | SpMA1 130nm | EG2:TER1(92%:8%) 30nm | --- | ST2:LiQ (50%: 50%) 40nm | --- |
| E5 | SpA1 70nm | HATCN 5nm | SpMA1 90nm | EG5:IC1:TEG1 (45%:45%:10%) 30nm | ST2 10nm | ST2:LiQ (50%: 50%) 30nm | --- |
| E6 | SpA1 70nm | HATCN 5nm | SpMA1 90nm | EG6:TEG1 (90%: 10%) 30nm | ST2 10nm | ST2:LiQ (50%: 50%) 30nm | --- |
| E7 | SpA1 70nm | HATCN 5nm | SpMA1 90nm | EG7:IC1:TEG1 (45%:45%:10%) 30nm | ST2 10nm | ST2:LiQ (50%: 50%) 30nm | --- |
| E8 | SpA1 70nm | HATCN 5nm | SpMA1 90nm | EG8:TEG1 (90%: 10%) 30nm | ST2 10nm | ST2:LiQ (50%: 50%) 30nm | --- |
| E6-1 | SpA1 70nm | HATCN 5nm | SpMA1 90nm | IC1:TEG1 (90%: 10%) 30nm | --- | EG6 40nm | LiQ 3nm |
| E6-2 | SpA1 70nm | HATCN 5nm | SpMA1 90nm | IC1:TEG1 (90%: 10%) 30nm | IC1 10nm | EG6:LiQ (50%: 50%) 30nm | --- |
| E6-3 | SpA1 70nm | HATCN 5nm | SpMA1 90nm | IC1:TEG1 (90%: 10%) 30nm | EG6 10nm | ST2:LiQ (50%: 50%) 30nm | --- |
| E6-4 | HATCN 5nm | SpMA1 70nm | SpMA2 15nm | EG6:L1:TEY1 (45%: 45%:10%) 25nm | --- | ST1 45nm | LiQ 3nm |

Tabelle 2: Daten der OLEDs

| Bsp. | U1000 (V) | SE1000 (cd/A) | CIE x/y bei 1000 cd/m$^2$ | $L_0$; $j_0$ | L1 % | LD (h) |
|---|---|---|---|---|---|---|
| V1 | 3.9 | 51 | 0.33/0.63 | 20mA/cm$^2$ | 80 | 90 |
| V2 | 4.3 | 53 | 0.33/0.62 | 20mA/cm$^2$ | 80 | 105 |
| V3 | 4.4 | 54 | 0.33/0.64 | 20mA/cm$^2$ | 80 | 110 |
| V4 | 3.8 | 58 | 0.32/0.64 | 20mA/cm$^2$ | 80 | 190 |
| V5 | 4.0 | 56 | 0.33/0.64 | 20mA/cm$^2$ | 80 | 170 |
| E1 | 3.8 | 52 | 0.33/0.62 | 20mA/cm$^2$ | 80 | 110 |
| E2 | 4.2 | 53 | 0.33/0.62 | 20mA/cm$^2$ | 80 | 130 |
| E3 | 3.7 | 59 | 0.33/0.63 | 20mA/cm$^2$ | 80 | 250 |
| E4 | 3.6 | 58 | 0.32/0.63 | 20mA/cm$^2$ | 80 | 210 |
| E1-1 | 4.3 | 12 | 0.66/0.34 | 4000 cd/m$^2$ | 80 | 310 |
| E2-1 | 4.5 | 11 | 0.67/0.34 | 4000 cd/m$^2$ | 80 | 320 |
| E5 | 4.1 | 48 | 0.33/0.63 | 20mA/cm$^2$ | 80 | 170 |
| E6 | 3.9 | 50 | 0.33/0.62 | 20mA/cm$^2$ | 80 | 80 |
| E7 | 3.4 | 62 | 0.34/0.63 | 20 mA/cm$^2$ | 80 | 190 |
| E8 | 3.8 | 49 | 0.33/0.62 | 20mA/cm$^2$ | 80 | 70 |
| E6-1 | 3.9 | 63 | 0.33/0.63 | 20 mA/cm$^2$ | 80 | 125 |
| E6-2 | 4.2 | 60 | 0.34/0.63 | 20 mA/cm$^2$ | 80 | 165 |
| E6-3 | 3.6 | 59 | 0.34/0.63 | 20 mA/cm$^2$ | 80 | 140 |
| E6-4 | 3.0 | 75 | 0.44/0.55 | 50mA/cm$^2$ | 90 | 80 |

Tabelle 3: Strukturformeln der Materialien für die OLEDs

| | |
|---|---|
| 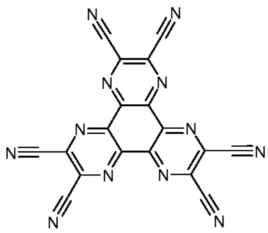 | 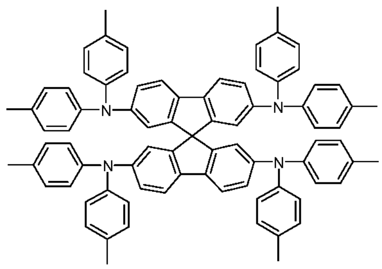 |
| HATCN | SpA1 |
| 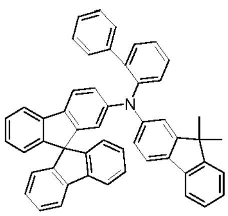 | 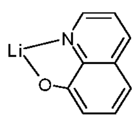 |
| SpMA1 | LiQ |

(fortgesetzt)

| | |
|---|---|
| 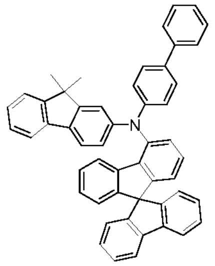 | 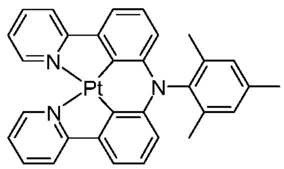 |
| SpMA2 | TER1 |
| 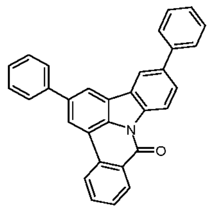 | 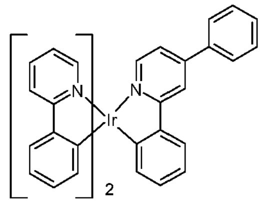 |
| L1 | TEY1 |
| 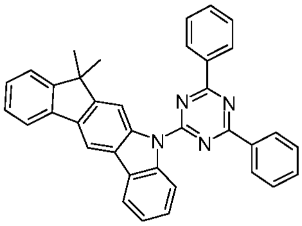 | 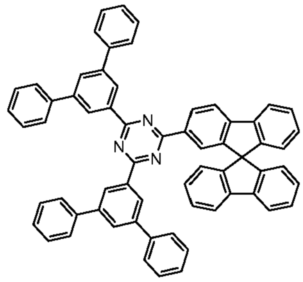 |
| IC1 | ST2 |
| 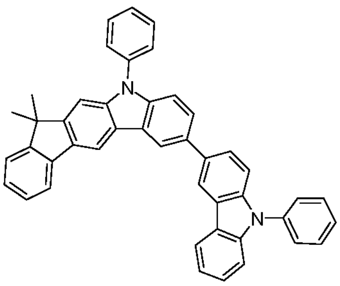 | 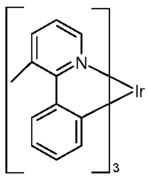 |
| IC3 | TEG1 |
| 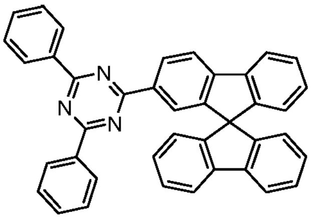 | 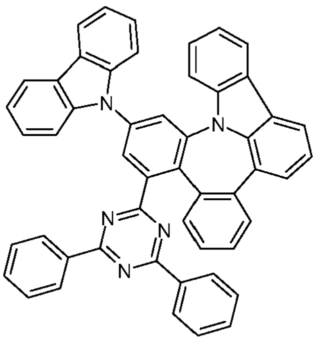 |
| ST1 | SdT1 |

(fortgesetzt)

| | |
|---|---|
| 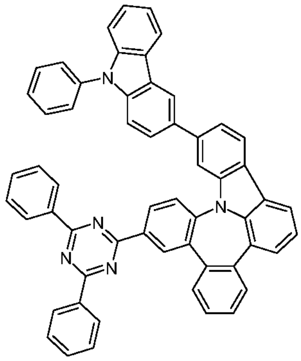 | 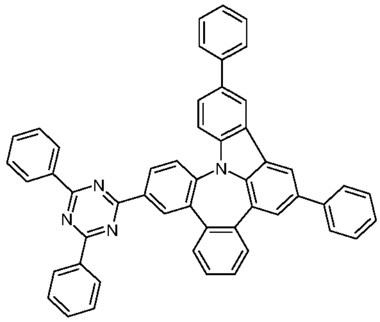 |
| SdT2 | SdT3 |
| 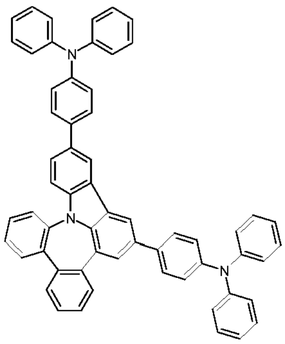 | 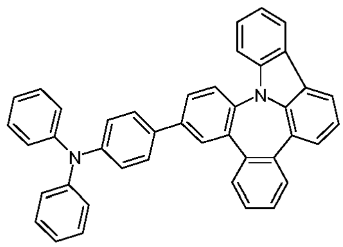 |
| SdT4 | SdT5 |
| 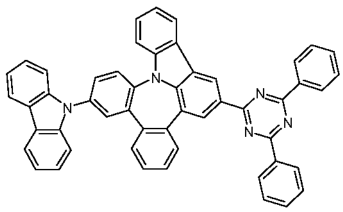 | 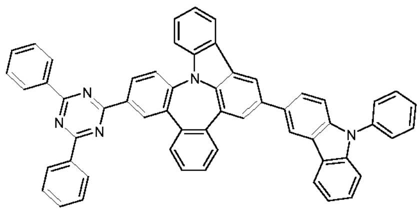 |
| EG1 | EG2 |
| 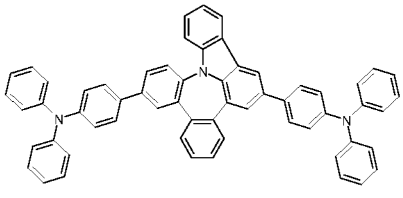 | 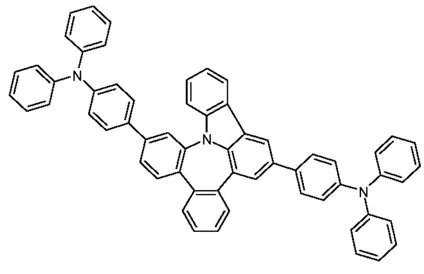 |
| EG3 | EG4 |
| 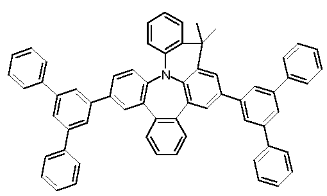 | 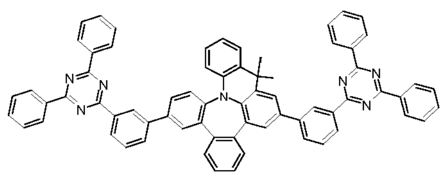 |
| EG5 | EG6 |

(fortgesetzt)

| | |
|---|---|
| 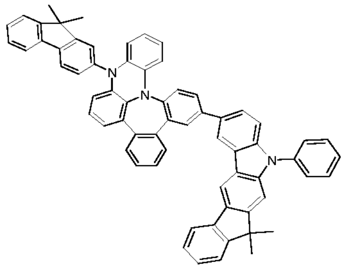 | 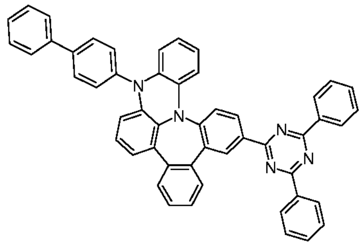 |
| EG7 | EG8 |

## Patentansprüche

**1.** Verbindung, umfassend Strukturen der Formel (II),

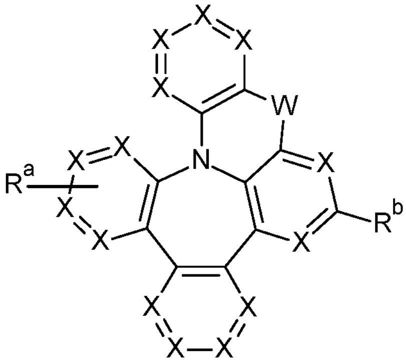

Formel (II),

wobei für die verwendeten Symbole gilt:

X ist bei jedem Auftreten gleich oder verschieden $CR^1$ oder C für die Anbindungsstelle des Restes $R^a$;
W ist eine Bindung, $NR^1$, $C(R^1)_2$, O oder S;
$R^a$ ist D, F, Cl, Br, I, $B(OR^1)_2$, CHO, $C(=O)R^1$, $CR^1=C(R^1)_2$, CN, $C(=O)OR^1$, $C(=O)N(R^1)_2$, $Si(R^1)_3$, $N(R^1)_2$, $NO_2$, $P(=O)(R^1)_2$, $OSO_2R^1$, $OR^1$, $S(=O)R^1$, $S(=O)_2R^1$, eine geradkettige Alkyl-, Alkoxy- oder Thioalkoxygruppe mit 1 bis 40 C-Atomen oder eine verzweigte oder cyclische Alkyl-, Alkoxy- oder Thioalkoxygruppe mit 3 bis 40 C-Atomen, die jeweils mit einem oder mehreren Resten $R^1$ substituiert sein kann, wobei eine oder mehrere nicht benachbarte $CH_2$-Gruppen durch $-R^1C=CR^1-$, -C≡C-, $Si(R^1)_2$, C=O, C=S, $C=NR^1$, -C(=O)O-, $-C(=O)NR^1-$, $NR^1$, $P(=O)(R^1)$, -O-, -S-, SO oder $SO_2$ ersetzt sein können und wobei ein oder mehrere H-Atome durch D, F, Cl, Br, I, CN oder $NO_2$ ersetzt sein können, oder ein aromatisches oder heteroaromatisches Ringsystem mit 5 bis 40 aromatischen Ringatomen, das jeweils durch einen oder mehrere Reste $R^1$ substituiert sein kann, oder eine Aryloxy- oder Heteroaryloxygruppe mit 5 bis 40 aromatischen Ringatomen, die durch einen oder mehrere Reste $R^1$ substituiert sein kann, oder eine Kombination dieser Systeme;
$R^b$ ist eine aromatische Gruppe mit 10 bis 40 C-Atomen oder eine heteroaromatische Gruppe mit 6 bis 40 C-Atomen, wobei die aromatische und/oder heteroaromatische Gruppe mindestens zwei benachbarte aromatische und/oder heteroaromatische Ringe umfasst, die jeweils kondensiert oder nicht-kondensiert und/oder durch einen oder mehrere Reste $R^1$ substituiert sein können;
$R^1$ ist bei jedem Auftreten gleich oder verschieden H, D, F, Cl, Br, I, $B(OR^2)_2$, CHO, $C(=O)R^2$, $CR^2=C(R^2)_2$, CN, $C(=O)OR^2$, $C(=O)N(R^2)_2$, $Si(R^2)_3$, $N(R^2)_2$, $NO_2$, $P(=O)(R^2)_2$, $OSO_2R^2$, $OR^2$, $S(=O)R^2$, $S(=O)_2R^2$, eine geradkettige Alkyl-, Alkoxy- oder Thioalkoxygruppe mit 1 bis 40 C-Atomen oder eine verzweigte oder cyclische Alkyl-, Alkoxy- oder Thioalkoxygruppe mit 3 bis 40 C-Atomen, die jeweils mit einem oder mehreren Resten $R^2$ substituiert sein kann, wobei eine oder mehrere nicht benachbarte $CH_2$-Gruppen durch $-R^2C=CR^2-$, -C=C-, $Si(R^2)_2$, $Ge(R^2)_2$, $Sn(R^2)_2$, C=O, C=S, C=Se, $C=NR^2$, -C(=O)O-, $-C(=O)NR^2-$, $NR^2$, $P(=O)(R^2)$, -O-, -S-, SO

oder $SO_2$ ersetzt sein können und wobei ein oder mehrere H-Atome durch D, F, Cl, Br, I, CN oder $NO_2$ ersetzt sein können, oder ein aromatisches oder heteroaromatisches Ringsystem mit 5 bis 40 aromatischen Ringatomen, das jeweils durch einen oder mehrere Reste $R^2$ substituiert sein kann, oder eine Aryloxy- oder Heteroaryloxygruppe mit 5 bis 40 aromatischen Ringatomen, die durch einen oder mehrere Reste $R^2$ substituiert sein kann, oder eine Kombination dieser Systeme;

$R^2$ ist bei jedem Auftreten gleich oder verschieden H, D, F, Cl, Br, I, $B(OR^3)_2$, CHO, $C(=O)R^3$, $CR^3=C(R^3)_2$, CN, $C(=O)OR^3$, $C(=O)N(R^3)_2$, $Si(R^3)_3$, $N(R^3)_2$, $NO_2$, $P(=O)(R^3)_2$, $OSO_2R^3$, $OR^3$, $S(=O)R^3$, $S(=O)_2R^3$, eine geradkettige Alkyl-, Alkoxy- oder Thioalkoxygruppe mit 1 bis 40 C-Atomen oder eine verzweigte oder cyclische Alkyl-, Alkoxy- oder Thioalkoxygruppe mit 3 bis 40 C-Atomen, die jeweils mit einem oder mehreren Resten $R^3$ substituiert sein kann, wobei eine oder mehrere nicht benachbarte $CH_2$-Gruppen durch $-R^3C=CR^3-$, -C=C-, $Si(R^3)_2$, $Si(R^2)_2$, $Ge(R^3)_2$, $Sn(R^3)_2$, C=O, C=S, C=Se, $C=NR^3$, -C(=O)O-, $-C(=O)NR^3-$, $NR^3$, $P(=O)(R^3)$, -O-, -S-, SO oder $SO_2$ ersetzt sein können und wobei ein oder mehrere H-Atome durch D, F, Cl, Br, I, CN oder $NO_2$ ersetzt sein können, oder ein aromatisches oder heteroaromatisches Ringsystem mit 5 bis 40 aromatischen Ringatomen, das jeweils durch einen oder mehrere Reste $R^3$ substituiert sein kann, oder eine Aryloxy- oder Heteroaryloxygruppe mit 5 bis 40 aromatischen Ringatomen, die durch einen oder mehrere Reste $R^3$ substituiert sein kann, oder eine Kombination dieser Systeme; dabei können zwei oder mehrere benachbarte Substituenten $R^2$ auch miteinander ein mono- oder polycyclisches, aliphatisches oder aromatisches Ringsystem bilden;

$R^3$ ist bei jedem Auftreten gleich oder verschieden H, D, F oder ein aliphatischer, aromatischer und/oder heteroaromatischer Kohlenwasserstoffrest mit 1 bis 20 C-Atomen, in dem auch H-Atome durch F ersetzt sein können; dabei können zwei oder mehrere benachbarte Substituenten $R^3$ auch miteinander ein mono- oder polycyclisches, aliphatisches oder aromatisches Ringsystem bilden, wobei die folgenden Verbindungen ausgeschlossen sind:

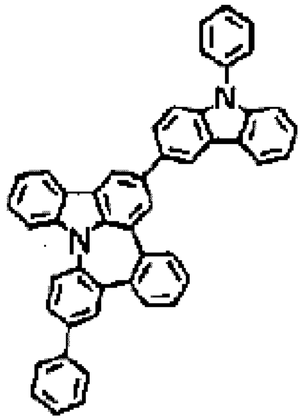
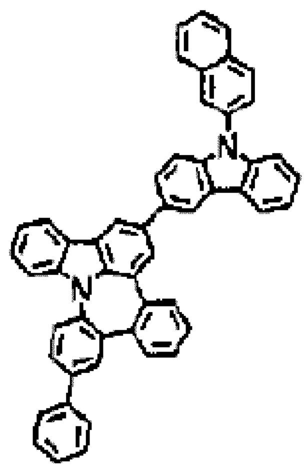
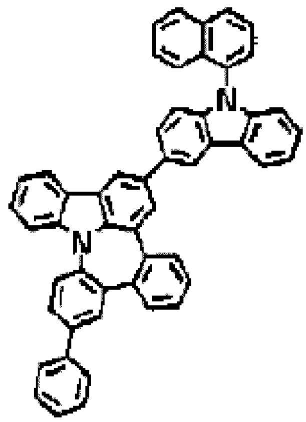
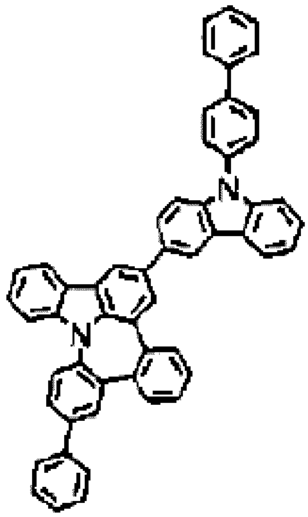
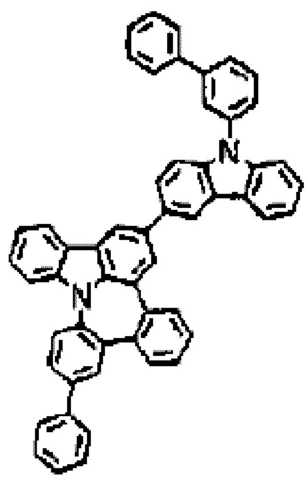
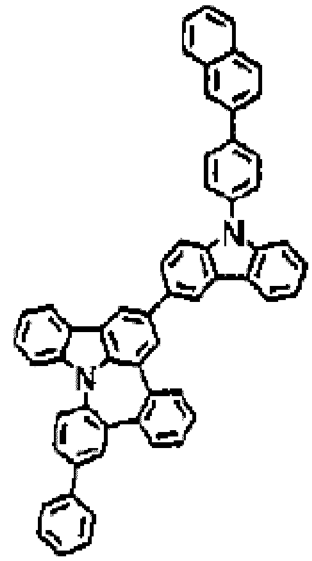

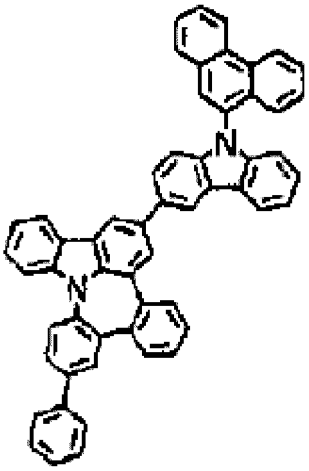

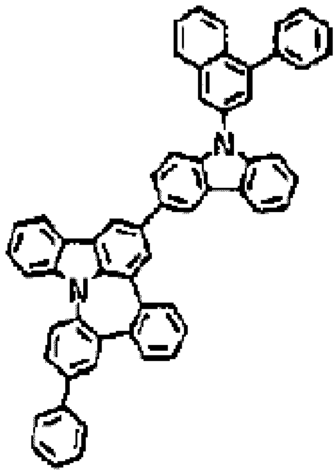

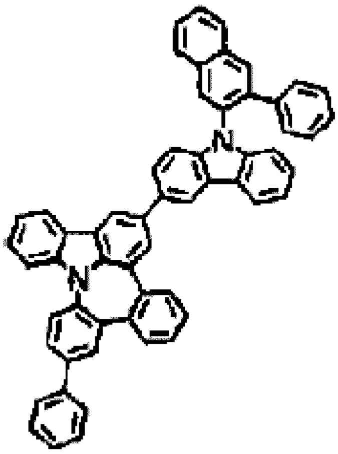

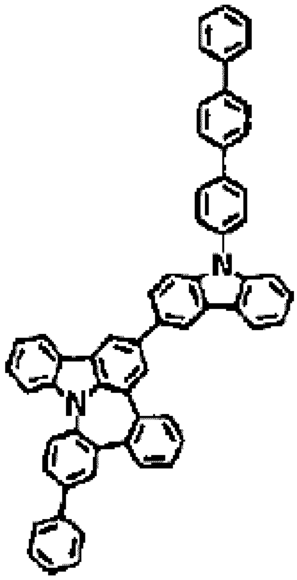

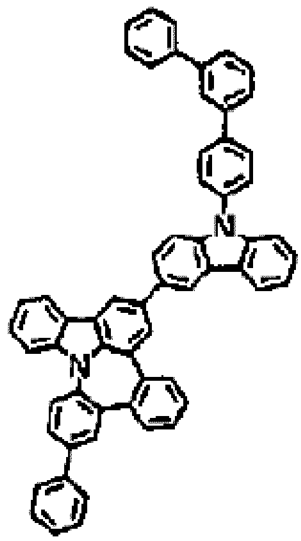

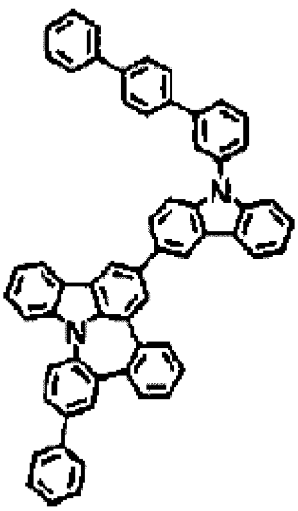

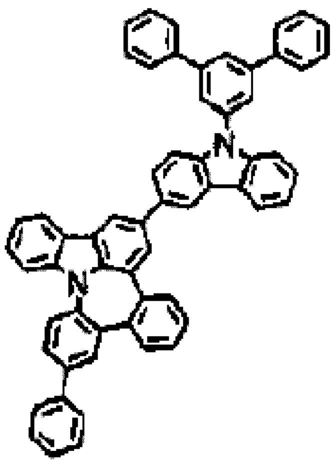

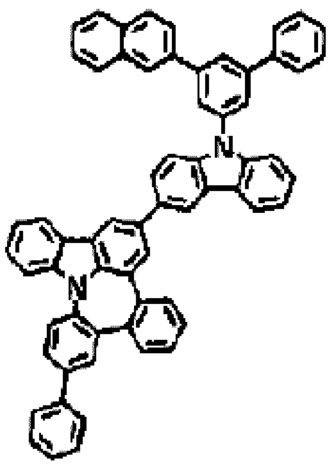

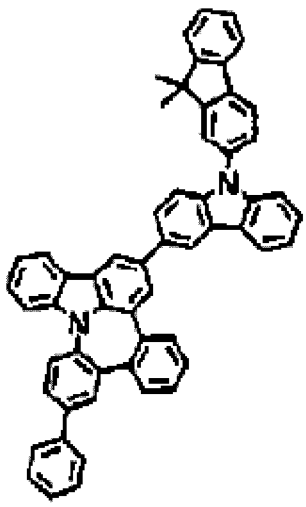

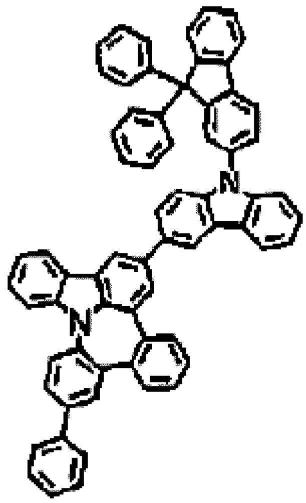

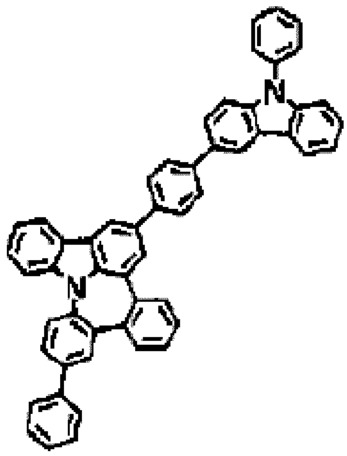

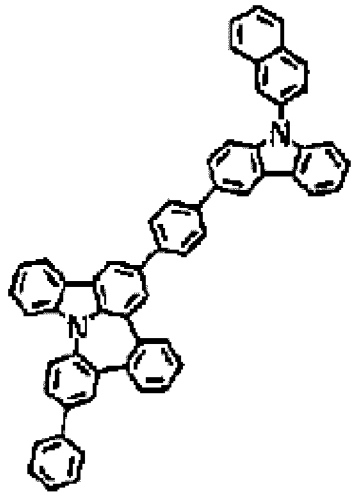

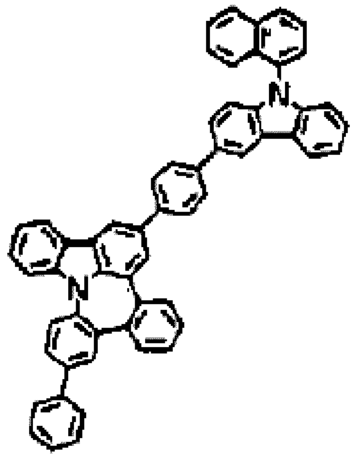

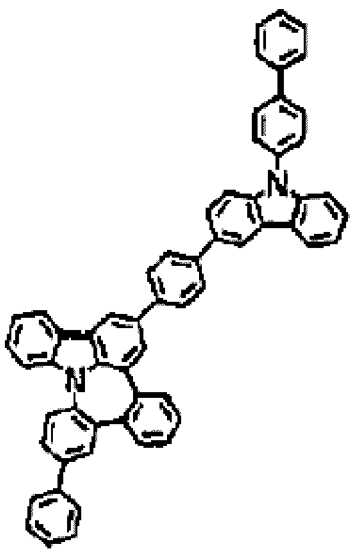

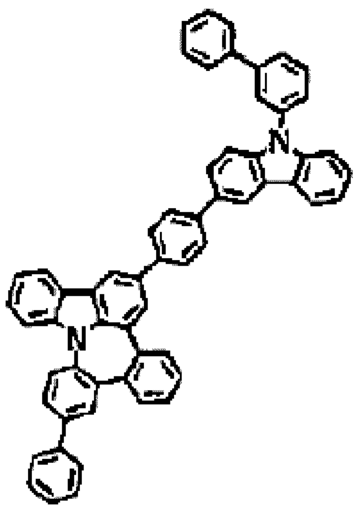

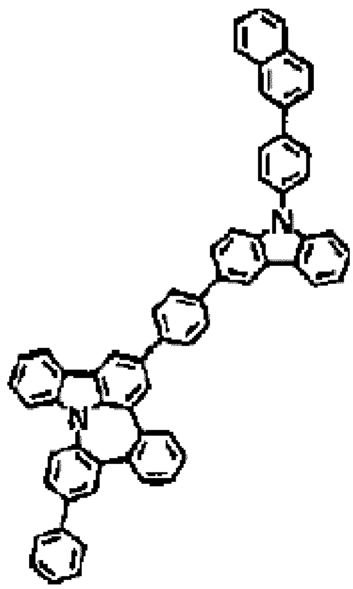

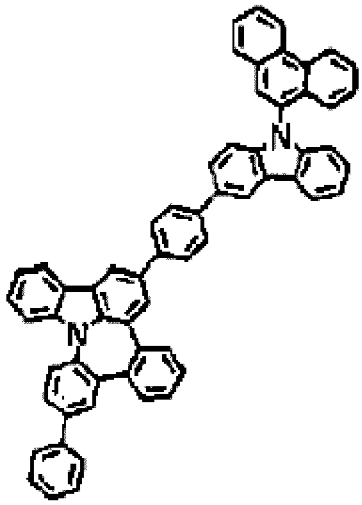

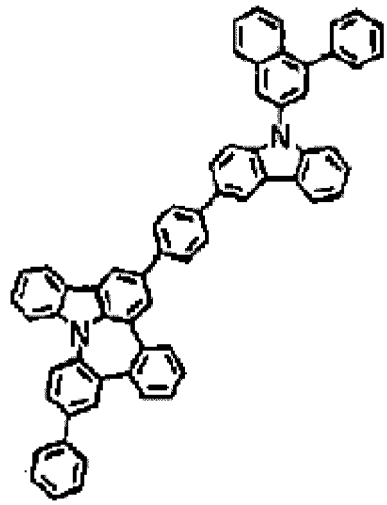

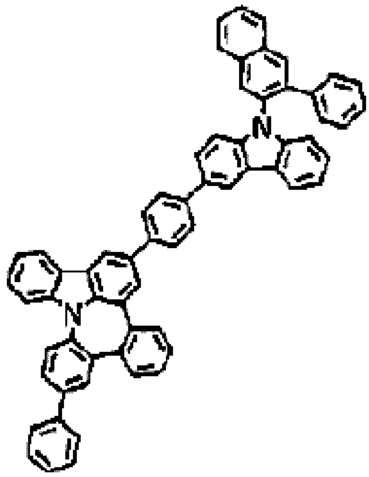

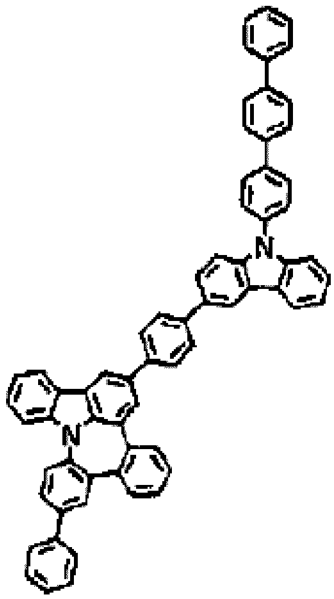

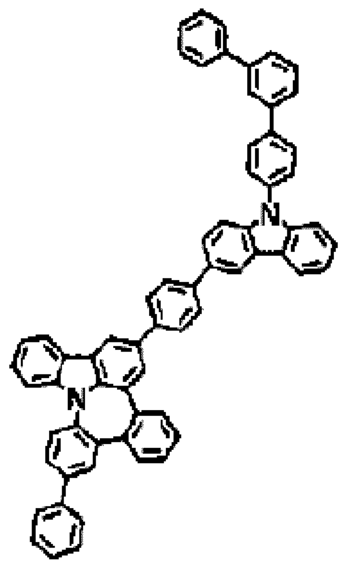

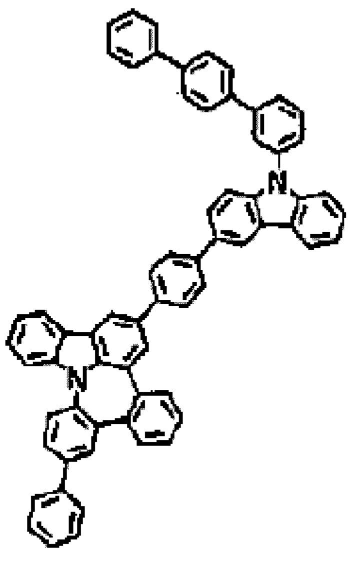

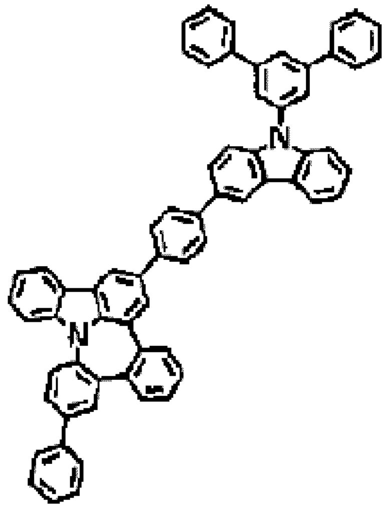

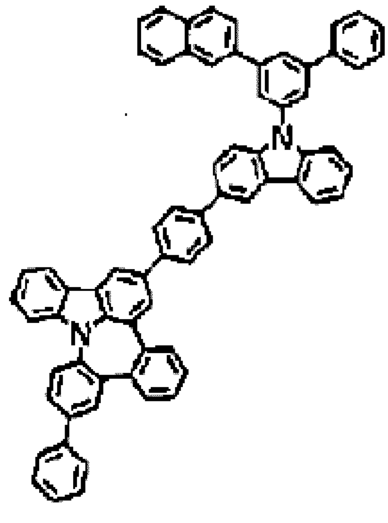

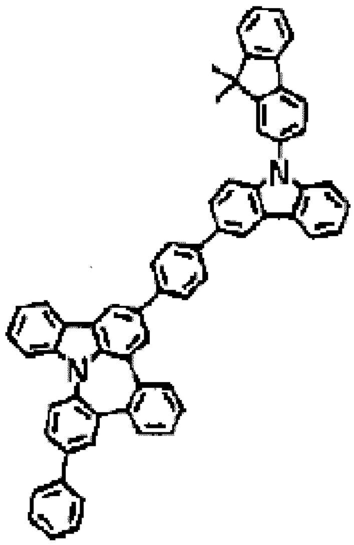 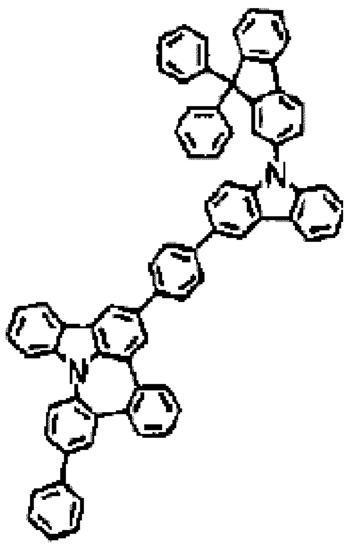

2. Verbindungen gemäß Anspruch 1, umfassend Strukturen der Formel (IV),

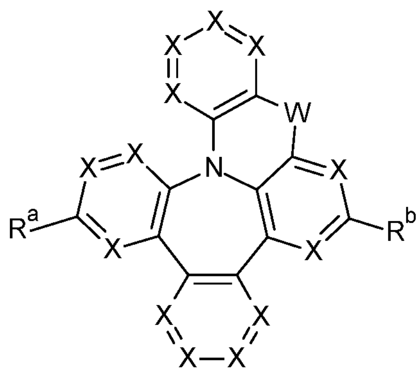

Formel (IV),

wobei die verwendeten Symbole die in Anspruch 1 genannte Bedeutung aufweisen.

3. Verbindungen gemäß mindestens einem der vorhergehenden Ansprüche, wobei mindestens einer der Reste $R^a$ und/oder $R^b$ eine Lochtransportgruppe oder eine Elektronentransportgruppe darstellt.

4. Verbindungen gemäß mindestens einem der vorhergehenden Ansprüche, wobei höchstens 4, besonders bevorzugt höchstens 3 und speziell bevorzugt höchstens 2 der Gruppen $CR^1$ für die X steht, ungleich der Gruppe CH ist.

5. Verbindungen gemäß mindestens einem der vorhergehenden Ansprüche, umfassend Strukturen der Formel (V),

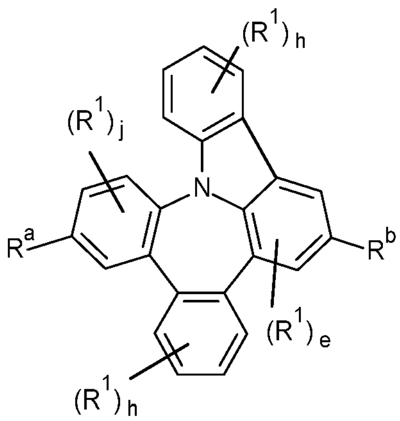

Formel (V),

worin $R^a$, $R^b$ und $R^1$ die in Anspruch 1 dargelegten Bedeutungen haben, e 0, 1 oder 2 ist, j 0, 1, 2 oder 3 ist, h 0, 1, 2, 3 oder 4 ist, wobei mindestens einer der Reste $R^a$, $R^b$ eine Lochtransportgruppe und/oder eine Elektronentransportgruppe darstellt.

**6.** Verbindungen gemäß mindestens einem der vorhergehenden Ansprüche, umfassend Strukturen der Formel (VI),

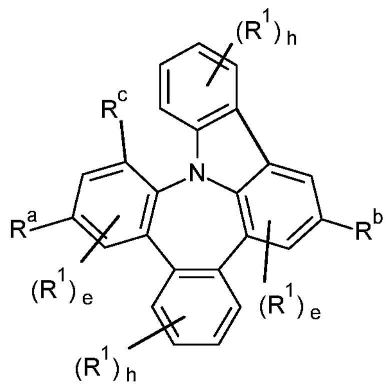

Formel (VI),

worin $R^a$, $R^b$ und $R^1$ die in Anspruch 1 dargelegten Bedeutungen haben, e 0, 1 oder 2 ist, h 0, 1, 2, 3 oder 4 ist, wobei der Rest $R^b$ eine Lochtransportgruppe oder eine Elektronentransportgruppe darstellt und der Rest $R^c$ eine aromatische Gruppe mit 10 bis 40 C-Atomen, oder eine heteroaromatische Gruppe mit 6 bis 40 C-Atomen ist, wobei die aromatische und/oder heteroaromatische Gruppe mindestens zwei benachbarte aromatische und/oder heteroaromatische Ringe umfasst, die jeweils kondensiert oder nicht-kondensiert und/oder durch einen oder mehrere Reste $R^1$ substituiert sein können.

**7.** Verbindungen gemäß mindestens einem der vorhergehenden Ansprüche, umfassend Strukturen der Formel (VII),

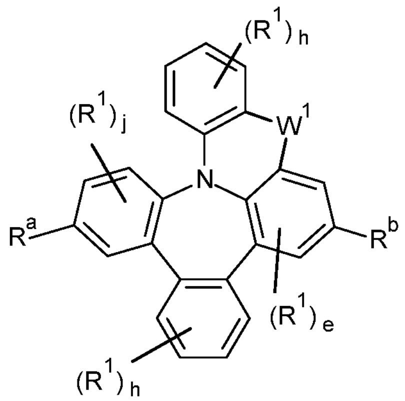

Formel (VII),

worin $R^a$, $R^b$ und $R^1$ die in Anspruch 1 dargelegten Bedeutungen haben, e 0, 1 oder 2 ist, j 0, 1, 2 oder 3 ist, h 0, 1, 2, 3 oder 4 ist, wobei mindestens einer der Reste $R^a$, $R^b$ eine Lochtransportgruppe und/oder eine Elektronentransportgruppe darstellt und $W^1$ $NR^1$, $C(R^1)_2$, O oder S ist.

**8.** Verbindungen gemäß mindestens einem der vorhergehenden Ansprüche, umfassend Strukturen der Formel (VIII),

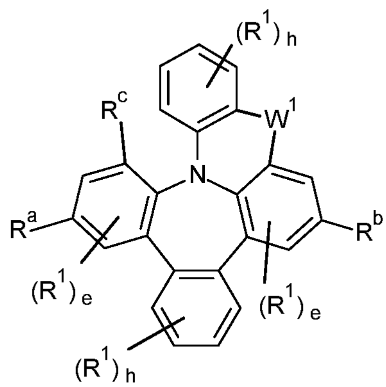

Formel (VIII),

worin $R^a$, $R^b$ und $R^1$ die in Anspruch 1 dargelegten Bedeutungen haben, e 0, 1 oder 2 ist, h 0, 1, 2, 3 oder 4 ist, wobei der Rest $R^b$ eine Lochtransportgruppe oder eine Elektronentransportgruppe darstellt und der Rest $R^c$ eine Arylgruppe mit 10 bis 40 C-Atomen, welche mindestens zwei Ringe umfasst, oder eine Heteroarylgruppe mit 6 bis 40 C-Atomen darstellt, welche mindestens zwei Ringe umfasst, wobei die jeweilige Gruppe jeweils durch einen oder mehrere Reste $R^1$ substituiert sein kann und $W^1$ $NR^1$, $C(R^1)_2$, O oder S ist.

**9.** Verbindungen gemäß mindestens einer der vorhergehenden Ansprüche, wobei der Rest $R^b$ in einer der Formeln (II), (IV), (V), (VI), (VII) und/oder (VIII) eine Lochtransportgruppe und der Rest $R^a$ in einer der Formeln (II), (IV), (V), (VI), (VII) und/oder (VIII) eine Lochtransportgruppe ist.

**10.** Verbindungen gemäß mindestens einer der vorhergehenden Ansprüche 1 bis 8, wobei der Rest $R^b$ in einer der Formeln (II), (IV), (V), (VI), (VII) und/oder (VIII) eine Elektronentransportgruppe und der Rest $R^a$ in einer der Formeln (II), (IV), (V), (VI), (VII) und/oder (VIII) eine Lochtransportgruppe ist.

**11.** Verbindungen gemäß mindestens einer der vorhergehenden Ansprüche 1 bis 8, wobei der Rest $R^b$ in einer der Formeln (II), (IV), (V), (VI), (VII) und/oder (VIII) eine Lochtransportgruppe und der Rest $R^a$ in einer der Formeln (II), (IV), (V), (VI), (VII) und/oder (VIII) eine Elektronentransportgruppe ist.

**12.** Verbindungen gemäß mindestens einer der vorhergehenden Ansprüche 1 bis 8, wobei der Rest $R^b$ in einer der

Formeln (II), (IV), (V), (VI), (VII) und/oder (VIII) eine Elektronentransportgruppe und der Rest $R^a$ in einer der Formeln (II), (IV), (V), (VI), (VII) und/oder (VIII) eine Elektronentransportgruppe ist.

**13.** Verbindungen gemäß mindestens einem der vorhergehenden Ansprüche, wobei in Strukturen gemäß Formel (II), (IV), (V), (VI), (VII) und/oder (VIII) mindestens ein Rest $R^1$, $R^a$, $R^b$ und/oder $R^c$ für eine Gruppe steht, die ausgewählt ist aus den Formeln ($R^1$-1) bis ($R^1$- 72)

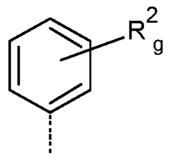

Formel ($R^1$-1)

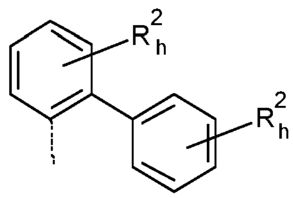

Formel ($R^1$-2)

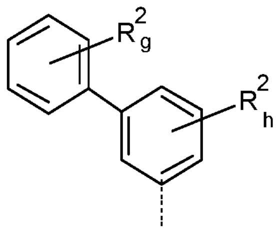

Formel ($R^1$-3)

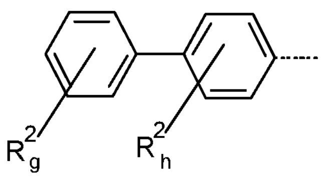

Formel ($R^1$-4)

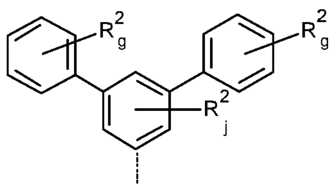

Formel ($R^1$-5)

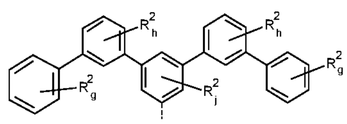

Formel ($R^1$-6)

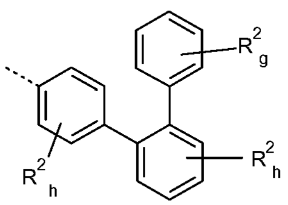

Formel ($R^1$-7)

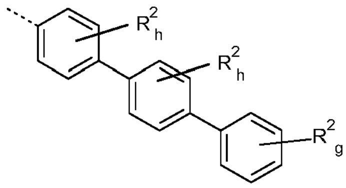

Formel ($R^1$-8)

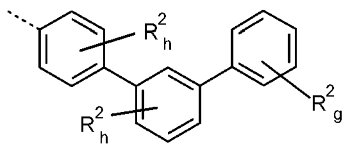

Formel ($R^1$-9)

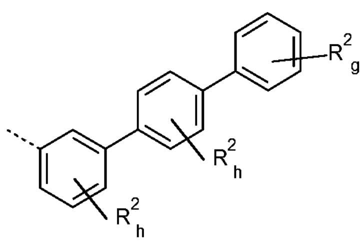

Formel ($R^1$-10)

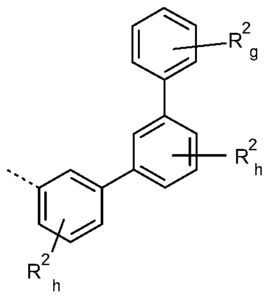

Formel ($R^1$-11)

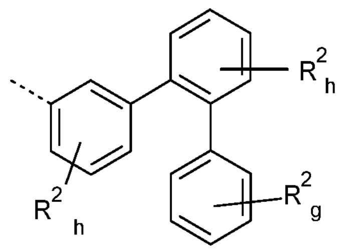

Formel ($R^1$-12)

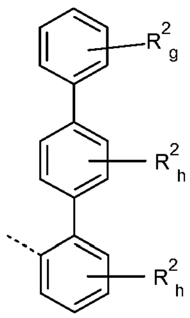

Formel ($R^1$-13)

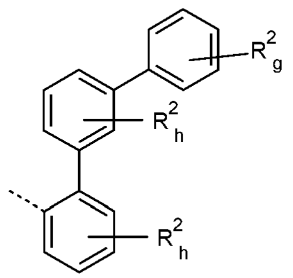

Formel ($R^1$-14)

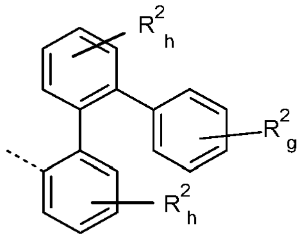

Formel ($R^1$-15)

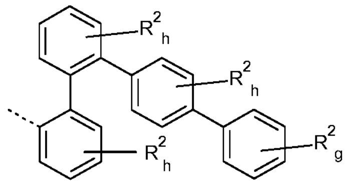

Formel ($R^1$-16)

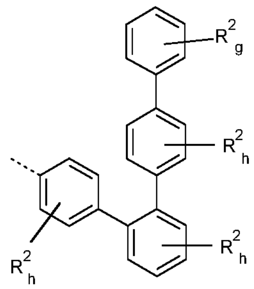

Formel ($R^1$-17)

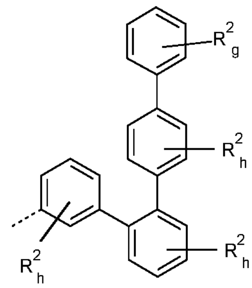

Formel ($R^1$-18)

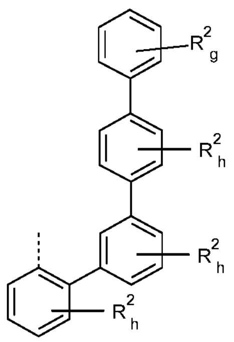

Formel ($R^1$-19)

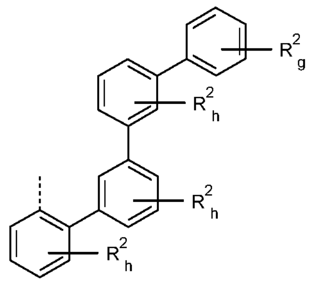

Formel ($R^1$-20)

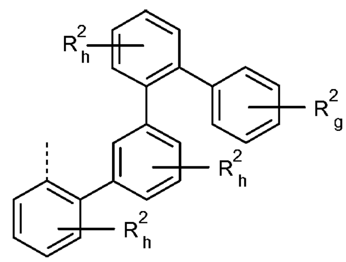

Formel ($R^1$-21)

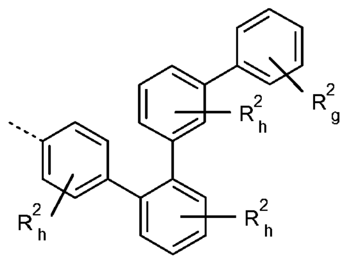

Formel ($R^1$-22)

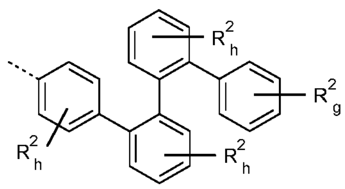

Formel ($R^1$-23)

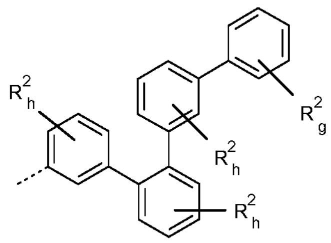

Formel ($R^1$-24)

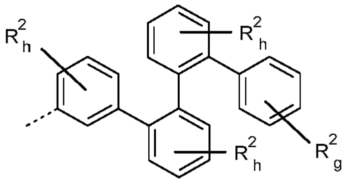

Formel ($R^1$-25)

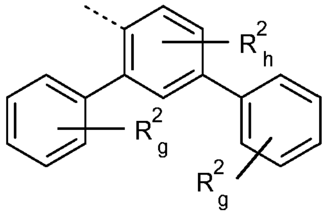

Formel ($R^1$-26)

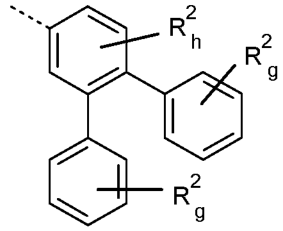

Formel ($R^1$-27)

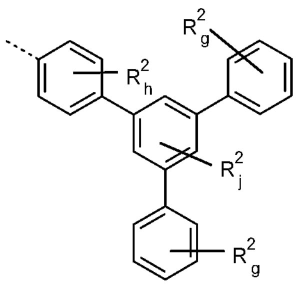

Formel ($R^1$-28)

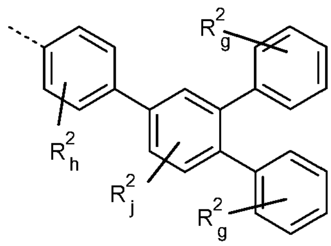

Formel ($R^1$-29)

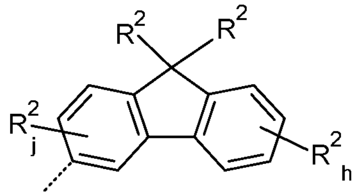

Formel ($R^1$-30)

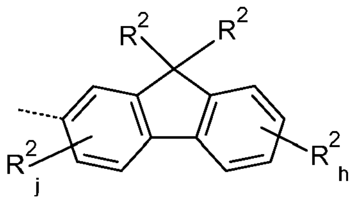

Formel ($R^1$-31)

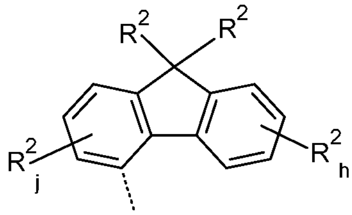

Formel ($R^1$-32)

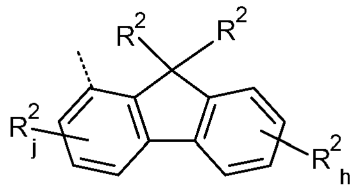

Formel ($R^1$-33)

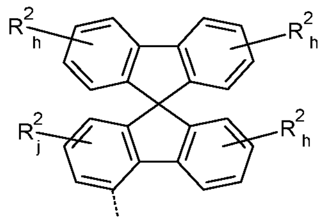

Formel ($R^1$-34)

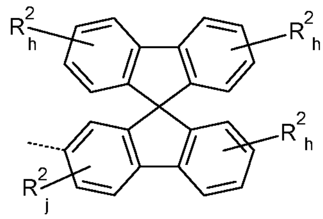

Formel ($R^1$-35)

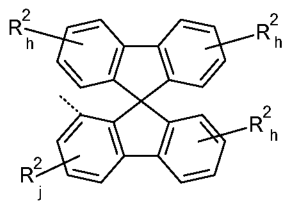

Formel ($R^1$-36)

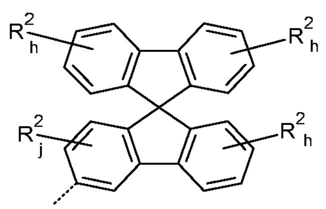

Formel ($R^1$-37)

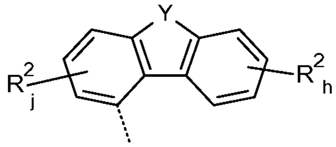

Formel ($R^1$-38)

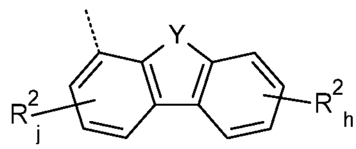

Formel ($R^1$-39)

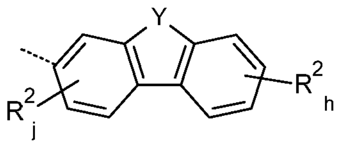
Formel ($R^1$-40)

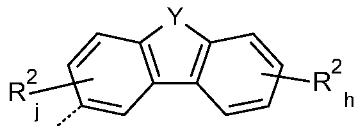
Formel ($R^1$-41)

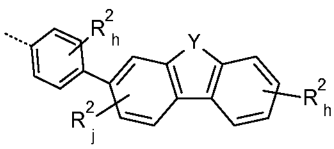
Formel ($R^1$-42)

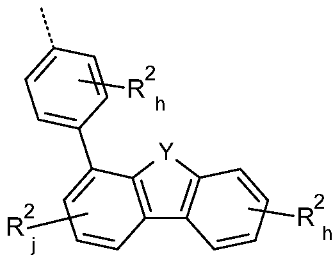
Formel ($R^1$-43)

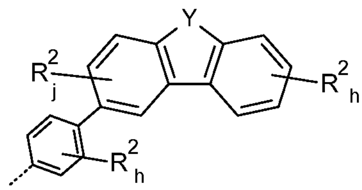
Formel ($R^1$-44)

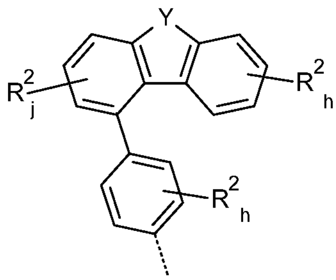
Formel ($R^1$-45)

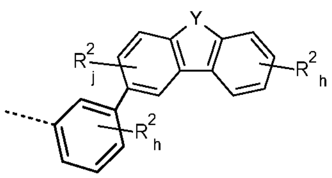
Formel ($R^1$-46)

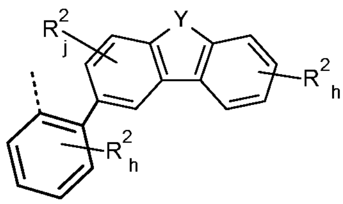
Formel ($R^1$-47)

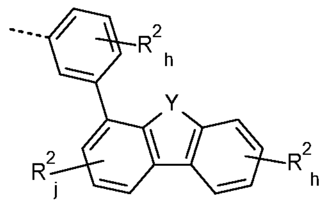
Formel ($R^1$-48)

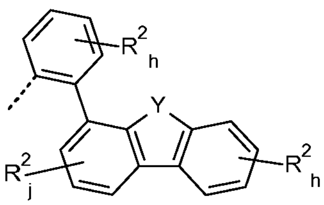
Formel ($R^1$-49)

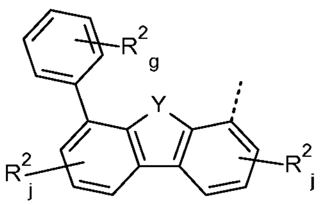
Formel ($R^1$-50)

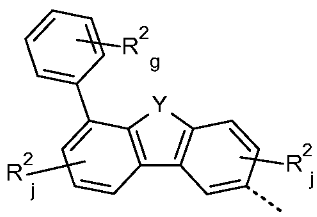
Formel ($R^1$-51)

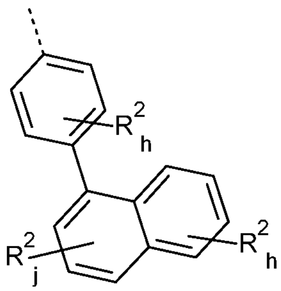
Formel ($R^1$-52)

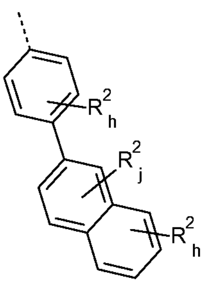
Formel ($R^1$-53)

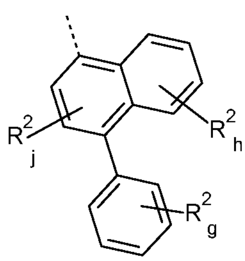
Formel ($R^1$-54)

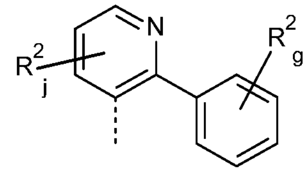
Formel ($R^1$-55)

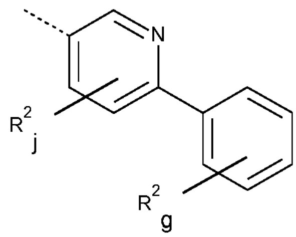
Formel ($R^1$-56)

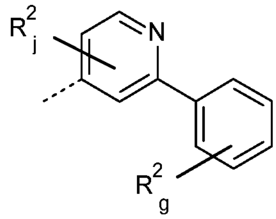
Formel ($R^1$-57)

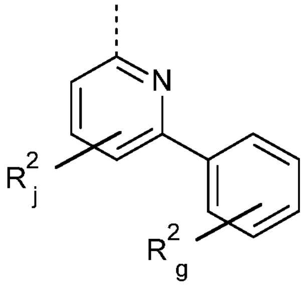
Formel ($R^1$-58)

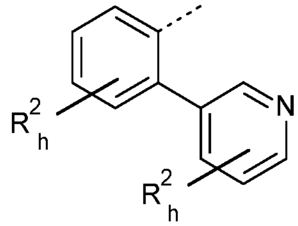
Formel ($R^1$-59)

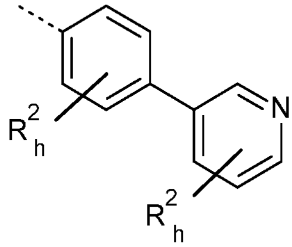
Formel ($R^1$-60)

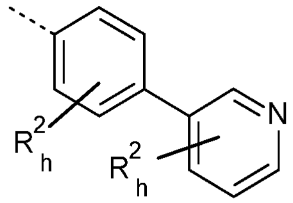
Formel ($R^1$-61)

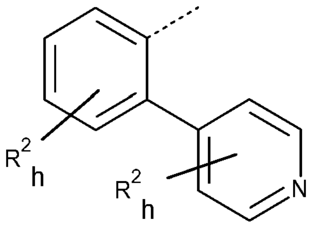
Formel ($R^1$-62)

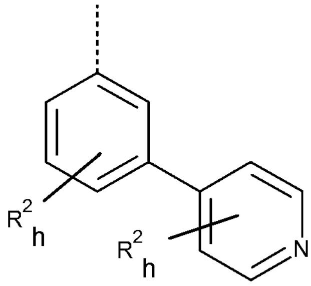
Formel ($R^1$-63)

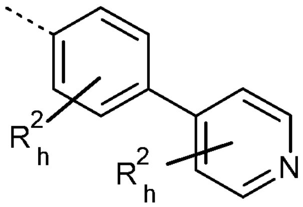
Formel ($R^1$-64)

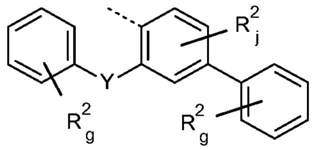
Formel ($R^1$-65)

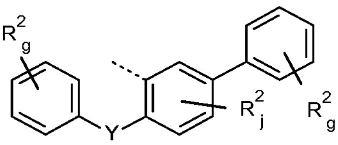
Formel ($R^1$-66)

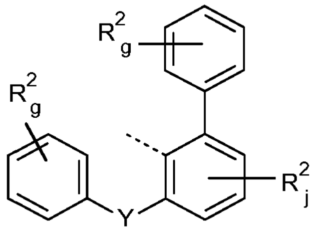
Formel ($R^1$-67)

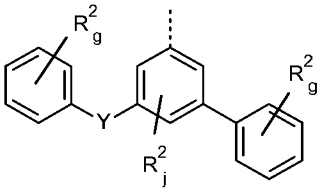
Formel ($R^1$-68)

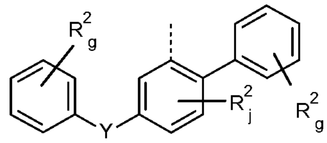
Formel ($R^1$-69)

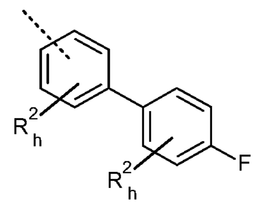

Formel ($R^1$-70)

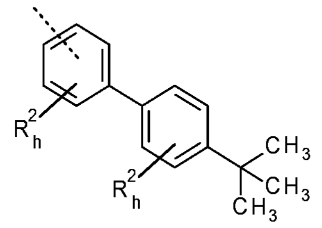

Formel ($R^1$-71)

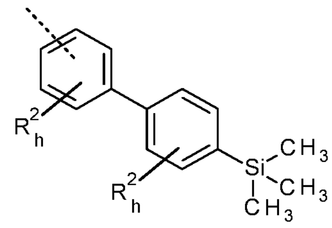

Formel ($R^1$-72),

wobei für die verwendeten Symbole gilt:

Y ist O, S oder $NR^2$, vorzugsweise O oder S;
j ist bei jedem Auftreten unabhängig 0, 1, 2 oder 3;
h ist bei jedem Auftreten unabhängig 0, 1, 2, 3 oder 4;
g ist bei jedem Auftreten unabhängig 0, 1, 2, 3, 4 oder 5: die gestrichelte Bindung markiert die Anbindungsposition; und
$R^2$ weist die in Anspruch 1 genannte Bedeutung auf.

**14.** Verbindungen gemäß Anspruch 13, wobei die Summe der Indices g, h und j in den Strukturen der Formel ($R^1$-1) bis ($R^1$-72) jeweils höchstens 3, vorzugsweise höchstens 2 und besonders bevorzugt höchstens 1 beträgt.

**15.** Verbindungen gemäß mindestens einem der vorhergehenden Ansprüche 3, 5 bis 8 und 10 bis 12, wobei die Elektronentransportgruppe mindestens eine Struktur gemäß den Formeln (E-1), (E-5) bis (E-10)

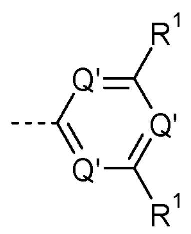

Formel (E-1)

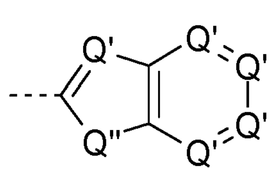

Formel (E-5)

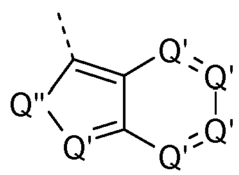

Formel (E-6)

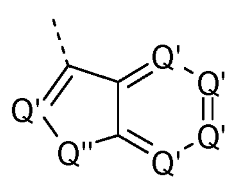

Formel (E-7)

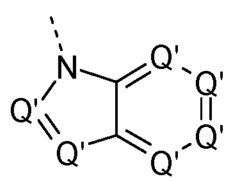

Formel (E-8)

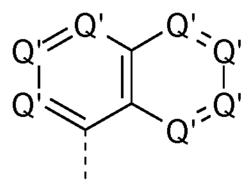

Formel (E-9)

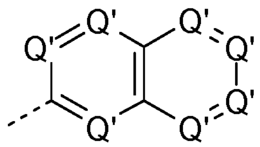

Formel (E-10),

aufweist, wobei die gestrichelte Bindung die Anbindungsposition markiert,

Q‘ bei jedem Auftreten gleich oder verschieden $CR^1$ oder N darstellt, und
Q" $NR^1$, O oder S darstellt;

wobei wenigstens ein Q’ gleich N und/oder wenigstens ein Q" gleich $NR^1$ ist und

$R^1$ wie in Anspruch 1 definiert ist.

**16.** Verbindungen gemäß mindestens einem der vorhergehenden Ansprüche 3, 5 bis 8 und 10 bis 12, wobei die Elektronentransportgruppe mindestens eine Struktur gemäß den Formeln (E-11) bis (E-23)

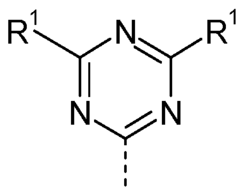

Formel (E-11)

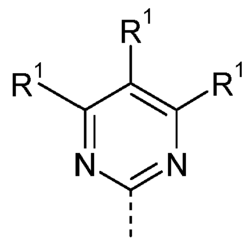

Formel (E-12)

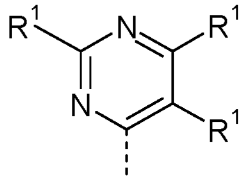

Formel (E-13)

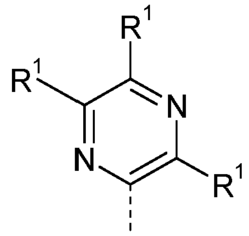

Formel (E-14)

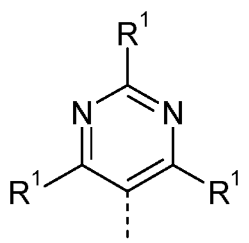

Formel (E-15)

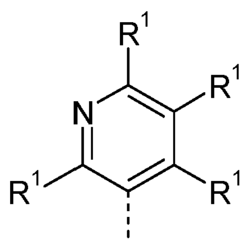

Formel (E-16)

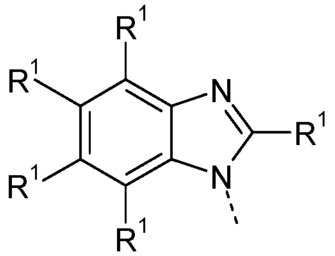

Formel (E-17)

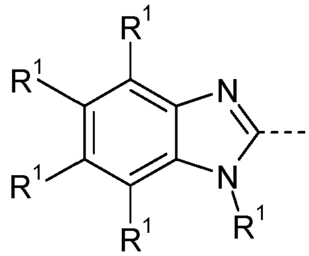

Formel (E-18)

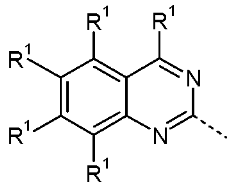

Formel (E-19)

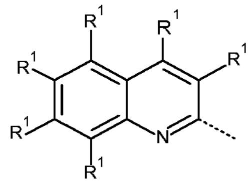

Formel (E-20)

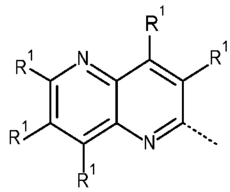

Formel (E-21)

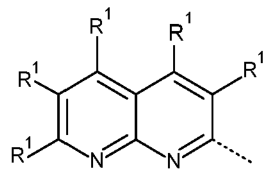

Formel (E-22)

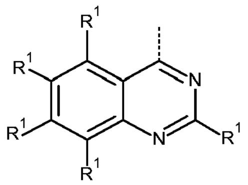

Formel (E-23)

aufweist, wobei die gestrichelte Bindung die Anbindungsposition markiert und $R^1$ die in Anspruch 1 genannte Bedeutung aufweist.

**17.** Verbindungen gemäß mindestens einem der vorhergehenden Ansprüche 3 und 5 bis 11, wobei die Lochtransportgruppe mindestens eine Struktur umfasst, die aus der Gruppe Carbazole, Indenocarbazole und Indolocarbazole ausgewählt ist.

**18.** Verbindungen gemäß mindestens einem der vorhergehenden Ansprüche 3 und 5 bis 11, wobei die Lochtransportgruppe mindestens eine Struktur gemäß den Formeln (L-2) bis (L-9)

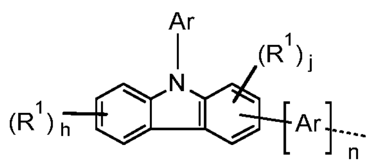

Formel (L-2)

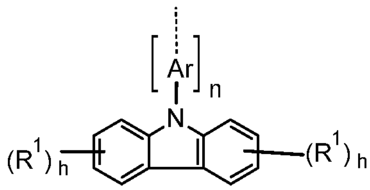

Formel (L-3)

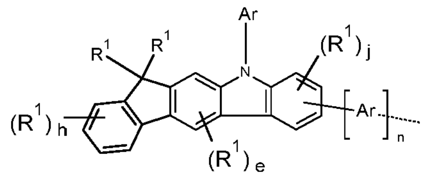

Formel (L-4)

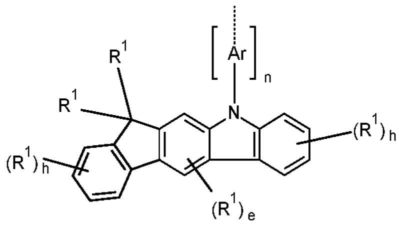

Formel (L-5)

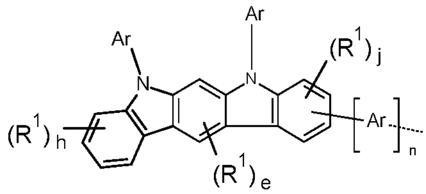

Formel (L-6)

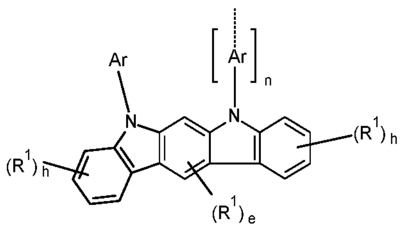

Formel (L-7)

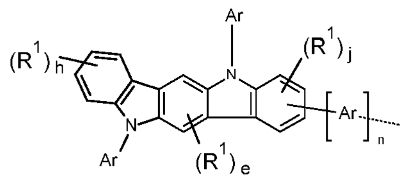

Formel (L-8)

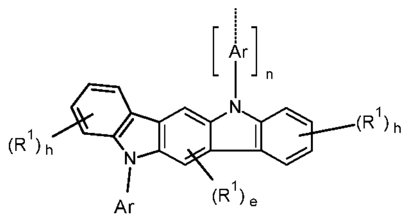

Formel (L-9)

aufweist, wobei die gestrichelte Bindung die Anbindungsposition markiert, e 0, 1 oder 2 ist, j 0, 1, 2 oder 3 ist, h 0, 1, 2, 3 oder 4 ist, n 0 oder 1 ist, Ar eine Arylgruppe mit 6 bis 40 C-Atomen oder eine Heteroarylgruppe mit 3 bis 40 C-Atomen darstellt, die durch einen oder mehrere Reste $R^1$ substituiert sein kann, und $R^1$ die in Anspruch 1 genannte Bedeutung aufweist.

**19.** Verbindungen gemäß mindestens einem der vorhergehenden Ansprüche ausgewählt aus der Gruppe der folgenden Verbindungen:

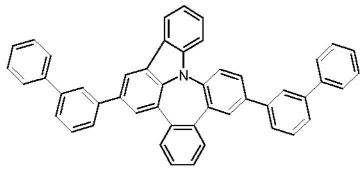

Formel 1

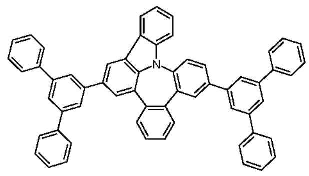

Formel 2

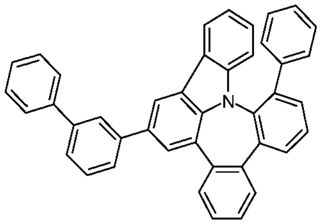

Formel 4

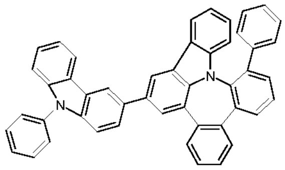

Formel 5

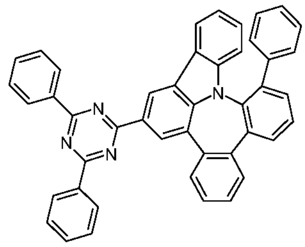

Formel 6

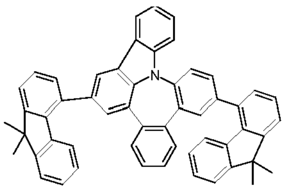

Formel 7

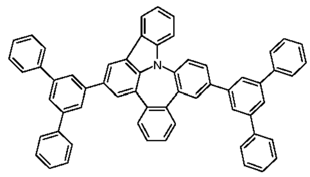

Formel 8

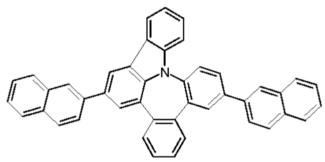

Formel 10

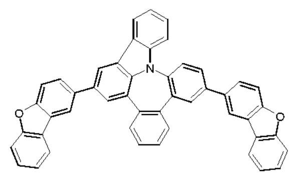

Formel 14

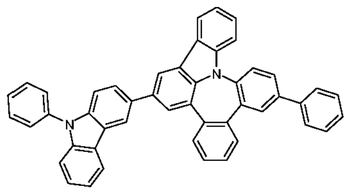

Formel 15

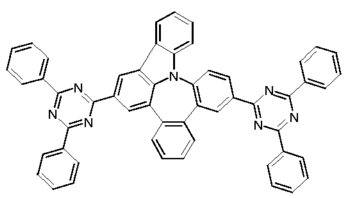

Formel 16

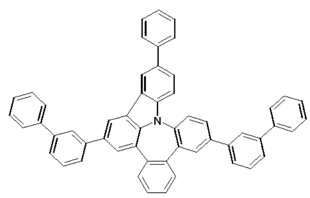

Formel 17

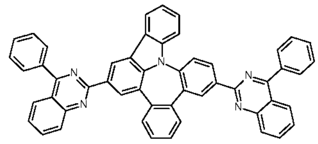

Formel 19

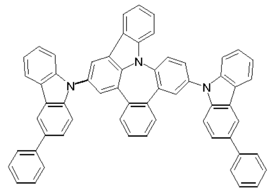

Formel 20

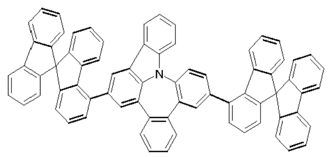

Formel 22

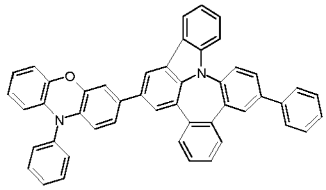

Formel 24

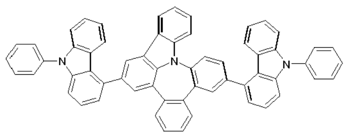

Formel 27

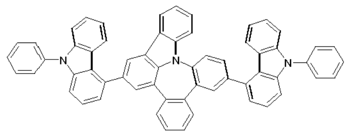

Formel 28

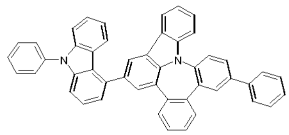

Formel 29

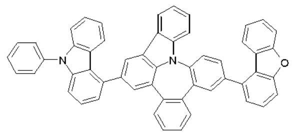

Formel 30

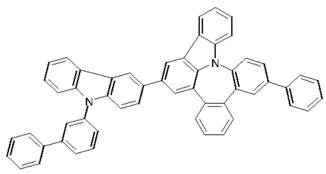

Formel 34

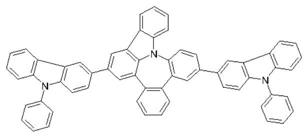

Formel 36

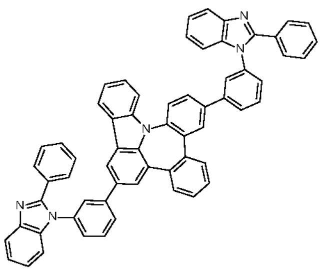

Formel 43

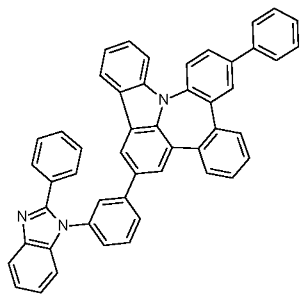

Formel 44

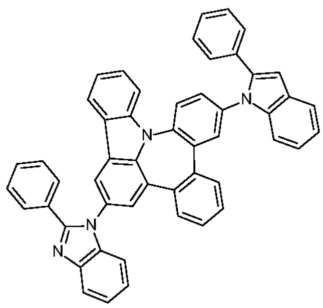

Formel 45

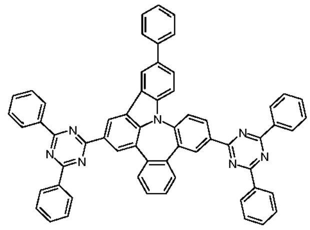

Formel 46

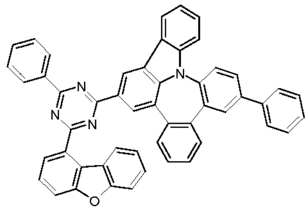

Formel 49

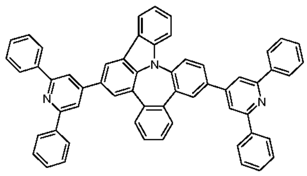

Formel 50

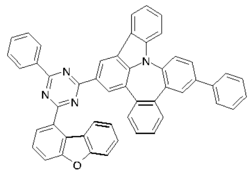

Formel 51

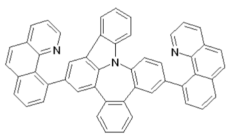

Formel 52

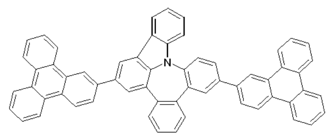

Formel 53

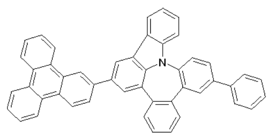

Formel 54

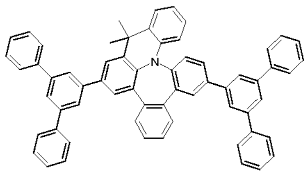

Formel 55

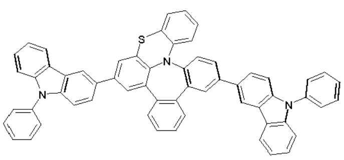

Formel 57

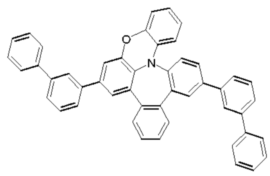

Formel 58

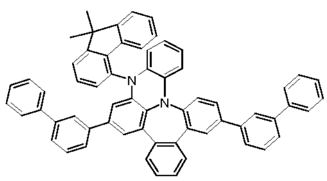

Formel 62

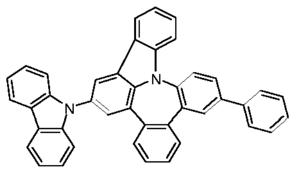

Formel 72

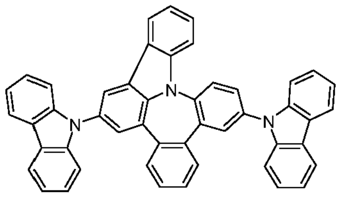

Formel 73

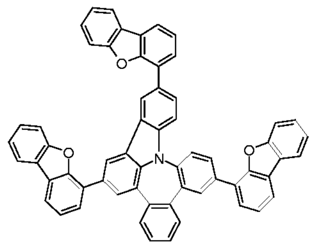

Formel 75

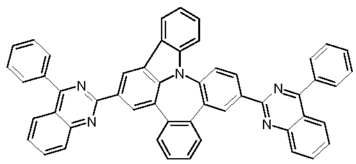

Formel 76.

**20.** Oligomer, Polymer oder Dendrimer enthaltend eine oder mehrere Verbindungen nach einem oder mehreren der Ansprüche 1 bis 19, wobei ein oder mehrere Bindungen der Verbindung zum Polymer, Oligomer oder Dendrimer vorhanden sind.

**21.** Zusammensetzung enthaltend wenigstens eine Verbindung nach einem oder mehreren der Ansprüche 1 bis 19 und/oder ein Oligomer, Polymer oder Dendrimer nach Anspruch 20 und wenigstens eine weitere Verbindung ausgewählt aus der Gruppe bestehend aus fluoreszierenden Emittern, phosphoreszierenden Emittern, Host Materialien, Matrix-Materialien, Elektronentransportmaterialien, Elektroneninjektionsmaterialien, Lochleitermaterialien, Lochinjektionsmaterialien, Elektronenblockiermaterialien und Lochblockiermaterialien.

**22.** Formulierung enthaltend mindestens eine Verbindung nach einem oder mehreren der Ansprüche 1 bis 19, ein Oligomer, Polymer oder Dendrimer nach Anspruch 20 und/oder wenigstens eine Zusammensetzung nach Anspruch 21 und mindestens ein Lösungsmittel.

**23.** Verfahren zur Herstellung einer Verbindung nach einem oder mehreren der Ansprüche 1 bis 19 oder eines Oligomers, Polymers oder Dendrimers nach Anspruch 20, wobei an einer Verbindung mit einem Azepinstrukturelement eine Ringschlussreaktion durchgeführt wird.

**24.** Verwendung einer Verbindung nach einem oder mehreren der Ansprüche 1 bis 19, eines Oligomers, Polymers oder Dendrimers nach Anspruch 20 oder einer Zusammensetzung nach Anspruch 21 in einer elektronischen Vorrichtung als Elektronenblockiermaterial, Lochinjektionsmaterial und/oder Lochtransportmaterial.

**25.** Elektronische Vorrichtung, enthaltend mindestens eine Verbindung nach einem oder mehreren der Ansprüche 1 bis 19, ein Oligomer, Polymer oder Dendrimer nach Anspruch 20 oder eine Zusammensetzung gemäß Anspruch 21.

**26.** Elektronische Vorrichtung nach Anspruch 25, wobei die elektronische Vorrichtung ausgewählt ist aus der Gruppe bestehend aus organischen Elektrolumineszenzvorrichtungen, organischen integrierten Schaltungen, organischen Feld-Effekt-Transistoren, organischen Dünnfilmtransistoren, organischen lichtemittierenden Transistoren, organischen Solarzellen, organischen optischen Detektoren, organischen Photorezeptoren, organischen Feld-Quench-Devices, lichtemittierenden elektrochemischen Zellen oder organischen Laser-dioden.

## Claims

1. Compound comprising structures of the formula (II),

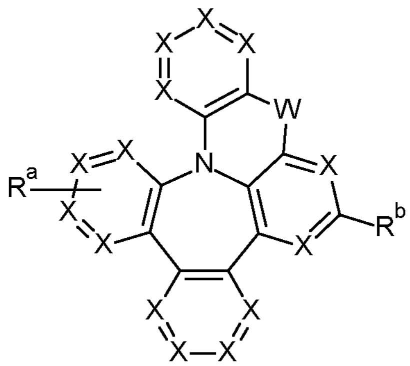

formula (II),

where the following applies to the symbols used:

X is on each occurrence, identically or differently, $CR^1$ or C for the bonding site of the radical $R^a$;
W is a bond, $NR^1$, $C(R^1)_2$, O or S;
$R^a$ is D, F, Cl, Br, I, $B(OR^1)_2$, CHO, $C(=O)R^1$, $CR^1=C(R^1)_2$, CN, $C(=O)OR^1$, $C(=O)N(R^1)_2$, $Si(R^1)_3$, $N(R^1)_2$, $NO_2$, $P(=O)(R^1)_2$, $OSO_2R^1$, $OR^1$, $S(=O)R^1$, $S(=O)_2R^1$, a straight-chain alkyl, alkoxy or thioalkoxy group having 1 to 40 C atoms or a branched or cyclic alkyl, alkoxy or thioalkoxy group having 3 to 40 C atoms, which may in each case be substituted by one or more radicals $R^1$, where one or more non-adjacent $CH_2$ groups may be replaced by $-R^1C=CR^1-$, -C=C-, $Si(R^1)_2$, C=O, C=S, $C=NR^1$, -C(=O)O-, $-C(=O)NR^1-$, $NR^1$, $P(=O)(R^1)$, -O-, -S-, SO or $SO_2$ and where one or more H atoms may be replaced by D, F, Cl, Br, I, CN or $NO_2$, or an aromatic or heteroaromatic ring system having 5 to 40 aromatic ring atoms, which may in each case be substituted by one or more radicals $R^1$, or an aryloxy or heteroaryloxy group having 5 to 40 aromatic ring atoms, which may be substituted by one or more radicals $R^1$, or a combination of these systems;
$R^b$ is an aromatic group having 10 to 40 C atoms or a hetero-aromatic group having 6 to 40 C atoms, where the aromatic and/or heteroaromatic group comprises at least two adjacent aromatic or heteroaromatic rings, which may in each case be condensed or non-condensed and/or substituted by one or more radicals $R^1$;
$R^1$ is on each occurrence, identically or differently, H, D, F, Cl, Br, I, $B(OR^2)_2$, CHO, $C(=O)R^2$, $CR^2=C(R^2)_2$, CN, $C(=O)OR^2$, $C(=O)N(R^2)_2$, $Si(R^2)_3$, $N(R^2)_2$, $NO_2$, $P(=O)(R^2)_2$, $OSO_2R^2$, $OR^2$, $S(=O)R^2$, $S(=O)_2R^2$, a straight-chain alkyl, alkoxy or thioalkoxy group having 1 to 40 C atoms or a branched or cyclic alkyl, alkoxy or thioalkoxy group having 3 to 40 C atoms, which may in each case be substituted by one or more radicals $R^2$, where one or more non-adjacent $CH_2$ groups may be replaced by $-R^2C=CR^2-$, $-C\equiv C-$, $Si(R^2)_2$, $Ge(R^2)_2$, $Sn(R^2)_2$, C=O, C=S, C=Se, $C=NR^2$, -C(=O)O-, $-C(=O)NR^2-$, $NR^2$, $P(=O)(R^2)$, -O-, -S-, SO or $SO_2$ and where one or more H atoms may be replaced by D, F, Cl, Br, I, CN or $NO_2$, or an aromatic or heteroaromatic ring system having 5 to 40 aromatic ring atoms, which may in each case be substituted by one or more radicals $R^2$, or an aryloxy or heteroaryloxy group having 5 to 40 aromatic ring atoms, which may be substituted by one or more radicals $R^2$, or a combination of these systems;
$R^2$ is on each occurrence, identically or differently, H, D, F, Cl, Br, I, $B(OR^3)_2$, CHO, $C(=O)R^3$, $CR^3=C(R^3)_2$, CN, $C(=O)OR^3$, $C(=O)N(R^3)_2$, $Si(R^3)_3$, $N(R^3)_2$, $NO_2$, $P(=O)(R^3)_2$, $OSO_2R^3$, $OR^3$, $S(=O)R^3$, $S(=O)_2R^3$, a straight-chain alkyl, alkoxy or thioalkoxy group having 1 to 40 C atoms or a branched or cyclic alkyl, alkoxy or thioalkoxy group having 3 to 40 C atoms, which may in each case be substituted by one or more radicals $R^3$, where one or more non-adjacent $CH_2$ groups may be replaced by $-R^3C=CR^3-$, -C=C-, $Si(R^3)_2$, $Si(R^2)_2$, $Ge(R^3)_2$, $Sn(R^3)_2$, C=O, C=S, C=Se, $C=NR^3$, -C(=O)O-, $-C(=O)NR^3-$, $NR^3$, $P(=O)(R^3)$, -O-, -S-, SO or $SO_2$ and where one or more H atoms may be replaced by D, F, Cl, Br, I, CN or $NO_2$, or an aromatic or hetero-aromatic ring system having 5 to 40 aromatic ring atoms, which may in each case be substituted by one or more radicals $R^3$, or an aryloxy or heteroaryloxy group having 5 to 40 aromatic ring atoms, which may be substituted by one or more radicals

$R^3$, or a combination of these systems; two or more adjacent substituents $R^2$ may also form a mono- or polycyclic, aliphatic or aromatic ring system with one another;
$R^3$ is on each occurrence, identically or differently, H, D, F or an aliphatic, aromatic and/or heteroaromatic hydrocarbon radical having 1 to 20 C atoms, in which, in addition, H atoms may be replaced by F; two or more adjacent substituents $R^3$ may also form a mono- or polycyclic, aliphatic or aromatic ring system with one another, where the following compounds are excluded:

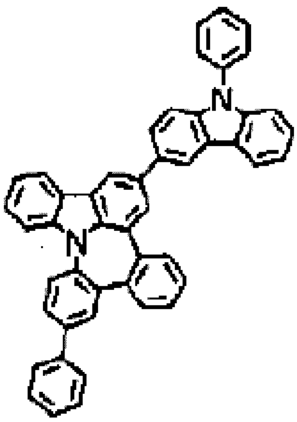

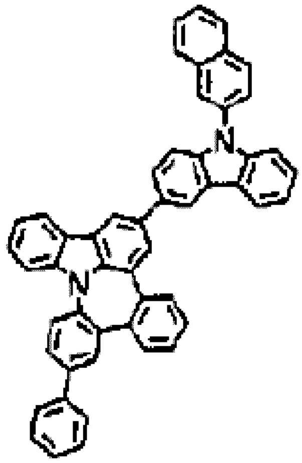

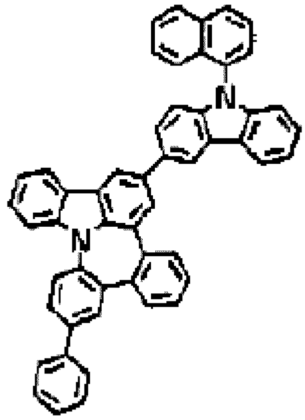

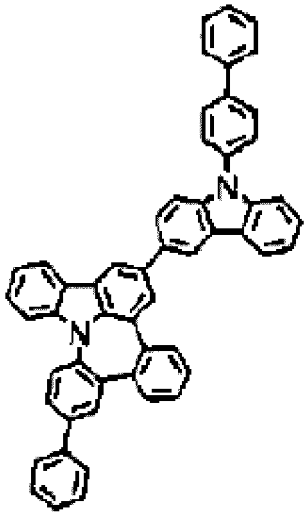

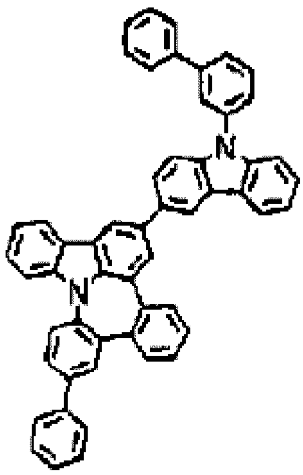

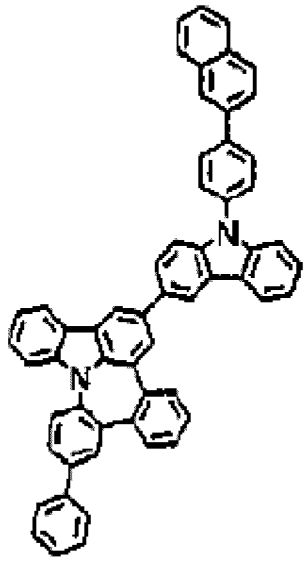

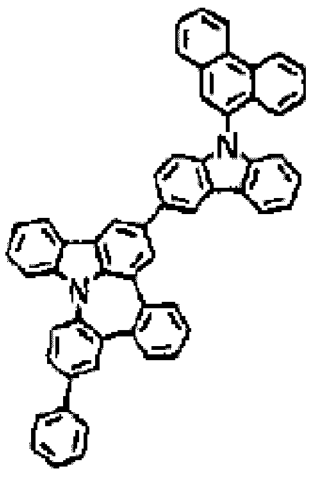

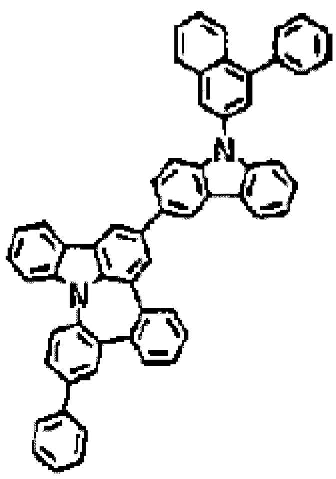

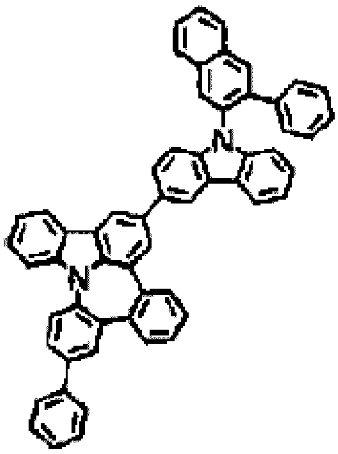

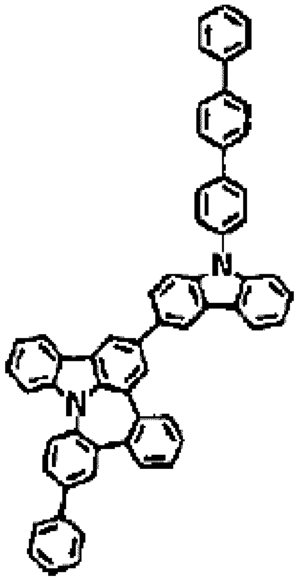

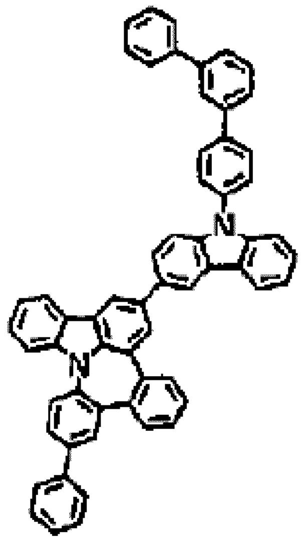

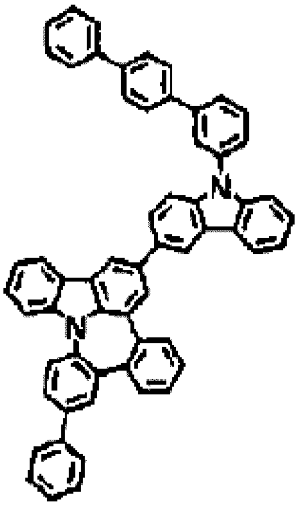

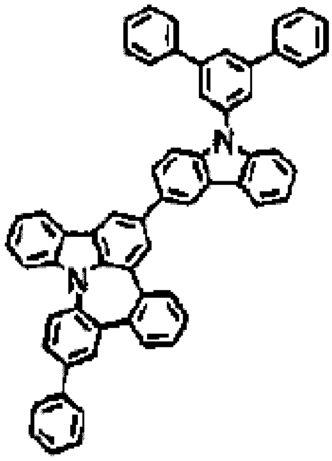

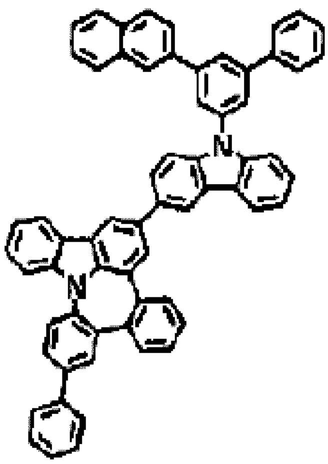

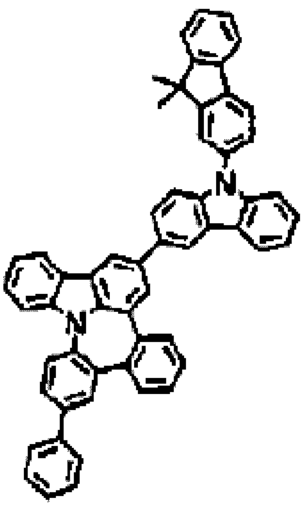

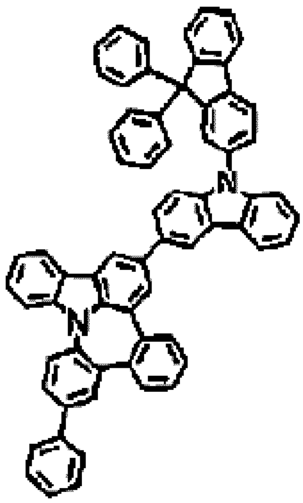

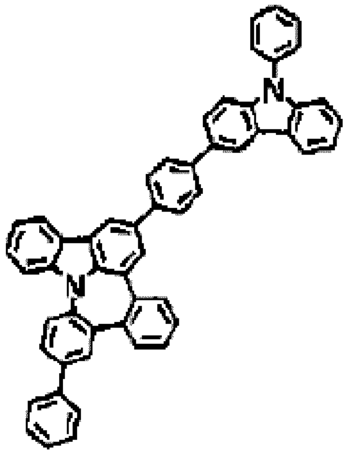

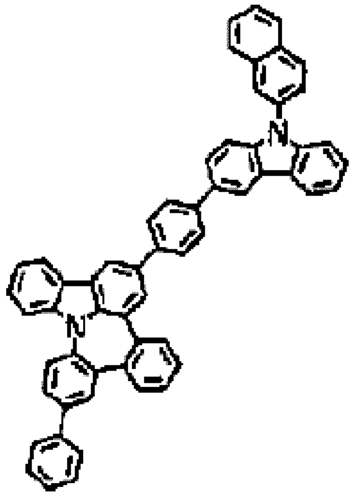

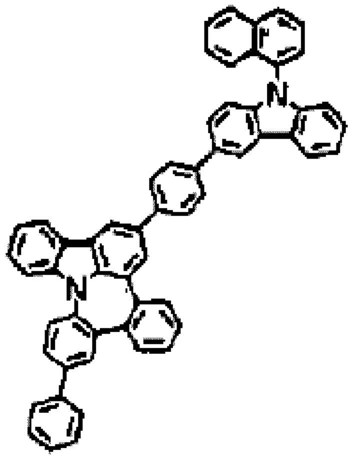

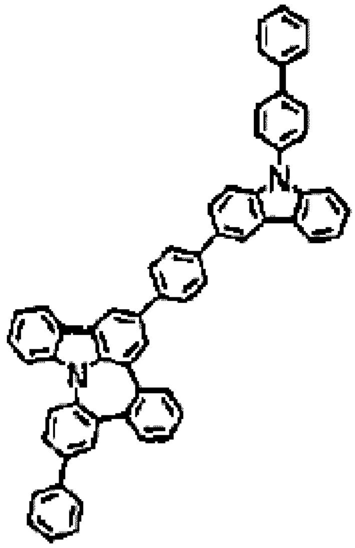

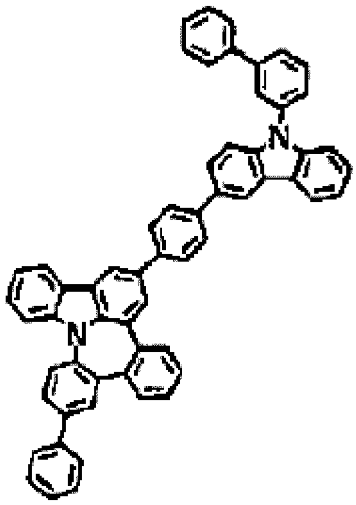

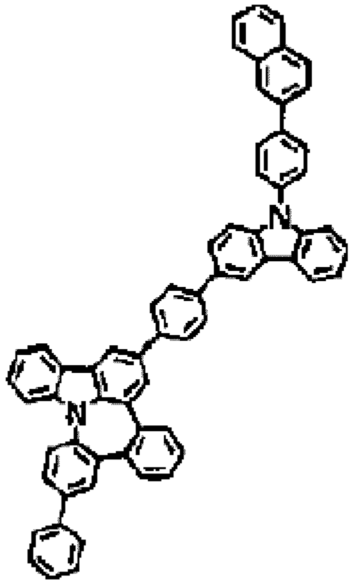

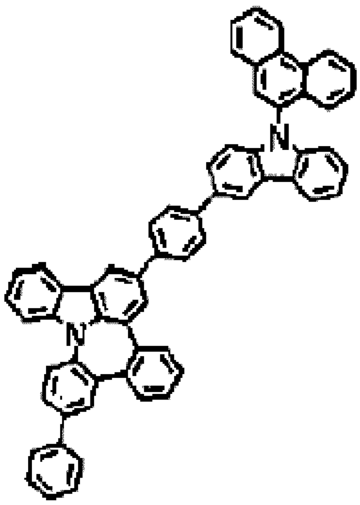

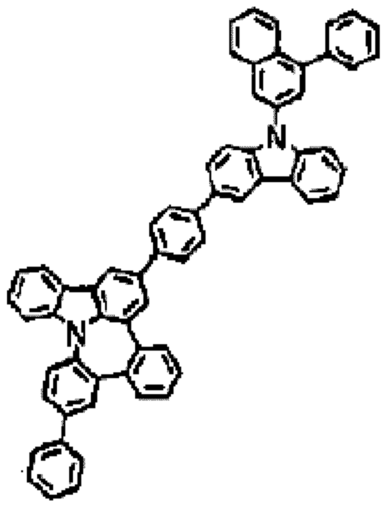

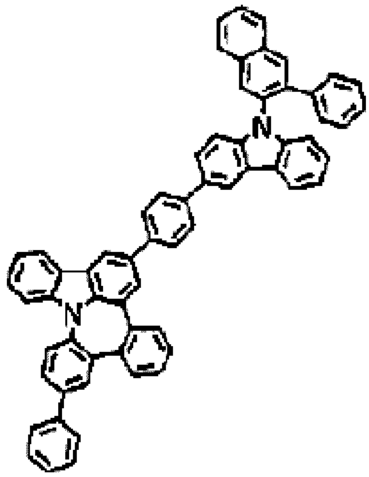
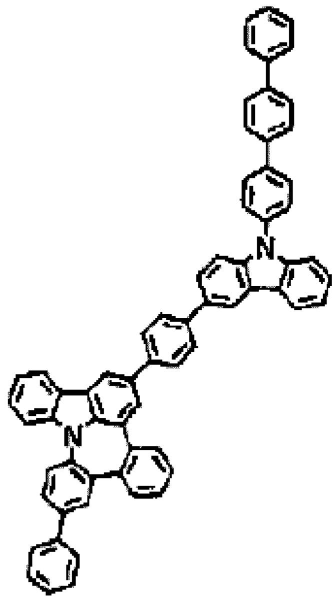
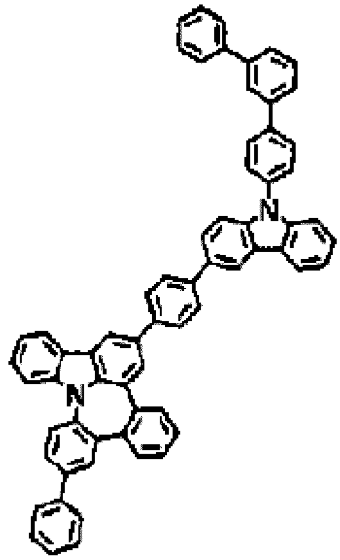
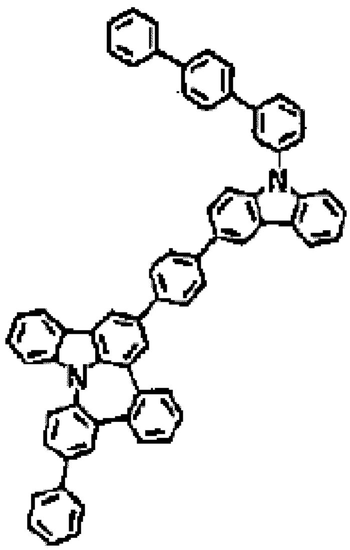
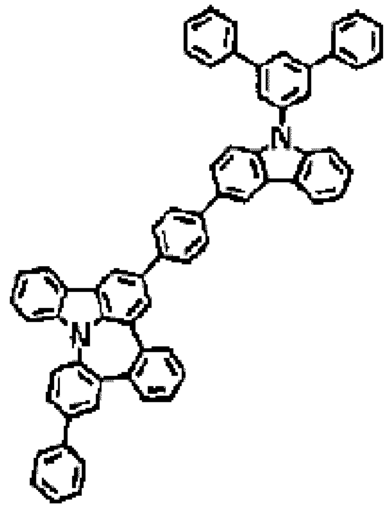
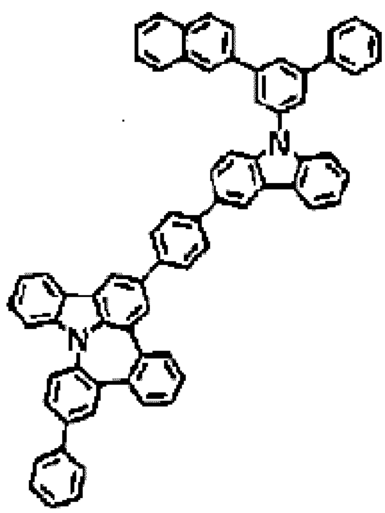
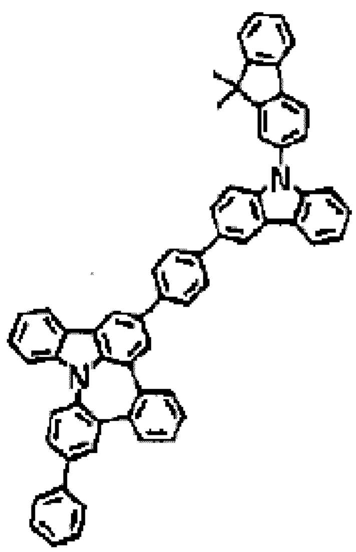
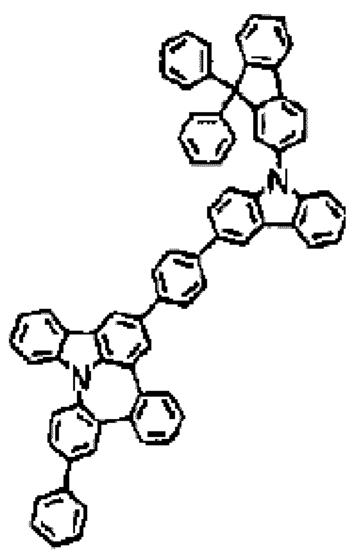

**2.** Compounds according to Claim 1, comprising structures of the formula (IV),

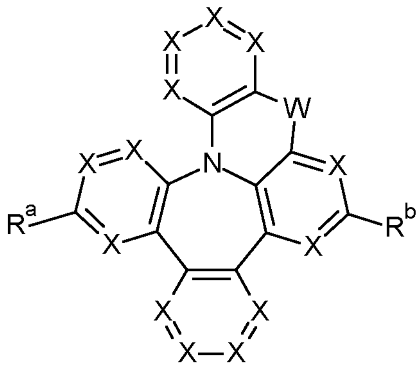

formula (IV),

where the symbols used have the meaning given in Claim 1.

**3.** Compounds according to at least one of the preceding claims, where at least one of the radicals $R^a$ and/or $R^b$ represents a hole-transport group or an electron-transport group.

**4.** Compounds according to at least one of the preceding claims, where at most 4, particularly preferably at most 3 and especially preferably at most 2 of the groups $CR^1$ for which X stands are not equal to the group CH.

**5.** Compounds according to at least one of the preceding claims, comprising structures of the formula (V),

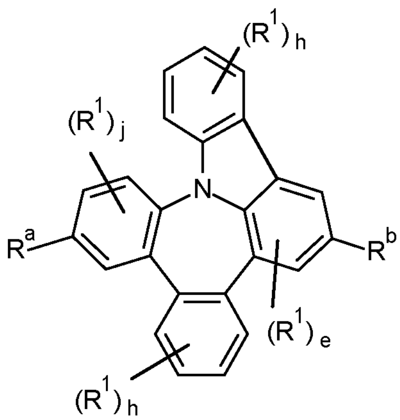

formula (V),

in which $R^a$, $R^b$ and $R^1$ have the meanings described in Claim 1, e is 0, 1 or 2, j is 0, 1, 2 or 3, h is 0, 1, 2, 3 or 4, where at least one of the radicals $R^a$, $R^b$ represents a hole-transport group and/or an electron-transport group.

**6.** Compounds according to at least one of the preceding claims, comprising structures of the formula (VI),

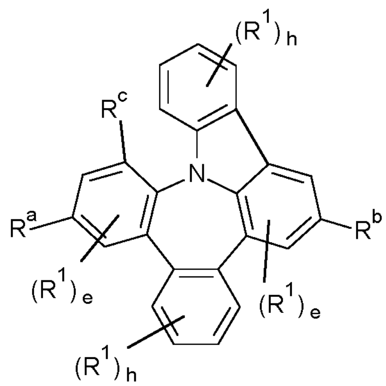

formula (VI),

in which $R^a$, $R^b$ and $R^1$ have the meanings described in Claim 1, e is 0, 1 or 2, h is 0, 1, 2, 3 or 4, where the radical $R^b$ represents a hole-transport group or an electron-transport group and the radical $R^c$ is an aromatic group having 10 to 40 C atoms or a heteroaromatic group having 6 to 40 C atoms, where the aromatic and/or heteroaromatic group comprises at least two adjacent aromatic and/or heteroaromatic rings, which may in each case be condensed or non-condensed and/or substituted by one or more radicals $R^1$.

**7.** Compounds according to at least one of the preceding claims, comprising structures of the formula (VII),

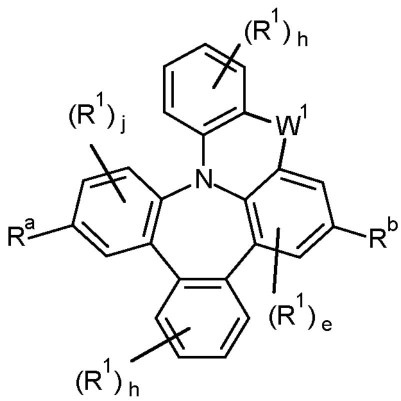

formula (VII),

in which $R^a$, $R^b$ and $R^1$ have the meanings described in Claim 1, e is 0, 1 or 2, j is 0, 1, 2 or 3, h is 0, 1, 2, 3 or 4, where at least one of the radicals $R^a$, $R^b$ represents a hole-transport group and/or an electron-transport group and $W^1$ is $NR^1$, $C(R^1)_2$, O or S.

**8.** Compounds according to at least one of the preceding claims, comprising structures of the formula (VIII),

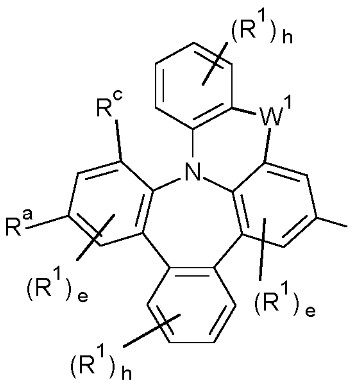

formula (VIII),

in which $R^a$, $R^b$ and $R^1$ have the meanings described in Claim 1, e is 0, 1 or 2, h is 0, 1, 2, 3 or 4, where the radical $R^b$ represents a hole-transport group or an electron-transport group and the radical $R^c$ represents an aryl group having 10 to 40 C atoms which comprises at least two rings, or a heteroaryl group having 6 to 40 C atoms which comprises at least two rings, where the respective group may in each case be substituted by one or more radicals $R^1$ and $W^1$ is $NR^1$, $C(R^1)_2$, O or S.

**9.** Compounds according to at least one of the preceding claims, where the radical $R^b$ in one of the formulae (II), (IV), (V), (VI), (VII) and/or (VIII) is a hole-transport group and the radical $R^a$ in one of the formulae (II), (IV), (V), (VI), (VII) and/or (VIII) is a hole-transport group.

**10.** Compounds according to at least one of the preceding Claims 1 to 8, where the radical $R^b$ in one of the formulae (II), (IV), (V), (VI), (VII) and/or (VIII) is an electron-transport group and the radical $R^a$ in one of the formulae (II), (IV), (V), (VI), (VII) and/or (VIII) is a hole-transport group.

**11.** Compounds according to at least one of the preceding Claims 1 to 8, where the radical $R^b$ in one of the formulae (II), (IV), (V), (VI), (VII) and/or (VIII) is a hole-transport group and the radical $R^a$ in one of the formulae (II), (IV), (V), (VI), (VII) and/or (VIII) is an electron-transport group.

**12.** Compounds according to at least one of the preceding Claims 1 to 8, where the radical $R^b$ in one of the formulae (II), (IV), (V), (VI), (VII) and/or (VIII) is an electron-transport group and the radical $R^a$ in one of the formulae (II), (IV), (V), (VI), (VII) and/or (VIII) is an electron-transport group.

**13.** Compounds according to at least one of the preceding claims, where at least one radical $R^1$, $R^a$, $R^b$ and/or $R^c$ in structures of the formula (II), (IV), (V), (VI), (VII) and/or (VIII) stands for a group selected from the formulae ($R^1$-1) to ($R^1$- 72)

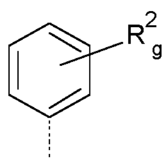

formula ($R^1$-1)

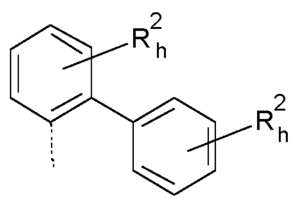

formula ($R^1$-2)

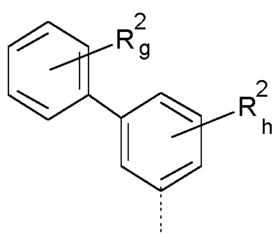

formula ($R^1$-3)

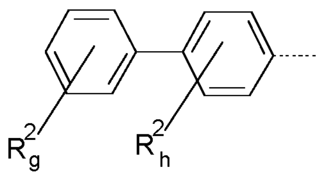
formula ($R^1$-4)

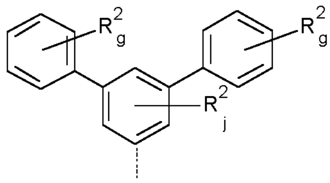
formula ($R^1$-5)

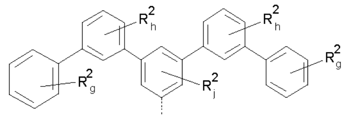
formula ($R^1$-6)

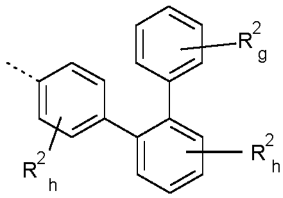
formula ($R^1$-7)

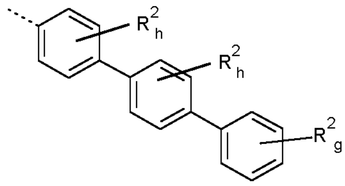
formula ($R^1$-8)

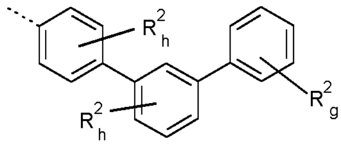
formula ($R^1$-9)

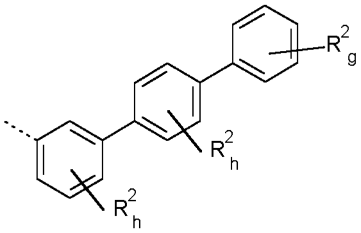
formula ($R^1$-10)

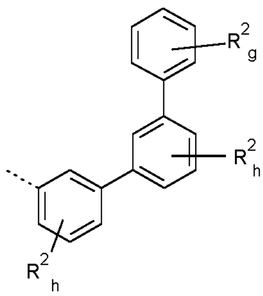
formula ($R^1$-11)

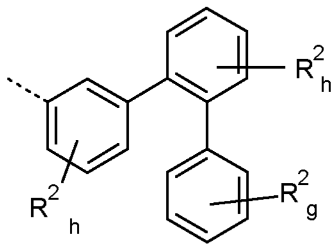
formula ($R^1$-12)

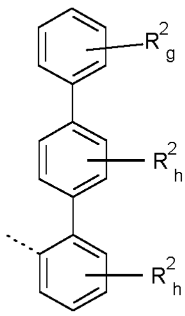
formula ($R^1$-13)

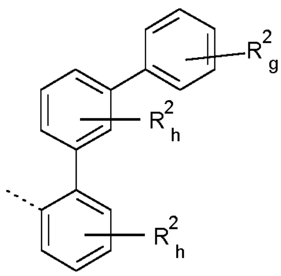
formula ($R^1$-14)

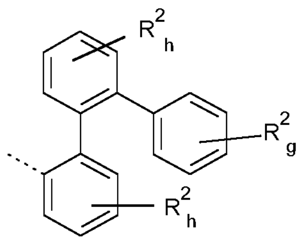
formula ($R^1$-15)

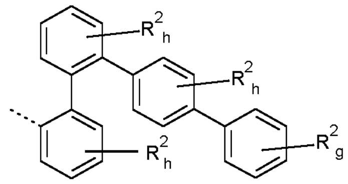

formula ($R^1$-16)

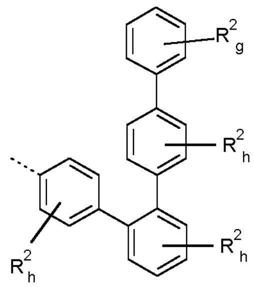

formula ($R^1$-17)

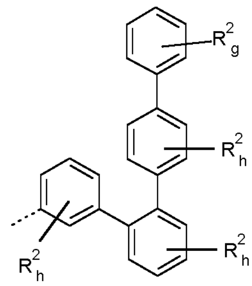

formula ($R^1$-18)

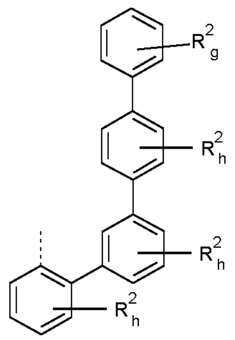

formula ($R^1$-19)

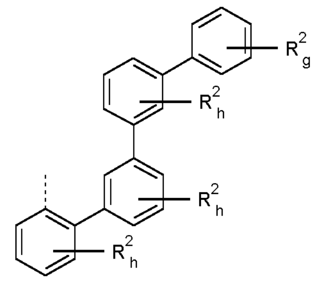

formula ($R^1$-20)

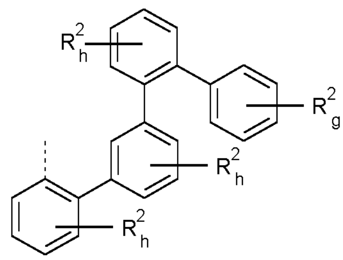

formula ($R^1$-21)

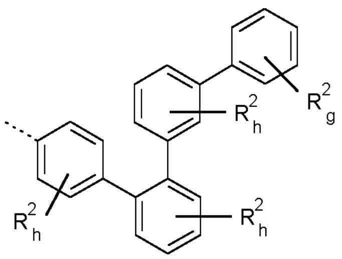

formula ($R^1$-22)

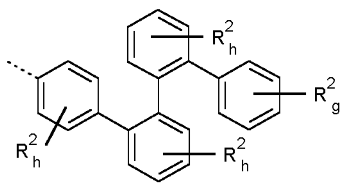

formula ($R^1$-23)

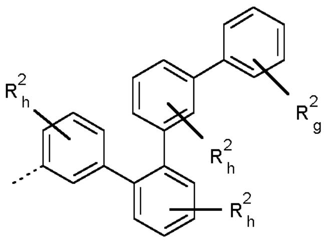

formula ($R^1$-24)

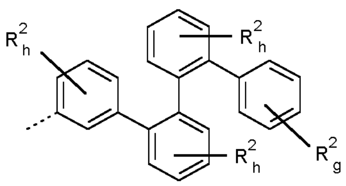

formula ($R^1$-25)

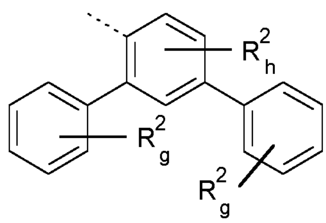

formula ($R^1$-26)

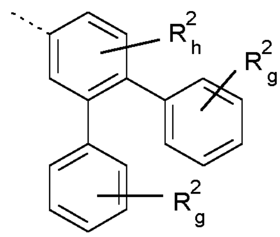

formula ($R^1$-27)

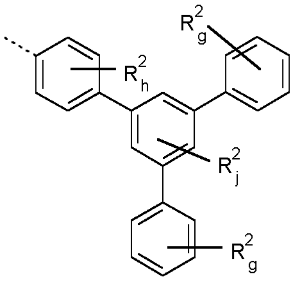

formula ($R^1$-28)

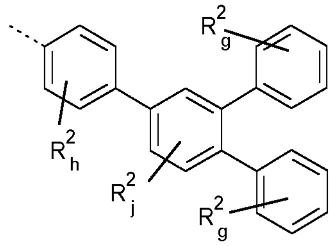

formula ($R^1$-29)

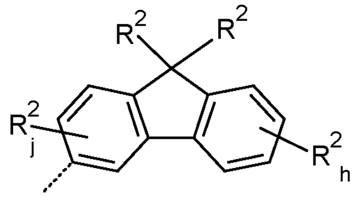

formula ($R^1$-30)

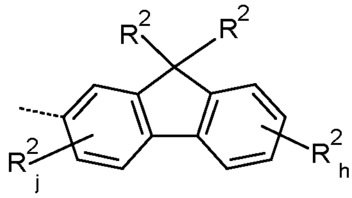

formula ($R^1$-31)

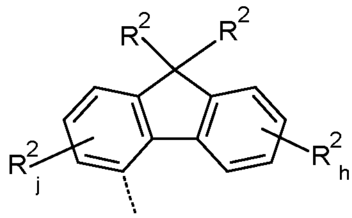

formula ($R^1$-32)

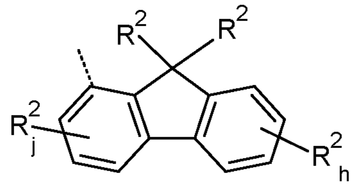

formula ($R^1$-33)

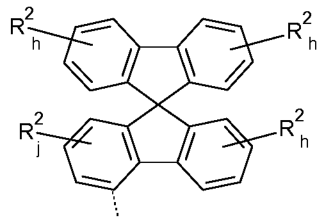

formula ($R^1$-34)

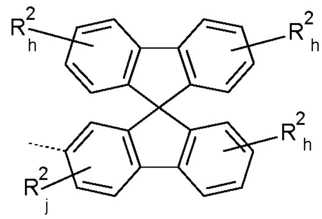

formula ($R^1$-35)

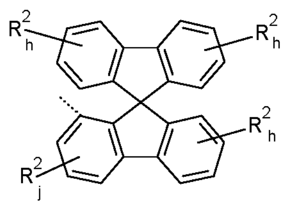

formula ($R^1$-36)

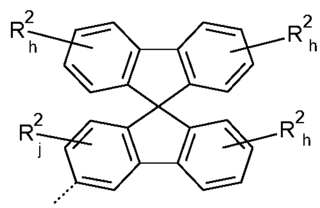

formula ($R^1$-37)

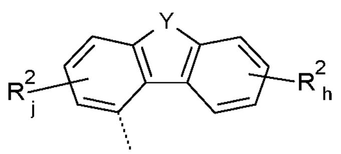

formula ($R^1$-38)

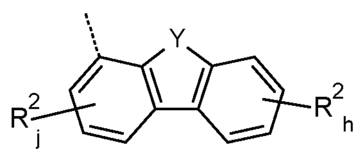

formula ($R^1$-39)

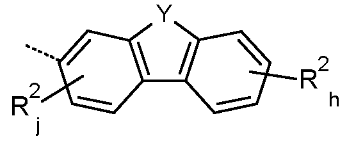

formula ($R^1$-40)

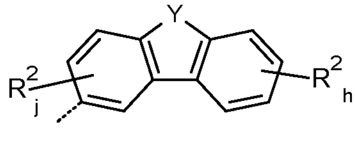

formula ($R^1$-41)

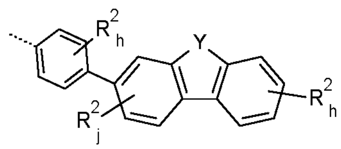

formula ($R^1$-42)

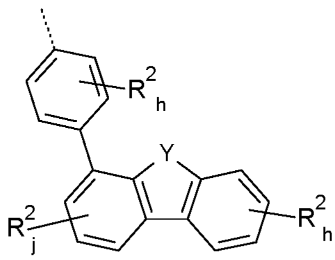

formula ($R^1$-43)

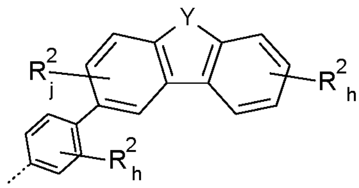

formula ($R^1$-44)

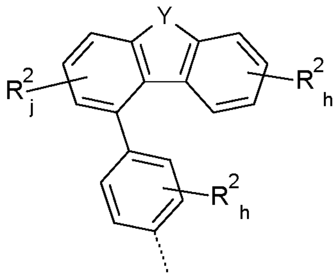

formula ($R^1$-45)

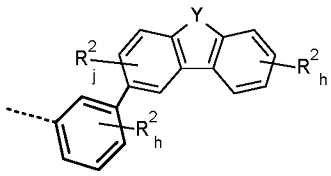

formula ($R^1$-46)

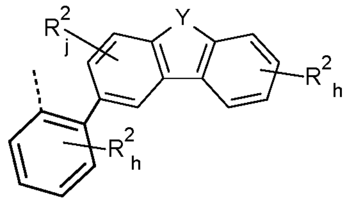

formula ($R^1$-47)

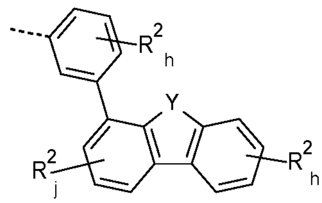

formula ($R^1$-48)

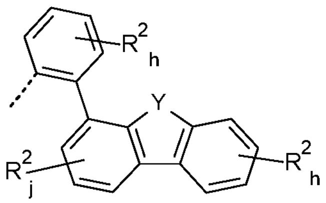

formula ($R^1$-49)

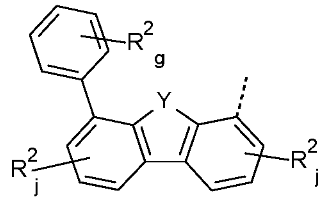

formula ($R^1$-50)

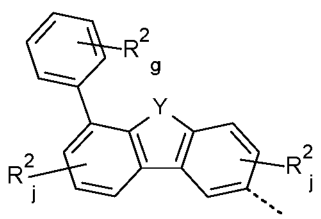

formula ($R^1$-51)

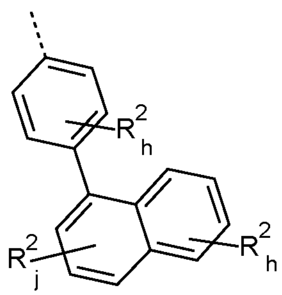

formula ($R^1$-52)

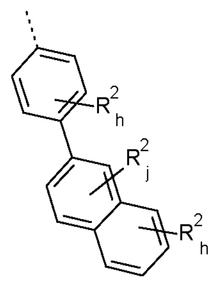

formula ($R^1$-53)

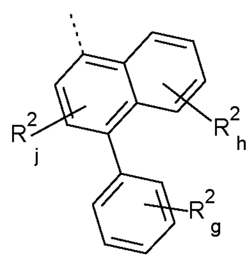

formula ($R^1$-54)

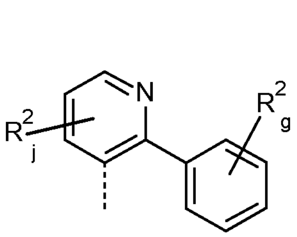

formula ($R^1$-55)

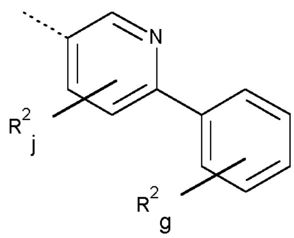

formula ($R^1$-56)

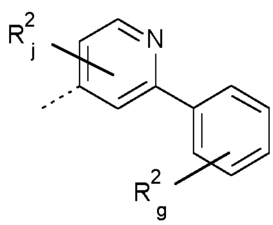

formula ($R^1$-57)

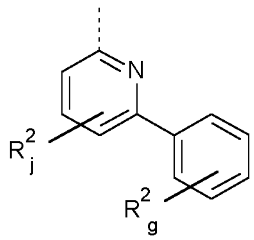
formula ($R^1$-58)

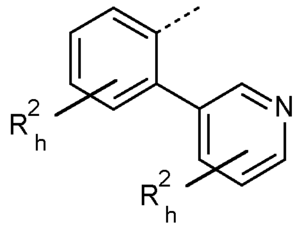
formula ($R^1$-59)

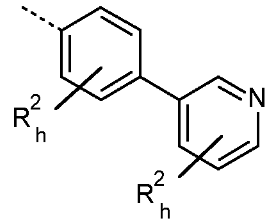
formula ($R^1$-60)

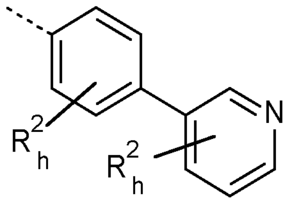
formula ($R^1$-61)

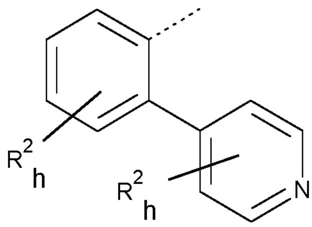
formula ($R^1$-62)

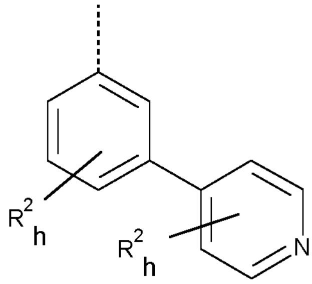
formula ($R^1$-63)

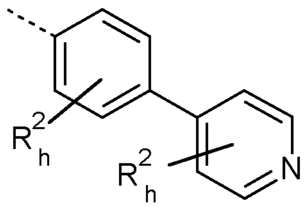
formula ($R^1$-64)

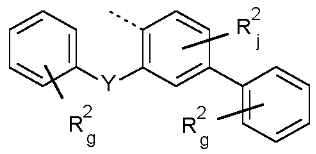
formula ($R^1$-65)

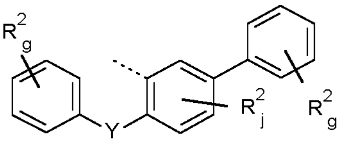
formula ($R^1$-66)

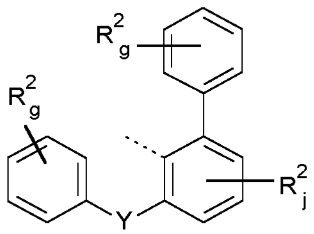
formula ($R^1$-67)

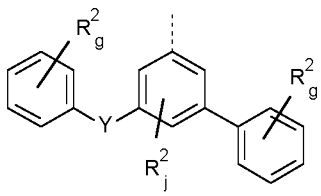
formula ($R^1$-68)

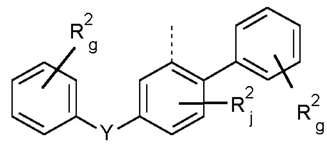
formula ($R^1$-69)

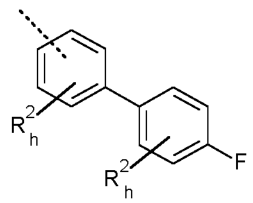
formula ($R^1$-70)

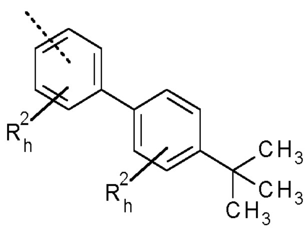
formula ($R^1$-71)

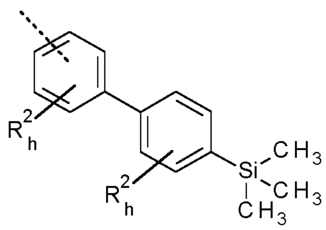
formula ($R^1$-72),

where the following applies to the symbols used:

Y is O, S or $NR^2$, preferably O or S;
j is on each occurrence, independently, 0, 1, 2 or 3;
h is on each occurrence, independently, 0, 1, 2, 3 or 4;
g is on each occurrence, independently, 0, 1, 2, 3, 4 or 5:

the dashed bond marks the bonding position; and

$R^2$ has the meaning given in Claim 1.

**14.** Compounds according to Claim 13, where the sum of the indices g, h and j in the structures of the formulae ($R^1$-1) to ($R^1$-72) is in each case at most 3, preferably at most 2 and particularly preferably at most 1.

**15.** Compounds according to at least one of the preceding Claims 3, 5 to 8 and 10 to 12, where the electron-transport group has at least one structure of the formulae (E-1), (E-5) to (E-10)

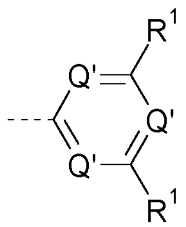

formula (E-1)

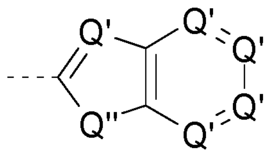

formula (E-5)

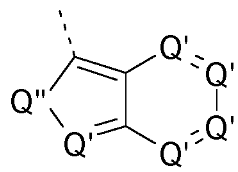

formula (E-6)

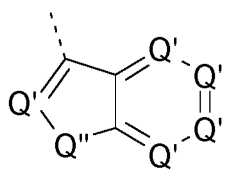

formula (E-7)

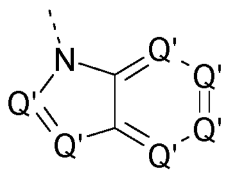

formula (E-8)

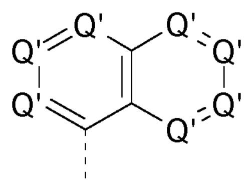

formula (E-9)

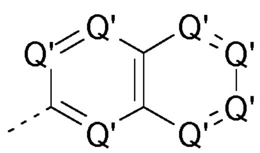

formula (E-10),

where the dashed bond marks the bonding position,

Q’ on each occurrence, identically or differently, represents $CR^1$ or N, and
Q" represents $NR^1$, O or S;

where at least one Q‘ is equal to N and/or at least one Q" is equal to $NR^1$ and

$R^1$ is as defined in Claim 1.

**16.** Compounds according to at least one of the preceding Claims 3, 5 to 8 and 10 to 12, where the electron-transport group has at least one structure of the formulae (E-11) to (E-23)

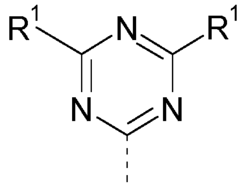

formula (E-11)

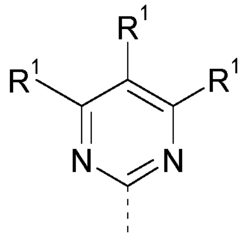

formula (E-12)

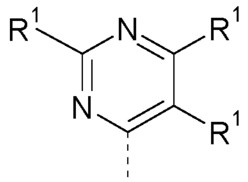

formula (E-13)

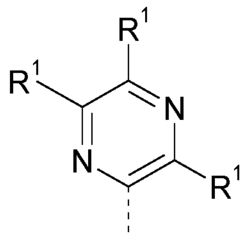

formula (E-14)

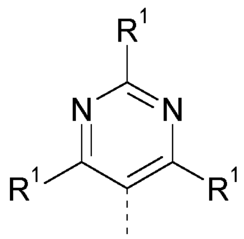

formula (E-15)

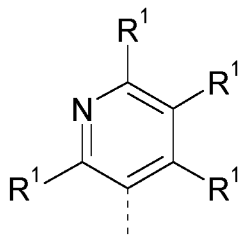

formula (E-16)

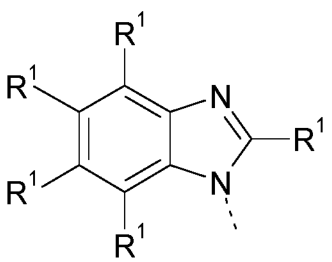

formula (E-17)

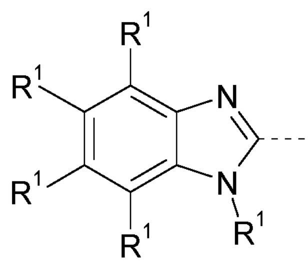

formula (E-18)

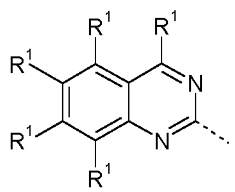

formula (E-19)

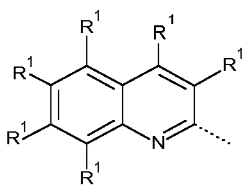

formula (E-20)

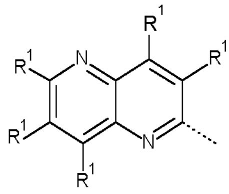

formula (E-21)

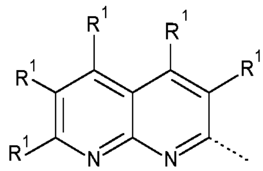

formula (E-22)

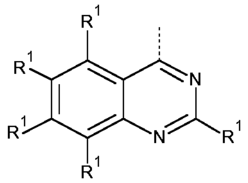

formula (E-23)

where the dashed bond marks the bonding position and $R^1$ has the meaning given in Claim 1.

**17.** Compounds according to at least one of the preceding Claims 3 and 5 to 11, where the hole-transport group comprises at least one structure selected from the group carbazoles, indenocarbazoles and indolocarbazoles.

**18.** Compounds according to at least one of the preceding claims 3 and 5 to 11, where the hole-transport group has at least one structure of the formulae (L-2) to (L-9)

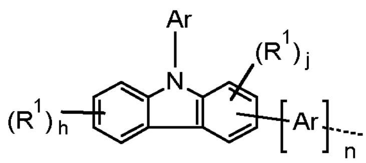

formula (L-2)

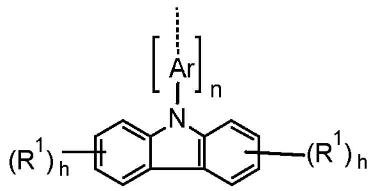

formula (L-3)

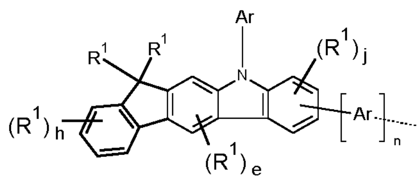

formula (L-4)

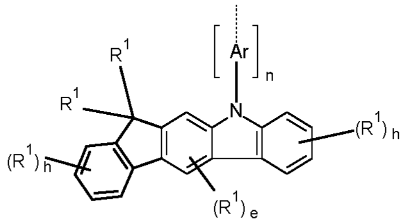

formula (L-5)

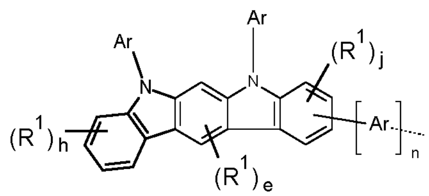

formula (L-6)

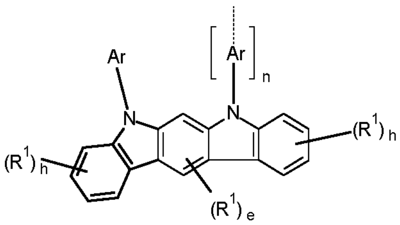

formula (L-7)

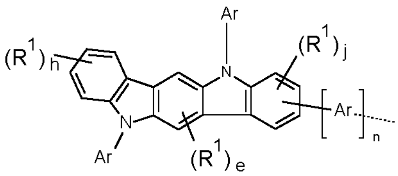

formula (L-8)

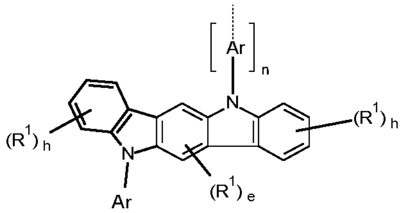

formula (L-9)

where the dashed bond marks the bonding position, e is 0, 1 or 2, j is 0, 1, 2 or 3, h is 0, 1, 2, 3 or 4, n is 0 or 1, Ar represents an aryl group having 6 to 40 C atoms or a heteroaryl group having 3 to 40 C atoms, which may be substituted by one or more radicals $R^1$, and $R^1$ has the meaning given in Claim 1.

**19.** Compounds according to at least one of the preceding claims, selected from the group of the following compounds:

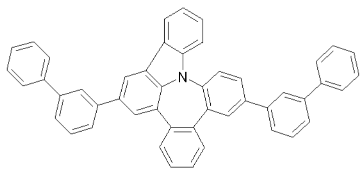

formula 1

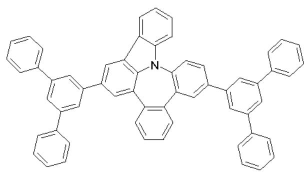

formula 2

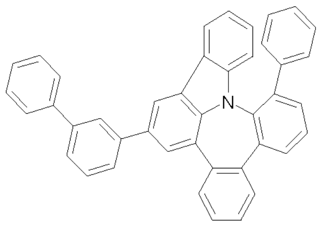

formula 4

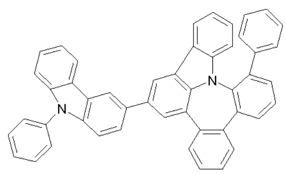

formula 5

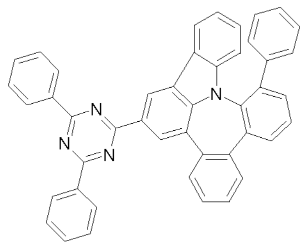

formula 6

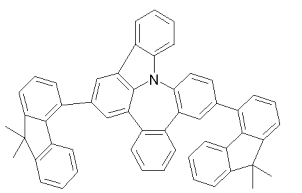

formula 7

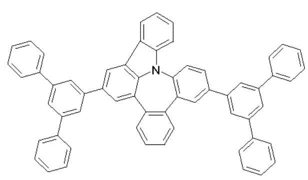

formula 8

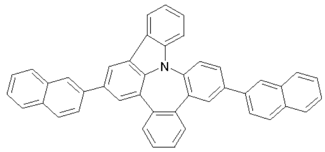

formula 10

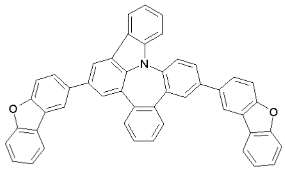

formula 14

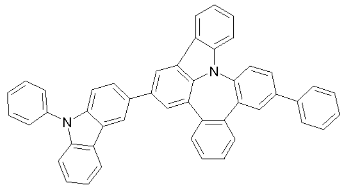

formula 15

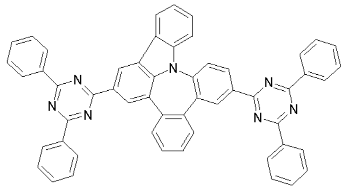

formula 16

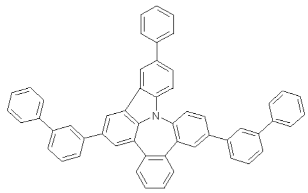

formula 17

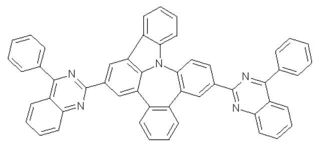

formula 19

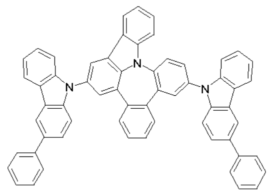

formula 20

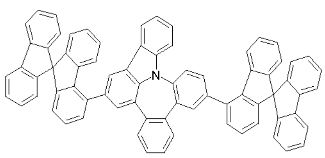

formula 22

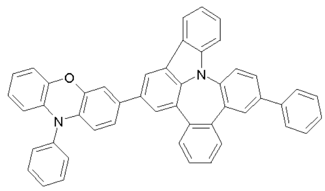

formula 24

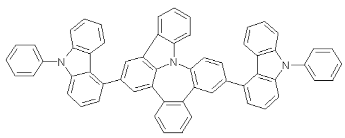

formula 27

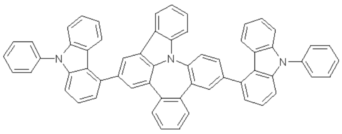

formula 28

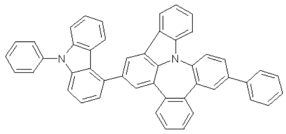

formula 29

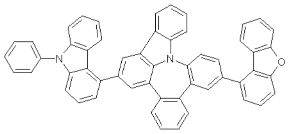

formula 30

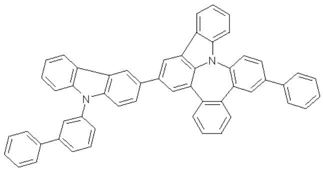

formula 34

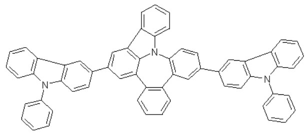

formula 36

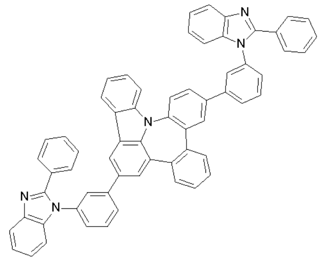

formula 43

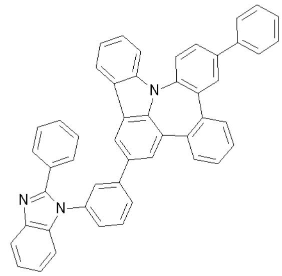

formula 44

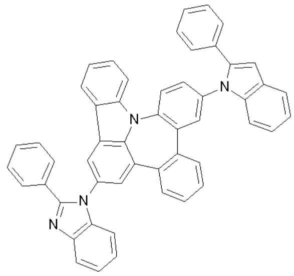

formula 45

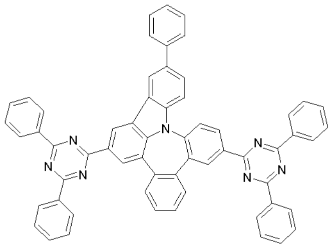

formula 46

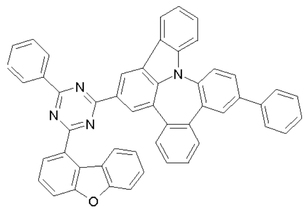

formula 49

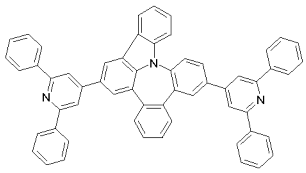

formula 50

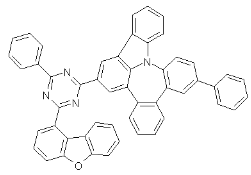

formula 51

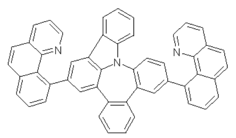

formula 52

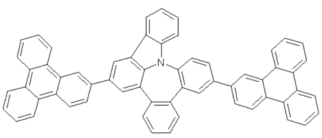

formula 53

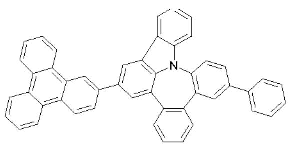

formula 54

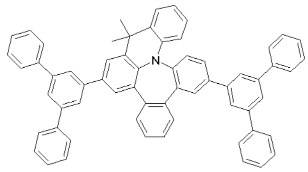

formula 55

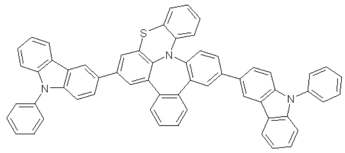

formula 57

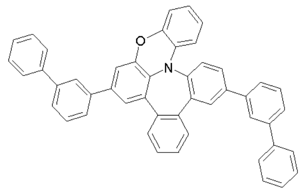

formula 58

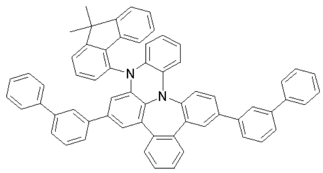

formula 62

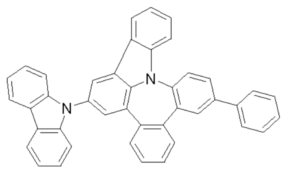

formula 72

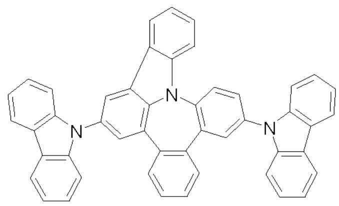

formula 73

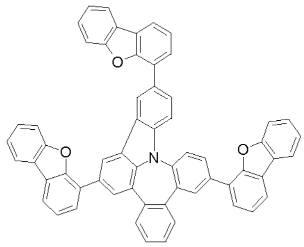

formula 75

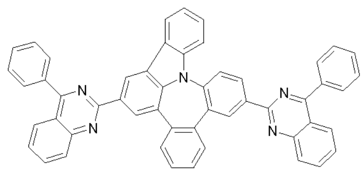

formula 76.

**20.** Oligomer, polymer or dendrimer containing one or more compounds according to one or more of Claims 1 to 19, where one or more bonds are present from the compound to the polymer, oligomer or dendrimer.

**21.** Composition comprising at least one compound according to one or more of Claims 1 to 19 and/or an oligomer, polymer or dendrimer according to Claim 20 and at least one further compound selected from the group consisting of fluorescent emitters, phosphorescent emitters, host materials, matrix materials, electron-transport materials, electron-injection materials, hole-conductor materials, hole-injection materials, electron-blocking materials and hole-blocking materials.

**22.** Formulation comprising at least one compound according to one or more of Claims 1 to 19, an oligomer, polymer or dendrimer according to Claim 20 and/or at least one composition according to Claim 21 and at least one solvent.

**23.** Process for the preparation of a compound according to one or more of Claims 1 to 19 or an oligomer, polymer or dendrimer according to Claim 20, where a ring-closure reaction is carried out on a compound containing an azepine structural element.

**24.** Use of a compound according to one or more of Claims 1 to 19, an oligomer, polymer or dendrimer according to Claim 20 or a composition according to Claim 21 in an electronic device as electron-blocking material, hole-injection material and/or hole-transport material.

**25.** Electronic device containing at least one compound according to one or more of Claims 1 to 19, an oligomer, polymer or dendrimer according to Claim 20 or a composition according to Claim 21.

**26.** Electronic device according to Claim 25, where the electronic device is selected from the group consisting of organic electroluminescent devices, organic integrated circuits, organic field-effect transistors, organic thin-film transistors, organic light-emitting transistors, organic solar cells, organic optical detectors, organic photoreceptors, organic field-quench devices, light-emitting electrochemical cells or organic laser diodes.

## Revendications

**1.** Composé comprenant des structures de formule (II),

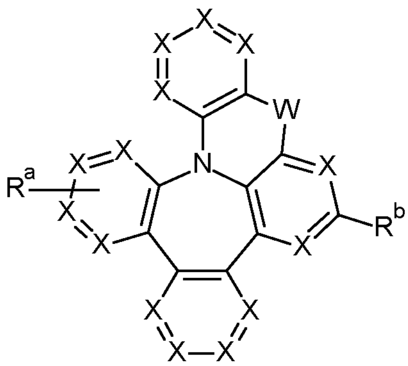

formule (II),

dans laquelle ce qui suit s'applique aux symboles utilisés :

X est à chaque occurrence, de manière identique ou différente, $CR^1$ ou C pour le site de liaison du radical $R^a$ ;
W est une liaison, $NR^1$, $C(R^1)_2$, O ou S ;
$R^a$ est D, F, Cl, Br, I, $B(OR^1)_2$, CHO, $C(=O)R^1$, $CR^1=C(R^1)_2$, CN, $C(=O)OR^1$, $C(=O)N(R^1)_2$, $Si(R^1)_3$, $N(R^1)_2$, $NO_2$, $P(=O)(R^1)_2$, $OSO_2R^1$, $OR^1$, $S(=O)R^1$, $S(=O)_2R^1$, un groupement alkyle, alcoxy ou thioalcoxy à chaîne linéaire ayant de 1 à 40 atomes de C ou un groupement alkyle, alcoxy ou thioalcoxy ramifié ou cyclique ayant de 3 à 40 atomes de C, qui peut dans chaque cas être substitué par un ou plusieurs radicaux $R^1$, où un ou plusieurs groupements $CH_2$ non adjacents peuvent être remplacés par-$R^1C=CR^1$-, -C=C-, $Si(R^1)_2$, C=O, C=S, $C=NR^1$, -C(=O)O-, $-C(=O)NR^1-$, $NR^1$, $P(=O)(R^1)$, -O-, -S-, SO ou $SO_2$ et où un ou plusieurs atomes de H peuvent être remplacés par D, F, Cl, Br, I, CN ou $NO_2$, ou un noyau aromatique ou hétéroaromatique ayant de 5 à 40 atomes de cycle aromatique, qui peut dans chaque cas être substitué par un ou plusieurs radicaux $R^1$, ou un groupement aryloxy ou hétéroaryloxy ayant de 5 à 40 atomes de cycle aromatique, qui peut être substitué par

un ou plusieurs radicaux $R^1$, ou une combinaison de ces systèmes ;

$R^b$ est un groupement aromatique ayant de 10 à 40 atomes de C ou un groupement hétéroaromatique ayant de 6 à 40 atomes de C, où le groupement aromatique et/ou hétéroaromatique comprend au moins deux cycles aromatiques ou hétéroaromatiques adjacents, qui peuvent dans chaque cas être condensés ou non condensés et/ou substitués par un ou plusieurs radicaux $R^1$ ;

$R^1$ est à chaque occurrence, de manière identique ou différente, H, D, F, Cl, Br, I, $B(OR^2)_2$, CHO, $C(=O)R^2$, $CR^2=C(R^2)_2$, CN, $C(=O)OR^2$, $C(=O)N(R^2)_2$, $Si(R^2)_3$, $N(R^2)_2$, $NO_2$, $P(=O)(R^2)_2$, $OSO_2R^2$, $OR^2$, $S(=O)R^2$, $S(=O)_2R^2$, un groupement alkyle, alcoxy ou thioalcoxy à chaîne linéaire ayant de 1 à 40 atomes de C ou un groupement alkyle, alcoxy ou thioalcoxy ramifié ou cyclique ayant de 3 à 40 atomes de C, qui peut dans chaque cas être substitué par un ou plusieurs radicaux $R^2$, où un ou plusieurs groupements $CH_2$ non adjacents peuvent être remplacés par $-R^2C=CR^2-$, -C=C-, $Si(R^2)_2$, $Ge(R^2)_2$, $Sn(R^2)_2$, C=O, C=S, C=Se, $C=NR^2$, -C(=O)O-, $-C(=O)NR^2-$, $NR^2$, $P(=O)(R^2)$, -O-, -S-, SO ou $SO_2$ et où un ou plusieurs atomes de H peuvent être remplacés par D, F, Cl, Br, I, CN ou $NO_2$, ou un noyau aromatique ou hétéroaromatique ayant de 5 à 40 atomes de cycle aromatique, qui peut dans chaque cas être substitué par un ou plusieurs radicaux $R^2$, ou un groupement aryloxy ou hétéroaryloxy ayant de 5 à 40 atomes de cycle aromatique, qui peut être substitué par un ou plusieurs radicaux $R^2$, ou une combinaison de ces systèmes ;

$R^2$ est à chaque occurrence, de manière identique ou différente, H, D, F, Cl, Br, I, $B(OR^3)_2$, CHO, $C(=O)R^3$, $CR^3=C(R^3)_2$, CN, $C(=O)OR^3$, $C(=O)N(R^3)_2$, $Si(R^3)_3$, $N(R^3)_2$, $NO_2$, $P(=O)(R^3)_2$, $OSO_2R^3$, $OR^3$, $S(=O)R^3$, $S(=O)_2R^3$, un groupement alkyle, alcoxy ou thioalcoxy à chaîne linéaire ayant de 1 à 40 atomes de C ou un groupement alkyle, alcoxy ou thioalcoxy ramifié ou cyclique ayant de 3 à 40 atomes de C, qui peut dans chaque cas être substitué par un ou plusieurs radicaux $R^3$, où un ou plusieurs groupements $CH_2$ non adjacents peuvent être remplacés par $-R^3C=CR^3-$, -C=C-, $Si(R^3)_2$, $Si(R^2)_2$, $Ge(R^3)_2$, $Sn(R^3)_2$, C=O, C=S, C=Se, $C=NR^3$, -C(=O)O-, $-C(=O)NR^3-$, $NR^3$, $P(=O)(R^3)$, -O-, -S-, SO ou $SO_2$ et où un ou plusieurs atomes de H peuvent être remplacés par D, F, Cl, Br, I, CN ou $NO_2$, ou un noyau aromatique ou hétéroaromatique ayant de 5 à 40 atomes de cycle aromatique, qui peut dans chaque cas être substitué par un ou plusieurs radicaux $R^3$, ou un groupement aryloxy ou hétéroaryloxy ayant de 5 à 40 atomes de cycle aromatique, qui peut être substitué par un ou plusieurs radicaux $R^3$, ou une combinaison de ces systèmes ; deux, ou plus, substituants $R^2$ adjacents peuvent également former un noyau mono- ou polycyclique, aliphatique ou aromatique les uns avec les autres ;

$R^3$ est à chaque occurrence, de manière identique ou différente, H, D, F ou un radical hydrocarboné aliphatique, aromatique et/ou hétéroaromatique ayant de 1 à 20 atomes de C, où, de plus, des atomes de H peuvent être remplacés par F ; deux, ou plus, substituants adjacents $R^3$ peuvent également former un noyau mono- ou polycyclique, aliphatique ou aromatique les uns avec les autres, où les composés suivants sont exclus :

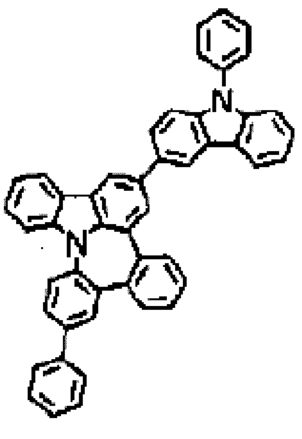
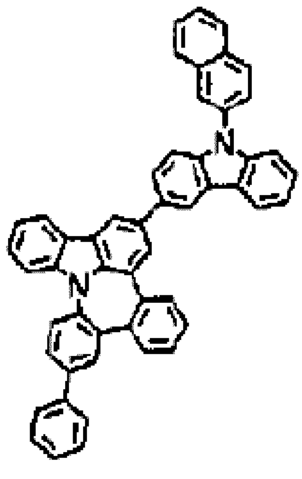
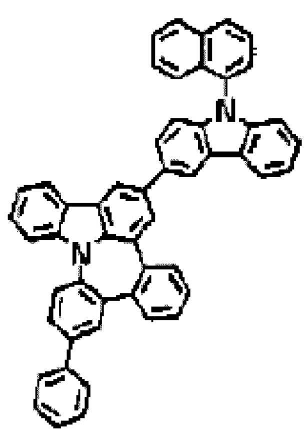

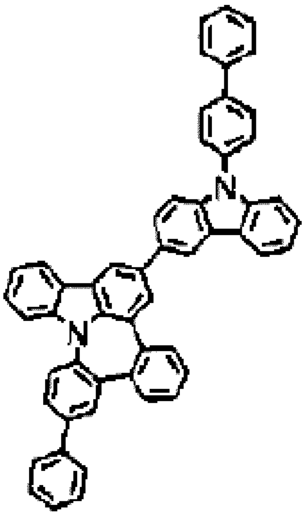

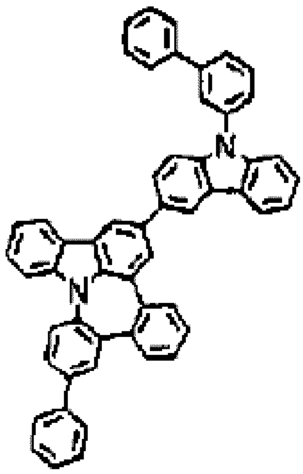

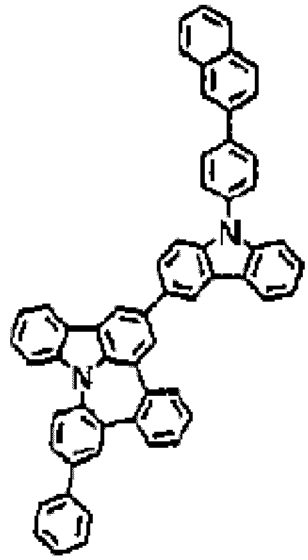

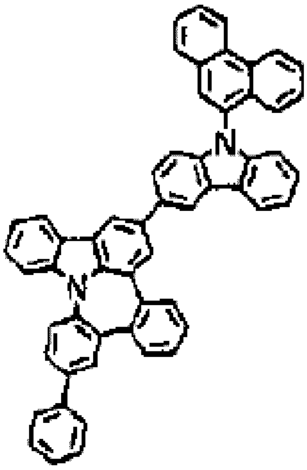

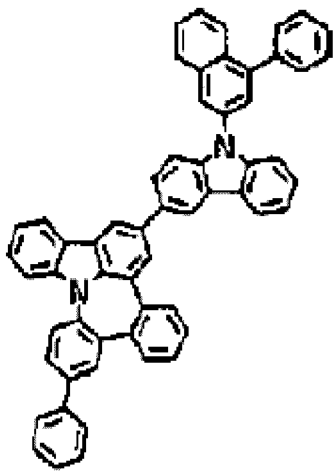

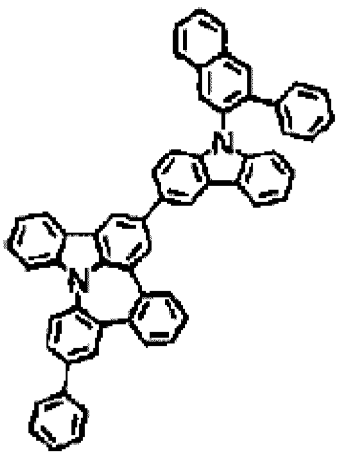

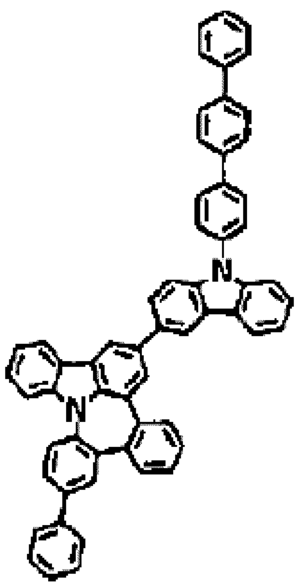

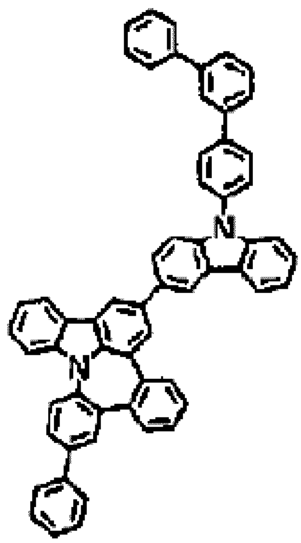

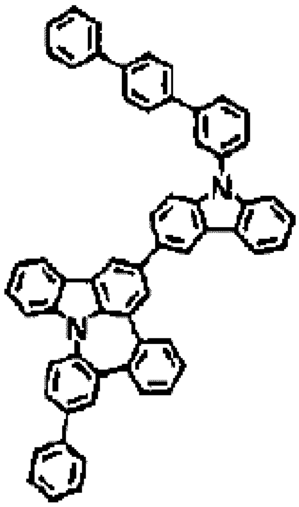

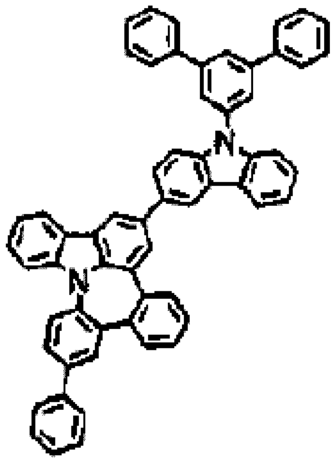

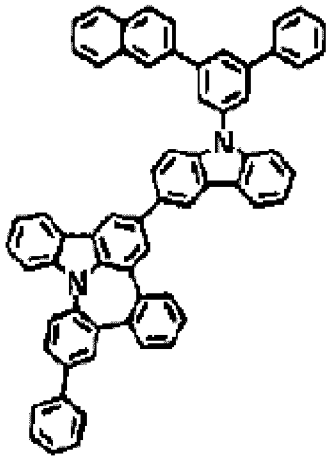

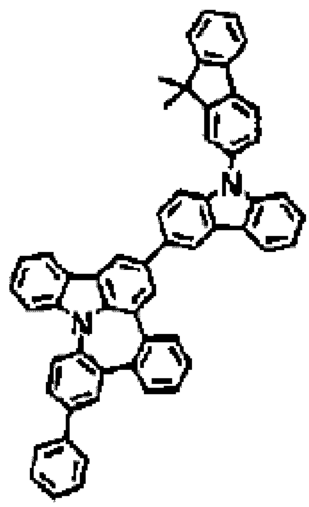

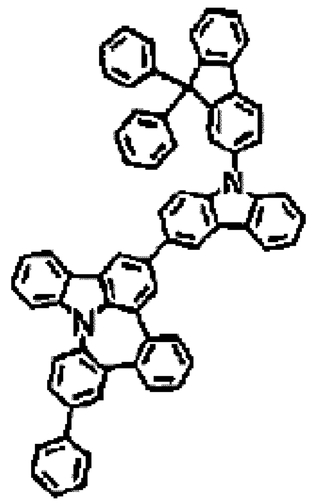

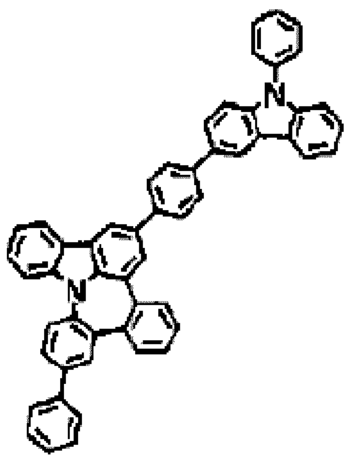

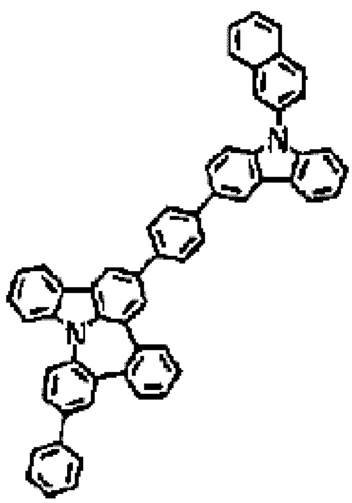

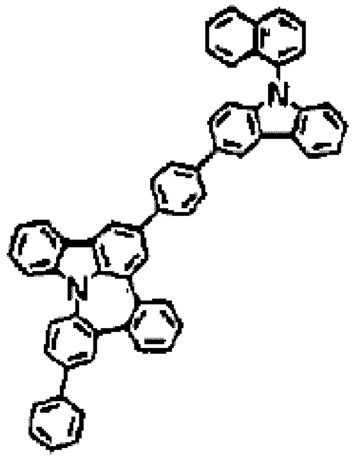

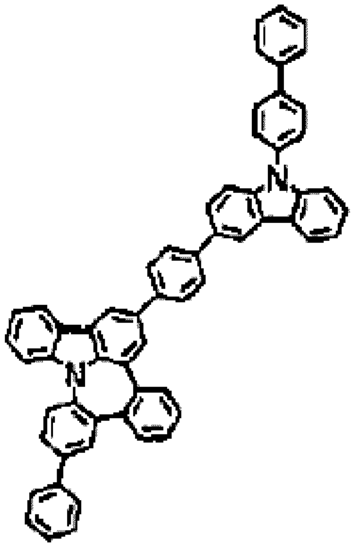

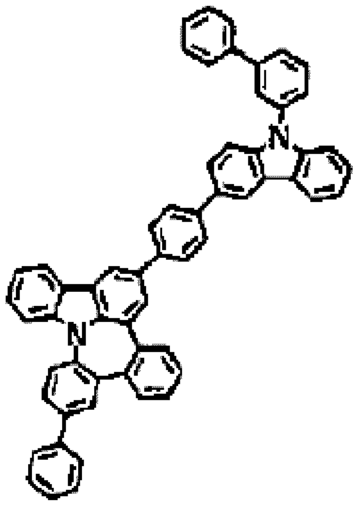

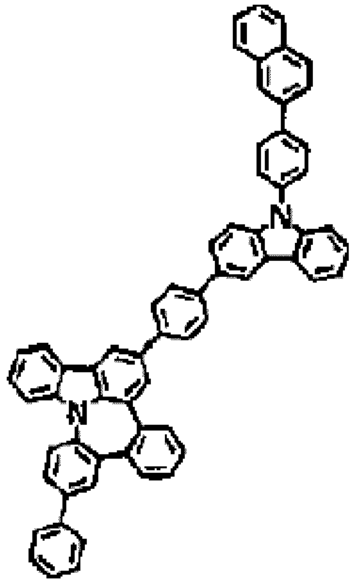

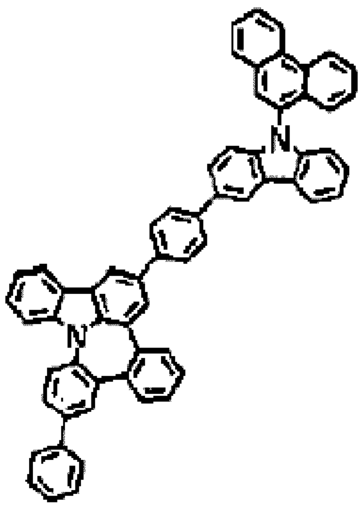

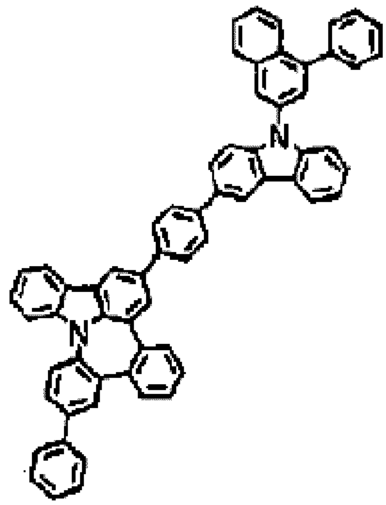

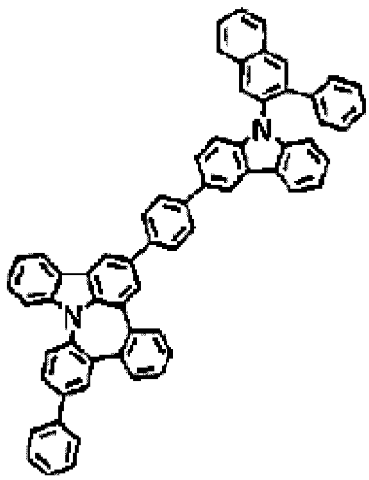

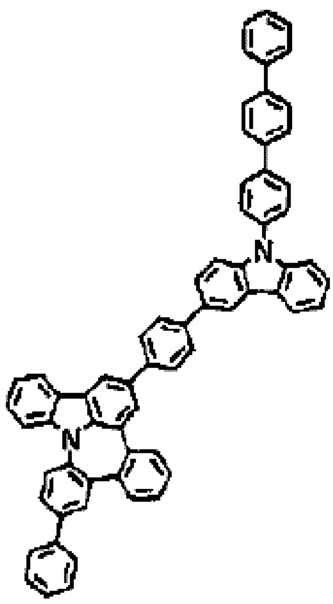

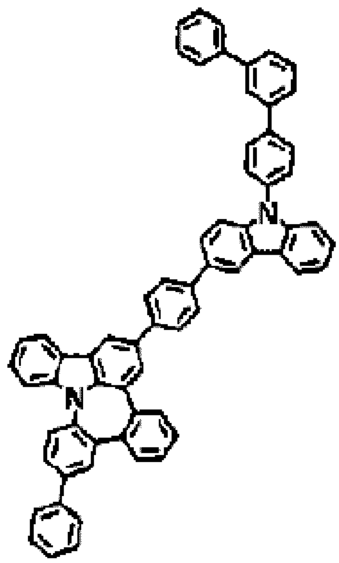

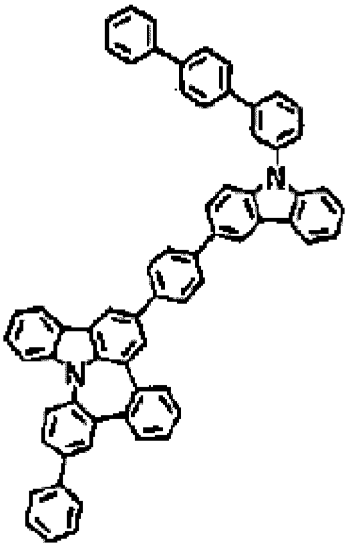
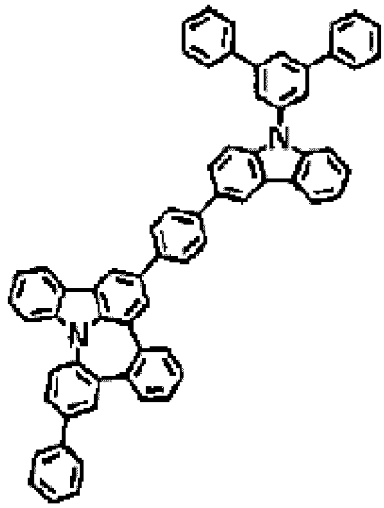
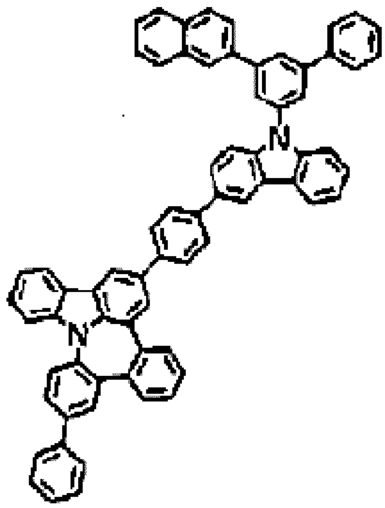

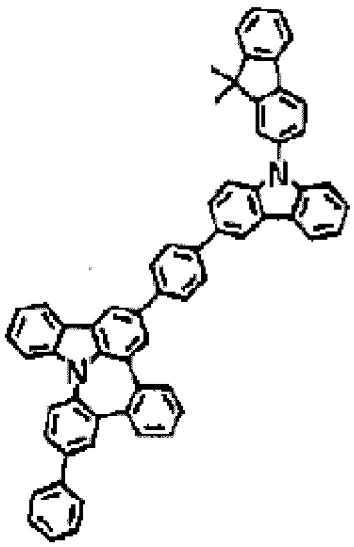
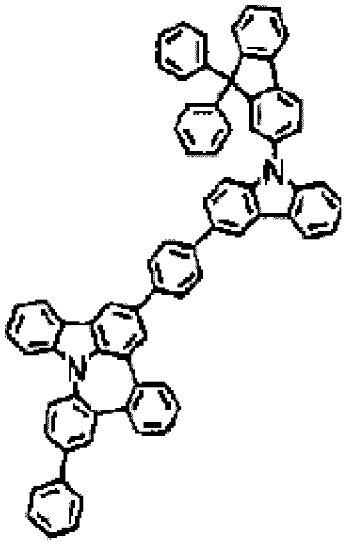

**2.** Composés selon la revendication 1, comprenant des structures de formule (IV),

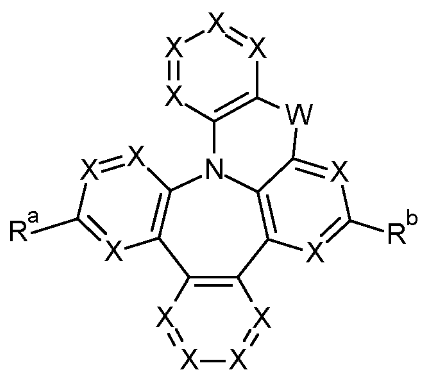

formule (IV),

dans laquelle les symboles utilisés revêtent la signification donnée selon la revendication 1.

**3.** Composés selon au moins l'une des revendications précédentes, dans lesquels au moins l'un des radicaux $R^a$ et/ou

$R^b$ représente un groupement de transport de trous ou un groupement de transport d'électrons.

**4.** Composés selon au moins l'une des revendications précédentes, dans lesquels au plus 4, particulièrement préférablement au plus 3 et notamment préférablement au plus 2 parmi les groupements $CR^1$, que X représente, ne sont pas équivalents au groupement CH.

**5.** Composés selon au moins l'une des revendications précédentes, comprenant des structures de formule (V),

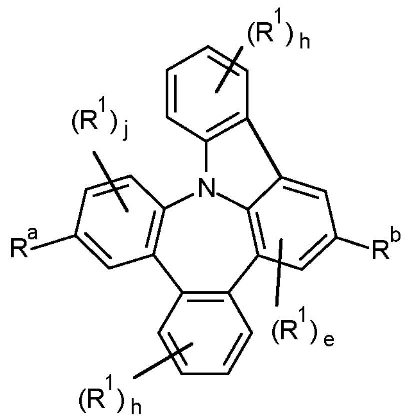

formule (V),

dans laquelle $R^a$, $R^b$ et $R^1$ revêtent les significations décrites selon la revendication 1, e vaut 0, 1 ou 2, j vaut 0, 1, 2 ou 3, h vaut 0, 1, 2, 3 ou 4, où au moins l'un des radicaux $R^a$, $R^b$ représente un groupement de transport de trous et/ou un groupement de transport d'électrons.

**6.** Composés selon au moins l'une des revendications précédentes, comprenant des structures de formule (VI),

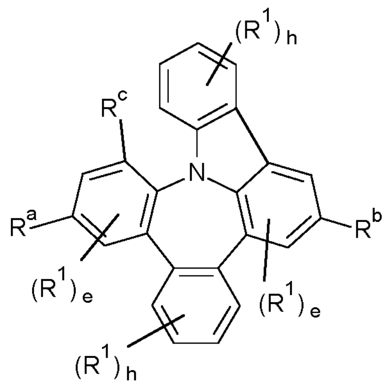

formule (VI),

dans laquelle $R^a$, $R^b$ et $R^1$ revêtent les significations décrites selon la revendication 1, e vaut 0, 1 ou 2, h vaut 0, 1, 2, 3 ou 4, où le radical $R^b$ représente un groupement de transport de trous ou un groupement de transport d'électrons et le radical $R^c$ est un groupement aromatique ayant de 10 à 40 atomes de C ou un groupement hétéroaromatique ayant de 6 à 40 atomes de C, où le groupement aromatique et/ou hétéroaromatique comprend au moins deux cycles aromatiques et/ou hétéroaromatiques adjacents, qui peuvent dans chaque cas être condensés ou non condensés et/ou substitués par un ou plusieurs radicaux $R^1$.

**7.** Composés selon au moins l'une des revendications précédentes, comprenant des structures de formule (VII),

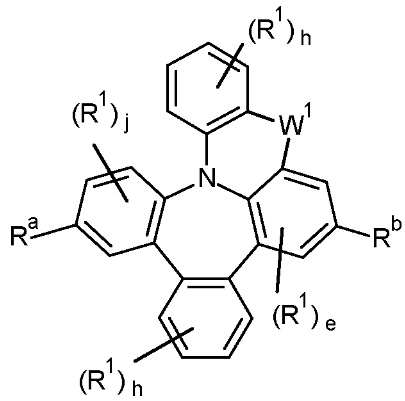

formule (VII),

dans laquelle $R^a$, $R^b$ et $R^1$ revêtent les significations décrites selon la revendication 1, e vaut 0, 1 ou 2, j vaut 0, 1, 2 ou 3, h vaut 0, 1, 2, 3 ou 4, où au moins l'un des radicaux $R^a$, $R^b$ représente un groupement de transport de trous et/ou un groupement de transport d'électrons et $W^1$ est $NR^1$, $C(R^1)_2$, O ou S.

8. Composés selon au moins l'une des revendications précédentes, comprenant des structures de formule (VIII),

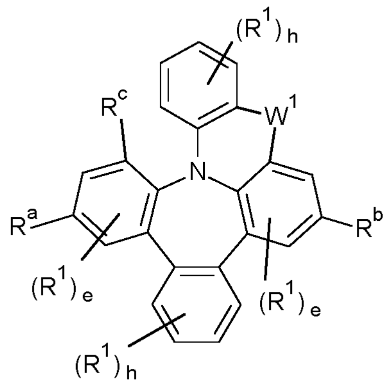

formule (VIII),

dans laquelle $R^a$, $R^b$ et $R^1$ revêtent les significations décrites selon la revendication 1, e vaut 0, 1 ou 2, h vaut 0, 1, 2, 3 ou 4, où le radical $R^b$ représente un groupement de transport de trous ou un groupement de transport d'électrons et le radical $R^c$ représente un groupement aryle ayant de 10 à 40 atomes de C qui comprend au moins deux cycles, ou un groupement hétéroaryle ayant de 6 à 40 atomes de C qui comprend au moins deux cycles, où le groupement respectif peut dans chaque cas être substitué par un ou plusieurs radicaux $R^1$ et $W^1$ est $NR^1$, $C(R^1)_2$, O ou S.

9. Composés selon au moins l'une des revendications précédentes, dans lesquels le radical Rb dans l'une des formules (II), (IV), (V), (VI), (VII) et/ou (VIII) est un groupement de transport de trous et le radical Ra dans l'une des formules (II), (IV), (V), (VI), (VII) et/ou (VIII) est un groupement de transport de trous.

10. Composés selon au moins l'une des revendications 1 à 8 précédentes, dans lesquels le radical $R^b$ dans l'une des formules (II), (IV), (V), (VI), (VII) et/ou (VIII) est un groupement de transport d'électrons et le radical $R^a$ dans l'une des formules (II), (IV), (V), (VI), (VII) et/ou (VIII) est un groupement de transport de trous.

11. Composés selon au moins l'une des revendications 1 à 8 précédentes, dans lesquels le radical $R^b$ dans l'une des formules (II), (IV), (V), (VI), (VII) et/ou (VIII) est un groupement de transport de trous et le radical $R^a$ dans l'une des formules (II), (IV), (V), (VI), (VII) et/ou (VIII) est un groupement de transport d'électrons.

12. Composés selon au moins l'une des revendications 1 à 8 précédentes, dans lesquels le radical $R^b$ dans l'une des formules (II), (IV), (V), (VI), (VII) et/ou (VIII) est un groupement de transport d'électrons et le radical $R^a$ dans l'une

des formules (II), (IV), (V), (VI), (VII) et/ou (VIII) est un groupement de transport d'électrons.

**13.** Composés selon au moins l'une des revendications précédentes, dans lesquels au moins un radical $R^1$, $R^a$, $R^b$ et/ou $R^c$ dans les structures de formule (II), (IV), (V), (VI), (VII) et/ou (VIII) représente un groupement choisi parmi les formules ($R^1$-1) à ($R^1$- 72)

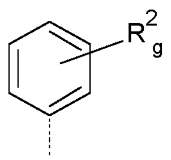

formule ($R^1$-1)

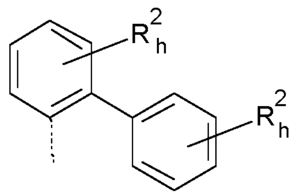

formule ($R^1$-2)

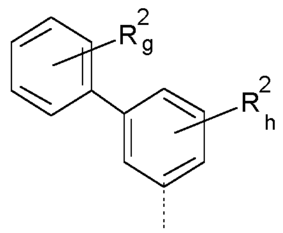

formule ($R^1$-3)

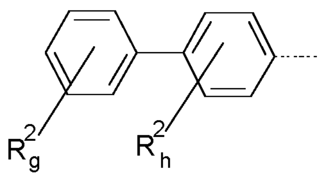

formule ($R^1$-4)

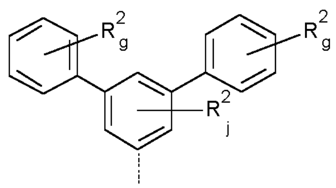

formule ($R^1$-5)

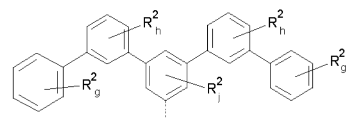

formule ($R^1$-6)

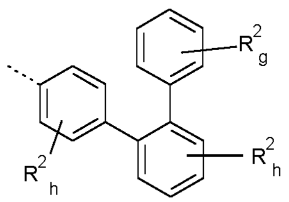

formule ($R^1$-7)

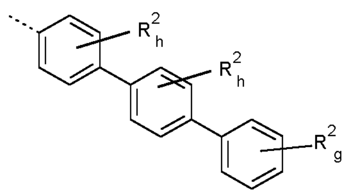

formule ($R^1$-8)

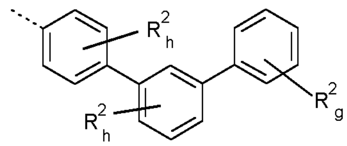

formule ($R^1$-9)

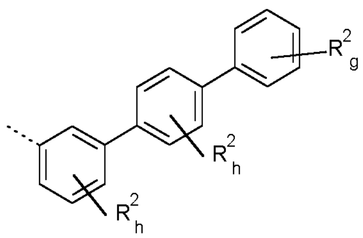

formule ($R^1$-10)

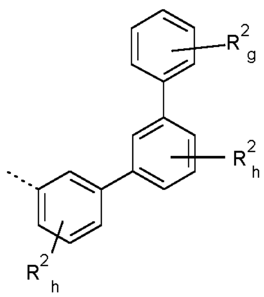

formule ($R^1$-11)

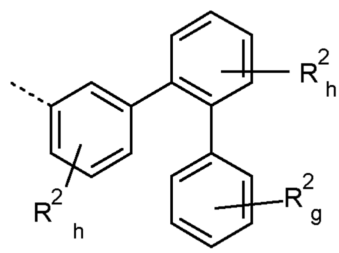

formule ($R^1$-12)

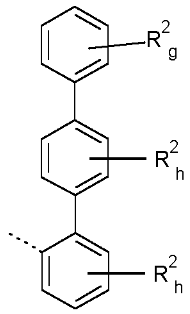
formule ($R^1$-13)

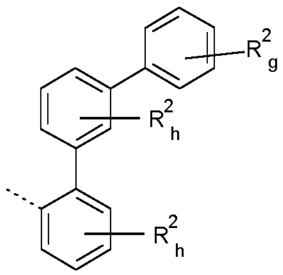
formule ($R^1$-14)

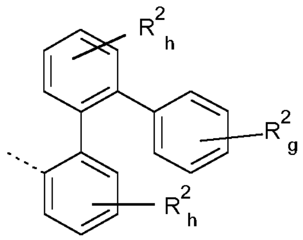
formule ($R^1$-15)

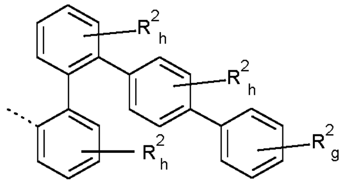
formule ($R^1$-16)

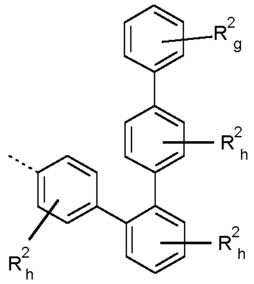
formule ($R^1$-17)

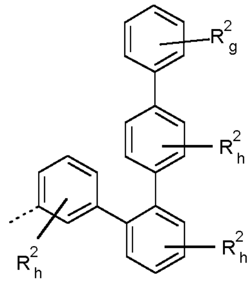
formule ($R^1$-18)

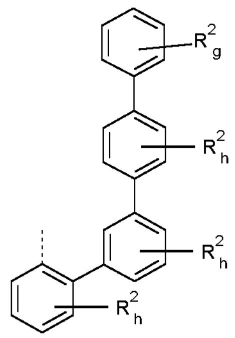
formule ($R^1$-19)

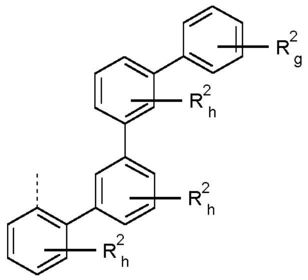
formule ($R^1$-20)

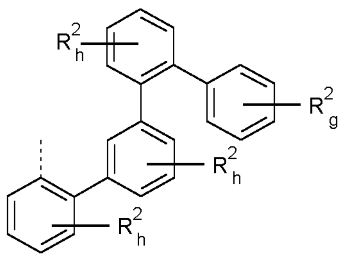
formule ($R^1$-21)

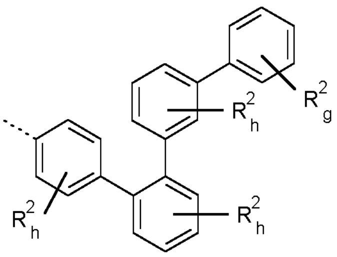
formule ($R^1$-22)

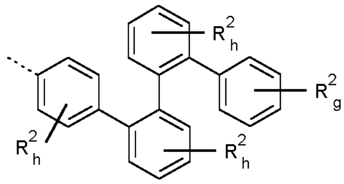
formule ($R^1$-23)

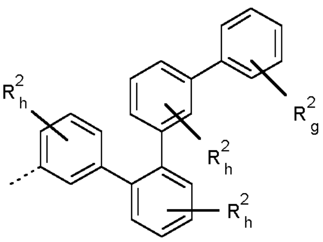
formule ($R^1$-24)

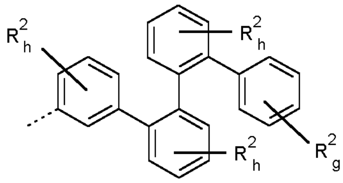

formule ($R^1$-25)

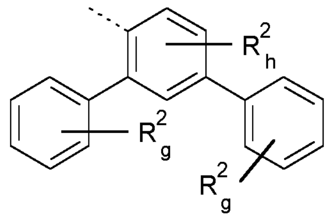

formule ($R^1$-26)

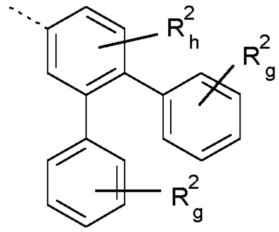

formule ($R^1$-27)

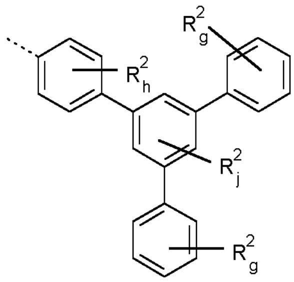

formule ($R^1$-28)

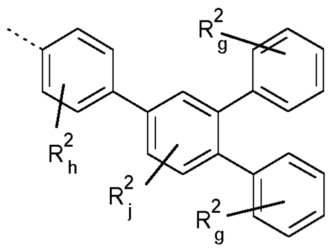

formule ($R^1$-29)

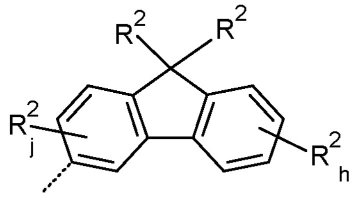

formule ($R^1$-30)

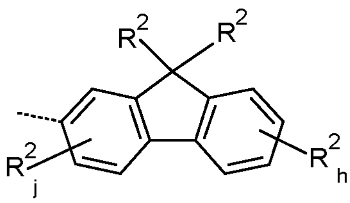

formule ($R^1$-31)

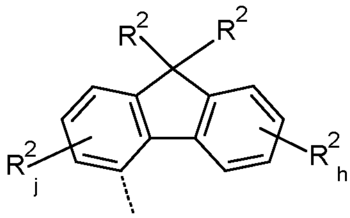

formule ($R^1$-32)

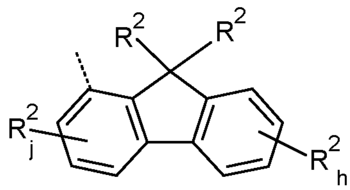

formule ($R^1$-33)

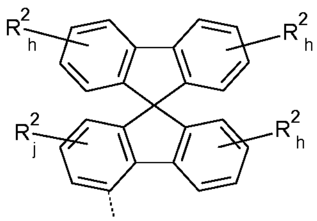

formule ($R^1$-34)

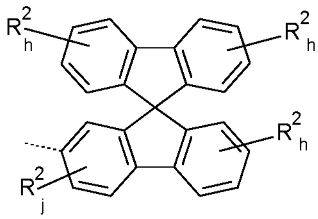

formule ($R^1$-35)

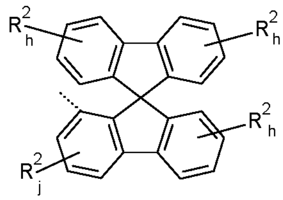

formule ($R^1$-36)

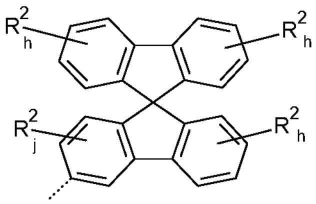

formule ($R^1$-37)

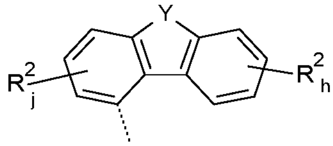

formule ($R^1$-38)

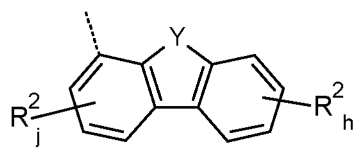

formule ($R^1$-39)

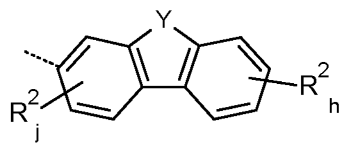

formule ($R^1$-40)

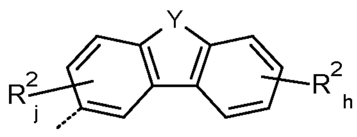

formule ($R^1$-41)

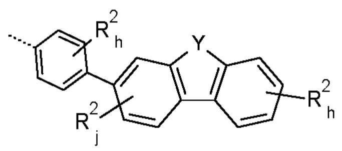

formule ($R^1$-42)

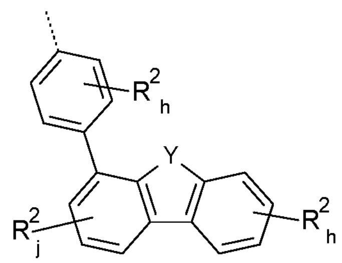

formule ($R^1$-43)

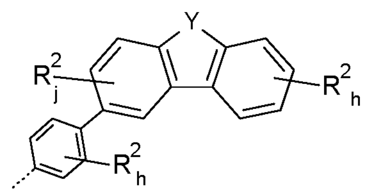

formule ($R^1$-44)

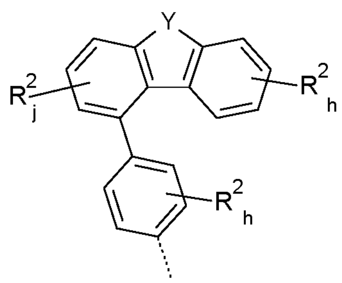

formule ($R^1$-45)

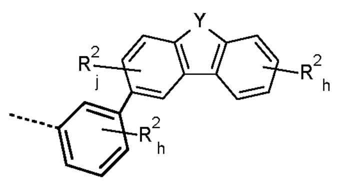

formule ($R^1$-46)

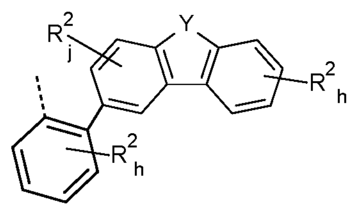

formule ($R^1$-47)

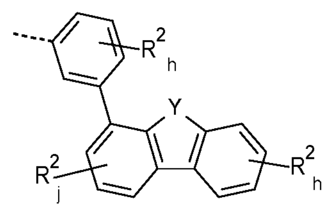

formule ($R^1$-48)

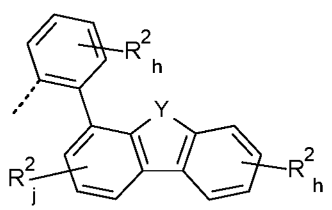

formule ($R^1$-49)

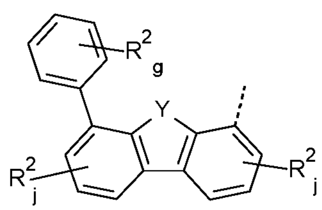

formule ($R^1$-50)

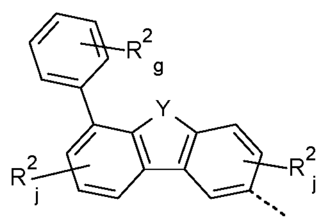

formule ($R^1$-51)

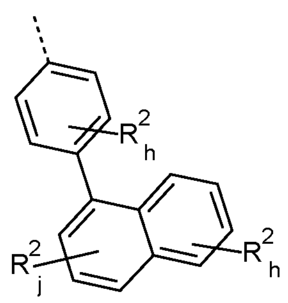

formule ($R^1$-52)

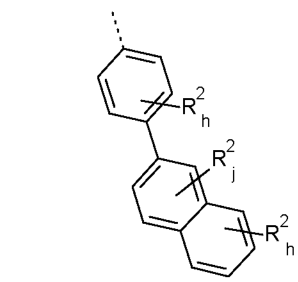

formule ($R^1$-53)

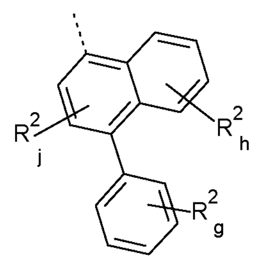

formule ($R^1$-54)

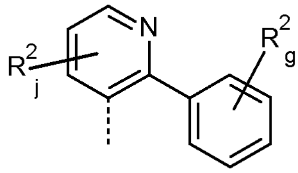

formule ($R^1$-55)

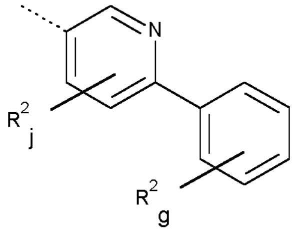

formule ($R^1$-56)

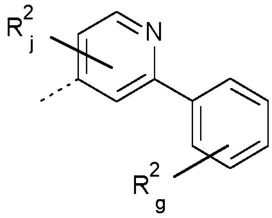

formule ($R^1$-57)

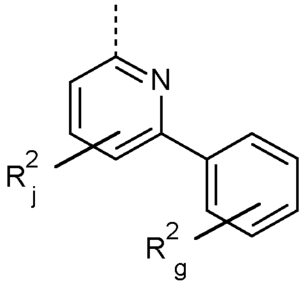

formule ($R^1$-58)

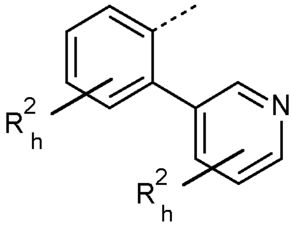

formule ($R^1$-59)

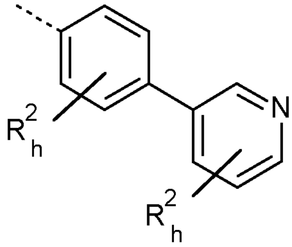

formule ($R^1$-60)

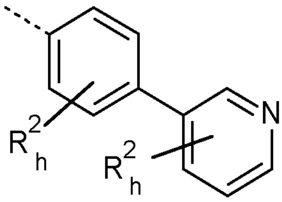

formule ($R^1$-61)

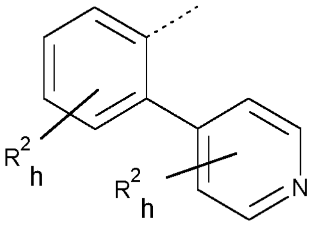

formule ($R^1$-62)

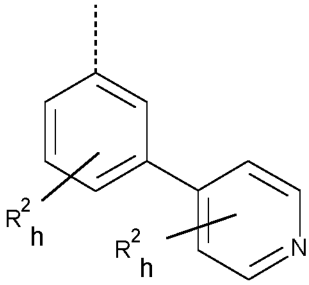

formule ($R^1$-63)

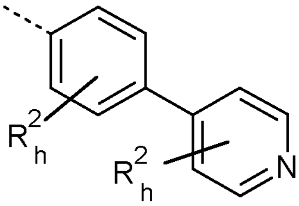

formule ($R^1$-64)

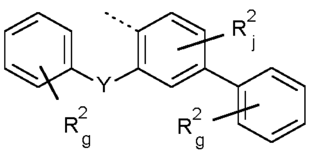

formule ($R^1$-65)

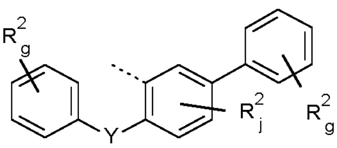

formule ($R^1$-66)

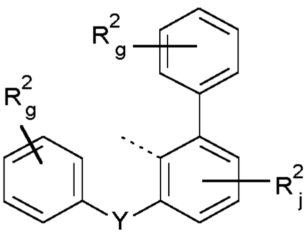

formule ($R^1$-67)

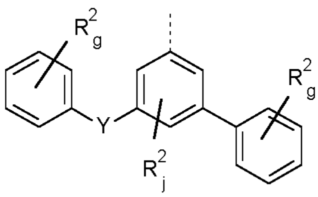

formule ($R^1$-68)

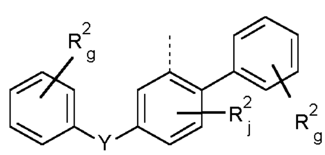

formule ($R^1$-69)

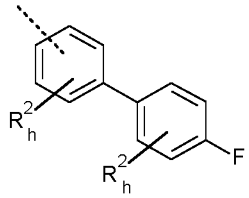

formule ($R^1$-70)

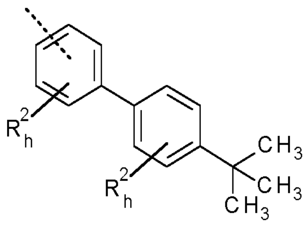

formule ($R^1$-71)

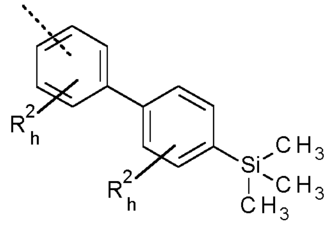

formule ($R^1$-72),

dans lesquelles ce qui suit s'applique aux symboles utilisés :

Y est O, S ou $NR^2$, préférablement O ou S ;
j vaut à chaque occurrence, indépendamment, 0, 1, 2 ou 3 ;
h vaut à chaque occurrence, indépendamment, 0, 1, 2, 3 ou 4 ;
g vaut à chaque occurrence, indépendamment, 0, 1, 2, 3, 4 ou 5 :

la liaison en pointillés marque la position de liaison ; et

$R^2$ revêt la signification donnée selon la revendication 1.

**14.** Composés selon la revendication 13, dans lesquels la somme des indices g, h et j dans les structures de formules (R1-1) à (R1-72) est dans chaque cas d'au plus 3, préférablement d'au plus 2 et particulièrement préférablement d'au plus 1.

**15.** Composés selon au moins l'une des revendications 3, 5 à 8 et 10 à 12 précédentes, dans lesquels le groupement de transport d'électrons présente au moins une structure de formules (E-1), (E-5) à (E-10)

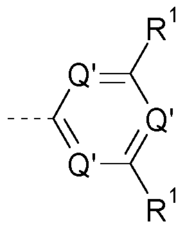

formule (E-1)

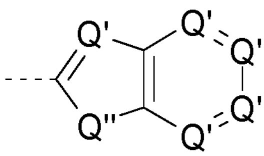

formule (E-5)

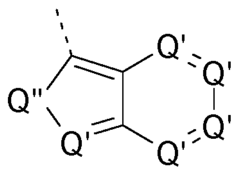

formule (E-6)

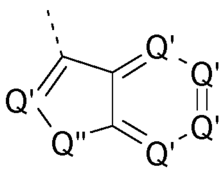

formule (E-7)

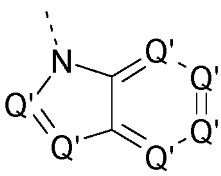

formule (E-8)

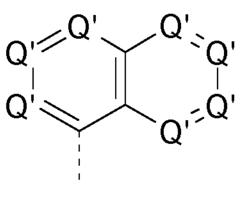

formule (E-9)

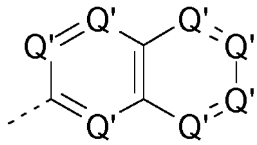

formule (E-10),

dans lesquelles la liaison en pointillés marque la position de liaison,

Q‘ à chaque occurrence, de manière identique ou différente, représente $CR^1$ ou N, and
Q" représente $NR^1$, O ou S ;

où au moins un Q‘ est égal à N et/ou au moins un Q" est égal à $NR^1$ et

$R^1$ est tel que défini selon la revendication 1.

**16.** Composés selon au moins l’une des revendications 3, 5 à 8 et 10 à 12 précédentes, dans lesquels le groupement de transport d’électrons présente au moins une structure de formules (E-11) à (E-23)

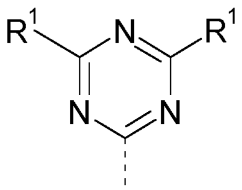

formule (E-11)

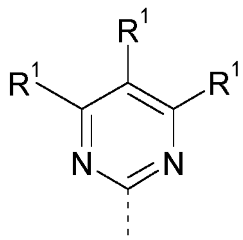

formule (E-12)

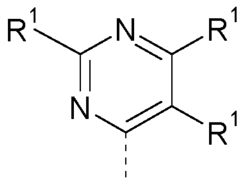

formule (E-13)

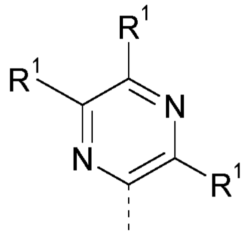

formule (E-14)

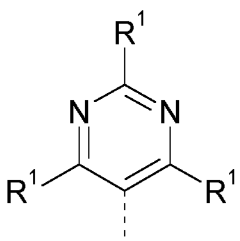

formule (E-15)

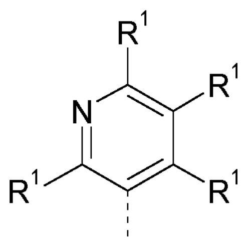

formule (E-16)

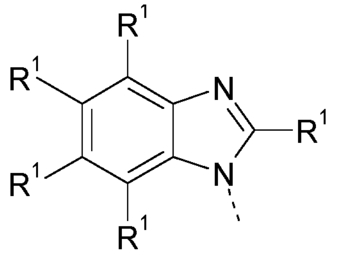

formule (E-17)

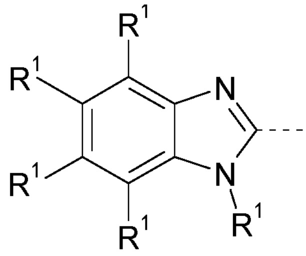

formule (E-18)

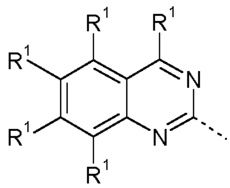

formule (E-19)

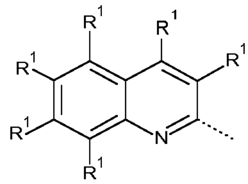

formule (E-20)

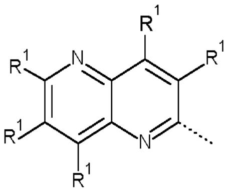

formule (E-21)

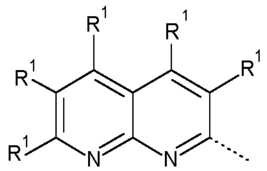

formule (E-22)

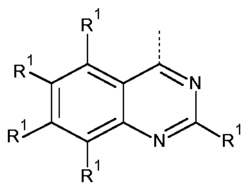

formule (E-23)

dans lesquelles la liaison en pointillés marque la position de liaison et $R^1$ revêt la signification donnée selon la revendication 1.

**17.** Composés selon au moins l'une des revendications 3 et 5 à 11 précédentes, dans lesquels le groupement de transport de trous comprend au moins une structure choisie dans le groupe constitué par les carbazoles, les indénocarbazoles et les indolocarbazoles.

**18.** Composés selon au moins l'une des revendications 3 et 5 à 11 précédentes, dans lesquels le groupement de transport de trous présente au moins une structure de formules (L-2) à (L-9)

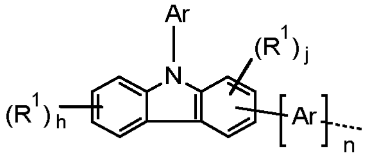

formule (L-2)

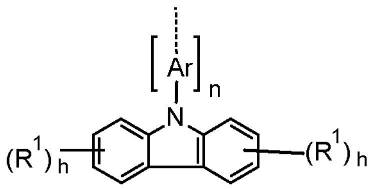

formule (L-3)

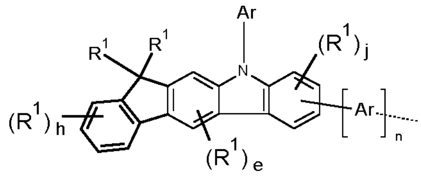

formule (L-4)

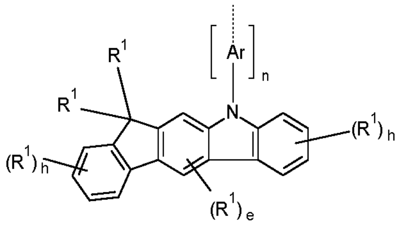

formule (L-5)

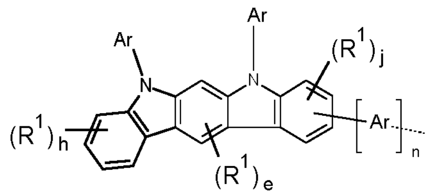

formule (L-6)

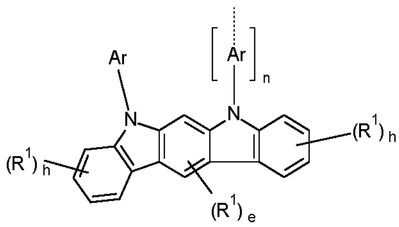

formule (L-7)

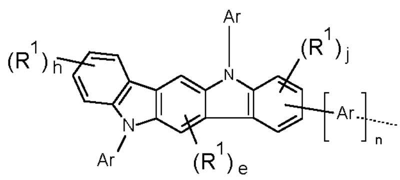

formule (L-8)

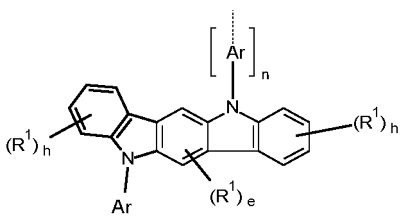

formule (L-9)

dans lesquelles la liaison en pointillés marque la position de liaison, e vaut 0, 1 ou 2, j vaut 0, 1, 2 ou 3, h vaut 0, 1, 2, 3 ou 4, n vaut 0 ou 1, Ar représente un groupement aryle ayant de 6 à 40 atomes de C ou un groupement hétéroaryle ayant de 3 à 40 atomes de C, qui peut être substitué par un ou plusieurs radicaux $R^1$, et $R^1$ revêt la signification donnée selon la revendication 1.

**19.** Composés selon au moins l'une des revendications précédentes, choisis dans le groupe constitué par les composés suivants :

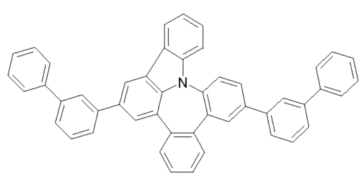

formule 1

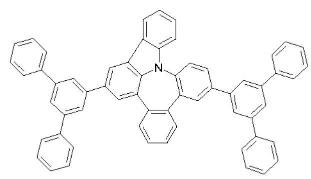

formule 2

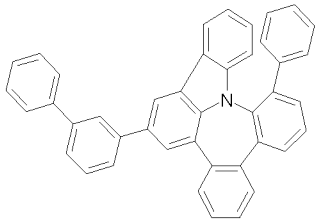

formule 4

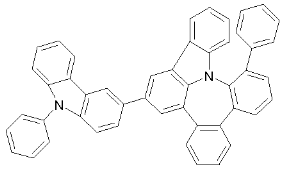

formule 5

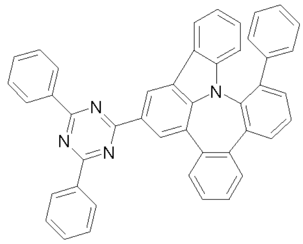

formule 6

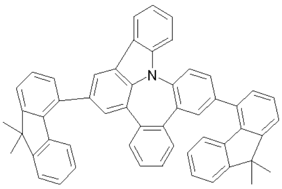

formule 7

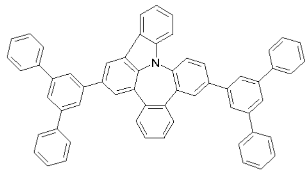

formule 8

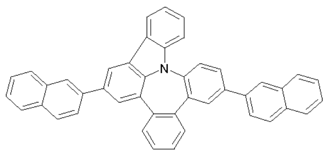

formule 10

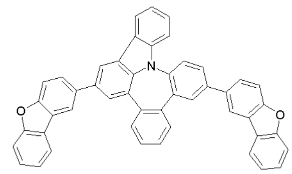

formule 14

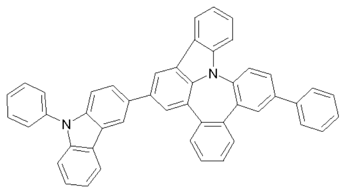

formule 15

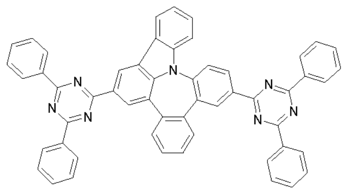

formule 16

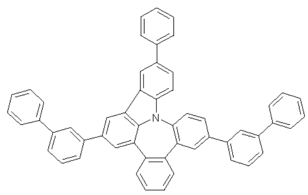

formule 17

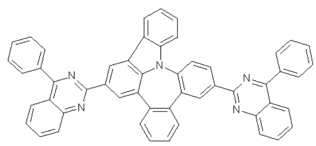

formule 19

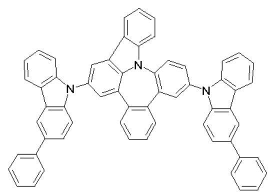

formule 20

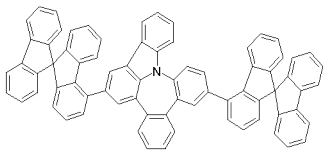

formule 22

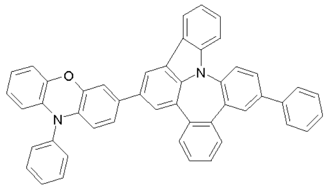

formule 24

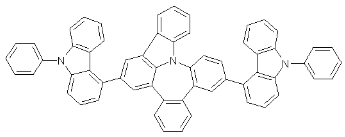

formule 27

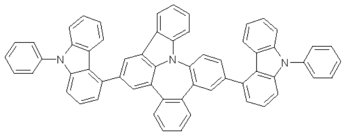

formule 28

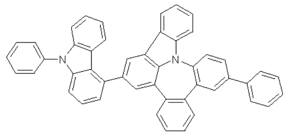

formule 29

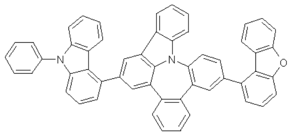

formule 30

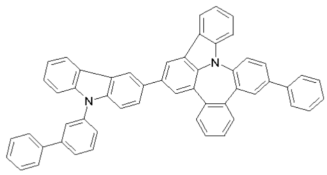

formule 34

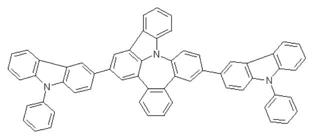

formule 36

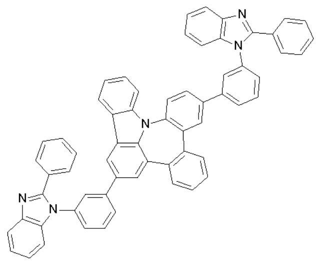

formule 43

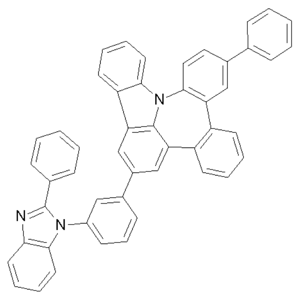

formule 44

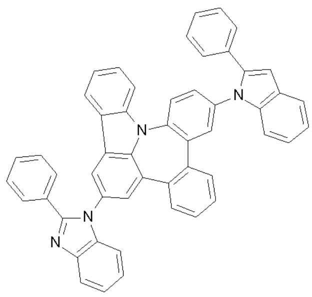

formule 45

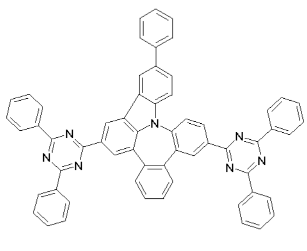

formule 46

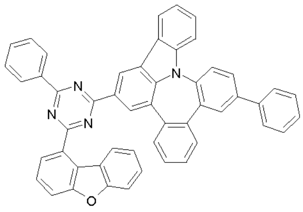

formule 49

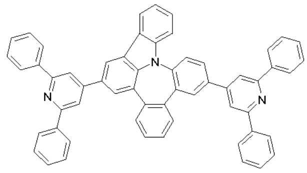

formule 50

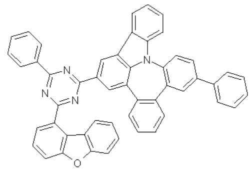

formule 51

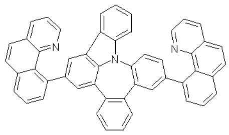

formule 52

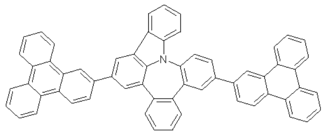

formule 53

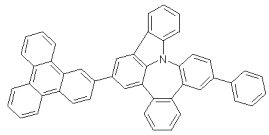

formule 54

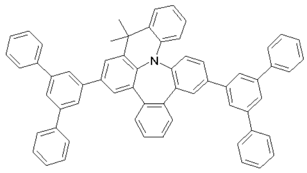

formule 55

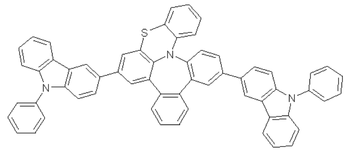

formule 57

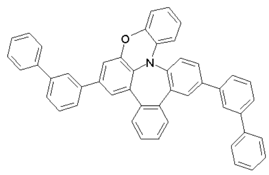

formule 58

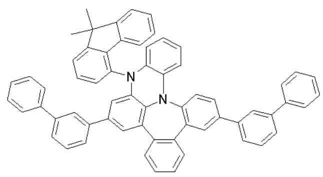

formule 62

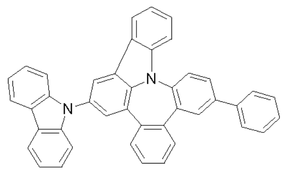

formule 72

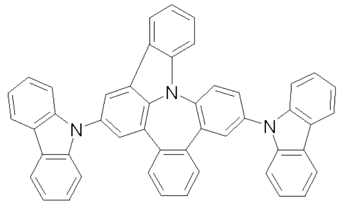

formule 73

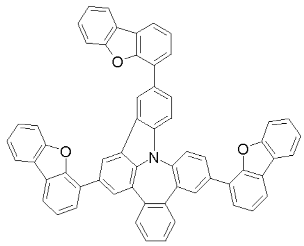

formule 75

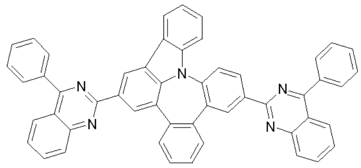

formule 76.

**20.** Oligomère, polymère ou dendrimère contenant un ou plusieurs composés selon l'une ou plusieurs parmi les revendications 1 à 19, une ou plusieurs liaisons étant présentes entre le composé et le polymère, l'oligomère ou le dendrimère.

**21.** Composition comprenant au moins un composé selon l'une ou plusieurs parmi les revendications 1 à 19 et/ou un oligomère, polymère ou dendrimère selon la revendication 20 et au moins un autre composé choisi dans le groupe constitué par les émetteurs fluorescents, les émetteurs phosphorescents, les matériaux hôtes, les matériaux de matrice, les matériaux de transport d'électrons, les matériaux d'injection d'électrons, les matériaux conducteurs de trous, les matériaux d'injection de trous, les matériaux de blocage d'électrons et les matériaux de blocage de trous.

**22.** Formulation comprenant au moins un composé selon l'une ou plusieurs parmi les revendications 1 à 19, un oligomère, polymère ou dendrimère selon la revendication 20 et/ou au moins une composition selon la revendication 21 et au moins un solvant.

**23.** Procédé de préparation d'un composé selon l'une ou plusieurs parmi les revendications 1 à 19 ou d'un oligomère, polymère ou dendrimère selon la revendication 20, dans lequel une réaction de fermeture de cycle est mise en oeuvre sur un composé contenant an élément structurel de type azépine.

**24.** Utilisation d'un composé selon l'une ou plusieurs parmi les revendications 1 à 19, d'un oligomère, polymère ou dendrimère selon la revendication 20 ou d'une composition selon la revendication 21, dans un dispositif électronique comme matériau de blocage d'électrons, matériau d'injection de trous et/ou matériau de transport de trous.

**25.** Dispositif électronique contenant au moins un composé selon l'une ou plusieurs parmi les revendications 1 à 19, un oligomère, polymère ou dendrimère selon la revendication 20 ou une composition selon la revendication 21.

**26.** Dispositif électronique selon la revendication 25, le dispositif électronique étant choisi dans le groupe constitué par les dispositifs électroluminescents organiques, les circuits intégrés organiques, les transistors organiques à effet de champ, les transistors organiques en couche mince, les transistors organiques émetteurs de lumière, les cellules solaires organiques, les détecteurs optiques organiques, les photorécepteurs organiques, les dispositifs organiques à extinction de champ, les cellules électrochimiques émettrices de lumière ou les diodes laser organiques.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- WO 9013148 A1
- JP 2014160813 A
- WO 0033617 A
- EP 2175005 A
- EP 842208 A
- WO 2000022026 A
- EP 707020 A
- EP 894107 A
- WO 2006061181 A
- WO 9218552 A
- WO 2004070772 A
- WO 2004113468 A
- EP 1028136 A
- WO 2005014689 A
- WO 2004041901 A
- WO 2004113412 A
- WO 2005040302 A
- WO 2005104264 A
- WO 2007017066 A
- US 7294849 B
- WO 200070655 A
- WO 200141512 A
- WO 200202714 A
- WO 200215645 A
- EP 1191613 A
- EP 1191612 A
- EP 1191614 A
- WO 2005033244 A
- WO 2005019373 A
- US 20050258742 A
- WO 2005011013 A
- WO 2004013080 A
- WO 2004093207 A
- WO 2006005627 A
- WO 2010006680 A
- WO 2005039246 A
- US 20050069729 A
- JP 2004288381 A
- EP 1205527 A
- WO 2008086851 A
- US 20090134784 A
- WO 2007063754 A
- WO 2008056746 A
- WO 2010136109 A
- WO 2011000455 A
- EP 1617710 A
- EP 1617711 A
- EP 1731584 A
- JP 2005347160 A
- WO 2007137725 A
- WO 2005111172 A
- WO 2006117052 A
- WO 2010054729 A
- WO 2010054730 A
- WO 2010015306 A
- EP 652273 A
- WO 2009062578 A
- WO 2009148015 A
- US 20090136779 A
- WO 2010050778 A
- WO 2011042107 A
- WO 2011088877 A
- WO 2010108579 A
- WO 2004058911 A


### In der Beschreibung aufgeführte Nicht-Patentliteratur


- **M. S. ARNOLD et al.** *Appl. Phys. Lett.,* 2008, vol. 92, 053301